# EUROPEAN PATENT SPECIFICATION

(11) **EP 4 244 218 B1**
(45) Date of publication and mention of the grant of the patent: **08.07.2026**
(21) Application number: 20825085.2
(22) Date of filing: 30.11.2020
(51) Int. Cl.: C07D 403/06, C07D 417/14, A61P 7/06, A61P 9/00, A61P 9/10, A61P 35/00

(54) **1-(2-(4-CYCLOPROPYL-1H-1,2,3-TRIAZOL-1-YL)ACETYL)-4-HYDROXY-N-(BENZYL)PYRROLIDINE-2-CARBOXAMIDE DERIVATIVES AS VHL INHIBITORS FOR THE TREATMENT OF ANEMIA AND CANCER**
1-(2-(4-CYCLOPROPYL-1H-1,2,3-TRIAZOL-1-YL)ACETYL)-4-HYDROXY-N-(BENZYL)PYRROLIDIN-2-CARBOXAMID-DERIVATE ALS VHL INHIBITOREN ZUR BEHANDLUNG VON ANÄMIE UND KREBS
DÉRIVÉS DE 1-(2-(4-CYCLOPROPYL-1H-1,2,3-TRIAZOL-1-YL)ACÉTYL)-4-HYDROXY-N-(BENZYL)PYRROLIDINE-2-CARBOXAMIDE EN TANT QU'INHIBITEURS DE VHL POUR LE TRAITEMENT DE L'ANÉMIE ET DU CANCER

(30) Priority: 11.11.2020 US 202063112609 P
(43) Date of publication of application: 20.09.2023
(73) Proprietor: Genentech, Inc., South San Francisco, CA 94080 (US)
(72) Inventor: FUHRMANN, Jakob, South San Francisco, California 94080 (US); WU, Hao, South San Francisco, California 94080 (US); FAIRBROTHER, Wayne, J., South San Francisco, California 94080 (US)
(74) Representative: Jochnowitz, Evan
(86) International application number: PCT/US2020/062627
(87) International publication number: WO 2022/103411

(56) References cited:
- WO-A2-2013/106643
- WO-A2-2013/106646
- DENNIS L BUCKLEY ET AL: "Small-Molecule Inhibitors of the Interaction between the E3 Ligase VHL and HIF1.alpha", ANGEWANDTE CHEMIE INTERNATIONAL EDITION, VERLAG CHEMIE, DE, vol. 51, no. 46, 12 November 2012 (2012-11-12), pages 11463 - 11467, XP002728004, ISSN: 1433-7851, DOI: 10.1002/ANIE.201206231

## Description

### FIELD OF THE DISCLOSURE

The present disclosure relates to compounds comprising a VHL ligand moiety and to compounds of the invention or a pharmaceutically acceptable salt thereof for use in methods of using such compounds as ligands of VHL. The present disclosure further relates to the compounds described herein, or pharmaceutical compositions thereof, to prevent and/or treat a range of diseases, disorders, and conditions.

### BACKGROUND OF THE DISCLOSURE

E3 ubiquitin ligases (of which over 600 are known in humans) confer substrate specificity for ubiquitination. There are known ligands which bind to these ligases. An E3 ubiquitin ligase binding group (E3LB) is a peptide or small molecule that can bind an E3 ubiquitin ligase.

A particular E3 ubiquitin ligase is von Hippel-Lindau (VHL) tumor suppressor, the substrate recognition subunit of the E3 ligase complex VCB (an important target in cancer, chronic anemia, and ischemia), which also consists of elongins B and C, Cul2, and Rbxl. The primary substrate of VHL is Hypoxia Inducible Factor 1α (HIF- 1α), a transcription factor that upregulates genes such as the pro-angiogenic growth factor VEGF and the red blood cell inducing cytokine erythropoietin in response to low oxygen levels. While HIF-1α is constitutively expressed, its intracellular levels are kept very low under normoxic conditions via its hydroxylation by prolyl hydroxylase domain (PHD) proteins and subsequent VHL-mediated ubiquitination.

The crystal structure of VHL with ligands has been obtained, confirming that a compound can mimic the binding mode of the transcription factor HIF-1α, the major substrate of VHL. These compounds bind VHL competing with the HIF-1α substrate, thereby reducing or blocking the activity of the VHL protein. There exists an ongoing need in the art for small molecule VHL ligands that are effective across a broad range of disease indications.

WO2013/106646 discloses 1-(2-(1H-1,2,3-triazol-1-yl)acetyl)-4-hydroxyprrolidine-2-carboxamide derivatives as VHL inhibitors for the treatment of e.g. anemia.

WO2013/106643 discloses 1-(2-(1H.1,2,3-triazol-1-yl)acetyl)-4-hydroxypyrrolidine-2-carboxaide derivatives as VHL inhibitors for the treatment of e.g. cancer.

Dennis L Buckley et al:"Small-molecule inhibitors of the ineraction between the E3 Ligase VHL and H1F1.alpha", ANGEWANDTE CHEMIE INTERNATIONAL EDITION, VERLAG CHEMIE, DE, vol 51, no 46, 12 November 2012 (2012-11-12) pages 11463-11467 discloses 1-(2-(1H-1,2,3-triazol-1-yl)acetyl)-4-hydroxyprrolidine-2-carboxamide derivatives as VHL inhibitors for the treatment of e.g. anemia.

### BRIEF DESCRIPTION OF THE DISCLOSURE

The present disclosure is directed to VHL ligands and, specifically, to VHL ligands that bind to a VHL E3 ubiquitin ligase.

In one aspect, the present disclosure is directed to a compound of formula (I): 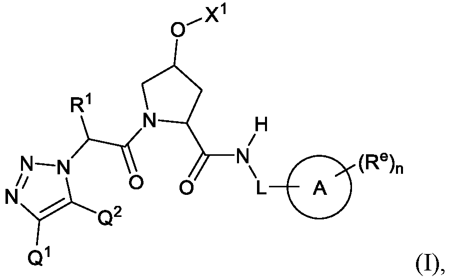 or a stereoisomer or tautomer thereof, or a pharmaceutically acceptable salt of any of the foregoing, wherein:
X¹ is, independently at each occurrence, H, C₁₋₁₂alkyl, or -C(O)-C₁₋₁₂alkyl;
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
L is, independently at each occurrence, absent or is C₁₋₁₂alkylene, wherein the C₁₋₁₂alkylene of L is independently optionally substituted with one or more R^{t}, wherein R^{t} is C₁₋₁₂alkyl or - C(O)NH₂, wherein the C₁₋₁₂alkyl of R^{t} is further optionally substituted with one or more halo;
ring A is, independently at each occurrence, C₆₋₂₀aryl or C₇₋₁₅cycloalkyl;
R^{e} is, independently at each occurrence, halo, C₆₋₂₀aryl, or 5-20 membered heteroaryl,
wherein the C₆₋₂₀aryl or 5-20 membered heteroaryl of R^{e} is independently optionally substituted with one or more C₁₋₁₂alkyl or halo;
n is, independently at each occurrence, 0, 1, 2, 3, 4, or 5; and
Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ or Q² is independently optionally substituted with one or more R⁹, wherein R⁹ is C₁₋₁₂alkyl, C₂₋₁₂alkenyl, C₂₋₁₂alkynyl, C₆₋₂₀aryl, C₁₋₁₂alkoxy, or 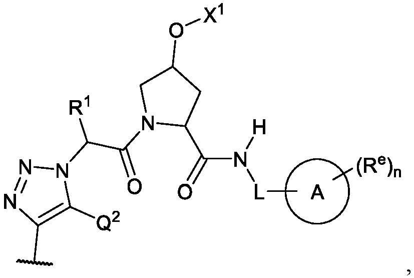 wherein the C₁₋₁₂alkyl or C₁₋₁₂alkoxy of R⁹ is independently further optionally substituted with one or more halo or - NHC(O)-C₁₋₁₂alkyl,
or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,
   wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, - C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In another aspect, the present disclosure is related to pharmaceutical compositions comprising one or more of the compounds described herein, or a stereoisomer or tautomer thereof, or a pharmaceutically acceptable salt of any of the foregoing, and one or more pharmaceutically acceptable excipients.

In one aspect, the present disclosure is directed to compounds of the invention or a pharmaceutical composition comprising a compound of the invention, for use in a method of inhibiting VHL using one or more of the compounds described herein, or a stereoisomer or tautomer thereof, or a pharmaceutically acceptable salt of any of the foregoing, or one or more of the pharmaceutical compositions described herein.

In a futher aspect, the present disclosure is directed to compounds of the invention or a pharmaceutical composition comprising a compound of the invention, for use in a method of preventing or treating a disease, disorder, or condition by administering to a subject in need thereof one or more of the compounds described herein, or a stereoisomer or tautomer thereof, or a pharmaceutically acceptable salt of any of the foregoing, or one or more of the pharmaceutical compositions described herein.

### DETAILED DESCRIPTION OF THE DISCLOSURE

The present disclosure is directed to compounds that bind an E3 ubiquitin ligase protein complex. In particular, compounds are described that bind to Von Hippel-Lindau (VHL), the substrate recognition subunit of the E3 ligase complex VCB.

The presently disclosed subject matter will now be described more fully hereinafter. However, many modifications and other embodiments of the presently disclosed subject matter set forth herein will come to mind to one skilled in the art to which the presently disclosed subject matter pertains having the benefit of the teachings presented in the foregoing descriptions. Therefore, it is to be understood that the presently disclosed subject matter is not to be limited to the specific embodiments disclosed and that modifications and other embodiments are intended to be included within the scope of the appended claims. In other words, the subject matter described herein covers alternatives, modifications, and equivalents. In the event that one or more of the incorporated literature, patents, and similar materials differs from or contradicts this application, including but not limited to defined terms, term usage, described techniques, or the like, this application controls. Unless otherwise defined, technical and scientific terms used herein have the same meaning as commonly understood by one of ordinary skill in the art to which this disclosure belongs, applying that term in context to its use in describing the present disclosure. The terminology used in the description is for describing particular embodiments only and is not intended to be limiting of the disclosure.

### I. Definitions

The terms "residue," "moiety," or "group" refers to a component that is covalently bound or linked to another component.

The term "covalently bound" or "covalently linked" refers to a chemical bond formed by sharing of one or more pairs of electrons.

A "patient" or "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the patient, individual, or subject is a human. In some embodiments, the patient may be a "cancer patient," i.e. one who is suffering or at risk for suffering from one or more symptoms of cancer.

The terms "cancer" and "cancerous" refer to or describe the physiological condition in mammals that is typically characterized by unregulated cell growth/proliferation. A "tumor" comprises one or more cancerous cells. Examples of cancer are provided elsewhere herein.

A "chemotherapeutic agent" or "anti-cancer agent" refers to a chemical compound useful in the treatment of cancer. Examples of chemotherapeutic agents include alkylating agents such as thiotepa and cyclosphosphamide (CYTOXAN^{®}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL^{®}), beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (see, e.g., Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN^{®}, morpholino-doxorubicin, cyanomorpholino-doxorubicin, 2-pyrrolino-doxorubicin, doxorubicin HCl liposome injection (DOXIL^{®}), liposomal doxorubicin TLC D-99 (MYOCET^{®}), peglylated liposomal doxorubicin (CAELYX^{®}), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR^{®}), tegafur (UFTORAL^{®}), capecitabine (XELODA^{®}), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); thiotepa; taxoid, e.g., paclitaxel (TAXOL^{®}), albumin-engineered nanoparticle formulation of paclitaxel (ABRAXANETM), and docetaxel (TAXOTERE^{®}); chloranbucil; 6-thioguanine; mercaptopurine; methotrexate; platinum agents such as cisplatin, oxaliplatin (e.g., ELOXATIN^{®}), and carboplatin; vincas, which prevent tubulin polymerization from forming microtubules, including vinblastine (VELBAN^{®}), vincristine (ONCOVIN^{®}), vindesine (ELDISINE^{®}, FILDESIN^{®}), and vinorelbine (NAVELBINE^{®}), etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN^{®}); bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTONEL^{®}); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE^{®} vaccine and gene therapy vaccines, for example, ALLOVECTIN^{®} vaccine, LEUVECTIN^{®} vaccine, and VAXID^{®} vaccine; topoisomerase 1 inhibitor (e.g., LURTOTECAN^{®}); rmRH (e.g., ABARELIX^{®}); BAY439006 (sorafenib; Bayer); SU-11248 (sunitinib, SUTENT^{®}, Pfizer); perifosine, COX-2 inhibitor (e.g., celecoxib or etoricoxib), proteosome inhibitor (e.g., PS341); bortezomib (VELCADE^{®}), CCI-779; tipifarnib (R11577); orafenib, ABT510; Bcl-2 inhibitor such as oblimersen sodium (GENASENSE^{®}, an antisence oligonucleotide); pixantrone; EGFR inhibitors (see definition below); tyrosine kinase inhibitors; serine-threonine kinase inhibitors such as rapamycin (sirolimus, RAPAMUNE^{®}); farnesyltransferase inhibitors such as lonafarnib (SCH 6636, SARASARTM); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATINTM) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX^{®}), 4-hydroxytamoxifen, toremifene (FARESTON^{®}), idoxifene, droloxifene, raloxifene (EVISTA^{®}), trioxifene, keoxifene, and selective estrogen receptor modulators (SERMs) such as SERM3; pure anti-estrogens without agonist properties, such as fulvestrant (FASLODEX^{®}), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN^{®}), and nonsteroidal aromatase inhibitors such as anastrazole (ARIMIDEX^{®}), letrozole (FEMARA^{®}) and aminoglutethimide, and other aromatase inhibitors include vorozole (RIVISOR^{®}), megestrol acetate (MEGASE^{®}), fadrozole, and 4(5)-imidazoles; lutenizing hormone-releaseing hormone agonists, including leuprolide (LUPRON^{®} and ELIGARD^{®}), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration, or palliation of the disease state, and remission or improved prognosis. In some embodiments, the compounds and compositions of the subject matter described herein are used to delay development of a disease or to slow the progression of a disease. In one embodiment, treatment is performed for prophylaxis only. In another embodiment, treatment is performed during the course of clinical pathology only (i.e., not for prophylaxis). In another embodiment, treatment is performed both during the course of clinical pathology and for prophylaxis.

A drug that is administered "concurrently" with one or more other drugs is administered during the same treatment cycle, on the same day of treatment as the one or more other drugs, and, optionally, at the same time as the one or more other drugs. For instance, for cancer therapies given every 3 weeks, the concurrently administered drugs are each administered on day-1 of a 3-week cycle.

The term "effective" is used to describe an amount of a compound, composition or component which, when used within the context of its intended use, achieves the desired therapeutic or prophylactic result. The term effective subsumes other effective amount or effective concentration terms, including therapeutically effective amounts, which are otherwise described or used in the present application. As used herein, the term "therapeutically effective amount" means any amount which, as compared to a corresponding subject who has not received such amount, results in treatment of a disease, disorder, or side effect, or a decrease in the rate of advancement of a disease or disorder. The term also includes within its scope amounts effective to enhance normal physiological function. For use in therapy, therapeutically effective amounts of a VHL ligand of the present disclosure, as well as stereoisomers or tautomes thereof, or pharmaceutically acceptable salts of any of the foregoing, may be administered as the raw chemical. Additionally, the active ingredient may be presented as a pharmaceutical composition.

As used herein, unless defined otherwise in a claim, the term "optionally" means that the subsequently described event(s) may or may not occur, and includes both event(s) that occur and event(s) that do not occur.

As used herein, unless defined otherwise, the phrase "optionally substituted", "substituted" or variations thereof denote an optional substitution, including multiple degrees of substitution, with one or more substituent group, for example, one, two, three, four or five. The phrase should not be interpreted as duplicative of the substitutions herein described and depicted.

The term "pharmaceutical formulation" or "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable excipient" refers to an ingredient in a pharmaceutical formulation, other than an active ingredient, which is nontoxic to a subject. A fdepharmaceutically acceptable excipient includes, but is not limited to, a buffer, carrier, stabilizer, or preservative.

The phrase "pharmaceutically acceptable salt," as used herein, refers to pharmaceutically acceptable organic or inorganic salts of a molecule. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p toluenesulfonate, and pamoate (i.e., 1,1' methylene bis - (2 hydroxy 3 naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

Other salts, which are not pharmaceutically acceptable, may be useful in the preparation of compounds described herein and these should be considered to form a further aspect of the subject matter. These salts, such as oxalic or trifluoroacetate, while not in themselves pharmaceutically acceptable, may be useful in the preparation of salts useful as intermediates in obtaining the compounds described herein and their pharmaceutically acceptable salts.

A "small molecule" or "small molecular compound" generally refers to an organic molecule that is less than about 5 kilodaltons (Kd) in size. In some embodiments, the small molecule is less than about 4 Kd, 3 Kd, about 2 Kd, or about 1 Kd. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, small molecules are non-polymeric. Small molecules are not proteins, polypeptides, oligopeptides, peptides, polynucleotides, oligonucleotides, polysaccharides, glycoproteins, proteoglycans, etc. A derivative of a small molecule refers to a molecule that shares the same structural core as the original small molecule, but which can be prepared by a series of chemical reactions from the original small molecule.

The term "alkyl" as used herein refers to a saturated linear or branched-chain monovalent hydrocarbon radical of any length from one to twelve carbon atoms (C₁-C₁₂), wherein the alkyl radical may be optionally substituted independently with one or more substituents described herein. In another embodiment, an alkyl radical is one to eight carbon atoms (C₁-C₈), or one to six carbon atoms (C₁-C₆), or one to four carbon atoms (C₁-C₄), or one to three carbon atoms (C₁-C₃). Examples of alkyl groups include, but are not limited to, methyl (Me, -CH₃), ethyl (Et, -CH₂CH₃), 1-propyl (n-Pr, n-propyl, -CH₂CH₂CH₃), 2-propyl (i-Pr, i-propyl, isopropyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, tert-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl, 1-octyl, and the like.

The term "alkylene" as used herein refers to a saturated linear or branched-chain divalent hydrocarbon radical of any length from one to twelve carbon atoms (C₁-C₁₂), wherein the alkylene radical may be optionally substituted independently with one or more substituents described herein. In another embodiment, an alkylene radical is one to eight carbon atoms (C₁-C₈), one to six carbon atoms (C₁-C₆), or one to four carbon atoms (C₁-C₄). Examples of alkylene groups include, but are not limited to, methylene (-CH₂-), ethylene (-CH₂CH₂-), propylene (-CH₂CH₂CH₂-), and the like.

The term "alkenyl" refers to linear or branched-chain monovalent hydrocarbon radical of any length from two to twelve carbon atoms (C₂-C₁₂) with at least one site of unsaturation, i.e., a carbon-carbon, sp2 double bond, wherein the alkenyl radical may be optionally substituted independently with one or more substituents described herein, and includes radicals having "cis" and "trans" orientations, or alternatively, "E" and "Z" orientations. Examples include, but are not limited to, ethylenyl or vinyl (-CH=CH₂), allyl (-CH₂CH=CH₂), and the like.

The term "alkenylene" refers to linear or branched-chain divalent hydrocarbon radical of any length from two to twelve carbon atoms (C₂-C₁₂) with at least one site of unsaturation, i.e., a carbon-carbon, sp2 double bond, wherein the alkenylene radical may be optionally substituted independently with one or more substituents described herein, and includes radicals having "cis" and "trans" orientations, or alternatively, "E" and "Z" orientations. Examples include, but are not limited to, ethylenylene or vinylene (-CH=CH-), allyl (-CH₂CH=CH-), and the like.

The term "alkynyl" refers to a linear or branched monovalent hydrocarbon radical of any length from two to twelve carbon atoms (C₂-C₁₂) with at least one site of unsaturation, i.e., a carbon-carbon, sp triple bond, wherein the alkynyl radical may be optionally substituted independently with one or more substituents described herein. Examples include, but are not limited to, ethynyl (-C≡CH), propynyl (propargyl, -CH₂C≡CH), and the like.

The term "alkynylene" refers to a linear or branched divalent hydrocarbon radical of any length from two to twelve carbon atoms (C₂-C₁₂) with at least one site of unsaturation, i.e., a carbon-carbon, sp triple bond, wherein the alkynylene radical may be optionally substituted independently with one or more substituents described herein. Examples include, but are not limited to, ethynylene (-C≡C-), propynylene (propargylene, -CH₂C≡C-), and the like.

The terms "carbocycle", "carbocyclyl", "carbocyclic ring" and "cycloalkyl" refer to a monovalent non-aromatic, saturated or partially unsaturated ring having 3 to 15 carbon atoms (C₃-C₁₅). Such rings may be monocyclic or polycyclic, with 3 to 15 carbons present in a monocyclic ring or 7 to 15 carbon atoms present in a polycyclic (e.g., bicyclic) ring. Bicyclic carbocycles having 7 to 12 atoms can be arranged, for example, as a bicyclo [4,5], [5,5], [5,6] or [6,6] system, and bicyclic carbocycles having 9 or 10 ring atoms can be arranged as a bicyclo [5,6] or [6,6] system, or as bridged systems such as bicyclo[2.2.1]heptane, bicyclo[2.2.2]octane and bicyclo[3.2.2]nonane. Polycyclic (e.g., bicyclic) rings that are overall fully saturated or partially unsaturated are encompassed within the definition of the terms "carbocycle", "carbocyclyl", "carbocyclic ring" and "cycloalkyl," incuding when one or more of the fused rings in the polycyclic ring is fully unsaturated (i.e., aromatic). Spiro moieties are also included within the scope of this definition. Examples of monocyclic carbocycles include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, indenyl, indanyl, 1,2-dihydronaphthalene, 1,2,3,4-tetrahydronaphthyl, and the like. Carbocyclyl groups are optionally substituted independently with one or more substituents described herein.

The term "cycloalkylene" refers to a divalent non-aromatic, saturated or partially unsaturated ring having 3 to 12 carbon atoms (C₃-C₁₂) as a monocyclic ring or 7 to 12 carbon atoms as a bicyclic ring. Bicyclic cycloalkylenes having 7 to 12 atoms can be arranged, for example, as a bicyclo [4,5], [5,5], [5,6] or [6,6] system, and bicyclic cycloalkylenes having 9 or 10 ring atoms can be arranged as a bicyclo [5,6] or [6,6] system, or as bridged systems such as bicyclo[2.2.1]heptane, bicyclo[2.2.2]octane and bicyclo[3.2.2]nonane. Spiro moieties are also included within the scope of this definition. Examples of monocyclic cycloalkylenes include, but are not limited to, cyclopropylene, cyclobutylene, cyclopentylene, 1-cyclopent-1-enylene, 1-cyclopent-2-enylene, 1-cyclopent-3-enylene, cyclohexylene, 1-cyclohex-1-enylene, 1-cyclohex-2-enylene, 1-cyclohex-3-enylene, cyclohexadienylene, cycloheptylene, cyclooctylene, cyclononylene, cyclodecylene, cycloundecylene, cyclododecylene, and the like. Cycloalkylene groups are optionally substituted independently with one or more substituents described herein.

"Aryl" means a monovalent aromatic hydrocarbon radical of 6-20 carbon atoms (C₆-C₂₀) derived by the removal of one hydrogen atom from a single carbon atom of a parent aromatic ring system. Some aryl groups are represented in the exemplary structures as "Ar". Typical aryl groups include, but are not limited to, radicals derived from benzene (phenyl), substituted benzenes, naphthalene, anthracene, biphenyl, and the like. Aryl groups are optionally substituted independently with one or more substituents described herein.

"Arylene" means a divalent aromatic hydrocarbon radical of 6-20 carbon atoms (C₆-C₂₀) derived by the removal of two hydrogen atom from a two carbon atoms of a parent aromatic ring system. Some arylene groups are represented in the exemplary structures as "Ar". Arylene includes bicyclic radicals comprising an aromatic ring fused to a saturated, partially unsaturated ring, or aromatic carbocyclic ring. Typical arylene groups include, but are not limited to, radicals derived from benzene (phenylene), substituted benzenes, naphthalene, anthracene, biphenylene, indenylene, indanylene, 1,2-dihydronaphthalene, 1,2,3,4-tetrahydronaphthyl, and the like. Arylene groups are optionally substituted with one or more substituents described herein.

The terms "heterocycle," "heterocyclyl" and "heterocyclic ring" are used interchangeably herein and refer to a saturated or a partially unsaturated (i.e., having one or more double and/or triple bonds within the ring) carbocyclic radical of 3 to about 20 ring atoms in which at least one ring atom is a heteroatom selected from nitrogen, oxygen, phosphorus and sulfur, the remaining ring atoms being C, where one or more ring atoms is optionally substituted independently with one or more substituents described herein. A heterocycle may be a monocycle having 3 to 7 ring members (2 to 6 carbon atoms and 1 to 4 heteroatoms selected from N, O, P, and S) or a bicycle having 7 to 10 ring members (4 to 9 carbon atoms and 1 to 6 heteroatoms selected from N, O, P, and S), for example: a bicyclo [4,5], [5,5], [5,6], or [6,6] system. Heterocycles are described in Paquette, Leo A.; "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566. "Heterocyclyl" also includes radicals where heterocycle radicals are fused with a saturated, partially unsaturated ring, or aromatic carbocyclic or heterocyclic ring. Examples of heterocyclic rings include, but are not limited to, morpholin-4-yl, piperidin-1-yl, piperazinyl, piperazin-4-yl-2-one, piperazin-4-yl-3-one, pyrrolidin-1-yl, thiomorpholin-4-yl, S-dioxothiomorpholin-4-yl, azocan-1-yl, azetidin-1-yl, octahydropyrido[1,2-a]pyrazin-2-yl, [1,4]diazepan-1-yl, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, 3-azabicyco[3.1.0]hexanyl, 3-azabicyclo[4.1.0]heptanyl, azabicyclo[2.2.2]hexanyl, 3H-indolyl quinolizinyl and N-pyridyl ureas. Spiro moieties are also included within the scope of this definition. Examples of a heterocyclic group wherein 2 ring atoms are substituted with oxo (=O) moieties are pyrimidinonyl and 1,1-dioxo-thiomorpholinyl. The heterocycle groups herein are optionally substituted independently with one or more substituents described herein.

The term "heterocyclylene" refers to a divalent saturated or a partially unsaturated (i.e., having one or more double and/or triple bonds within the ring) carbocyclic radical of 3 to about 20 ring atoms in which at least one ring atom is a heteroatom selected from nitrogen, oxygen, phosphorus and sulfur, the remaining ring atoms being C, where one or more ring atoms is optionally substituted independently with one or more substituents described herein. A heterocyclylene may be a monocycle having 3 to 7 ring members (2 to 6 carbon atoms and 1 to 4 heteroatoms selected from N, O, P, and S) or a bicycle having 7 to 10 ring members (4 to 9 carbon atoms and 1 to 6 heteroatoms selected from N, O, P, and S), for example: a bicyclo [4,5], [5,5], [5,6], or [6,6] system. Heterocycles are described in Paquette, Leo A.; "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566. "Heterocyclylene" also includes divalent radicals where heterocycle radicals are fused with a saturated, partially unsaturated ring, or aromatic carbocyclic or heterocyclic ring. Examples of heterocyclylenes include, but are not limited to, morpholin-4-ylene, piperidin-1-ylene, piperazinylene, piperazin-4-ylene-2-one, piperazin-4-ylene-3-one, pyrrolidin-1-ylene, thiomorpholin-4-ylene, S-dioxothiomorpholin-4-ylene, azocan-1-ylene, azetidin-1-ylene, octahydropyrido[1,2-a]pyrazin-2-ylene, [1,4]diazepan-1-ylene, pyrrolidinylene, tetrahydrofuranylene, dihydrofuranylene, tetrahydrothienylene, tetrahydropyranylene, dihydropyranylene, tetrahydrothiopyranylene, piperidino, morpholino, thiomorpholino, thioxanylene, piperazinylene, homopiperazinylene, azetidinylene, oxetanylene, thietanylene, homopiperidinylene, oxepanylene, thiepanylene, oxazepinylene, diazepinylene, thiazepinylene, 2-pyrrolinylene, 3-pyrrolinylene, indolinylene, 2H-pyranylene, 4H-pyranylene, dioxanylene, 1,3-dioxolanylene, pyrazolinylene, dithianylene, dithiolanylene, dihydropyranylene, dihydrothienylene, dihydrofuranylene, pyrazolidinylimidazolinylene, imidazolidinylene, 3-azabicyco[3.1.0]hexanylene, 3-azabicyclo[4.1.0]heptanylene, azabicyclo[2.2.2]hexanylene, 3H-indolyl quinolizinyl and N-pyridyl ureas. Spiro moieties are also included within the scope of this definition. Examples of a heterocyclylene group wherein 2 ring atoms are substituted with oxo (=O) moieties are pyrimidinonylene and 1,1-dioxo-thiomorpholinylene. The heterocyclylene groups herein are optionally substituted independently with one or more substituents described herein.

The term "heteroaryl" refers to a monovalent aromatic radical of 5-, 6-, or 7-membered rings, and includes fused ring systems (at least one of which is aromatic) of 5-20 atoms, containing one or more heteroatoms independently selected from nitrogen, oxygen, and sulfur. Examples of heteroaryl groups are pyridinyl (including, for example, 2-hydroxypyridinyl), imidazolyl, imidazopyridinyl, 1-methyl-1H-benzo[d]imidazole, [1,2,4]triazolo[1,5-a]pyridine, pyrimidinyl (including, for example, 4-hydroxypyrimidinyl), pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. Heteroaryl groups are optionally substituted independently with one or more substituents described herein.

The term "heteroarylene" refers to a divalent aromatic radical of 5-, 6-, or 7-membered rings, and includes fused ring systems (at least one of which is aromatic) of 5-20 atoms, containing one or more heteroatoms independently selected from nitrogen, oxygen, and sulfur. Examples of heteroarylene groups are pyridinylene (including, for example, 2-hydroxypyridinylene), imidazolylene, imidazopyridinylene, 1-methyl-1H-benzo[d]imidazole, [1,2,4]triazolo[1,5-a]pyridine, pyrimidinylene (including, for example, 4-hydroxypyrimidinylene), pyrazolylene, triazolylene, pyrazinylene, tetrazolylene, furylene, thienylene, isoxazolylene, thiazolylene, oxadiazolylene, oxazolylene, isothiazolylene, pyrrolylene, quinolinylene, isoquinolinylene, tetrahydroisoquinolinylene, indolylene, benzimidazolylene, benzofuranylene, cinnolinylene, indazolylene, indolizinylene, phthalazinylene, pyridazinylene, triazinylene, isoindolylene, pteridinylene, purinylene, oxadiazolylene, thiadiazolylene, thiadiazolylene, furazanylene, benzofurazanylene, benzothiophenylene, benzothiazolylene, benzoxazolylene, quinazolinylene, quinoxalinylene, naphthyridinylene, and furopyridinylene. Heteroarylene groups are optionally substituted independently with one or more substituents described herein.

The heterocycle or heteroaryl groups may be carbon (carbon-linked), or nitrogen (nitrogen-linked) bonded where such is possible. By way of example and not limitation, carbon bonded heterocycles or heteroaryls are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline.

By way of example, and not limitation, nitrogen bonded heterocycles or heteroaryls are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline.

The term "acyl" refers to both substituted and unsubstituted acyl. In certain embodiments, an "acyl" may be -C(O)-R¹⁶, wherein R¹⁶ is selected from the group consisting of substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, and substituted or unsubstituted heterocyclyl. In one particular embodiment, it is a substituted C₁-C₃ alkyl.

The term "oxo" refers to "=O".

As provided herein, a symbol comprising a closed circle, drawn with a solid line, with a label at the center of the same (e.g., 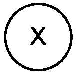 ), denotes a ring moiety, wherein, unless otherwise indicated, the ring moiety may comprise any suitable number and type of annular atoms. For example, the ring moiety may comprise, without limitation, a cycloalkyl moiety, an aryl moiety, a heterocyclyl moiety, or a heteroaryl moiety, as defined herein, comprising any suitable number and type of annular atoms. Such a symbol may be used interchangeably with the term "ring". By way of illustration, the symbol " 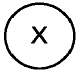 " and the term "ring X" are interchangeable, and both refer to a ring moiety X, wherein, unless otherwise indicated, the ring moiety X may comprise any suitable number and type of annular atoms.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The terms "co-administration" and "co-administering" or "combination therapy" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent, preferably at effective amounts, at the same time. In certain preferred aspects, one or more of the present compounds described herein, are coadministered in combination with at least one additional bioactive agent, especially including an anticancer agent. In particularly preferred aspects, the co-administration of compounds results in synergistic activity and/or therapy, including anticancer activity.

The term "compound", as used herein, unless otherwise indicated, refers to any specific chemical compound disclosed herein and includes tautomers, regioisomers, geometric isomers, and where applicable, stereoisomers, including optical isomers (enantiomers) and other stereoisomers (diastereomers) thereof, as well as pharmaceutically acceptable salts and derivatives (including prodrug forms) thereof where applicable, in context. Within its use in context, the term compound generally refers to a single compound, but also may include other compounds such as stereoisomers, regioisomers and/or optical isomers (including racemic mixtures) as well as specific enantiomers or enantiomerically enriched mixtures of disclosed compounds. The term also refers, in context to prodrug forms of compounds which have been modified to facilitate the administration and delivery of compounds to a site of activity. It is noted that in describing the present compounds, numerous substituents and variables associated with same, among others, are described. It is understood by those of ordinary skill that molecules which are described herein are stable compounds as generally described hereunder. When the bond 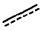 is shown, both a double bond and single bond are represented within the context of the compound shown. When a crossed double bond ( 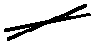 ) is shown, both the E and Z configurations are represented within the context of the compound shown; and the compound may contain the E isomer or the Z isomer or a mixture of both the E and Z isomers.

The term "VCB E3 Ubiquitin Ligase," "Von Hippel-Lindau (or VHL) E3 Ubiquitin Ligase," "VHL," or "Ubiquitin Ligase," which are generally used interchangeably unless the context indicates otherwise, is used to describe a target enzyme(s) binding site of ubiquitin ligase moieties as described herein. VCB E3 is a protein that in combination with an E2 ubiquitin-conjugating enzyme causes the attachment of ubiquitin to a lysine on a target protein; the E3 ubiquitin ligase targets specific protein substrates for degradation by the proteasome. Thus, E3 ubiquitin ligase alone or in complex with an E2 ubiquitin conjugating enzyme is responsible for the transfer of ubiquitin to targeted proteins. In general, the ubiquitin ligase is involved in polyubiquitination such that a second ubiquitin is attached to the first; a third is attached to the second, and so forth. Polyubiquitination marks proteins for degradation by the proteasome. However, there are some ubiquitination events that are limited to mono-ubiquitination, in which only a single ubiquitin is added by the ubiquitin ligase to a substrate molecule. Mono-ubiquitinated proteins are not targeted to the proteasome for degradation, but may instead be altered in their cellular location or function, for example, via binding other proteins that have domains capable of binding ubiquitin. Further complicating matters, different lysines on ubiquitin can be targeted by an E3 to make chains. The most common lysine is Lys48 on the ubiquitin chain. This is the lysine used to make polyubiquitin, which is recognized by the proteasome.

As used herein, a moiety that binds the E3 VHL ubiquitin ligase or a component thereof, is referred to a VHL ligand.

In certain embodiments disclosed herein, certain groups (e.g., alkyl, alkenyl, alkynyl, cycloalkyl, aryl, heteroaryl or heterocyclyl) are described as "substituted". In some such embodiments, the "substituted" group may be substituted with 1, 2, 3, 4, 5, or more substituents, as indicated herein. In certain embodiments, alkyl, alkenyl, alkynyl, cycloalkyl, aryl, heteroaryl or heterocyclyl may be substituted with one or more substituents independently selected from, but not limited to, alkyl, alkenyl, alkynyl, cycloalkyl heterocyclyl, aryl, heteroaryl, halo (i.e., halogen), haloalkyl, oxo, OH, CN, -O-alkyl, S-alkyl, NH-alkyl, N(alkyl)₂, O-cycloalkyl, S-cycloalkyl, NH-cycloalkyl, N(cycloalkyl)₂, N(cycloalkyl)(alkyl), NH₂, SH, SO₂-alkyl, P(O)(O-alkyl)(alkyl), P(O)(O-alkyl)₂, Si(OH)₃, Si(alkyl)₃, Si(OH)(alkyl)₂, CO-alkyl, CO₂H, NO₂, SF₅, SO₂NH-alkyl, SO₂N(alkyl)₂, SONH-alkyl, SON(alkyl)₂, CONH-alkyl, CON(alkyl)₂, N(alkyl)CONH(alkyl), N(alkyl)CON(alkyl)₂, NHCONH(alkyl), NHCON(alkyl)₂, NHCONH₂, N(alkyl)SO₂NH(alkyl), N(alkyl)SO₂N(alkyl)₂, NHSO₂NH(alkyl), NHSO₂N(alkyl)₂, and NHSO₂NH₂.

Still additional definitions and abbreviations are provided elsewhere herein.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

In the claims, as well as in the specification above, transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

As used herein in the specification and in the claims, the phrase "at least one," in reference to a list of one or more elements, should be understood to mean at least one element selected from anyone or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a nonlimiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

### II. Compounds

E3 ubiquitin ligases (of which over 600 are known in humans) confer substrate specificity for ubiquitination. There are known ligands which bind to these ligases. An E3 ubiquitin ligase binding group (E3LB) is a peptide or small molecule that can bind an E3 ubiquitin ligase.

A particular E3 ubiquitin ligase is von Hippel-Lindau (VHL) tumor suppressor, the substrate recognition subunit of the E3 ligase complex VCB, which also consists of elongins B and C, Cul2, and Rbxl. The primary substrate of VHL is Hypoxia Inducible Factor lα (HIF- lα), a transcription factor that upregulates genes such as the pro-angiogenic growth factor VEGF and the red blood cell inducing cytokine erythropoietin in response to low oxygen levels.

In one embodiment, provided herein is a compound of formula (I): 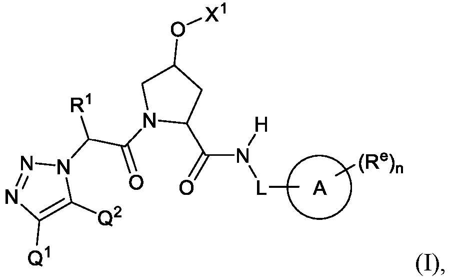 or a stereoisomer or tautomer thereof, or a pharmaceutically acceptable salt of any of the foregoing, wherein
X¹ is, independently at each occurrence, H, C₁₋₁₂alkyl, or -C(O)-C₁₋₁₂alkyl;
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
L is, independently at each occurrence, absent or is C₁₋₁₂alkylene, wherein the C₁₋₁₂alkylene of L is independently optionally substituted with one or more R^{t}, wherein R^{t} is C₁₋₁₂alkyl or - C(O)NH₂, wherein the C₁₋₁₂alkyl of R^{t} is further optionally substituted with one or more halo;
ring A is, independently at each occurrence, C₆₋₂₀aryl or C₇₋₁₅cycloalkyl;
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo;
n is, independently at each occurrence, 0, 1, 2, 3, 4, or 5; and
Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ or Q² is independently optionally substituted with one or more R⁹, wherein R⁹ is C₁₋₁₂alkyl, C₂₋₁₂alkenyl, C₂₋₁₂alkynyl, C₆₋₂₀aryl, C₁₋₁₂alkoxy, or 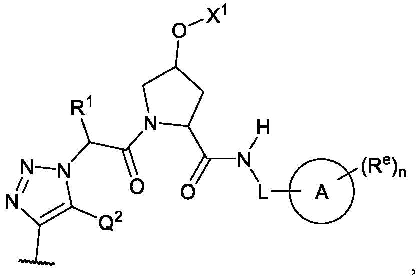 wherein the C₁₋₁₂alkyl or C₁₋₁₂alkoxy of R^{q} is independently further optionally substituted with one or more halo or - NHC(O)-C₁₋₁₂alkyl,
or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,
   wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, - C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In some embodiments, provided herein is a compound of formula (I), or a stereoisomer or tautomer thereof, or a pharmaceutically acceptable salt of any of the foregoing, wherein L is, independently at each occurrence, absent.

In other embodiments, provided herein is a compound of formula (I), or a stereoisomer or tautomer thereof, or a pharmaceutically acceptable salt of any of the foregoing, wherein L is, independently at each occurrence, C₁₋₁₂alkylene, wherein the C₁₋₁₂alkylene of L is independently optionally substituted with one or more R^{t}, wherein R^{t} is independently C₁₋₁₂alkyl or -C(O)NH₂, wherein the C₁₋₁₂alkyl of R^{t} is further independently optionally substituted with one or more halo. In certain embodiments, L is, independently at each occurrence, C₁₋₆alkylene, wherein the C₁₋₆alkylene of L is independently optionally substituted with one or more R^{t}, wherein R^{t} is independently C₁₋₆alkyl or -C(O)NH₂, wherein the C₁₋₆alkyl of R^{t} is further independently optionally substituted with one or more halo. In some embodiments, L is, independently at each occurrence, C₁₋₃alkylene, wherein the C₁₋₃alkylene of L is independently optionally substituted with one or more R^{t}, wherein R^{t} is independently C₁₋₆alkyl or -C(O)NH₂, wherein the C₁₋₆alkyl of R^{t} is further independently optionally substituted with one or more halo. In some embodiments, L is, independently at each occurrence, ethylene, wherein the ethylene of L is independently optionally substituted with one or more R^{t}, wherein R^{t} is independently C₁₋₆alkyl or -C(O)NH₂, wherein the C₁₋₆alkyl of R^{t} is further independently optionally substituted with one or more halo. In some embodiments, L is, independently at each occurrence, methylene, wherein the methylene of L is independently optionally substituted with one or more R^{t}, wherein R^{t} is independently C₁₋₆alkyl or -C(O)NH₂, wherein the C₁₋₆alkyl of R^{t} is further independently optionally substituted with one or more halo.

In other embodiments, provided herein is a compound of formula (I), or a stereoisomer or tautomer thereof, or a pharmaceutically acceptable salt of any of the foregoing, wherein L is, independently at each occurrence, unsubstituted ethylene. In certain embodiments, L is, independently at each occurrence, ethylene, wherein the ethylene of L is independently substituted with one R^{t}, wherein R^{t} is independently C₁₋₆alkyl or -C(O)NH₂, wherein the C₁₋₆alkyl of R^{t} is further independently optionally substituted with one or more halo. In some embodiments, L is, independently at each occurrence, ethylene, wherein the ethylene of L is substituted with one -C(O)NH₂.

In certain embodiments, provided herein is a compound of formula (I), or a stereoisomer or tautomer thereof, or a pharmaceutically acceptable salt of any of the foregoing, wherein L is, independently at each occurrence, unsubstituted methylene. In certain embodiments, L is, independently at each occurrence, methylene, wherein the methylene of L is substituted with one R^{t}, wherein R^{t} is independently C₁₋₆alkyl or -C(O)NH₂, wherein the C₁₋₆alkyl of R^{t} is further independently optionally substituted with one or more halo. In certain embodiments, L is, independently at each occurrence, methylene, wherein the methylene of L is substituted with one C₁₋₆alkyl. In some embodiments, L is, independently at each occurrence, methylene, wherein the methylene is L is susbtitued with methyl. In other embodiments, L is, independently at each occurrence, methylene, wherein the methylene of L is substituted with -CF₃.

In some embodiments, provided herein is a compound of formula (I), or a stereoisomer or tautomer thereof, or a pharmaceutically acceptable salt of any of the foregoing, wherein ring A, independently at each occurrence, is C₆₋₂₀aryl. In certain embodiments, ring A is, independently at each occurrence, C₆₋₁₆aryl. In still other embodiments, ring A is, independently at each occurrence, C₆₋₁₂aryl. In some embodiments, ring A is, independently at each occurrence, C₆₋₁₀aryl. In other embodiments, ring A is, independently at each occurrence, C₆₋₈aryl. In certain embodiments, ring A is, independently at each occurrence, phenyl.

In other embodiments, provided herein is a compound of formula (I), or a stereoisomer or tautomer thereof, or a pharmaceutically acceptable salt of any of the foregoing, wherein ring A is, independently at each occurrence, C₇₋₁₅cycloalkyl. In some embodiments, ring A is, independently at each occurrence, C₇₋₁₂cycloalkyl. In other embodiments, ring A is, independently at each occurrence, C₇₋₁₀cycloalkyl. In certain embodiments, ring A is, independently at each occurrence, C₇₋₈cycloalkyl. In some embodiments, ring A is, independently at each occurrence, C₁₀₋₁₅cycloalkyl. In certain embodiments, ring A is, independently at each occurrence, C₁₂₋₁₅cycloalkyl.

In some embodiments, n is, independently at each occurrence, 0, 1, 2, 3, 4, or 5. In other embodiments, n is, independently at each occurrence, 0, 1, 2, 3, or 4. In other embodiments, n is, independently at each occurrence, 0, 1, 2, or 3. In other embodiments, n is, independently at each occurrence, 0, 1, or 2. In certain embodiments, n is, independently at each occurrence, 0 or 1. In some embodiments, n is, independently at each occurrence, 2. In other embodiments, n is, independently at each occurrence, 1. In certain embodiments, n is, independently at each occurrence, 0.

In some embodiments, provided herein is a compound of formula (I), or a stereoisomer or tautomer thereof, or a pharmaceutically acceptable salt of any of the foregoing, wherein R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo.

In some embodiments, R^{e} is, independently at each occurrence, halo. In certain embodiments, R^{e} is, independently at each occurrence, halo, wherein the halo is independently fluoro or chloro. In some embodiments, R^{e} is, independently at each occurrence, halo, wherein the halo is chloro.

In other embodiments, R^{e} is, independently at each occurrence, C₆₋₂₀aryl, wherein the C₆₋₂₀aryl of R^{e} is independently optionally substituted with one or more C₁₋₁₂alkyl or halo. In some embodiments, R^{e} is, independently at each occurrence, unsubstituted C₆₋₂₀aryl. In other embodiments, R^{e} is, independently at each occurrence, C₆₋₂₀aryl, wherein the C₆₋₂₀aryl of R^{e} is independently optionally substituted with one or more halo. In some embodiments, R^{e} is, independently at each occurrence, C₆₋₂₀aryl, wherein the C₆₋₂₀aryl of R^{e} is independently optionally substituted with one or more halo, wherein the halo is fluoro. In some embodiments, R^{e} is, independently at each occurrence, 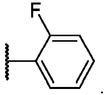 In other embodiments, R^{e} is, independently at each occurrence,

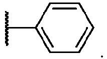

In certain embodiments, R^{e} is, independently at each occurrence, 5-20 membered heteroaryl, wherein the 5-20 membered heteroaryl of R^{e} is independently optionally substituted with one or more C₁₋₁₂alkyl or halo. In some embodiments, R^{e} is, independently at each occurrence, 5-20 membered heteroaryl, wherein the 5-20 membered heteroaryl of R^{e} is independently optionally substituted with one or more C₁₋₁₂alkyl. In some embodiments, R^{e} is, independently at each occurrence, 5-20 membered heteroaryl, wherein the 5-20 membered heteroaryl of R^{e} independently comprises 1, 2, 3, or 4 annular heteroatoms. In some embodiments, the 5-20 membered heteroaryl of R^{e} independently comprises 1 or 2 annular heteroatoms. In other embodiments, the 5-20 membered heteroaryl of R^{e} independently comprises 2 annular heteroatoms. In still other embodiments, the 5-20 membered heteroaryl of R^{e} independently comprises 1 annular heteroatom. In some embodiments, R^{e} is, independently at each occurrence, 5-20 membered heteroaryl, wherein the 5-20 membered heteroaryl of R^{e} independently comprises 1, 2, 3, or 4 annular heteroatoms independently selected from the group consisting of N, S, and O. In some embodiments, the 1, 2, 3, or 4 heteroatoms are all the same heteroatom. In other embodiments, the 1, 2, 3, or 4 heteroatoms comprise a combination of different heteroatoms.

In some embodiments, R^{e} is, independently at each occurrence, 5-20 membered heteroaryl, wherein the 5-20 membered heteroaryl of R^{e} is independently a 5-16 membered heteroaryl. In some embodiments, R^{e} is, independently at each occurrence, 5-12 membered heteroaryl. In other embodiments, R^{e} is, independently at each occurrence, 5-10 membered heteroaryl. In still other embodiments, R^{e} is, independently at each occurrence, 5-7 membered heteroaryl. In some embodiments, R^{e} is, independently at each occurrence, 5-6 membered heteroaryl. In other embodiments, R^{e} is, independently at each occurrence, 6-membered heteroaryl. In certain embodiments, R^{e} is, independently at each occurrence, 5-membered heteroaryl. In certain embodiments, R^{e} is, independently at each occurrence, thiazolyl, wherein the thiazolyl of R^{e} is independently optionally substituted with one or more C₁₋₁₂alkyl or halo. In certain embodiments, R^{e} is, independently at each occurrence, thiazolyl, wherein the thiazolyl of R^{e} is independently optionally substituted with one or more C₁₋₆alkyl. In some embodiments, R^{e} is, independently at each occurrence, thiazolyl, wherein the thiazolyl of R^{e} is independently optionally substituted with one or more methyl. In some embodiments, R^{e} is, independently at each occurrence,

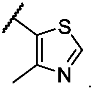

In some embodiments, provided herein is a compound of formula (I), or a stereoisomer or tautomer thereof, or a pharmaceutically acceptable salt of any of the foregoing, wherein L is, independently at each occurrence, C₁₋₁₂alkylene, wherein the C₁₋₁₂alkylene of L is independently optionally substituted with one or more R^{t}, wherein R^{t} is independently C₁₋₁₂alkyl or -C(O)NH₂, wherein the C₁₋₁₂alkyl of R^{t} is further independently optionally substituted with one or more halo, and ring A is is, independently at each occurrence, C₆₋₂₀aryl. In other embodiments, L is, independently at each occurrence, C₁₋₆alkylene, wherein the C₁₋₆alkylene of L is independently optionally substituted with one or more R^{t}, wherein R^{t} is independently C₁₋₁₂alkyl or -C(O)NH₂, wherein the C₁₋₁₂alkyl of R^{t} is further independently optionally substituted with one or more halo, and ring A is, independently at each occurrence, C₆₋₂₀aryl. In some embodiments, L is, independently at each occurrence, C₁₋₆alkylene, wherein the C₁₋₆alkylene of L is independently optionally substituted with one or more R^{t}, wherein R^{t} is independently C₁₋₁₂alkyl or -C(O)NH₂, wherein the C₁₋₁₂alkyl of R^{t} is independently further optionally substituted with one or more halo, and ring A is, independently at each occurrence, phenyl. In other embodiments, L is, independently at each occurrence, ethylene, wherein the ethylene of L is independently optionally substituted with one or more R^{t}, wherein R^{t} is independently C₁₋₁₂alkyl or -C(O)NH₂, wherein the C₁₋₁₂alkyl of R^{t} is independently further optionally substituted with one or more halo, and ring A is, independently at each occurrence, phenyl.

In some embodiments, provided herein is a compound of formula (I), or a stereoisomer or tautomer thereof, or a pharmaceutically acceptable salt of any of the foregoing, wherein L is, independently at each occurrence, C₁₋₁₂alkylene, wherein the C₁₋₁₂alkylene of L is independently optionally substituted with one or more R^{t}, wherein R^{t} is independently C₁₋₁₂alkyl or -C(O)NH₂, wherein the C₁₋₁₂alkyl of R^{t} is further independently optionally substituted with one or more halo, ring A is, independently at each occurrence, C₆₋₂₀aryl, n is,independently at each occurrence, 1, and R^{e} is, independently at each occurrence, 5-20 membered heteroaryl, wherein the 5-20 membered heteroaryl of R^{e} is independently optionally substituted with one or more C₁₋₁₂alkyl or halo. In certain embodiments, L is, independently at each occurrence, C₁₋₆alkylene, wherein the C₁₋₆alkylene of L is independently optionally substituted with one or more R^{t}, wherein R^{t} is independently C₁₋₁₂alkyl or -C(O)NH₂, wherein the C₁₋₁₂alkyl of R^{t} is further independently optionally substituted with one or more halo, ring A is, independently at each occurrence, C₆₋₂₀aryl, n is, independently at each occurrence, 1, and R^{e} is, independently at each occurrence, 5-20 membered heteroaryl, wherein the 5-20 membered heteroaryl of R^{e} is independently optionally substituted with one or more C₁₋₁₂alkyl or halo. In some embodiments, L is, independently at each occurrence, C₁₋₆alkylene, wherein the C₁₋₆alkylene of L is independently optionally substituted with one or more C₁₋₁₂alkyl, ring A is, independently at each occurrence, C₆₋₂₀aryl, n is, independently at each occurrence, 1, and R^{e} is, independently at each occurrence, 5-20 membered heteroaryl, wherein the 5-20 membered heteroaryl of R^{e} is independently optionally substituted with one or more C₁₋₁₂alkyl.

In certain embodiments, provided herein is a compound of formula (I), or a stereoisomer or tautomer thereof, or a pharmaceutically acceptable salt of any of the foregoing, wherein the compound of formula (I) is a compound of formula (IA): 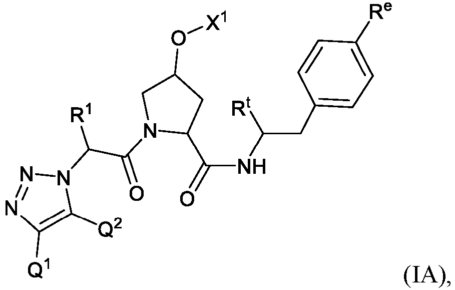 or a stereoisomer or tautomer thereof, or a pharmaceutically acceptable salt of any of the foregoing, wherein:
X¹ is, independently at each occurrence, H, C₁₋₁₂alkyl, or -C(O)-C₁₋₁₂alkyl;
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo;
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo; and
Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ or Q² is independently optionally substituted with one or more R⁹, wherein R⁹ is C₁₋₁₂alkyl, C₂₋₁₂alkenyl, C₂₋₁₂alkynyl, C₆₋₂₀aryl, C₁₋₁₂alkoxy, or 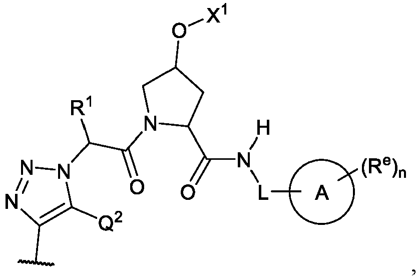 wherein the C₁₋₁₂alkyl or C₁₋₁₂alkoxy of R^{q} is independently further optionally substituted with one or more halo or - NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,
wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, - C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In embodiments, X¹ is H; R¹ is C₁₋₁₂alkyl, C₂₋₁₂alkenyl or C₃₋₁₅cycloalkyl, wherein the C₁₋₁₂alkyl is independently optionally substituted with one or more C₆₋₂₀aryl; R^{t} is C₁₋₁₂alkyl or -C(O)NH₂, wherein the C₁₋₁₂alkyl of R^{t} is further optionally substituted with one or more halo; R^{e} is C₆₋₂₀aryl or 5-20 membered heteroaryl, wherein the C₆₋₂₀aryl or 5-20 membered heteroaryl of R^{e} is independently optionally substituted with one or more C₁₋₁₂alkyl or halo; and Q¹ and Q² are, independently of each other and independently at each occurrence, H, halo, cyano, C₁₋₁₂alkyl, C₃₋₁₅cycloalkyl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R⁹ is C₁₋₁₂alkyl, C₂₋₁₂alkynyl, C₆₋₂₀aryl, C₁₋₁₂alkoxy, or 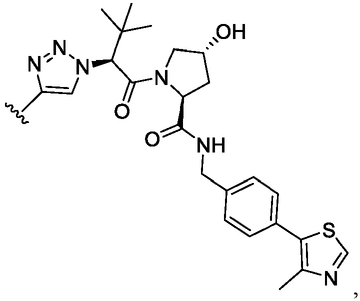 wherein the C₁₋₁₂alkyl or C₁₋₁₂alkoxy of R^{q} is independently further optionally substituted with one or more halo or -NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, -C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In embodiments, X¹ is H; R¹ is C₁₋₁₂alkyl, C₂₋₁₂alkenyl or C₃₋₁₅cycloalkyl, wherein the C₁₋₁₂alkyl is independently optionally substituted with one or more C₆₋₂₀aryl; R^{t} is H, methyl, -CF₃, or -C(O)NH₂, wherein the methyl of R^{t} is further optionally substituted with one or more halo; R^{e} is, independently at each occurrence, 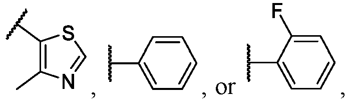 and Q¹ and Q² are, independently of each other and independently at each occurrence, H, halo, cyano, C₁₋₅alkyl, C₃₋₁₅cycloalkyl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R⁹ is C₁₋₁₂alkyl, C₂₋₁₂alkynyl, C₆₋₂₀aryl, C₁₋₁₂alkoxy, or 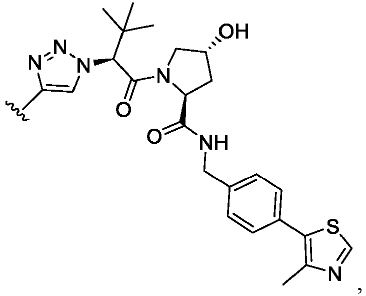 wherein the C₁₋₁₂alkyl or C₁₋₁₂alkoxy of R^{q} is independently further optionally substituted with one or more halo or -NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, -C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In certain embodiments, provided herein is a compound of formula (I), or a stereoisomer or tautomer thereof, or a pharmaceutically acceptable salt of any of the foregoing, wherein the compound of formula (I) is a compound of formula (IA-1): 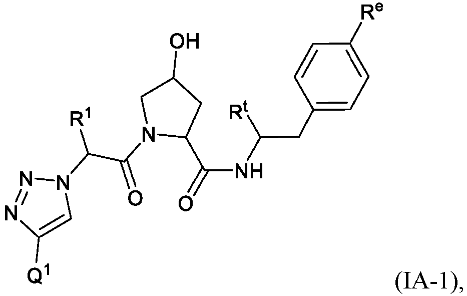 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo;
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo; and
Q¹ is selected from the group consisting of H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ is independently optionally substituted with one or more R^{q}, wherein R⁹ is C₁₋₁₂alkyl, C₂₋₁₂alkenyl, C₂₋₁₂alkynyl, C₆₋₂₀aryl, C₁₋₁₂alkoxy, or 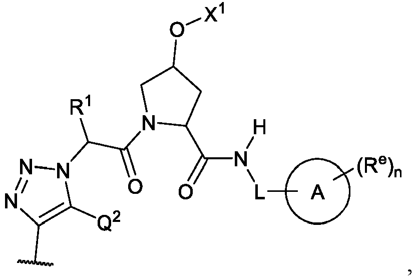 wherein the C₁₋₁₂alkyl or C₁₋₁₂alkoxy of R^{q} is independently further optionally substituted with one or more halo or -NHC(O)-C₁₋₁₂alkyl.

In certain embodiments, provided herein is a compound of formula (I), or a stereoisomer or tautomer thereof, or a pharmaceutically acceptable salt of any of the foregoing, wherein the compound of formula (I) is a compound of formula (IA-2): 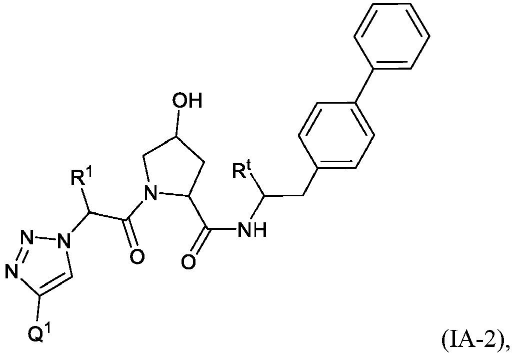 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo; and
Q¹ is selected from the group consisting of H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 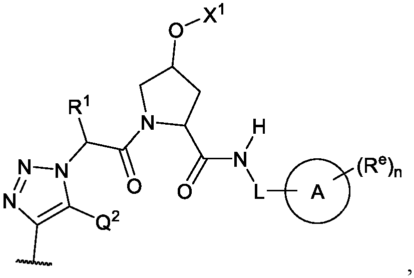 wherein the C₁₋₁₂alkyl or C₁₋₁₂alkoxy of R^{q} is independently further optionally substituted with one or more halo or -NHC(O)-C₁₋₁₂alkyl.

In certain embodiments, provided herein is a compound of formula (I), or a stereoisomer or tautomer thereof, or a pharmaceutically acceptable salt of any of the foregoing, wherein the compound of formula (I) is a compound of formula (IA-3): 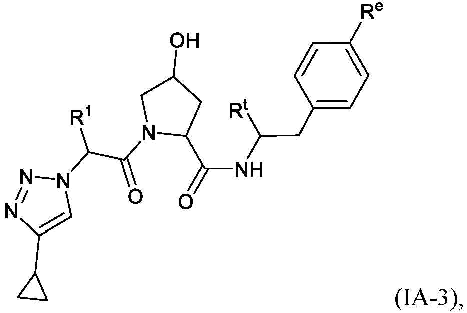 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo; and
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo.

In certain embodiments, provided herein is a compound of formula (I), or a stereoisomer or tautomer thereof, or a pharmaceutically acceptable salt of any of the foregoing, wherein the compound of formula (I) is a compound of formula (IA-4): 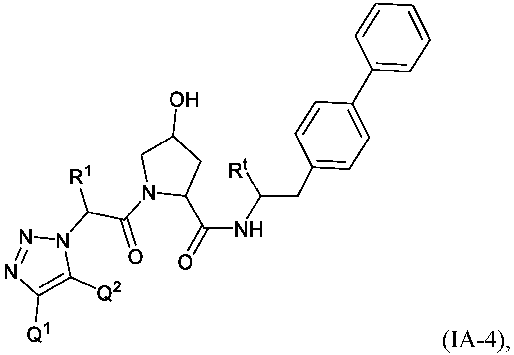 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo; and
Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ or Q² is independently optionally substituted with one or more R^{q}, wherein R⁹ is C₁₋₁₂alkyl, C₂₋₁₂alkenyl, C₂₋₁₂alkynyl, C₆₋₂₀aryl, C₁₋₁₂alkoxy, or 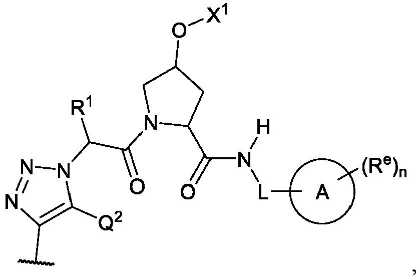 wherein the C₁₋₁₂alkyl or C₁₋₁₂alkoxy of R^{q} is independently further optionally substituted with one or more halo or - NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,
   wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, - C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In certain embodiments, provided herein is a compound of formula (I), or a stereoisomer or tautomer thereof, or a pharmaceutically acceptable salt of any of the foregoing, wherein the compound of formula (I) is a compound of formula (IB): 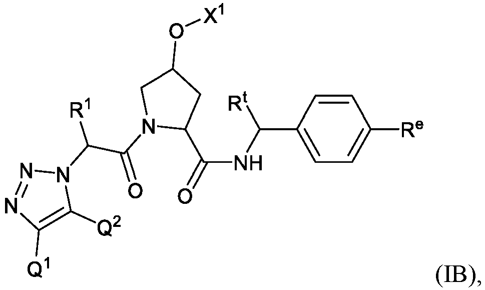 or a stereoisomer or tautomer thereof, or a pharmaceutically acceptable salt of any of the foregoing, wherein:
X¹ is, independently at each occurrence, H, C₁₋₁₂alkyl, or -C(O)-C₁₋₁₂alkyl;
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo;
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo; and
Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ or Q² is independently optionally substituted with one or more R^{q}, wherein R⁹ is C₁₋₁₂alkyl, C₂₋₁₂alkenyl, C₂₋₁₂alkynyl, C₆₋₂₀aryl, C₁₋₁₂alkoxy, or 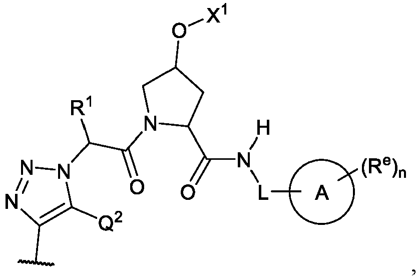 wherein the C₁₋₁₂alkyl or C₁₋₁₂alkoxy of R^{q} is independently further optionally substituted with one or more halo or - NHC(O)-C₁₋₁₂alkyl, or
Q¹ and Q² are taken, together with the atoms to which they are attached, to form a C₃₋₁₅cycloalkyl, 3-15 membered heterocyclyl, C₆₋₂₀aryl, or 5-20 membered heteroaryl,
   wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, - C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In embodiments, X¹ is H; R¹ is C₁₋₁₂alkyl, C₂₋₁₂alkenyl or C₃₋₁₅cycloalkyl, wherein the C₁₋₁₂alkyl is independently optionally substituted with one or more C₆₋₂₀aryl; R^{t} is C₁₋₁₂alkyl or -C(O)NH₂, wherein the C₁₋₁₂alkyl of R^{t} is further optionally substituted with one or more halo; R^{e} is C₆₋₂₀aryl or 5-20 membered heteroaryl, wherein the C₆₋₂₀aryl or 5-20 membered heteroaryl of R^{e} is independently optionally substituted with one or more C₁₋₁₂alkyl or halo; and Q¹ and Q² are, independently of each other and independently at each occurrence, H, halo, cyano, C₁₋₁₂alkyl, C₃₋₁₅cycloalkyl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R^{q} is C₁₋₁₂alkyl, C₂₋₁₂alkynyl, C₆₋₂₀aryl, C₁₋₁₂alkoxy, or 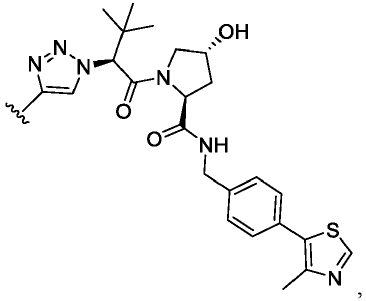 wherein the C₁₋₁₂alkyl or C₁₋₁₂alkoxy of R^{q} is independently further optionally substituted with one or more halo or -NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, -C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In embodiments, X¹ is H; R¹ is C₁₋₁₂alkyl, C₂₋₁₂alkenyl or C₃₋₁₅cycloalkyl, wherein the C₁₋₁₂alkyl is independently optionally substituted with one or more C₆₋₂₀aryl; R^{t} is H, methyl, -CF₃, or -C(O)NH₂, wherein the methyl of R^{t} is further optionally substituted with one or more halo; R^{e} is, independently at each occurrence, 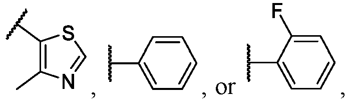 and Q¹ and Q² are, independently of each other and independently at each occurrence, H, halo, cyano, C₁₋₅alkyl, C₃₋₁₅cycloalkyl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R^{q} is C₁₋₁₂alkyl, C₂₋₁₂alkynyl, C₆₋₂₀aryl, C₁₋₁₂alkoxy, or 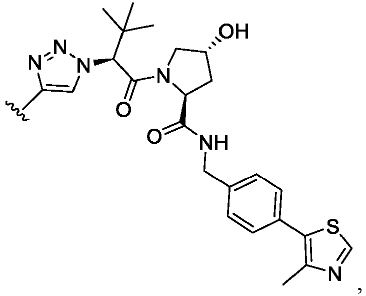 wherein the C₁₋₁₂alkyl or C₁₋₁₂alkoxy of R^{q} is independently further optionally substituted with one or more halo or -NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, -C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In certain embodiments, provided herein is a compound of formula (I), or a stereoisomer or tautomer thereof, or a pharmaceutically acceptable salt of any of the foregoing, wherein the compound of formula (I) is a compound of formula (IB-1): 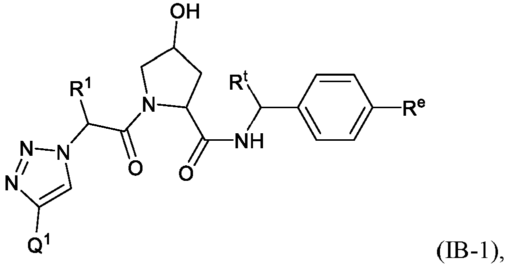 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo;
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo; and
Q¹ is selected from the group consisting of H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 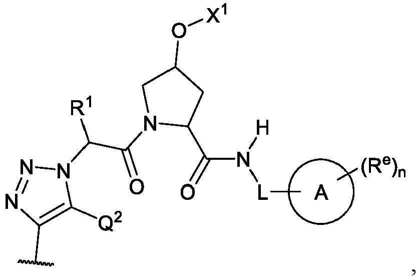 wherein the C₁₋₁₂alkyl or C₁₋₁₂alkoxy of R^{q} is independently further optionally substituted with one or more halo or -NHC(O)-C₁₋₁₂alkyl.

In certain embodiments, provided herein is a compound of formula (I), or a stereoisomer or tautomer thereof, or a pharmaceutically acceptable salt of any of the foregoing, wherein the compound of formula (I) is a compound of formula (IB-2): 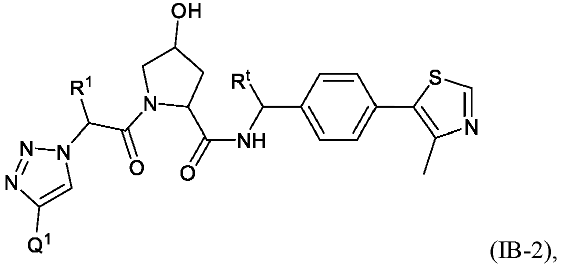 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo; and
Q¹ is selected from the group consisting of H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
   wherein the C₁₋₁₂alkyl or C₃₋₁₅cycloalkyl of Q¹ is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 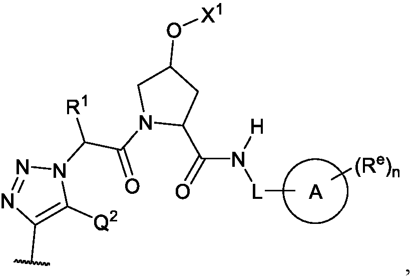 wherein the C₁₋₁₂alkyl or C₁₋₁₂alkoxy of R^{q} is independently further optionally substituted with one or more halo or -NHC(O)-C₁₋₁₂alkyl.

In certain embodiments, provided herein is a compound of formula (I), or a stereoisomer or tautomer thereof, or a pharmaceutically acceptable salt of any of the foregoing, wherein the compound of formula (I) is a compound of formula (IB-3): 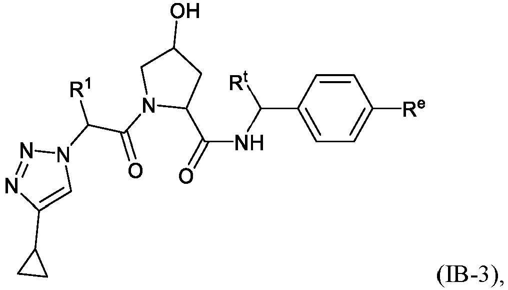 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
R^{t} is H, C₁₋₁₂alkyl or -C(O)NH₂, wherein the C₁₋₁₂alkyl of R^{t} is further optionally substituted with one or more halo; and
R^{e} is, independently at each occurrence, halo, C₆₋₂₀aryl, or 5-20 membered heteroaryl, wherein the C₆₋₂₀aryl or 5-20 membered heteroaryl of R^{e} is independently optionally substituted with one or more C₁₋₁₂alkyl or halo.

In certain embodiments, provided herein is a compound of formula (I), or a stereoisomer or tautomer thereof, or a pharmaceutically acceptable salt of any of the foregoing, wherein the compound of formula (I) is a compound of formula (IB-4): 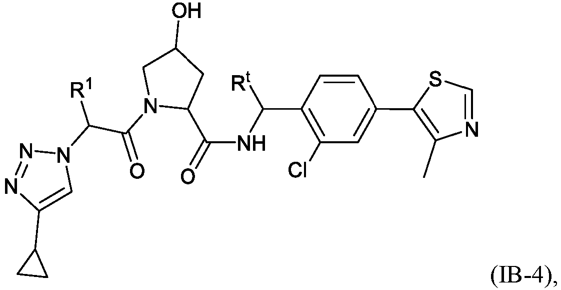 or a stereoisomer or tautomer thereof, or a pharmaceutically acceptable salt of any of the foregoing, wherein:
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
   wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl; and
R^{t} is H, C₁₋₁₂alkyl or -C(O)NH₂, wherein the C₁₋₁₂alkyl of R^{t} is further optionally substituted with one or more halo.

In some embodiments, L is, independently at each occurrence, unsubstituted methylene, ring A is, independently at each occurrence, C₆₋₂₀aryl, n is, independently at each occurrence, 1, and R^{e} is, independently at each occurrence, 5-20 membered heteroaryl, wherein the 5-20 membered heteroaryl of R^{e} is independently optionally substituted with one or more C₁₋₁₂alkyl. In other embodiments, L is, independently at each occurrence, unsubstituted methylene, ring A is, independently at each occurrence, C₆₋₂₀aryl, n is, independently at each occurrence, 1, and R^{e} is, independently at each occurrence, thiazolyl, wherein the thiazolyl of R^{e} is independently optionally substituted with one or more C₁₋₁₂alkyl. In certain embodiments, provided herein is a compound of formula (I), or a stereoisomer or tautomer thereof, or a pharmaceutically acceptable salt of any of the foregoing, wherein the compound of formula (I) is a compound of formula (IC): 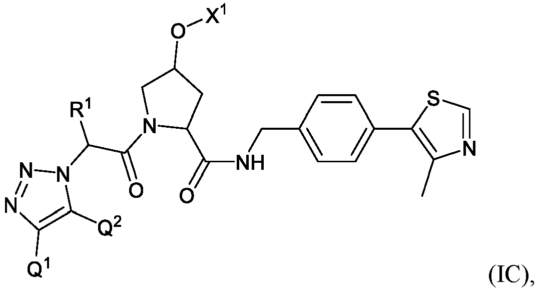 or a stereoisomer or tautomer thereof, or a pharmaceutically acceptable salt of any of the foregoing.

In other embodiments, L is, independently at each occurrence, methylene, wherein the methylene of L is independently substituted with one or more C₁₋₁₂alkyl, ring A is, independently at each occurrence, C₆₋₂₀aryl, n is, independently at each occurrence, 1, and R^{e} is, independently at each occurrence, 5-20 membered heteroaryl, wherein the 5-20 membered heteroaryl of R^{e} is independently optionally substituted with one or more C₁₋₁₂alkyl. In other embodiments, L is, independently at each occurrence, methylene, wherein the methylene of L is substituted with one or more C₁₋₁₂alkyl, ring A is, independently at each occurrence, C₆₋₂₀aryl, n is, independently at each occurrence, 1, and R^{e} is, independently at each occurrence, thiazolyl, wherein the thiazolyl of R^{e} is independently optionally substituted with one or more C₁₋₁₂alkyl.

In certain embodiments, provided herein is a compound of formula (I), or a stereoisomer or tautomer thereof, or a pharmaceutically acceptable salt of any of the foregoing, wherein the compound of formula (I) is a compound of formula (ID): 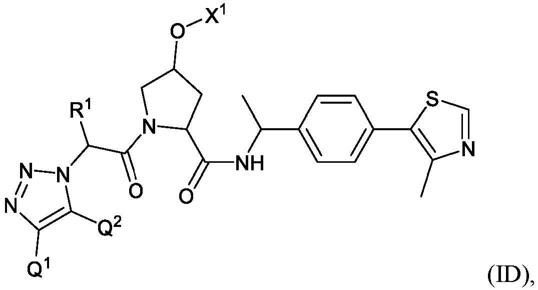 or a stereoisomer or tautomer thereof, or a pharmaceutically acceptable salt of any of the foregoing.

In other embodiments, L is, independently at each occurrence, methylene, wherein the methylene of L is substituted with one or more C₁₋₁₂alkyl, ring A is, independently at each occurrence, C₆₋₂₀aryl, n is, independently at each occurrence, 2, one of the R^{e} is, independently at each occurrence, 5-20 membered heteroaryl, wherein the 5-20 membered heteroaryl of R^{e} is independently optionally substituted with one or more C₁₋₁₂alkyl, and the other R^{e} is, independently at each occurrence, halo. In other embodiments, L is, independently at each occurrence, methylene, wherein the methylene of L is substituted with one or more C₁₋₁₂alkyl, ring A is, independently at each occurrence, C₆₋₂₀aryl, n is, independently at each occurrence, 2, one of the R^{e} is, independently at each occurrence, thiazolyl, wherein the thiazolyl of R^{e} is independently optionally substituted with one or more C₁₋₁₂alkyl, and the other R^{e} is, independently at each occurrence, halo. In some embodiments, the halo of R^{e} is, independently at each occurrence, chloro. In certain embodiments, provided herein is a compound of formula (I), or a stereoisomer or tautomer thereof, or a pharmaceutically acceptable salt of any of the foregoing, wherein the compound of formula (I) is a compound of formula (IE): 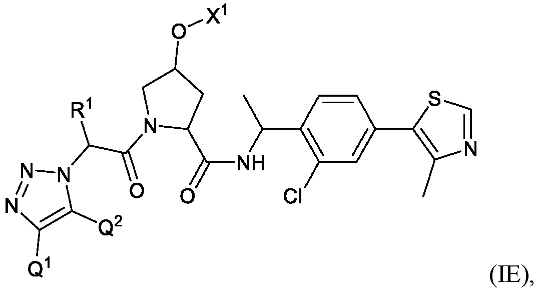 or a stereoisomer or tautomer thereof, or a pharmaceutically acceptable salt of any of the foregoing.

In certain embodiments, provided herein is a compound of formula (I), or a stereoisomer or tautomer thereof, or a pharmaceutically acceptable salt of any of the foregoing, wherein L is, independently at each occurrence, C₁₋₁₂alkylene, wherein the C₁₋₁₂alkylene of L is independently optionally substituted with one or more R^{t}, wherein R^{t} is independently C₁₋₁₂alkyl or -C(O)NH₂, wherein the C₁₋₁₂alkyl of R^{t} is further independently optionally substituted with one or more halo, ring A is, independently at each occurrence, C₆₋₂₀aryl, n is, independently at each occurrence, 1, and R^{e} is, independently at each occurrence, C₆₋₂₀aryl, wherein the C₆₋₂₀aryl of R^{e} is independently optionally substituted with one or more C₁₋₁₂alkyl or halo. In some embodiments, L is, independently at each occurrence, C₁₋₆alkylene, wherein the C₁₋₆alkylene of L is independently optionally substituted with one or more R^{t}, wherein R^{t} is, independently at each occurrence, C₁₋₁₂alkyl or -C(O)NH₂, wherein the C₁₋₁₂alkyl of R^{t} is further independently optionally substituted with one or more halo, ring A is, independently at each occurrence, C₆₋₂₀aryl, n is, independently at each occurrence, 1, and R^{e} is, independently at each occurrence, C₆₋₂₀aryl, wherein the C₆₋₂₀aryl of R^{e} is independently optionally substituted with one or more C₁₋₁₂alkyl or halo. In certain embodiments, L is, independently at each occurrence, C₁₋₆alkylene, wherein the C₁₋₆alkylene of L is independently optionally substituted with one or more -C(O)NH₂, ring A is, independently at each occurrence, C₆₋₂₀aryl, n is, independently at each occurrence, 1, and R^{e} is, independently at each occurrence, C₆₋₂₀aryl, wherein the C₆₋₂₀aryl of R^{e} is independently optionally substituted with one or more C₁₋₁₂alkyl or halo.

In certain embodiments, L is, independently at each occurrence, ethylene, wherein the ethylene of L is independently substituted with one or more -C(O)NH₂, ring A is, independently at each occurrence, phenyl, n is, independently at each occurrence, 1, and R^{e} is, independently at each occurrence, unsubstituted phenyl. In some embodiments, provided herein is a compound of formula (I), or a stereoisomer or tautomer thereof, or a pharmaceutically acceptable salt of any of the foregoing, wherein the compound of formula (I) is a compound of formula (IF): 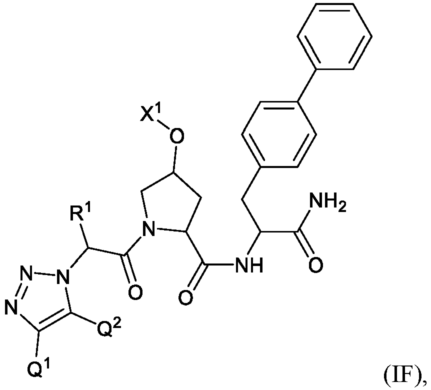 or a stereoisomer or tautomer thereof, or a pharmaceutically acceptable salt of any of the foregoing.

In some embodiments, L is, independently at each occurrence, methylene, wherein the methylene of L is independently substituted with one or more C₁₋₁₂alkyl, ring A is, independently at each occurrence, phenyl, n is, independently at each occurrence, 1, and R^{e} is, independently at each occurrence, phenyl, wherein the phenyl of R^{e} is independently substituted with one or more halo. In certain embodiments, L is, independently at each occurrence, methylene, wherein the methylene of L is independently substituted with one or more C₁₋₆alkyl, ring A is, independently at each occurrence, phenyl, n is, independently at each occurrence, 1, and R^{e} is, independently at each occurrence, phenyl, wherein the phenyl of R^{e} is independently substituted with one or more halo, wherein the halo is fluoro. In some embodiments, provided herein is a compound of formula (I), or a stereoisomer or tautomer thereof, or a pharmaceutically acceptable salt of any of the foregoing, wherein the compound of formula (I) is a compound of formula (IG): 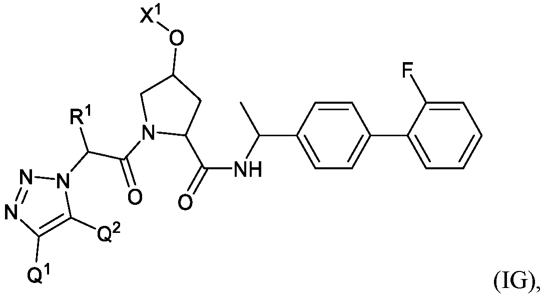 or a stereoisomer or tautomer thereof, or a pharmaceutically acceptable salt of any of the foregoing.

In some embodiments, provided herein is a compound of formula (I), or a stereoisomer or tautomer thereof, or a pharmaceutically acceptable salt of any of the foregoing, wherein L is, independently at each occurrence, absent and ring A is, independently at each occurrence, C₇₋₁₅cycloalkyl. In other embodiments, L is, independently at each occurrence, absent, ring A is, independently at each occurrence, C₇₋₁₅cycloalkyl, n is, independently at each occurrence, 1, and R^{e} is, independently at each occurrence, 5-20 membered heteroaryl, wherein the 5-20 membered heteroaryl of R^{e} is independently optionally substituted with one or more C₁₋₁₂alkyl. In some embodiments, L is,independently at each occurrence, absent, ring A is, independently at each occurrence, C₇₋₁₅cycloalkyl, n is, independently at each occurrence, 1, and R^{e} is, independently at each occurrence, thiazolyl, wherein the thiazolyl of R^{e} is independently optionally substituted with one or more C₁₋₁₂alkyl. In some embodiments, provided herein is a compound of formula (I), or a stereoisomer or tautomer thereof, or a pharmaceutically acceptable salt of any of the foregoing, wherein the compound of formula (I) is a compound of formula (IH): 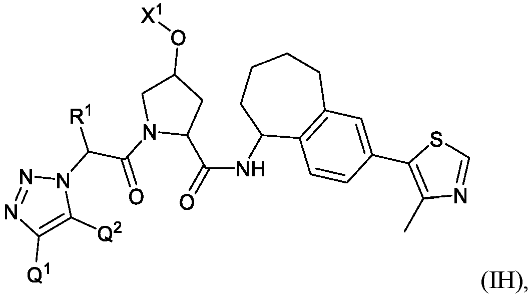 or a stereoisomer or tautomer thereof, or a pharmaceutically acceptable salt of any of the foregoing.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 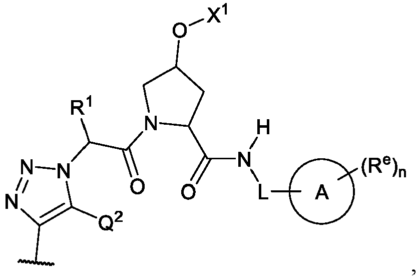 wherein the C₁₋₁₂alkyl or C₁₋₁₂alkoxy of R^{q} is independently further optionally substituted with one or more halo or -NHC(O)-C₁₋₁₂alkyl. In some embodiments, Q¹ is H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 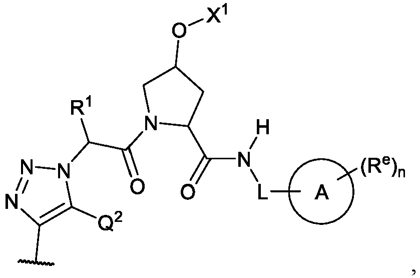 wherein the C₁₋₁₂alkyl or C₁₋₁₂alkoxy of R^{q} is independently further optionally substituted with one or more halo or -NHC(O)-C₁₋₁₂alkyl, and Q² is H. In some embodiments, Q² is, independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q² is independently optionally substituted with one or more R^{q}, wherein R^{q} is, independently at each occurrence, C₁₋₁₂alkyl, C₂₋₁₂alkenyl, C₂₋₁₂alkynyl, C₆₋₂₀aryl, C₁₋₁₂alkoxy, or 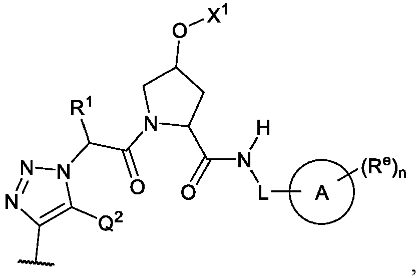 wherein the C₁₋₁₂alkyl or C₁₋₁₂alkoxy of R^{q} is independently further optionally substituted with one or more halo or -NHC(O)-C₁₋₁₂alkyl, and Q¹ is H. In other embodiments, Q¹ and Q² are, independently of each other and independently at each occurrence, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R^{q} is, independently at each occurrence, C₁₋₁₂alkyl, C₂₋₁₂alkenyl, C₂₋₁₂alkynyl, C₆₋₂₀aryl, C₁₋₁₂alkoxy, or 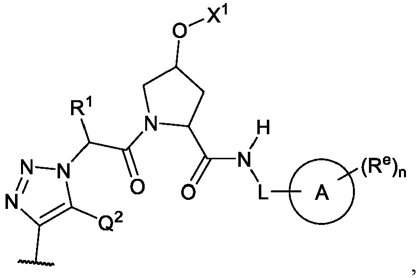 wherein the C₁₋₁₂alkyl or C₁₋₁₂alkoxy of R^{q} is independently further optionally substituted with one or more halo or -NHC(O)-C₁₋₁₂alkyl. In still other embodiments, Q¹ and Q² are, independently of each other and independently at each occurrence, H.

In some embodiments, Q¹ is C₃₋₁₅cycloalkyl, wherein the C₃₋₁₅cycloalkyl of Q¹ is optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 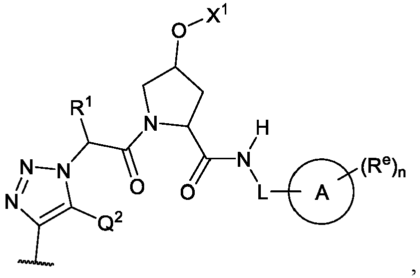 wherein the C₁₋₁₂alkyl or C₁₋₁₂alkoxy of R^{q} is independently further optionally substituted with one or more halo or -NHC(O)-C₁₋₁₂alkyl. **In** other embodiments, Q¹ is C₃₋₁₂cycloalkyl, wherein the C₃₋₁₂cycloalkyl of Q¹ is optionally substituted with one or more R^{q}. **In** some embodiments, Q¹ is C₃₋₁₀cycloalkyl, wherein the C₃₋₁₀cycloalkyl of Q¹ is optionally substituted with one or more R^{q}. **In** certain embodiments, Q¹ is C₃₋₈cycloalkyl, wherein the C₃₋₈cycloalkyl of Q¹ is optionally substituted with one or more R^{q}. **In** some embodiments, Q¹ is C₃₋₆cycloalkyl, wherein the C₃₋₆cycloalkyl of Q¹ is optionally substituted with one or more R^{q}. **In** some embodiments, Q¹ is C₃₋₅cycloalkyl, wherein the C₃₋₅cycloalkyl of Q¹ is optionally substituted with one or more R^{q}. **In** some embodiments, Q¹ is cyclopropyl, wherein the cyclopropyl of Q¹ is optionally substituted with one or more R^{q}, wherein R^{q} is C₁₋₁₂alkyl, C₂₋₁₂alkynyl, or 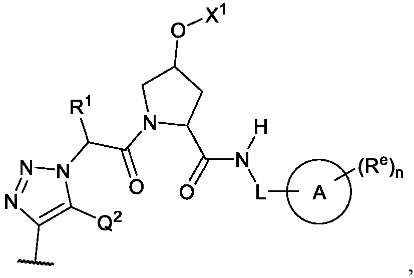 wherein the C₁₋₁₂alkyl of R^{q} is independently further optionally substituted with one or more halo or -NHC(O)-C₁₋₁₂alkyl. In some embodiments, Q¹ is cyclopropyl, wherein the cyclopropyl of Q¹ is optionally substituted with one or more R^{q}, wherein R^{q} is C₁₋₁₂alkyl, C₂₋₁₂alkynyl, or 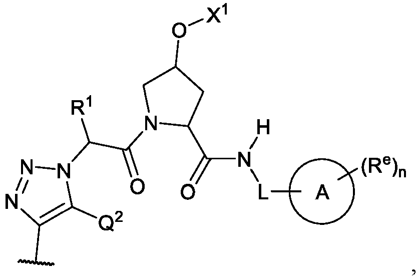 wherein the C₁₋₁₂alkyl of R^{q} is independently further optionally substituted with one or more halo or - NHC(O)-C₁₋₁₂alkyl, and Q² is H. In some embodiments, Q¹ is unsubstituted cyclopropyl. In certain embodiments, Q¹ is unsubstituted cyclopropyl, and Q² is H.

In some embodiments, Q¹ is C₁₋₁₂alkyl, wherein the C₁₋₁₂alkyl of Q¹ is optionally substituted with one or more R^{q}, wherein R^{q} is independently C₆₋₂₀aryl or C₁₋₁₂alkoxy, wherein the C₁₋₁₂alkoxy of R^{q} is independently further optionally substituted with one or more halo or -NHC(O)-C₁₋₁₂alkyl, and Q² is, independently at each occurrence, H. In some embodiments, Q¹ is methyl and Q² is, independently at each occurrence, H. In other embodiments, Q¹ is H and Q² is, independently at each occurrence, C₁₋₁₂alkyl, wherein the C₁₋₁₂alkyl of Q² is independently optionally substituted with one or more R^{q}, wherein R^{q} is, independently at each occurrence, C₆₋₂₀aryl or C₁₋₁₂alkoxy, wherein the C₁₋₁₂alkoxy of R^{q} is independently further optionally substituted with one or more halo or -NHC(O)-C₁₋₁₂alkyl. In some embodiments, Q¹ is H and Q² is, independently at each occurrence, methyl. In other embodiments, Q¹ and Q² are, independently of each other and independently at each occurrence, C₁₋₁₂alkyl, wherein the C₁₋₁₂alkyl of Q¹ or Q² is independently optionally substituted with one or more R^{q}, wherein R^{q} is, independently at each occurrence, C₆₋₂₀aryl or C₁₋₁₂alkoxy, wherein the C₁₋₁₂alkoxy of R^{q} is independently further optionally substituted with one or more halo or -NHC(O)-C₁₋₁₂alkyl. In some embodiments, Q¹ and Q² are, independently of each other and independently at each occurrence, methyl.

In some embodiments, Q¹ is -C(O)-O(R^{a}) or -C(O)-N(R^{b})(R^{c}), wherein R^{a}, R^{b}, and R^{c} are each independently H or C₁₋₁₂alkyl, and Q² is, independently at each occurrence, H. In other embodiments, Q¹ is -C(O)-O(R^{a}) or -C(O)-N(R^{b})(R^{c}), wherein R^{a}, R^{b}, and R^{c} are each independently H or C₁₋₁₂alkyl, and Q² is, independently at each occurrence, C₁₋₁₂alkyl. In some embodiments, Q¹ is -C(O)-O(R^{a}) or -C(O)-N(R^{b})(R^{c}), wherein R^{a}, R^{b}, and R^{c} are each independently H or C₁₋₁₂alkyl, and Q² is,independently at each occurrence, methyl.

In some embodiments, Q¹ is 5-20 membered heteroaryl. In some embodiments, Q¹ is 5-16 membered heteroaryl. In other embodiments, Q¹ is 5-12 membered heteroaryl. In still other embodiments, Q¹ is 5-10 membered heteroaryl. In some embodiments, Q¹ is 5-8 membered heteroaryl. In other embodiments, Q¹ is 5-6 membered heteroaryl. In some embodiments, Q¹ is 5-membered heteroaryl. In certain embodimens, Q¹ is furanyl. In other embodiments, Q¹ is thiophenyl. In some embodiments, Q¹ is 5-20 membered heteroaryl and Q² is, independently at each occurrence, H.

In some embodiments, Q¹ is cyano. In certain embodiments, Q¹ is cyano and Q² is, independently at each occurrence, H. In some embodiments, Q¹ is halo. In some embodiments, Q¹ is halo and Q² is, independently at each occurrence, H. In certain embodiments, Q¹ is fluoro and Q² is, independently at each occurrence, H.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein Q¹ and Q² are taken, together with the atoms to which they are attached, to form a C₃₋₁₅cycloalkyl, 3-15 membered heterocyclyl, C₆₋₂₀aryl, or 5-20 membered heteroaryl, wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, -C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo or OH. In certain embodiments, Q¹ and Q² are taken, together with the atoms to which they are attached, to form a C₆₋₂₀aryl, wherein the C₆₋₂₀aryl formed by Q¹ and Q² is independently optionally substituted with one or more R^{s}. In some embodiments, Q¹ and Q² are taken, together with the atoms to which they are attached, to form an unsubstituted C₆₋₂₀aryl. In certain embodiments, Q¹ and Q² are taken, together with the atoms to which they are attached, to form an unsubstituted C₆₋₁₀aryl. In certain embodiments, Q¹ and Q² are taken, together with the atoms to which they are attached, to form an unsubstituted C₆aryl.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl, wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In certain embodiments, R¹ is, independently at each occurrence, C₁₋₁₂alkyl, wherein the C₁₋₁₂alkyl of R¹ is independently optionally substituted with one or more C₆₋₂₀aryl, -S(O)₂-C₁₋₁₂alkyl, or -C(O)-C₁₋₁₂alkyl. In some embodiments, R¹ is, independently at each occurrence, unsubstituted C₁₋₁₂alkyl. In other embodiments, R¹ is, independently at each occurrence, C₁₋₆alkyl, wherein the C₁₋₆alkyl of R¹ is independently optionally substituted with one or more C₆₋₂₀aryl, -S(O)₂-C₁₋₁₂alkyl, or -C(O)-C₁₋₁₂alkyl. In other embodiments, R¹ is, independently at each occurrence, C₁₋₆alkyl, wherein the C₁₋₆alkyl of R¹ is independently optionally substituted with one or more C₆₋₂₀aryl. In some embodiments, R¹ is, independently at each occurrence, unsubstituted C₁₋₆alkyl. In some embodiments, R¹ is, independently at each occurrence, methyl, *tert*-butyl, *sec*-butyl, *iso-*propyl, or *tert*-pentyl. In certain embodiments, R¹ is, independently at each occurrence, methyl, *tert*-butyl, or iso-propyl. In some embodiments, R¹ is, independently at each occurrence, *tert-*butyl or iso-propyl. In some embodiments, R¹ is, independently at each occurrence, *tert*-butyl. In other embodiments, R¹ is, independently at each occurrence, iso-propyl.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein R¹ is, independently at each occurrence, C₃₋₁₅cycloalkyl, wherein the C₃₋₁₅cycloalkyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In some embodiments, R¹ is, independently at each occurrence, C₃₋₁₂cycloalkyl, wherein the C₃₋₁₂cycloalkyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In other embodiments, R¹ is, independently at each occurrence, C₃₋₁₀cycloalkyl, wherein the C₃₋₁₀cycloalkyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In still other embodiments, R¹ is, independently at each occurrence, C₃₋₈cycloalkyl, wherein the C₃₋₈cycloalkyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In certain embodiments, R¹ is, independently at each occurrence, C₃₋₆cycloalkyl, wherein the C₃₋₆cycloalkyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In other embodiments, R¹ is, independently at each occurrence, C₃₋₅cycloalkyl, wherein the C₃₋₅cycloalkyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In certain embodiments, R¹ is, independently at each occurrence, cyclobutyl, wherein the cyclobutyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In certain embodiments, R¹ is, independently at each occurrence, unsubstituted cyclobutyl. In some embodiments, R¹ is, independently at each occurrence, cyclohexyl, wherein the cyclohexyl of R¹ is independently optionally substituted with one or more C₁₋₁₂alkyl, C₆₋₂₀aryl, -S(O)₂-C₁₋₁₂alkyl, or -C(O)-C₁₋₁₂alkyl. In certain embodiments, R¹ is, independently at each occurrence, unsubstituted cyclohexyl. In some embodiments, R¹ is, independently at each occurrence, unsubstituted adamantyl.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein R¹ is, independently at each occurrence, 3-15 membered heterocyclyl, wherein the 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein the chiral carbon atom to which R¹ is attached is in the S stereochemical configuration. In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein the chiral carbon atom to which R¹ is attached is in the R stereochemical configuration.

In some embodiments, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein X¹ is, independently at each occurrence, H, C₁₋₁₂alkyl, or -C(O)-C₁₋₁₂alkyl. In some embodiments, X¹ is, independently at each occurrence, H. In other embodiments, X¹ is, independently at each occurrence, -C(O)-C₁₋₁₂alkyl. In other embodiments, X¹ is, independently at each occurrence, -C(O)-CH₃. In some embodiments, X¹ is, independently at each occurrence, C₁₋₁₂alkyl. In certain embodiments, the C₁₋₁₂alkyl of X¹ is unsubtituted.

In some embodiments, provided herein is a compound of formula (I), or a stereoisomer or tautomer thereof, or a pharmaceutically acceptable salt of any of the foregoing, wherein: X¹ is, independently at each occurrence, H, C₁₋₁₂alkyl, or -C(O)-C₁₋₁₂alkyl; R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl, wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl; L is, independently at each occurrence, absent or is C₁₋₁₂alkylene, wherein the C₁₋₁₂alkylene of L is independently optionally substituted with one or more R^{t}, wherein R^{t} is C₁₋₁₂alkyl or -C(O)NH₂, wherein the C₁₋₁₂alkyl of R^{t} is further optionally substituted with one or more halo; ring A is, independently at each occurrence, C₆₋₂₀aryl or C₇₋₁₅cycloalkyl; n is, independently at each occurrence, 1, 2, 3, 4, or 5; R^{e} is, independently at each occurrence, halo, C₆₋₂₀aryl, or 5-20 membered heteroaryl, provided that at least one R^{e} is C₆₋₂₀aryl or 5-20 membered heteroaryl comprising one or more annular sulfur atoms, wherein the C₆₋₂₀aryl or 5-20 membered heteroaryl of R^{e} is independently optionally substituted with one or more C₁₋₁₂alkyl or halo; and Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl, wherein the C₁₋₁₂alkyl or C₃₋₁₅cycloalkyl of Q¹ or Q² is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 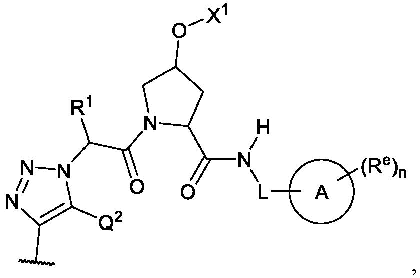 wherein the C₁₋₁₂alkyl or C₁₋₁₂alkoxy of R^{q} is independently further optionally substituted with one or more halo or - NHC(O)-C₁₋₁₂alkyl, or Q¹ and Q² are taken, together with the atoms to which they are attached, to form a C₃₋₁₅cycloalkyl, 3-15 membered heterocyclyl, C₆₋₂₀aryl, or 5-20 membered heteroaryl, wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, -C(O)NH₂, - SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH.

In some embodiments of the foregoing, the at least one R^{e} that is C₆₋₂₀aryl or 5-20 membered heteroaryl comprising one or more annular sulfur atoms is bonded to ring A at the *ortho* position of ring A. In other embodiments, the at least one R^{e} that is C₆₋₂₀aryl or 5-20 membered heteroaryl comprising one or more annular sulfur atoms is bonded to ring A at the *meta* position of ring A. In other embodiments, the at least one R^{e} that is C₆₋₂₀aryl or 5-20 membered heteroaryl comprising one or more annular sulfur atoms is bonded to ring A at the *para* position of ring A.

In some embodiments of the foregoing, provided herein is a compound of formula (I), or a stereoisomer or tautomer thereof, or a pharmaceutically acceptable salt of any of the foregoing, wherein, when L is, independently at each occurrence, unsubstituted methylene, ring A is, independently at each occurrence, C₆₋₂₀aryl; n is, independently at each occurrence, 1; R^{e} is, independently at each occurrence, 5-20 membered heteroaryl, wherein the 5-20 membered heteroaryl of R^{e} comprises one or more annular sulfur atom and is independently optionally substituted with one or more C₁₋₁₂alkyl; and Q¹ is unsubstituted cyclopropyl, then R¹ is C₁₋₃alkyl, C₂₋₁₂alkenyl, C₂₋₁₂alkynyl, C₃₋₁₅cycloalkyl, or 3-15 membered heterocyclyl, wherein the C₁₋₃alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl.

In other embodiments of the foregoing, provided herein is a compound of formula (I), or a stereoisomer or tautomer thereof, or a pharmaceutically acceptable salt of any of the foregoing, wherein, when L is, independently at each occurrence, unsubstituted methylene, ring A is, independently at each occurrence, C₆₋₂₀aryl; n is, independently at each occurrence, 1; R^{e} is, independently at each occurrence, thiazolyl, wherein the thiazolyl of R^{e} is independently optionally substituted with one or more C₁₋₁₂alkyl; and Q¹ is unsubstituted cyclopropyl, then R¹ is C₁₋₃alkyl, C₂₋₁₂alkenyl, C₂₋₁₂alkynyl, C₃₋₁₅cycloalkyl, or 3-15 membered heterocyclyl, wherein the C₁₋₃alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl.

In some embodiments of the foregoing, provided herein is a compound of formula (I), or a stereoisomer or tautomer thereof, or a pharmaceutically acceptable salt of any of the foregoing, wherein, when L is, independently at each occurrence, unsubstituted methylene, ring A is, independently at each occurrence, C₆₋₂₀aryl; n is, independently at each occurrence, 1; R^{e} is, independently at each occurrence, thiazolyl, wherein the thiazolyl of R^{e} is independently optionally substituted with one or more methyl; and Q¹ is unsubstituted cyclopropyl, then R¹ is C₁₋₃alkyl, C₂₋₁₂alkenyl, C₂₋₁₂alkynyl, C₃₋₁₅cycloalkyl, or 3-15 membered heterocyclyl, wherein the C₁₋₃alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl.

In some aspects of the foregoing, the compound of formula (I), such as a compound of (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, includes (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)benzyl)pyrrolidine-2-carboxamide, or a pharmaceutically acceptable salt thereof. In some aspects of the foregoing, the compound of formula (I), such as a compound of (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, does not include (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)benzyl)pyrrolidine-2-carboxamide, or pharmaceutically acceptable salt thereof.

It is to be understood that any variation or embodiment of X¹, R¹, Q¹, Q², ring A, n, L, R^{a}, R^{b}, R^{c}, R^{e}, R^{q}, R^{s}, and R^{t} provided herein can be combined with every other variation or embodiment of X¹, R¹, Q¹, Q², ring A, n, L, R^{a}, R^{b}, R^{c}, R^{e}, R^{q}, R^{s}, and R^{t}, the same as if each and every combination had been individually and specifically described.

In one embodiment, provided herein is a compound of formula (I), such as a compound of formula (IA), (IA-1), (IA-2), (IA-3), (IA-4), (IB), (IB-1), (IB-2), (IB-3), (IB-4), (IC), (ID), (IE), (IF), (IG), or (IH), or a stereoisomer or tautomer thereof, or a pharmaceutically acceptable salt of any of the foregoing, wherein the compound is selected from the compounds in Table 1.

**Table 1**

| # | Compound Structure | Compound Name |
|---|---|---|
| 1 | 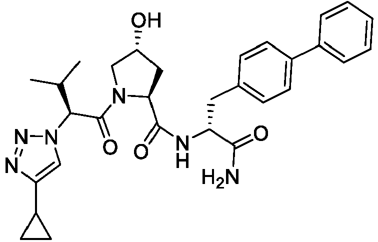 | (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3-methylbutanoyl)-4-hydroxypyrrolidine-2-carboxamide |
| 2 | 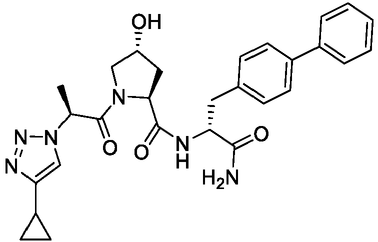 | (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)propanoyl)-4-hydroxypyrrolidine-2-carboxamide |
| 3 | 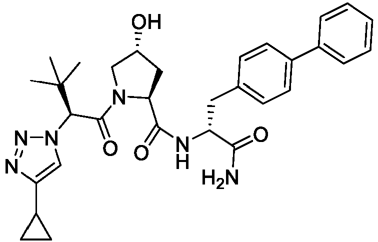 | (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3,3-dimethylbutanoyl)-4-hydroxypyrrolidine-2-carboxamide |
| 4 | 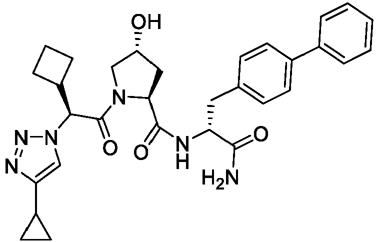 | (2S,4R)-N-((R)-3-([1,1'-biphenyl]-4-yl)-1-amino-1-oxopropan-2-yl)-1-((S)-2-cyclobutyl-2-(4-cyclopropyl-1H-1,2,3-triazol-1-yl)acetyl)-4-hydroxypyrrolidine-2-carboxamide |
| 5 | 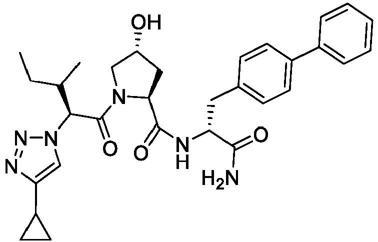 | (2S,4R)-N-((R)-3-([1,1'-biphenyl]-4-yl)-1-amino-1-oxopropan-2-yl)-1-((2S,3S)-2-(4-cyclopropyl-1H-1,2,3-triazol-1-yl)-3-methylpentanoyl)-4-hydroxypyrrolidine-2-carboxamide |
| 6 | 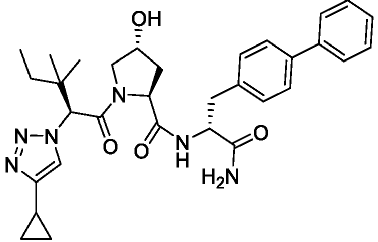 | (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3,3-dimethylpentanoyl)-4-hydroxypyrrolidine-2-carboxamide |
| 7 | 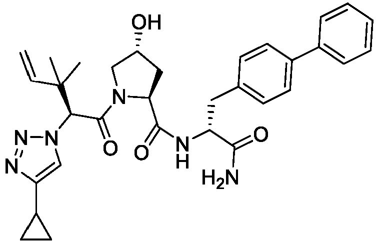 | (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3,3-dimethylpent-4-enoyl)-4-hydroxypyrrolidine-2-carboxamide |
| 8 | 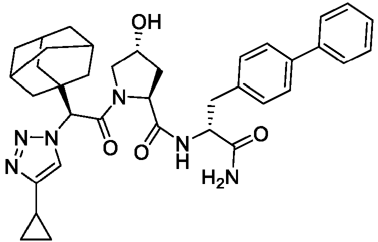 | (2S,4R)-N-((R)-3-([1,1'-biphenyl]-4-yl)-1-amino-1-oxopropan-2-yl)-1-((2S)-2-(adamantan-1-yl)-2-(4-cyclopropyl-1H-1,2,3-triazol-1-yl)acetyl)-4-hydroxypyrrolidine-2-carboxamide |
| 9 | 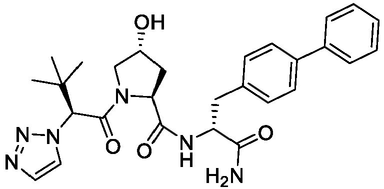 | (2S,4R)-N-((R)-3-([1,1'-biphenyl]-4-yl)-1-amino-1-oxopropan-2-yl)-1-((S)-3,3-dimethyl-2-(1H-1,2,3-triazol-1-yl)butanoyl)-4-hydroxypyrrolidine-2-carboxamide |
| 10 | 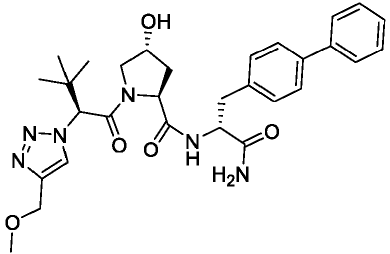 | (2S,4R)-N-((R)-3-([1,1'-biphenyl]-4-yl)-1-amino-1-oxopropan-2-yl)-4-hydroxy-1-((S)-2-(4-(methoxymethyl)-1H-1,2,3-triazol-1-yl)-3,3-dimethylbutanoyl)pyrrolidine-2-carboxamide |
| 11 | 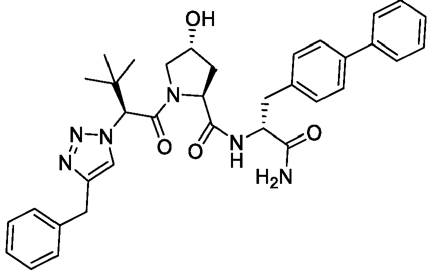 | (2S,4R)-N-((R)-3-([1,1'-biphenyl]-4-yl)-1-amino-1-oxopropan-2-yl)-1-((S)-2-(4-benzyl-1H-1,2,3-triazol-1-yl)-3,3-dimethylbutanoyl)-4-hydroxypyrrolidine-2-carboxamide |
| 12 | 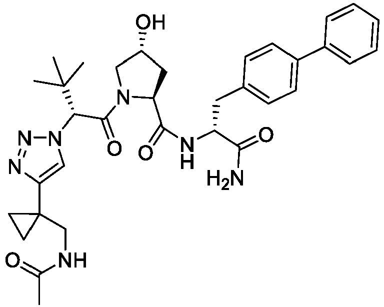 | (2S,4R)-N-((R)-3-([1,1'-biphenyl]-4-yl)-1-amino-1-oxopropan-2-yl)-1-((R)-2-(4-(1-(acetamidomethyl)cyclopropyl)-1H-1,2,3-triazol-1-yl)-3,3-dimethylbutanoyl)-4-hydroxypyrrolidine-2-carboxamide |
| 13 | 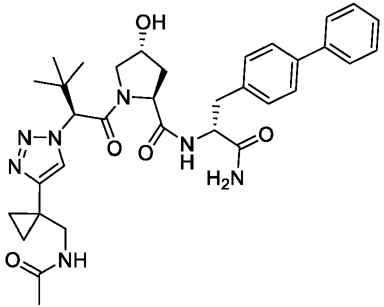 | (2S,4R)-N-((R)-3-([1,1'-biphenyl]-4-yl)-1-amino-1-oxopropan-2-yl)-1-((S)-2-(4-(1-(acetamidomethyl)cyclopropyl)-1H-1,2,3-triazol-1-yl)-3,3-dimethylbutanoyl)-4-hydroxypyrrolidine-2-carboxamide |
| 14 | 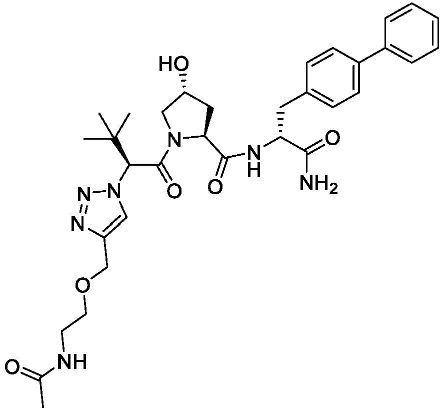 | (2S,4R)-N-((R)-3-([1,1'-biphenyl]-4-yl)-1-amino-1-oxopropan-2-yl)-1-((S)-2-(4-((2-acetamidoethoxy)methyl)-1H-1,2,3-triazol-1-yl)-3,3-dimethylbutanoyl)-4-hydroxypyrrolidine-2-carboxamide |
| 15 | 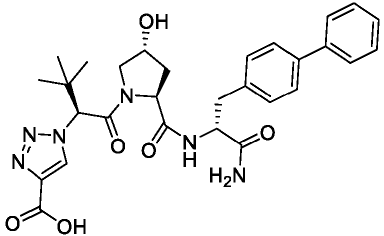 | 1-((S)-1-((2S,4R)-2-((R)-3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-1H-1,2,3-triazole-4-carboxylic acid |
| 16 | 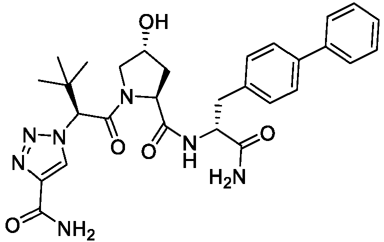 | 1-((S)-1-((2S,4R)-2-((R)-3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-1H-1,2,3-triazole-4-carboxamide |
| 17 | 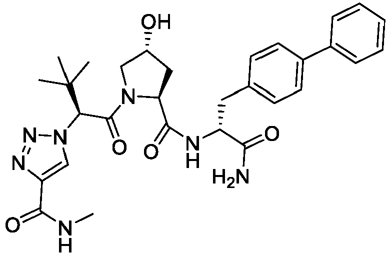 | 1-((S)-1-((2S,4R)-2-((R)-3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-N-methyl-1H-1,2,3-triazole-4-carboxamide |
| 18 | 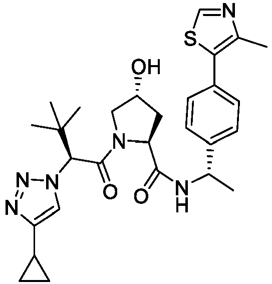 | (2S,4R)-1-((S)-2-(4-cyclopropyl-1H-1,2,3-triazol-1-yl)-3,3-dimethylbutanoyl)-4-hydroxy-N-((S)-1-(4-(4-methylthiazol-5-yl)phenyl)ethyl)pyrrolidine-2-carboxamide |
| 19 | 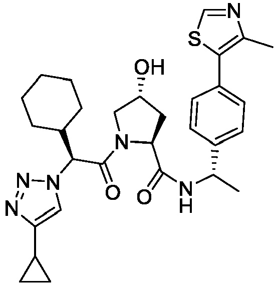 | (25,4R)-1-((S)-2-cyclohexyl-2-(4-cyclopropyl-1H-1,2,3-triazol-1-yl)acetyl)-4-hydroxy-N-((S)-1-(4-(4-methylthiazol-5-yl)phenyl)ethyl)pyrrolidine-2-carboxamide |
| 20 | 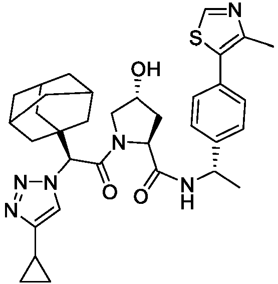 | (2S,4R)-1-((2S)-2-(adamantan-1-yl)-2-(4-cyclopropyl-1H-1,2,3-triazol-1-yl)acetyl)-4-hydroxy-N-((S)-1-(4-(4-methylthiazol-5-yl)phenyl)ethyl)pyrrolidine-2-carboxamide |
| 21 | 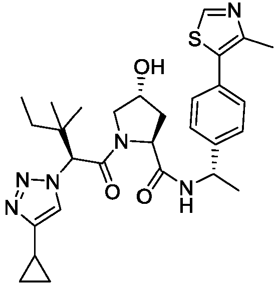 | (2S,4R)-1-((S)-2-(4-cyclopropyl-1H-1,2,3-triazol-1-yl)-3,3-dimethylpentanoyl)-4-hydroxy-N-((S)-1-(4-(4-methylthiazol-5-yl)phenyl)ethyl)pyrrolidine-2-carboxamide |
| 22 | 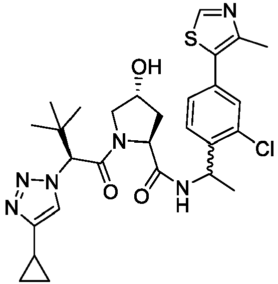 | (2S,4R)-N-(1-(2-chloro-4-(4-methylthiazol-5-yl)phenyl)ethyl)-1-((S)-2-(4-cyclopropyl-1H-1,2,3-triazol-1-yl)-3,3-dimethylbutanoyl)-4-hydroxypyrrolidine-2-carboxamide |
| 23 | 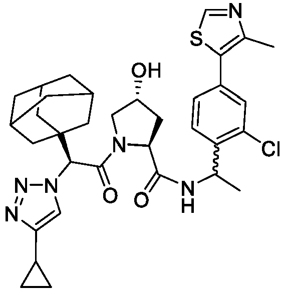 | (2S,4R)-1-((2S)-2-(adamantan-1-yl)-2-(4-cyclopropyl-1H-1,2,3-triazol-1-yl)acetyl)-N-(1-(2-chloro-4-(4-methylthiazol-5-yl)phenyl)ethyl)-4-hydroxypyrrolidine-2-carboxamide |
| 24 | 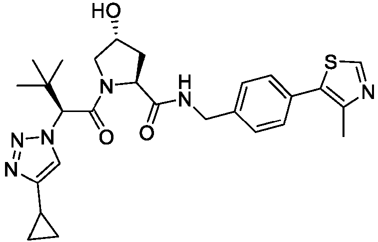 | (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)benzyl)pyrrolidine-2-carboxamide |
| 25 | 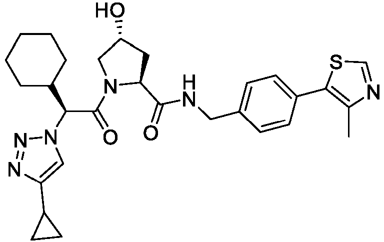 | (2S,4R)-1-((S)-2-cyclohexyl-2-(4-cyclopropyl-1H-1,2,3-triazol-1-yl)acetyl)-4-hydroxy-N-(4-(4-methylthiazol-5-yl)benzyl)pyrrolidine-2-carboxamide |
| 26 | 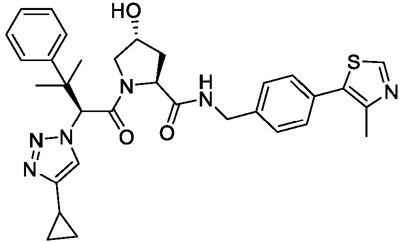 | (2S,4R)-1-((S)-2-(4-cyclopropyl-1H-1,2,3-triazol-1-yl)-3-methyl-3-phenylbutanoyl)-4-hydroxy-N-(4-(4-methylthiazol-5-yl)benzyl)pyrrolidine-2-carboxamide |
| 27 | 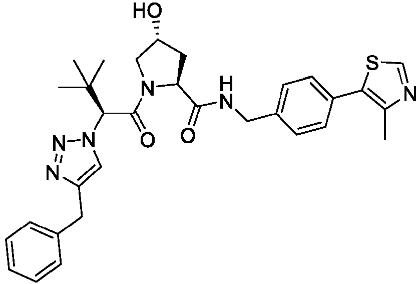 | (2S,4R)-1-((S)-2-(4-benzyl-1H-1,2,3-triazol-1-yl)-3,3-dimethylbutanoyl)-4-hydroxy-N-(4-(4-methylthiazol-5-yl)benzyl)pyrrolidine-2-carboxamide |
| 28 | 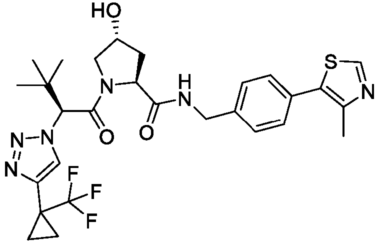 | (2S,4R)-1-((S)-3,3-dimethyl-2-(4-(1-(trifluoromethyl)cyclopropyl)-1H-1,2,3-triazol-1-yl)butanoyl)-4-hydroxy-N-(4-(4-methylthiazol-5-yl)benzyl)pyrrolidine-2-carboxamide |
| 29 | 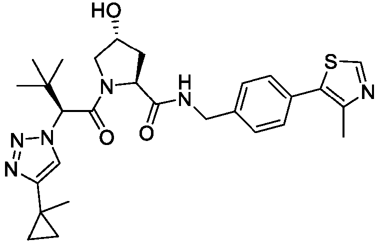 | (25,4R)-1-((S)-3,3-dimethyl-2-(4-(1-methylcyclopropyl)-1H-1,2,3-triazol-1-yl)butanoyl)-4-hydroxy-N-(4-(4-methylthiazol-5-yl)benzyl)pyrrolidine-2-carboxamide |
| 30 | 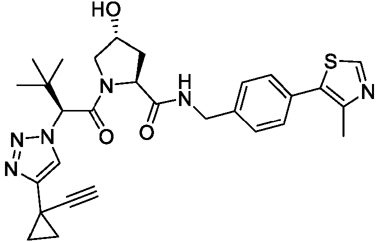 | (2S,4R)-1-((S)-2-(4-(1-ethynylcyclopropyl)-1H-1,2,3-triazol-1-yl)-3,3-dimethylbutanoyl)-4-hydroxy-N-(4-(4-methylthiazol-5-yl)benzyl)pyrrolidine-2-carboxamide |
| 31 | 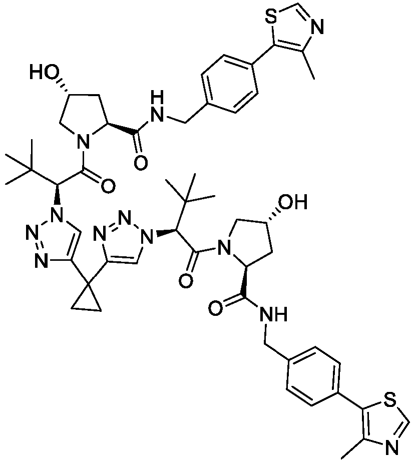 | (2S,2'S,4R,4'R)-1, 1'-((2S,2'S)-2,2'-(cyclopropane-1,1-diylbis(1H-1,2,3-triazole-4,1-diyl))bis(3,3-dimethylbutanoyl))bis(4-hydroxy-N-(4-(4-methylthiazol-5-yl)benzyl)pyrrolidine-2-carboxamide) |
| 32 | 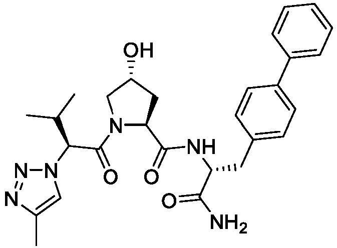 | (2S,4R)-N-((R)-3-([1,1'-biphenyl]-4-yl)-1-amino-1-oxopropan-2-yl)-4-hydroxy-1-((S)-3-methyl-2-(4-methyl-1H-1,2,3-triazol-1-yl)butanoyl)pyrrolidine-2-carboxamide |
| 33 | 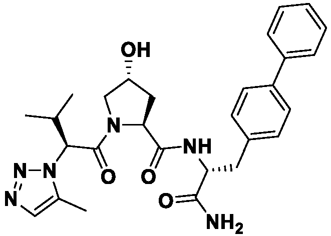 | (2S,4R)-N-((R)-3-([1,1'-biphenyl]-4-yl)-1-amino-1-oxopropan-2-yl)-4-hydroxy-1-((S)-3-methyl-2-(5-methyl-1H-1,2,3-triazol-1-yl)butanoyl)pyrrolidine-2-carboxamide |
| 34 | 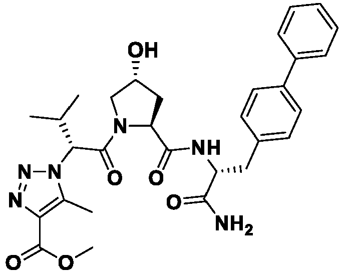 | methyl 1-((R)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate |
| 35 | 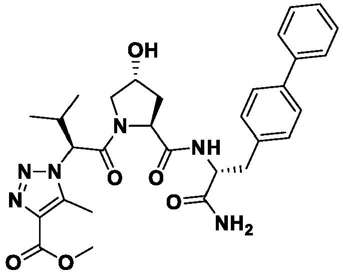 | methyl 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate |
| 36 | 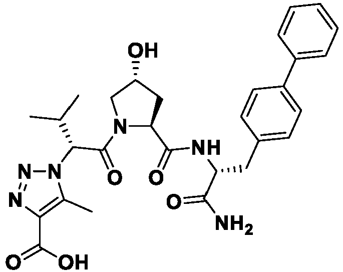 | 1-((R)-1-((2S,4R)-2-((R)-3([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid |
| 37 | 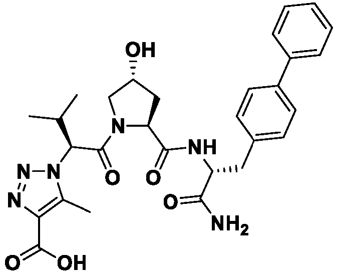 | 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid |
| 38 | 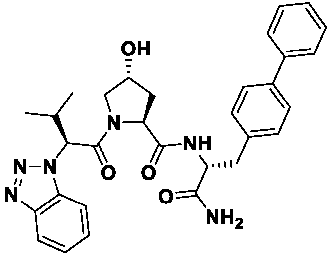 | (2S,4R)-1-((S)-2-(1H-benzo[d][1,2,3]triazol-1-yl)-3-methylbutanoyl)-N-((R)-3-([1,1'-biphenyl]-4-yl)-1-amino-1-oxopropan-2-yl)-4-hydroxypyrrolidine-2-carboxamide |
| 39 | 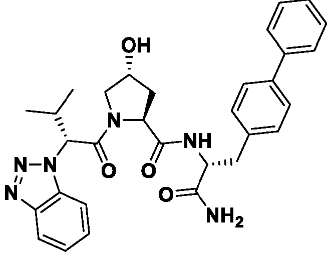 | (2S,4R)-1-((R)-2-(1H-benzo[d][1,2,3]triazol-1-yl)-3-methylbutanoyl)-N-((R)-3-([1,1'-biphenyl]-4-yl)-1-amino-1-oxopropan-2-yl)-4-hydroxypyrrolidine-2-carboxamide |
| 40 | 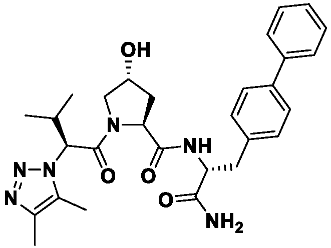 | (2S,4R)-N-((R)-3-([1,1'-biphenyl]-4-yl)-1-amino-1-oxopropan-2-yl)-1-((S)-2-(4, 5-dimethyl-1H-1,2,3 -triazol-1-yl)-3-methylbutanoyl)-4-hydroxypyrrolidine-2-carboxamide |
| 41 | 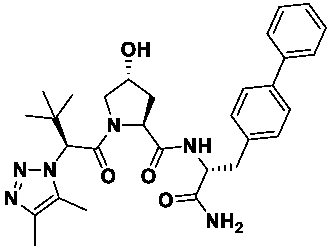 | (2S,4R)-N-((R)-3-([1,1'-biphenyl]-4-yl)-1-amino-1-oxopropan-2-yl)-1-((S)-2-(4, 5-dimethyl-1H-1,2,3 -triazol-1-yl)-3,3-dimethylbutanoyl)-4-hydroxypyrrolidine-2-carboxamide |
| 42 | 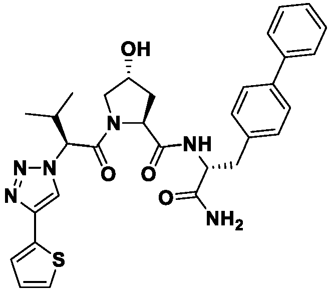 | (2S,4R)-N-((R)-3-([1,1'-biphenyl]-4-yl)-1-amino-1-oxopropan-2-yl)-4-hydroxy-1-((S)-3-methyl-2-(4-(thiophen-2-yl)-1H-1,2,3-triazol-1-yl)butanoyl)pyrrolidine-2-carboxamide |
| 43 | 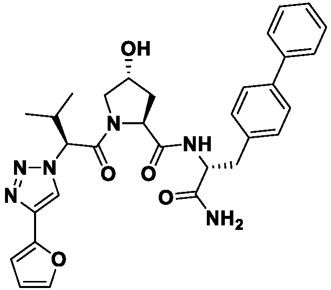 | (2S,4R)-N-((R)-3-([1,1'-biphenyl]-4-yl)-1-amino-1-oxopropan-2-yl)-1-((S)-2-(4-(furan-2-yl)-1H-1,2,3-triazol-1-yl)-3-methylbutanoyl)-4-hydroxypyrrolidine-2-carboxamide |
| 44 | 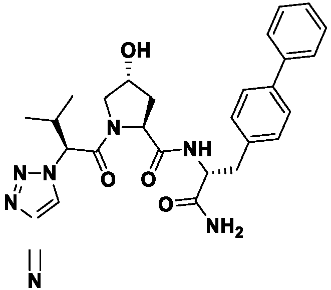 | (2S,4R)-N-((R)-3-([1,1'-biphenyl]-4-yl)-1-amino-1-oxopropan-2-yl)-1-((S)-2-(4-cyano-1H-1,2,3-triazol-1-yl)-3-methylbutanoyl)-4-hydroxypyrrolidine-2-carboxamide |
| 45 | 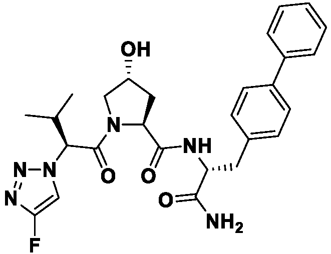 | (2S,4R)-N-((R)-3-([1,1'-biphenyl]-4-yl)-1-amino-1-oxopropan-2-yl)-1-((S)-2-(4-fluoro-1H-1,2,3-triazol-1-yl)-3-methylbutanoyl)-4-hydroxypyrrolidine-2-carboxamide |
| 46 | 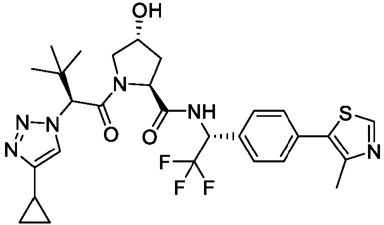 | (2S,4R)-1-((S)-2-(4-cyclopropyl-1H-1,2,3-triazol-1-yl)-3,3-dimethylbutanoyl)-4-hydroxy-N-((R)-2,2,2-trifluoro-1-(4-(4-methylthiazol-5-yl)phenyl)ethyl)pyrrolidine-2-carboxamide |
| 47 | 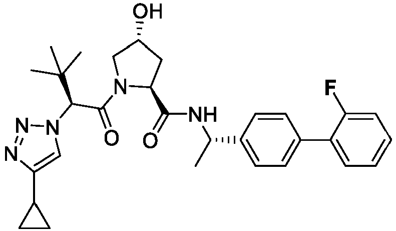 | (2S,4R)-1-((S)-2-(4-cyclopropyl-1H-1,2,3-triazol-1-yl)-3,3-dimethylbutanoyl)-N-((S)-1-(2'-fluoro-[1,1'-biphenyl]-4-yl)ethyl)-4-hydroxypyrrolidine-2-carboxamide |
| 48 | 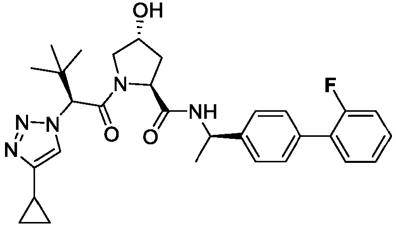 | (2S,4R)-1-((S)-2-(4-cyclopropyl-1H-1,2,3-triazol-1-yl)-3,3-dimethylbutanoyl)-N-((R)-1-(2'-fluoro-[1,1'-biphenyl]-4-yl)ethyl)-4-hydroxypyrrolidine-2-carboxamide |
| 49 | 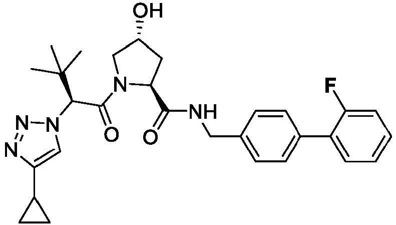 | (2S,4R)-1-((S)-2-(4-cyclopropyl-1H-1,2,3-triazol-1-yl)-3,3-dimethylbutanoyl)-N-((2'-fluoro-[1,1'-biphenyl]-4-yl)methyl)-4-hydroxypyrrolidine-2-carboxamide |
| 50 | 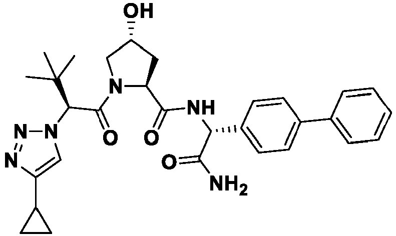 | (2S,4R)-N-((R)-1-([1,1'-biphenyl]-4-yl)-2-amino-2-oxoethyl)-1-((S)-2-(4-cyclopropyl-1H-1,2,3-triazol-1-yl)-3,3-dimethylbutanoyl)-4-hydroxypyrrolidine-2-carboxamide |
| 51 | 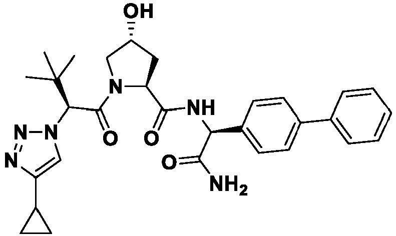 | (2S,4R)-N-((S)-1-([1,1'-biphenyl]-4-yl)-2-amino-2-oxoethyl)-1-((S)-2-(4-cyclopropyl-1H-1,2,3-triazol-1-yl)-3,3-dimethylbutanoyl)-4-hydroxypyrrolidine-2-carboxamide |
| 52 | 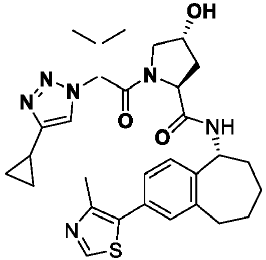 | (2S,4R)-1-((S)-2-(4-cyclopropyl-1H-1,2,3-triazol-1-yl)-3,3-dimethylbutanoyl)-4-hydroxy-N-((R)-2-(4-methylthiazol-5-yl)-6,7,8,9-tetrahydro-5H-benzo[7]annulen-5-yl)pyrrolidine-2-carboxamide |
| 53 | 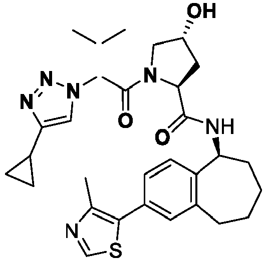 | (2S,4R)-1-((S)-2-(4-cyclopropyl-1H-1,2,3-triazol-1-yl)-3,3-dimethylbutanoyl)-4-hydroxy-N-((S)-2-(4-methylthiazol-5-yl)-6,7,8,9-tetrahydro-5H-benzo[7]annulen-5-yl)pyrrolidine-2-carboxamide |
| 54 | 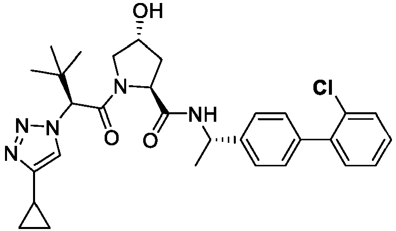 | (2S,4R)-N-((S)-1-(2'-chloro-[1,1'-biphenyl]-4-yl)ethyl)-1-((S)-2-(4-cyclopropyl-1H-1,2,3-triazol-1-yl)-3,3-dimethylbutanoyl)-4-hydroxypyrrolidine-2-carboxamide |

In one embodiment, provided herein is a compound of formula (I), or a stereoisomer or tautomer thereof, or a pharmaceutically acceptable salt of any of the foregoing, wherein the compound is selected from the group consisting of 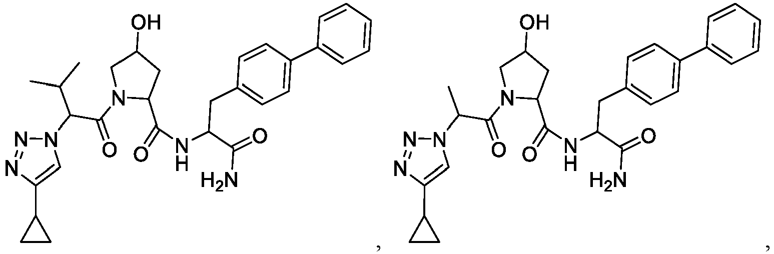 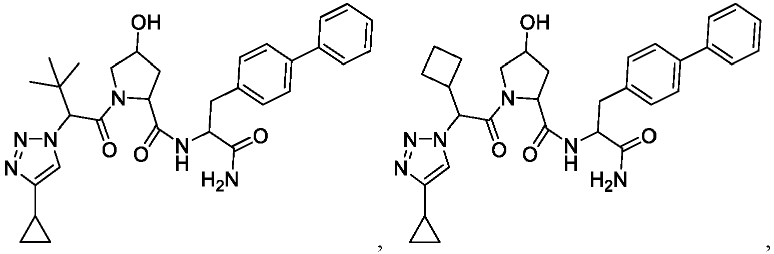 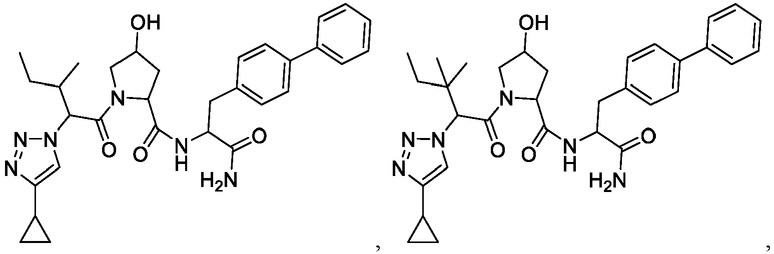 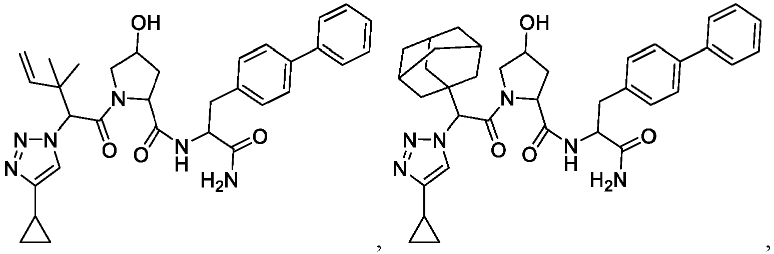 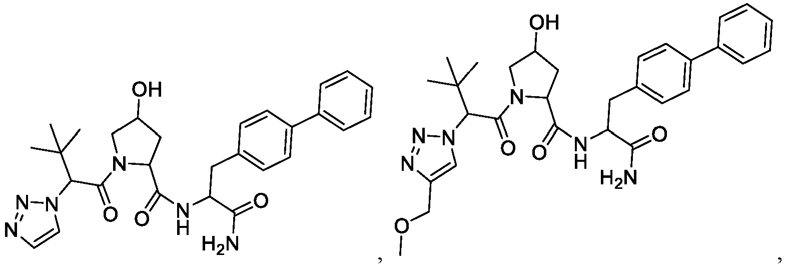 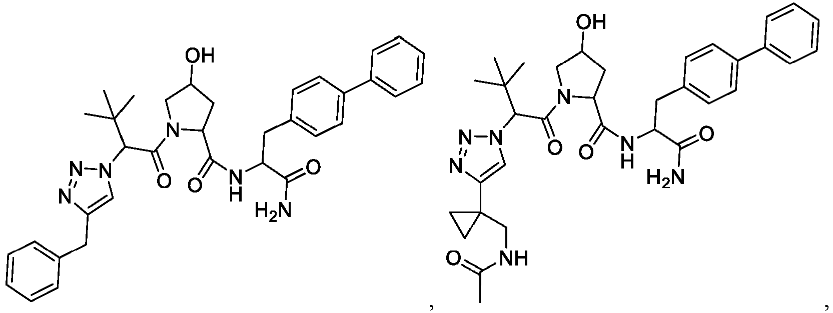 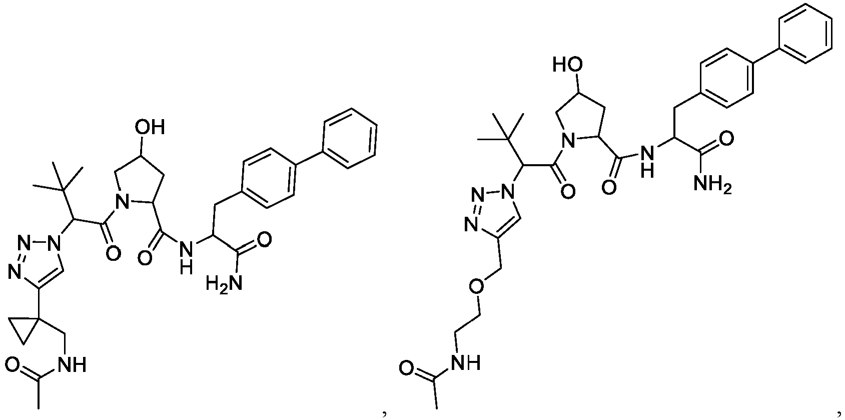 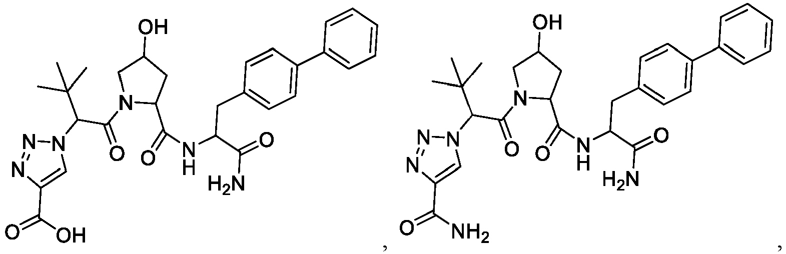 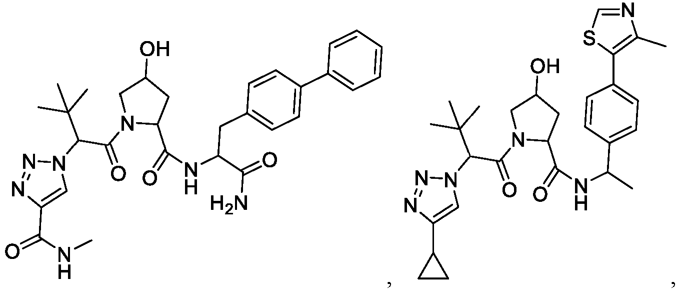 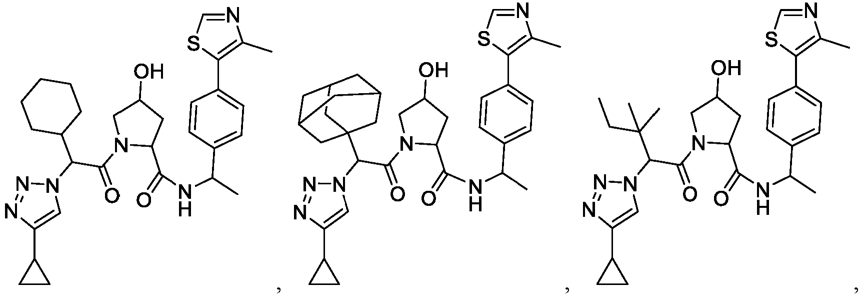 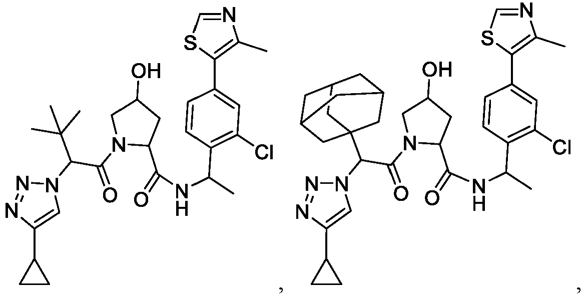 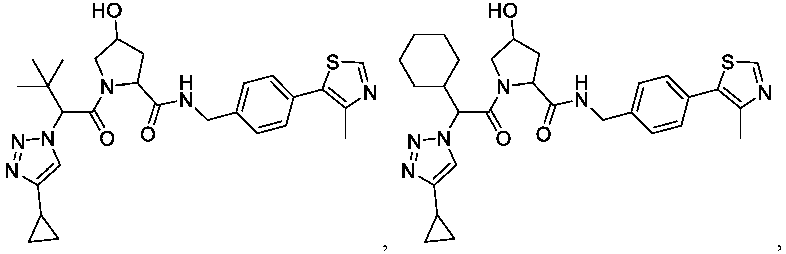 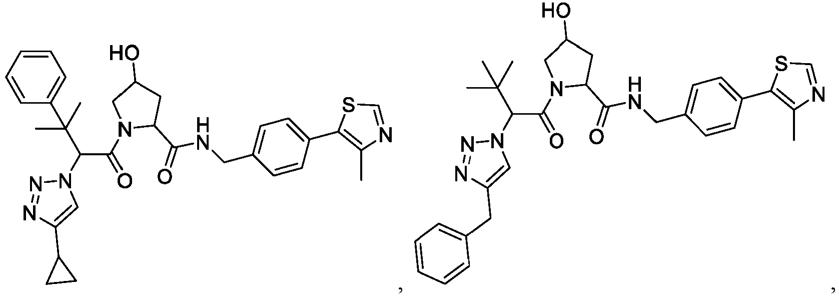 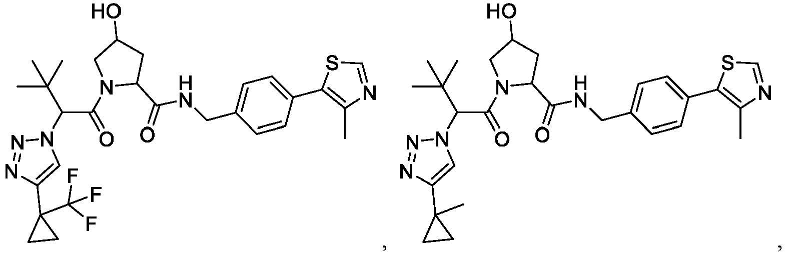 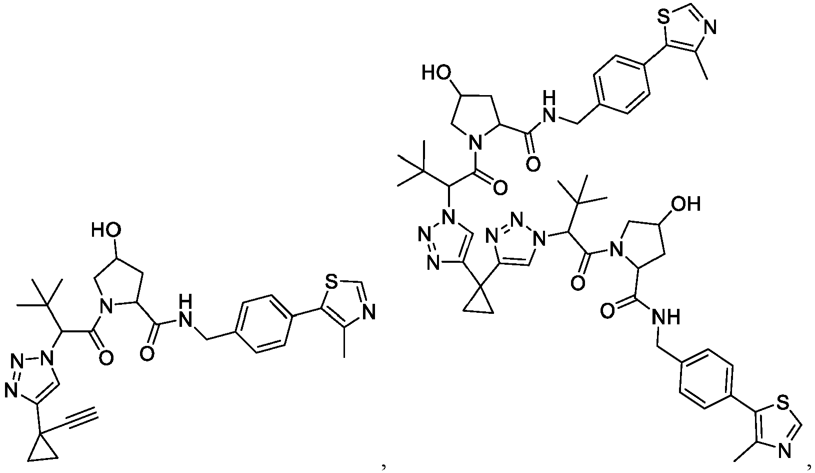 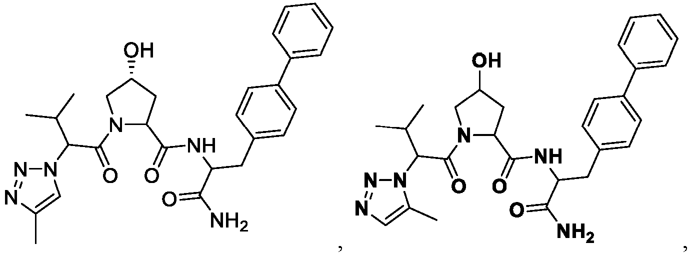 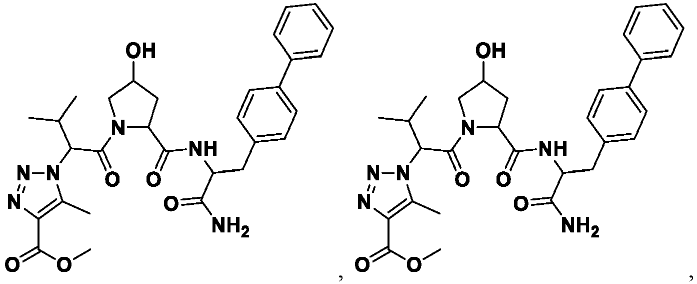 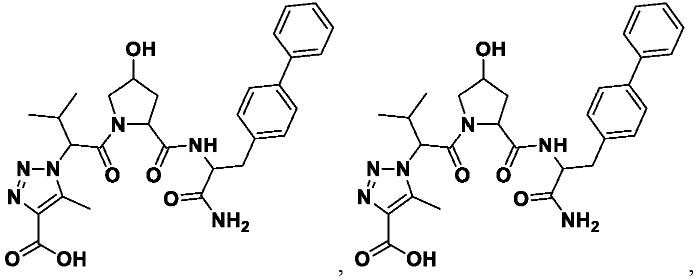 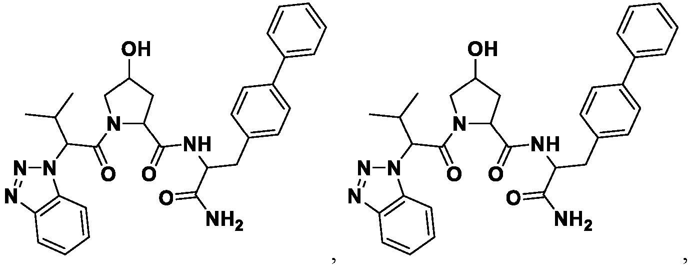 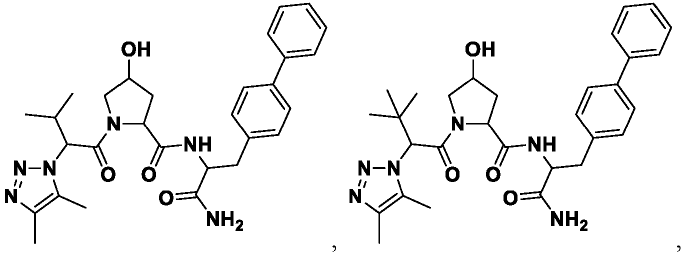 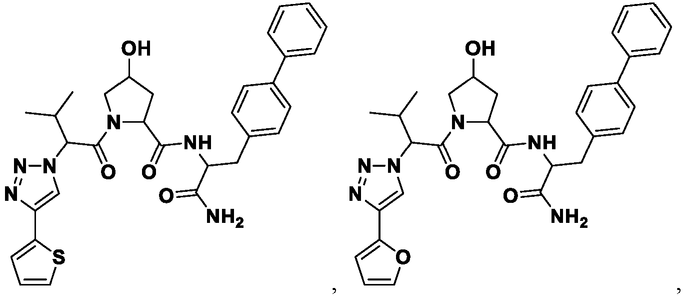 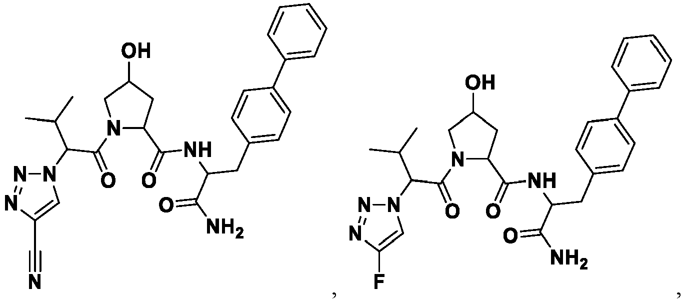 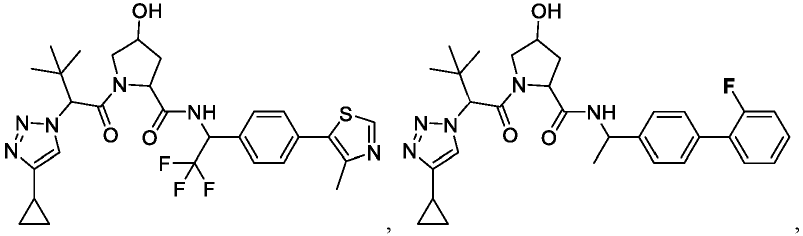 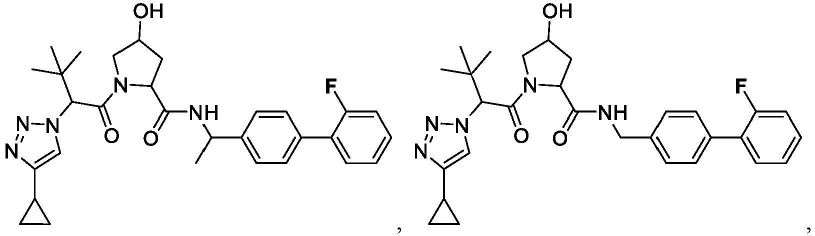 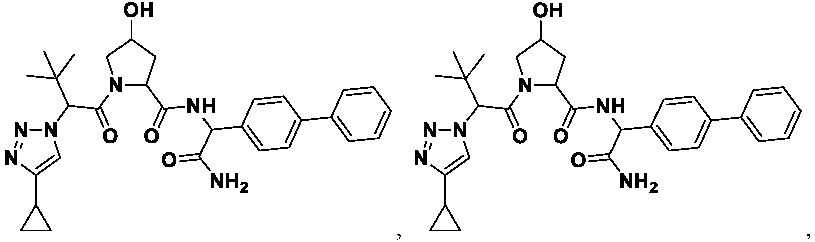 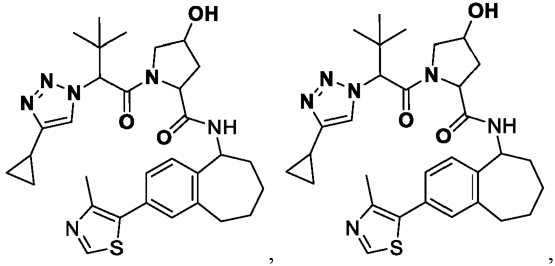 and 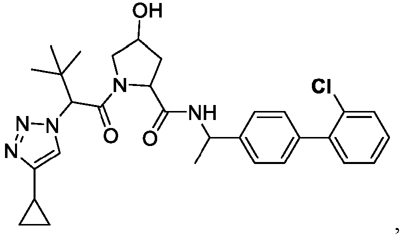 or a stereoisomer or tautomer thereof, or a pharmaceutically acceptable salt of any of the foregoing.

In one embodiment, provided herein is a compound of formula (I), or a stereoisomer or tautoer thereof, or a pharmaceutically acceptable salt of any of the foregoing, wherein the compound is selected from the group consisting of:
N-(3-([1,1'-biphenyl]-4-yl)-1-amino-1-oxopropan-2-yl)-1-(2-(4-cyclopropyl-1H-1,2,3-triazol-1-yl)-3-methylbutanoyl)-4-hydroxypyrrolidine-2-carboxamide;
N-(3-([1,1'-biphenyl]-4-yl)-1-amino-1-oxopropan-2-yl)-1-(2-(4-cyclopropyl-1H-1,2,3-triazol-1-yl)propanoyl)-4-hydroxypyrrolidine-2-carboxamide;
N-(3-([1,1'-biphenyl]-4-yl)-1-amino-1-oxopropan-2-yl)-1-(2-(4-cyclopropyl-1H-1,2,3-triazol-1-yl)-3,3-dimethylbutanoyl)-4-hydroxypyrrolidine-2-carboxamide;
N-(3-([1,1'-biphenyl]-4-yl)-1-amino-1-oxopropan-2-yl)-1-(2-cyclobutyl-2-(4-cyclopropyl-1H-1,2,3-triazol-1-yl)acetyl)-4-hydroxypyrrolidine-2-carboxamide;
N-(3-([1,1'-biphenyl]-4-yl)-1-amino-1-oxopropan-2-yl)-1-(2-(4-cyclopropyl-1H-1,2,3-triazol-1-yl)-3-methylpentanoyl)-4-hydroxypyrrolidine-2-carboxamide;
N-(3-([1,1'-biphenyl]-4-yl)-1-amino-1-oxopropan-2-yl)-1-(2-(4-cyclopropyl-1H-1,2,3-triazol-1-yl)-3,3-dimethylpentanoyl)-4-hydroxypyrrolidine-2-carboxamide;
N-(3-([1,1'-biphenyl]-4-yl)-1-amino-1-oxopropan-2-yl)-1-(2-(4-cyclopropyl-1H-1,2,3-triazol-1-yl)-3,3-dimethylpent-4-enoyl)-4-hydroxypyrrolidine-2-carboxamide;
N-(3-([1,1'-biphenyl]-4-yl)-1-amino-1-oxopropan-2-yl)-1-(2-(adamantan-1-yl)-2-(4-cyclopropyl-1H-1,2,3-triazol-1-yl)acetyl)-4-hydroxypyrrolidine-2-carboxamide;
N-(3-([1,1'-biphenyl]-4-yl)-1-amino-1-oxopropan-2-yl)-1-(3,3-dimethyl-2-(1H-1,2,3-triazol-1-yl)butanoyl)-4-hydroxypyrrolidine-2-carboxamide;
N-(3-([1,1'-biphenyl]-4-yl)-1-amino-1-oxopropan-2-yl)-4-hydroxy-1-(2-(4-(methoxymethyl)-1H-1,2,3-triazol-1-yl)-3,3-dimethylbutanoyl)pyrrolidine-2-carboxamide;
N-(3-([1,1'-biphenyl]-4-yl)-1-amino-1-oxopropan-2-yl)-1-(2-(4-benzyl-1H-1,2,3-triazol-1-yl)-3,3-dimethylbutanoyl)-4-hydroxypyrrolidine-2-carboxamide;
N-(3-([1,1'-biphenyl]-4-yl)-1-amino-1-oxopropan-2-yl)-1-(2-(4-(1-(acetamidomethyl)cyclopropyl)-1H-1,2,3-triazol-1-yl)-3,3-dimethylbutanoyl)-4-hydroxypyrrolidine-2-carboxamide;
N-(3-([1,1'-biphenyl]-4-yl)-1-amino-1-oxopropan-2-yl)-1-(2-(4-((2-acetamidoethoxy)methyl)-1H-1,2,3-triazol-1-yl)-3,3-dimethylbutanoyl)-4-hydroxypyrrolidine-2-carboxamide;
1-(1-(2-((3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-1H-1,2,3-triazole-4-carboxylic acid;
1-(1-(2-((3-([1,1'-biphenyl]-4-yl)-1-amino-1-oxopropan-2-yl)carbamoyll)-4-hydroxypyrrolidin-1-yl)-3,3-dimethyl-1-oxobutan-2-yl)-1H-1,2,3-triazole-4-carboxamide;
1-(1-(2-((3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-N-methyl-1H-1,2,3-triazole-4-carboxamide;
1-(2-(4-cyclopropyl-1H-1,2,3-triazol-1-yl)-3,3-dimethylbutanoyl)-4-hydroxy-N-(1-(4-(4-methylthiazol-5-yl)phenyl)ethyl)pyrrolidine-2-carboxamide;
1-(2-cyclohexyl-2-(4-cyclopropyl-1H-1,2,3-triazol-1-yl)acetyl)-4-hydroxy-N-(1-(4-(4-methylthiazol-5-yl)phenyl)ethyl)pyrrolidine-2-carboxamide;
1-(2-(adamantan-1-yl)-2-(4-cyclopropyl-1H-1,2,3-triazol-1-yl)acetyl)-4-hydroxy-N-(1-(4-(4-methylthiazol-5-yl)phenyl)ethyl)pyrrolidine-2-carboxamide;
1-(2-(4-cyclopropyl-1H-1,2,3-triazol-1-yl)-3,3-dimethylpentanoyl)-4-hydroxy-N-(1-(4-(4-methylthiazol-5-yl)phenyl)ethyl)pyrrolidine-2-carboxamide;
N-(1-(2-chloro-4-(4-methylthiazol-5-yl)phenyl)ethyl)-1-(2-(4-cyclopropyl-1H-1,2,3-triazol-1-yl)-3,3-dimethylbutanoyl)-4-hydroxypyrrolidine-2-carboxamide;
1-(2-(adamantan-1-yl)-2-(4-cyclopropyl-1H-1,2,3-triazol-1-yl)acetyl)-N-(1-(2-chloro-4-(4-methylthiazol-5-yl)phenyl)ethyl)-4-hydroxypyrrolidine-2-carboxamide;
1-(2-(4-cyclopropyl-1H-1,2,3-triazol-1-yl)-3,3-dimethylbutanoyl)-4-hydroxy-N-(4-(4-methylthiazol-5-yl)benzyl)pyrrolidine-2-carboxamide;
1-(2-cyclohexyl-2-(4-cyclopropyl-1H-1,2,3-triazol-1-yl)acetyl)-4-hydroxy-N-(4-(4-methylthiazol-5-yl)benzyl)pyrrolidine-2-carboxamide;
1-(2-(4-cyclopropyl-1H-1,2,3-triazol-1-yl)-3-methyl-3-phenylbutanoyl)-4-hydroxy-N-(4-(4-methylthiazol-5-yl)benzyl)pyrrolidine-2-carboxamide;
1-(2-(4-benzyl-1H-1,2,3-triazol-1-yl)-3,3-dimethylbutanoyl)-4-hydroxy-N-(4-(4-methylthiazol-5-yl)benzyl)pyrrolidine-2-carboxamide;
1-(3,3-dimethyl-2-(4-(1-(trifluoromethyl)cyclopropyl)-1H-1,2,3-triazol-1-yl)butanoyl)-4-hydroxy-N-(4-(4-methylthiazol-5-yl)benzyl)pyrrolidine-2-carboxamide;
1-(3,3-dimethyl-2-(4-(1-methylcyclopropyl)-1H-1,2,3-triazol-1-yl)butanoyl)-4-hydroxy-N-(4-(4-methylthiazol-5-yl)benzyl)pyrrolidine-2-carboxamide;
1-(2-(4-(1-ethynylcyclopropyl)-1H-1,2,3-triazol-1-yl)-3,3-dimethylbutanoyl)-4-hydroxy-N-(4-(4-methylthiazol-5-yl)benzyl)pyrrolidine-2-carboxamide;
1,1'-(2,2'-(cyclopropane-1,1-diylbis(1H-1,2,3-triazole-4,1-diyl))bis(3,3-dimethylbutanoyl))bis(4-hydroxy-N-(4-(4-methylthiazol-5-yl)benzyl)pyrrolidine-2-carboxamide);
N-(3-([1,1'-biphenyl]-4-yl)-1-amino-1-oxopropan-2-yl)-4-hydroxy-1-(3-methyl-2-(4-methyl-1H-1,2,3-triazol-1-yl)butanoyl)pyrrolidine-2-carboxamide;
N-(3-([1,1'-biphenyl]-4-yl)-1-amino-1-oxopropan-2-yl)-4-hydroxy-1-(3-methyl-2-(5-methyl-1H-1,2,3-triazol-1-yl)butanoyl)pyrrolidine-2-carboxamide;
methyl 1-(1-(2-((3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate;
1-(1-(2-((3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid;
1-(2-(1H-benzo[d][1,2,3]triazol-1-yl)-3-methylbutanoyl)-N-(3-([1,1'-biphenyl]-4-yl)-1-amino-1-oxopropan-2-yl)-4-hydroxypyrrolidine-2-carboxamide;
N-(3-([1,1'-biphenyl]-4-yl)-1-amino-1-oxopropan-2-yl)-1-(2-(4,5-dimethyl-1H-1,2,3-triazol-1-yl)-3-methylbutanoyl)-4-hydroxypyrrolidine-2-carboxamide;
N-(3-([1,1'-biphenyl]-4-yl)-1-amino-1-oxopropan-2-yl)-1-(2-(4,5-dimethyl-1H-1,2,3-triazol-1-yl)-3,3-dimethylbutanoyl)-4-hydroxypyrrolidine-2-carboxamide;
N-(3-([1,1'-biphenyl]-4-yl)-1-amino-1-oxopropan-2-yl)-4-hydroxy-1-(3-methyl-2-(4-(thiophen-2-yl)-1H-1,2,3-triazol-1-yl)butanoyl)pyrrolidine-2-carboxamide;
N-(3-([1,1'-biphenyl]-4-yl)-1-amino-1-oxopropan-2-yl)-1-(2-(4-(furan-2-yl)-1H-1,2,3-triazol-1-yl)-3-methylbutanoyl)-4-hydroxypyrrolidine-2-carboxamide;
N-(3-([1,1'-biphenyl]-4-yl)-1-amino-1-oxopropan-2-yl)-1-(2-(4-cyano-1H-1,2,3-triazol-1-yl)-3-methylbutanoyl)-4-hydroxypyrrolidine-2-carboxamide;
N-(3-([1,1'-biphenyl]-4-yl)-1-amino-1-oxopropan-2-yl)-1-(2-(4-fluoro-1H-1,2,3-triazol-1-yl)-3-methylbutanoyl)-4-hydroxypyrrolidine-2-carboxamide;
1-(2-(4-cyclopropyl-1H-1,2,3-triazol-1-yl)-3,3-dimethylbutanoyl)-4-hydroxy-N-(2,2,2-trifluoro-1-(4-(4-methylthiazol-5-yl)phenyl)ethyl)pyrrolidine-2-carboxamide;
1-(2-(4-cyclopropyl-1H-1,2,3-triazol-1-yl)-3,3-dimethylbutanoyl)-N-(1-(2'-fluoro-[1,1'-biphenyl]-4-yl)ethyl)-4-hydroxypyrrolidine-2-carboxamide;
1-(2-(4-cyclopropyl-1H-1,2,3-triazol-1-yl)-3,3-dimethylbutanoyl)-N-((2'-fluoro-[1,1'-biphenyl]-4-yl)methyl)-4-hydroxypyrrolidine-2-carboxamide;
N-(1-([1,1'-biphenyl]-4-yl)-2-amino-2-oxoethyl)-1-(2-(4-cyclopropyl-1H-1,2,3-triazol-1-yl)-3,3-dimethylbutanoyl)-4-hydroxypyrrolidine-2-carboxamide;
1-(2-(4-cyclopropyl-1H-1,2,3-triazol-1-yl)-3,3-dimethylbutanoyl)-4-hydroxy-N-(2-(4-methylthiazol-5-yl)-6,7,8,9-tetrahydro-5H-benzo[7]annulen-5-yl)pyrrolidine-2-carboxamide; and
N-(1-(2'-chloro-[1,1'-biphenyl]-4-yl)ethyl)-1-(2-(4-cyclopropyl-1H-1,2,3-triazol-1-yl)-3,3-dimethylbutanoyl)-4-hydroxypyrrolidine-2-carboxamide,
or a stereoisomer or tautomer thereof, or a pharmaceutically acceptable salt of any of the foregoing.

The Compound Names included in Table 1 and in the list in the paragraph above were are auto-generated using ChemDraw^{®} software version 18.2.0.48.

A VHL ligand, as described herein, can exist in solid or liquid form. In the solid state, the ligand may exist in crystalline or noncrystalline form, or as a mixture thereof. The skilled artisan will appreciate that pharmaceutically acceptable solvates may be formed for crystalline or non-crystalline compounds. In crystalline solvates, solvent molecules are incorporated into the crystalline lattice during crystallization. Solvates may involve non-aqueous solvents such as, but not limited to, ethanol, isopropanol, DMSO, acetic acid, ethanolamine, or ethyl acetate, or they may involve water as the solvent that is incorporated into the crystalline lattice. Solvates wherein water is the solvent incorporated into the crystalline lattice are typically referred to as "hydrates." Hydrates include stoichiometric hydrates as well as compositions containing variable amounts of water. The subject matter described herein includes such solvates.

The skilled artisan will further appreciate that certain VHL ligands described herein that exist in crystalline form, including the various solvates thereof, may exhibit polymorphism (i.e. the capacity to occur in different crystalline structures). These different crystalline forms are typically known as "polymorphs." The subject matter disclosed herein includes such polymorphs. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. The skilled artisan will appreciate that different polymorphs may be produced, for example, by changing or adjusting the reaction conditions or reagents, used in making the compound. For example, changes in temperature, pressure, or solvent may result in polymorphs. In addition, one polymorph may spontaneously convert to another polymorph under certain conditions.

VHL ligands described herein, or a pharmaceutically acceptable salt thereof, may exist in stereoisomeric forms (e.g., it contains one or more asymmetric carbon atoms). The individual stereoisomers (enantiomers and diastereomers) and mixtures of these are included within the scope of the subject matter disclosed herein. Likewise, it is understood that a compound or salt of formula (I) may exist in tautomeric forms other than that shown in the formula and these are also included within the scope of the subject matter disclosed herein. It is to be understood that the subject matter disclosed herein includes combinations and subsets of the particular groups described herein. The scope of the subject matter disclosed herein includes mixtures of stereoisomers as well as purified enantiomers or enantiomerically/diastereomerically enriched mixtures. It is to be understood that the subject matter disclosed herein includes combinations and subsets of the particular groups defined hereinabove.

The subject matter disclosed herein also includes isotopically-labelled forms of the compounds described herein, but for the fact that one or more atoms are replaced by an atom having an atomic mass or mass number different from the atomic mass or mass number usually found in nature. Examples of isotopes that can be incorporated into compounds described herein and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I and ¹²⁵I.

VHL ligands as disclosed herein, and pharmaceutically acceptable salts thereof, that contain the aforementioned isotopes and/or other isotopes of other atoms are within the scope of the subject matter disclosed herein. Isotopically-labelled compounds are disclosed herein, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are commonly used for their ease of preparation and detectability. ¹¹C and ¹⁸F isotopes are useful in PET (positron emission tomography), and ¹²⁵I isotopes are useful in SPECT (single photon emission computerized tomography), all useful in brain imaging. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds of formula I can generally be prepared by carrying out the procedures disclosed in the Schemes and/or in the Examples below, by substituting a readily available isotopically labelled reagent for a non-isotopically labelled reagent.

In some embodiments, a VHL ligand provided herein is integrated into a heterobifunctional molecule. In some embodiments, the heterobifunctional molecule is a chemical inducer of degradation (CIDE) having (i) a VHL ligand, as provided herein, and (ii) a moiety that is capable of binding to a protein of interest that is targeted for degradation, wherein (i) and (ii) are covalently linked. In some embodiments, (i) and (ii) are covalently linked through a linker moiety, such as a polyethylene glycol (PEG) chain or an alkyl chain. In some embodiments, the CIDE is capable of selectively degrading a target protein by forming a ternary complex between the target protein, the heterobifunctional molecule described herein, and a ubiquitin ligase. In some embodiments, the ubiquitin ligase is a VHL E3 ubiquitin ligase. By way of illustration, and not limitation, the target protein may be, for example, a structural protein, an enzyme, a receptor, or a cell surface protein.

In some embodiments, the heterobifunctional molecule is a compound of formula (II):

[A]-[B]-[C] (II),

wherein [A] is a moiety of a VHL ligand provided herein, [B] is a linker moiety, and [C] is a protein-binding moiety.

### III. Formulations

In an additional aspect, the description provides therapeutic or pharmaceutical compositions comprising an effective amount of at least one of the compounds as described herein, including, e.g., at least one VHL ligand. Pharmaceutical compositions comprising an effective amount of at least one VHL ligand of the present disclosure, and optionally one or more of the compounds otherwise described herein, in effective amounts, in combination with a pharmaceutically effective amount of a carrier, additive, or excipient, and optionally an additional bioactive agent, represents a further aspect of the disclosure.

In certain embodiments, the compositions comprise pharmaceutically acceptable salts, in particular, acid or base addition salts of compounds as described herein. The acids that are used to prepare the pharmaceutically acceptable acid addition salts of the aforementioned base compounds include those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3 naphthoate)]salts, among numerous others.

Pharmaceutically acceptable base addition salts may also be used to produce pharmaceutically acceptable salt forms of the compounds or derivatives. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (eg, calcium, zinc and magnesium), ammonium or watersoluble amine addition salts such as N-methylglucamine-(meglumine), and the lower alkanolammonium and other base salts of pharmaceutically acceptable organic amines, among others.

The compositions as described herein may in certain embodiments be administered in single or divided unit doses by the oral, parenteral or topical routes. Administration of the compounds may range from continuous (intravenous drip) to several oral administrations per day (for example, Q.I.D.) and may include oral, topical, parenteral, intramuscular, intravenous, sub-cutaneous, transdermal (which may include a penetration enhancement agent), buccal, sublingual and suppository administration, by inhalation spray, rectally, vaginally, or via an implanted reservoir, among other routes of administration. Enteric coated oral tablets may also be used to enhance bioavailability of the compounds from an oral route of administration. The most effective dosage form will depend upon the pharmacokinetics of the particular agent chosen as well as the severity of disease in the patient. Administration of compounds according to the present disclosure as sprays, mists, or aerosols for intra-nasal, intra-tracheal or pulmonary administration may also be used. The present disclosure therefore also is directed to pharmaceutical compositions comprising an effective amount of compound according to the present disclosure, optionally in combination with a pharmaceutically acceptable carrier, additive or excipient. Compounds according to the present disclosure may be administered in immediate release, intermediate release or sustained or controlled release forms. Sustained or controlled release forms are preferably administered orally, but may also be administered in suppository and transdermal or other topical forms. Intramuscular injections in liposomal form may also be used to control or sustain the release of compound at an injection site.

Thus in one aspect, pharmaceutical formulations of VHL ligands, as described herein, can be prepared for parenteral administration with a pharmaceutically acceptable parenteral vehicle and in a unit dosage injectable form. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. A VHL ligand having the desired degree of purity is optionally mixed with one or more pharmaceutically acceptable excipients (Remington's Pharmaceutical Sciences (1980) 16th edition, Osol, A. Ed.), in the form of a lyophilized formulation for reconstitution or an aqueous solution.

The compositions of the present disclosure may be formulated in a conventional manner using one or more pharmaceutically acceptable carriers and may also be administered in controlled-release formulations. The compounds of the disclosure can be formulated in accordance with standard pharmaceutical practice as a pharmaceutical composition. According to this aspect, there is provided a pharmaceutical composition comprising a VHL ligand, as described herein, in association with one or more pharmaceutically acceptable excipients.

A typical formulation is prepared by mixing the compounds of the disclosure with excipients, such as carriers and/or diluents. Suitable carriers, diluents and other excipients are well known to those skilled in the art and include materials such as carbohydrates, waxes, water soluble and/or swellable polymers, hydrophilic or hydrophobic materials, gelatin, oils, solvents, water and the like. The particular carrier, diluent or other excipient used will depend upon the means and purpose for which the compound is being applied. Other pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), etc. and mixtures thereof. Acceptable diluents, carriers, excipients and stabilizers are nontoxic to recipients at the dosages and concentrations employed, and include buffers such as phosphate, citrate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the VHL ligand or aid in the manufacturing of the pharmaceutical product. The formulations may be prepared using conventional dissolution and mixing procedures.

Formulation may be conducted by mixing at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of compound, but may range from about 3 to about 8. Formulation in an acetate buffer at pH 5 is a suitable embodiment.

The pharmaceutical compositions may be in the form of a sterile injectable preparation, such as a sterile injectable aqueous or oleaginous suspension. In particular, formulations to be used for in vivo administration must be sterile. Such sterilization is readily accomplished by filtration through sterile filtration membranes. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such 1,3-butanediol. The sterile injectable preparation may also be prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables, as well as natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The pharmaceutical compositions as described herein may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, the pharmaceutical compositions as described herein may be administered in the form of suppositories for rectal administration. These can be prepared by mixing the agent with a suitable non-irritating excipient, which is solid at room temperature but liquid at rectal temperature and therefore will melt in the rectum to release the drug. Such materials include cocoa butter, beeswax and polyethylene glycols.

The pharmaceutical compositions as described herein may also be administered topically. Suitable topical formulations are readily prepared for each of these areas or organs. Topical application for the lower intestinal tract can be effected in a rectal suppository formulation (see above) or in a suitable enema formulation. Topically-acceptable transdermal patches may also be used.

For topical applications, the pharmaceutical compositions may be formulated in a suitable ointment containing the active component suspended or dissolved in one or more carriers. Carriers for topical administration of the compounds of this disclosure include, but are not limited to, mineral oil, liquid petrolatum, white petrolatum, propylene glycol, polyoxyethylene, polyoxypropylene compound, emulsifying wax and water. In certain preferred aspects of the disclosure, the compounds may be coated onto a stent which is to be surgically implanted into a patient in order to inhibit or reduce the likelihood of occlusion occurring in the stent in the patient.

Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of this disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The VHL ligand compositions ordinarily can be stored as a solid composition, a lyophilized formulation or as an aqueous solution.

The pharmaceutical compositions comprising a VHL ligand of the present disclosure can be formulated, dosed and administered in a fashion, i.e., amounts, concentrations, schedules, course, vehicles and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the compound to be administered will be governed by such considerations, and is the minimum amount necessary to prevent, ameliorate, or treat the disorder. Such amount is preferably below the amount that is toxic to the host or renders the host significantly more susceptible to unwanted side effects.

The VHL ligand can be formulated into pharmaceutical dosage forms to provide an easily controllable dosage of the drug and to enable patient compliance with the prescribed regimen. The pharmaceutical composition (or formulation) for application may be packaged in a variety of ways depending upon the method used for administering the drug. Generally, an article for distribution includes a container having deposited therein the pharmaceutical formulation in an appropriate form. Suitable containers are well known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease or condition being treated.

A patient or subject in need of therapy using compounds according to the present disclosure can be treated by administering to the patient (subject) an effective amount of the compound according to the present disclosure including pharmaceutically acceptable salts, solvates or polymorphs, thereof optionally in a pharmaceutically acceptable carrier or diluent, either alone, or in combination with other known erythopoiesis stimulating agents as otherwise identified herein.

The active compound is included in the pharmaceutically acceptable carrier or diluent in an amount sufficient to deliver to a patient a therapeutically effective amount for the desired indication, without causing serious toxic effects in the patient treated. A preferred dose of the active compound for the herein-mentioned conditions is in the range from about 10 ng/kg to 300 mg/kg, preferably 0.1 to 100 mg/kg per day, more generally 0.5 to about 25 mg per kilogram body weight of the recipient/patient per day. One typical daily dosage might range from about 1 µg/kg to 100 mg/kg or more, depending on the factors mentioned above. A typical topical dosage will range from 0.01-5% wt/wt in a suitable carrier.

The compound is conveniently administered in any suitable unit dosage form, including but not limited to one containing less than 1 mg, 1 mg to 3000 mg, preferably 5 to 500 mg of active ingredient per unit dosage form. An oral dosage of about 25-250 mg is often convenient.

The active ingredient is preferably administered to achieve peak plasma concentrations of the active compound of about 0.00001-30 mM, preferably about 0.1-30 mM. This may be achieved, for example, by the intravenous injection of a solution or formulation of the active ingredient, optionally in saline, or an aqueous medium or administered as a bolus of the active ingredient. Oral administration is also appropriate to generate effective plasma concentrations of active agent.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions may also be pharmaceutically acceptable carriers. These may be prepared according to methods known to those skilled in the art, for example, as described in U.S. Pat. No.4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound are then introduced into the container. The container is then swirled by hand to free lipid material from the sides of the container and to disperse lipid aggregates, thereby forming the liposomal suspension.

The term "pharmaceutically acceptable salt" is used throughout the specification to describe, where applicable, a salt form of one or more of the compounds described herein which are presented to increase the solubility of the compound in the gastric juices of the patient's gastrointestinal tract in order to promote dissolution and the bioavailability of the compounds. Pharmaceutically acceptable salts include those derived from pharmaceutically acceptable inorganic or organic bases and acids, where applicable. Suitable salts include those derived from alkali metals such as potassium and sodium, alkaline earth metals such as calcium, magnesium and ammonium salts, among numerous other acids and bases well known in the pharmaceutical art. Sodium and potassium salts are particularly preferred as neutralization salts of the phosphates according to the present disclosure.

The term "pharmaceutically acceptable derivative" is used throughout the specification to describe any pharmaceutically acceptable prodrug form (such as an ester, amide other prodrug group), which, upon administration to a patient, provides directly or indirectly the present compound or an active metabolite of the present compound.

The subject matter further provides veterinary compositions comprising at least one active ingredient as above defined together with a veterinary carrier therefore. Veterinary carriers are materials useful for the purpose of administering the composition and may be solid, liquid or gaseous materials which are otherwise inert or acceptable in the veterinary art and are compatible with the active ingredient. These veterinary compositions may be administered parenterally or by any other desired route.

### IV. Indications and Methods of Treatment

Any reference to methods of treatment in the subsequent paragraphs of this description are to be interpreted as references to the compounds, pharmaceutical compositions and medicaments of the present invention for use in a method for treatment of the human (or animal) body by therapy (or for diagnosis).

It is contemplated that the VHL ligands disclosed herein may be used to treat various diseases, disorders, or conditions. Thus, it is understood that any one of the compounds provided herein may find use in the treatment of a disease or condition modulated by VHL such as any of the diseases and conditions listed herein. It is also understood that any of the compounds provided herein may find use in the preparation of a medicament for treatment of a condition modulated by VHL such as any of the diseases and conditions listed herein.

It is contemplated that the compounds disclosed herein may be used in therapy. It is further contemplated that the compounds disclosed herein may be used to treat a disease or indication associated with VHL activity, such as the diseases and indications in Zhang et al., J. Med. Chem. 219, 62, 5725-5749. Thus, it is understood that any one of the compounds provided herein may find use in the treatment of a condition modulated by VHL. In some embodiments, the VHL ligands disclosed herein may be used to treat a cancer implicated by VHL modulation. In some embodiments, the VHL ligands disclosed herein may be used to treat a solid tumor. In some embodiments, the solid tumor is breast cancer (such as triple-negative breast cancer), lung cancer, multiple myeloma or renal cell carcinoma (RCC).

In alternative aspects, the present invention relates to compounds for use in a method for enhancing erythropoiesis in a patient or subject in need, the method comprising administering to said patient or subject an effective amount of at least one compound as described hereinabove, optionally in combination with an additional erythropoiesis stimulating compound. The method according to the present invention may be used to increase the number of red blood cells (erythrocytes) and/or the hematocrit of the patient by virtue of the administration of effective amounts of at least one compound described herein. Additional method aspects of the present invention relate to treating anemia, including chronic anemia or ischemia in a patient or subject in need, the method comprising administering to a patient in need an effective amount of at least one compound according to the present invention. The methods according to the present invention may be used to treat anemia, including chronic anemia such as anemia associate with chronic kidney disease, dialysis and chemotherapy and ischemia, including local ischemia, stroke and cardiovascular ischemia and limit the damage which occurs as a consequence of those disease states and/or conditions.

Additional method aspects of the present invention relate to enhancing wound healing and reducing scar tissue formation during wound healing by administering one or more compounds according to the present invention to a patient in need. Further methods include inducing local angiogenesis in a patient or subject in need by administering an effective amount of at least one compound of the present invention, optionally in combination with an additional erythropoiesis stimulating compound. Methods of stimulating erythropoiesis in a subject or patient, including increasing the number of red blood cells and/or hematocrit of the patient, treating anemia, including chronic anemia and anemia associated with chronic kidney disease, dialysis, and cancer chemotherapy, ischemia, stroke and damage to cardiovascular tissue during cardiovascular ischemia as well as enhancing wound healing processes and preventing/reducing scarring associated with or secondary to the healing process represent additional aspects of the present invention.

Other methods of the present invention relate to the local enhancement of angiogenesis through the induction of VEGF in a patient or subject using at least one compound according to the present invention, optionally in combination with an erythropoiesis stimulating compound as otherwise described herein. An additional method of the present invention relates to the reduction and/or inhibition of occlusion in a surgically implanted stent in a patient or subject.

The compounds described herein may be administered to a patient to treat a number of diseases, disorders, or conditions. In some embodiments, administration of a compound, as described herein, provides stimulation of erythropoiesis in a patient or subject, including inducement of EPO production in the patient or subject. In other embodiments, administration of a compound, as described herein, is provided for the treatment of chronic anemia and ischemia (which limits brain injury during episodes of localized anemia, ischemia and/or stroke and damage to cardiovascular tissue during cardiovascular ischemia), as well as enhancing wound healing processes. Methods of stimulating erythropoiesis in a subject or patient, including increasing the number of red blood cells and/or hematocrit of the patient, treating anemia, including chronic anemia and anemia associated with chronic kidney disease, dialysis, and cancer chemotherapy, ischemia, stroke and damage to cardiovascular tissue during cardiovascular ischemia as well as enhancing wound healing processes and preventing/reducing scarring secondary to healing represent additional treatment aspects of the present invention. Local enhancement of angiogenesis through induction of VEGF including wound healing and reduction of stent occlusion remain additional aspects of the present invention.

Also provided herein is compound as described herein for use in the manufacture of a medicament for use in the treatment of a number of diseases, disorders, and conditions. In one embodiments, provided herein is compound as described herein for use in the manufacture of a medicament for use in the treatment of anemia. In some embodiments, the anemia is chronic anemia or anemia associated with chronic kidney disease, dialysis, or cancer chemotherapy, or any combination thereof. In other embodiments, provided herein is a compound as described herein for use in the manufacture of a medicament for use in the treatment of ischemia, stroke, or damage to the cardiovascular system during ischemia, or any combination thereof. In some embodiments, provided herein is a compound as described herein for use in the manufacture of a medicament for use in the enhancement of wound healing in a human in need thereof. In other embodiments, provided herein is a compound as described herein for use in the manufacture of a medicament for use in the reduction of scarring secondary to wound healing in a human in need thereof. In some embodiments, provided herein is a compound as described herein for use in the manufacture of a medicament for use in the enhancement of angiogenesis or arteriogenesis, or both, in a human in need thereof. In certain embodiments, the enhancement of angiogenesis or arteriogenesis, or both, occurs locally in the human. In some embodiments, provided herein is a compound as described herein for use in the manufacture of a medicament for use in reducing the likelihood of stent occlusion in a human in need thereof.

Also provided herein is a compound, as described elsewhere herein, for use in the treatment of anemia. In some embodiments, the anemia is chronic anemia or anemia associated with chronic kidney disease, dialysis, or cancer chemotherapy, or any combination thereof. In other embodiments, provided herein is a compound, as described elsewhere herein, for use in the treatment of ischemia, stroke, or damage to the cardiovascular system during ischemia, or any combination thereof. In some embodiments, provided herein is a compound, as described elsewhere herein, for use in the enhancement of wound healing in a human in need thereof. In other embodiments, provided herein is a compound, as described elsewhere herein, for use in the reduction of scarring secondary to wound healing in a human in need thereof. In some embodiments, provided herein is a compound, as described elsewhere herein, for use in the enhancement of angiogenesis or arteriogenesis, or both, in a human in need thereof. In some embodiments, the enhancement of the angiogenesis or arteriogenesis, or both, occurs locally in the human. In some embodiments, provided herein is a compound, as described elsewhere herein, for use in reducing the likelihood of stent occlusion in a human in need thereof.

The term "coadministration" or "combination therapy" shall mean that at least two compounds or compositions are administered to the patient at the same time, such that effective amounts or concentrations of each of the two or more compounds may be found in the patient at a given point in time. Although compounds according to the present invention may be co-administered to a patient at the same time, the term embraces both administration of two or more agents at the same time or at different times, provided that effective concentrations of all coadministered compounds or compositions are found in the·subject at a given time. In certain preferred aspects of the present invention, one or more of the present compounds described above, are coadministered in combination with at least one additional bioactive agent having erythropoiesis stimulating activity as otherwise described herein in order to enhance erythopoeisis, treat chronic anemia and ischemia (limit brain injury during episodes of localized anemia, ischemia and/or stroke and damage to cardiovascular tissue during cardiovascular ischemia), as well as enhancing wound healing processes and stimulating angiogenesis and inhibiting or preventing occlusion in a surgically implanted stent. In particularly preferred aspects of the invention, the co-administration of compounds results in synergistic erythropoietic activity and/or therapy.

The term "additional erythropoisis stimulating agent" shall mean a traditional polypeptide such as EPO (procrit or epogen) or darbapoietin alfa (a synthetic form of erythropoietin).

The compositions of the present invention may be formulated in a conventional manner using one or more pharmaceutically acceptable carriers and may also be administered in controlled-release formulations. Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylenepolyoxypropylene-block polymers, polyethylene glycol and wool fat.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrastemal, intrathecal, intrahepatic, intralesional and intracranial injection or infusion techniques. Preferably, the compositions are administered orally, intraperitoneally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally-acceptable diluent or solvent, for example as a solution in 1, 3-butanediol.

Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

The pharmaceutical compositions of this invention may be orally administered in any orally acceptable dosage form including, but not limited to, capsules, tablets, aqueous suspensions or solutions. In the case of tablets for oral use, carriers which are commonly used include lactose and com starch. Lubricating agents, such as magnesium stearate, are also typically added. For oral administration in a capsule form, useful diluents include lactose and dried com starch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically. Suitable topical formulations are readily prepared for each of these areas or organs. Topical application for the lower intestinal tract can be effected in a rectal suppository formulation (see above) or in a suitable enema formulation. Topically-acceptable transdermal patches may also be used. For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. In certain preferred aspects of the invention, the compounds may be coated onto a stent which is to be surgically implanted into a patient in order to inhibit or reduce the likelihood of occlusion occurring in the stent in the patient.

Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of compound in a pharmaceutical composition of the instant invention that may be combined with the carrier materials to produce a single dosage form will vary depending upon the host and disease treated, the particular mode of administration. Preferably, the compositions should be formulated to contain between about 0.05 milligram to about 750 milligrams or more, more preferably about 1 milligram to about 600 milligrams, and even more preferably about 10 milligrams to about 500 milligrams of active ingredient, alone or in combination with at least one other compound according to the present invention or erythropoiesis stimulating agent (EPO, darbapoietin alfa) in order to *inter alia* enhance erythopoeisis, treat chronic anemia and ischemia (limits brain injury during episodes of localized anemia, ischemia and/or stroke and damage to cardiovascular tissue during cardiovascular ischemia), as well as enhancing wound healing processes.and stimulating angiogenesis and inhibiting or preventing occlusion in a surgically implanted stent.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease or condition being treated.

A patient or subject in need of therapy using compounds according to the present invention can be treated by administering to the patient (subject) an effective amount of the compound according to the present invention including pharmaceutically acceptable salts, solvates or polymorphs, thereof optionally in a pharmaceutically acceptable carrier or diluent, either alone, or in combination with other known erythopoiesis stimulating agents as otherwise identified herein. These compounds can be administered by any appropriate route, for example, orally, parenterally, intravenously, intradermally, subcutaneously, or topically, including transdermally, in liquid, cream, gel, or solid form, or by aerosol form. The active compound is included in the pharmaceutically acceptable carrier or diluent in an amount sufficient to deliver to a patient a therapeutically effective amount for the desired indication, without causing serious toxic effects in the patient treated. A preferred dose of the active compound for all of the herein-mentioned conditions is in the range from about 10 ng/kg to 300 mg/kg, preferably 0.1 to 100 mg/kg per day, more generally 0.5 to about 25 mg per kilogram body weight of the recipient/patient per day. A typical topical dosage will range from 0.01-5% wt/wt in a suitable carrier. The compound is conveniently administered in any suitable unit dosage form, including but not limited to one containing less than 1mg, 1 mg to 3000 mg, preferably 5 to 500 mg of active ingredient per unit dosage form. An oral dosage of about 25-250 mg is often convenient.

The active ingredient is preferably administered to achieve peak plasma concentrations of the active compound of about 0.00001-30 mM, preferably about 0.1-30 µM. This may be achieved, for example, by the intravenous injection of a solution or formulation of the active ingredient, optionally in saline, or an aqueous medium or administered as a bolus of the active ingredient. Oral administration is also appropriate to generate effective plasma concentrations of active agent.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition.

The active ingredient may be administered at once, or may be divided into a number of smaller doses to be administered at varying intervals of time. Oral compositions will generally include an inert diluent or an edible carrier. They may be enclosed in gelatin capsules or compressed into tablets. For the purpose of oral therapeutic administration, the active compound or its prodrug derivative can be incorporated with excipients and used in the form of tablets, troches, or capsules. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a dispersing agent such as alginic acid, Primogel, or com starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or enteric agents.

The active compound or pharmaceutically acceptable salt thereof can be administered as a component of an elixir, suspension, syrup, wafer, chewing gum or the like. A syrup may contain, in addition to the active compounds, sucrose as a sweetening agent and certain preservatives, dyes and colorings and flavors. The active compound or pharmaceutically acceptable salts thereof can also be mixed with other active materials that do not impair the desired action, or with materials that supplement the desired action, such as erythropoietin stimulating agents, including EPO and darbapoietin alfa, among others. In certain preferred aspects of the invention, one or more compounds according to the present invention are coadministered with another bioactive agent, such as an erythropoietin stimulating agent or a wound healing agent, including an antibiotic, as otherwise described herein.

Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic. If administered intravenously, preferred carriers are physiological saline or phosphate buffered saline (PBS). In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions may also be pharmaceutically acceptable carriers. These may be prepared according to methods known to those skilled in the art, for example, as described in U.S. Pat. No. 4,522,811 (which is incorporated herein by reference in its entirety). For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound are then introduced into the container. The container is then swirled by hand to free lipid material from the sides of the container and to disperse lipid aggregates, thereby forming the liposomal suspension.

### V. Articles of Manufacture

In another aspect, described herein are articles of manufacture, for example, a "kit", containing materials useful for the treatment of the diseases and disorders described above is provided. The kit comprises a container comprising a VHL ligand. The kit may further comprise a label or package insert, on or associated with the container. The term "package insert" is used to refer to instructions customarily included in commercial packages of therapeutic products, that contain information about the indications, usage, dosage, administration, contraindications and/or warnings concerning the use of such therapeutic products.

Suitable containers include, for example, bottles, vials, syringes, blister pack, etc. A "vial" is a container suitable for holding a liquid or lyophilized preparation. In one embodiment, the vial is a single-use vial, e.g. a 20-cc single-use vial with a stopper. The container may be formed from a variety of materials such as glass or plastic. The container may hold a VHL ligand or a formulation thereof which is effective for treating the condition and may have a sterile access port (for example, the container may be an intravenous solution bag or a vial having a stopper pierceable by a hypodermic injection needle).

At least one active agent in the composition is a VHL ligand of the present disclosure. The label or package insert indicates that the composition is used for treating the condition of choice, such as cancer. In addition, the label or package insert may indicate that the patient to be treated is one having a disorder such as a hyperproliferative disorder, neurodegeneration, cardiac hypertrophy, pain, migraine or a neurotraumatic disease or event. In one embodiment, the label or package inserts indicates that the composition comprising a VHL ligand can be used to treat a disorder resulting from abnormal cell growth. The label or package insert may also indicate that the composition can be used to treat other disorders. Alternatively, or additionally, the article of manufacture may further comprise a second container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of the VHL ligand and, if present, the second pharmaceutical formulation. For example, if the kit comprises a first composition comprising a VHL ligand, and a second pharmaceutical formulation, the kit may further comprise directions for the simultaneous, sequential or separate administration of the first and second pharmaceutical compositions to a patient in need thereof.

In another embodiment, the kits are suitable for the delivery of solid oral forms of a VHL ligand, such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one embodiment, a kit may comprise (a) a first container with a VHL ligand contained therein; and optionally (b) a second container with a second pharmaceutical formulation contained therein, wherein the second pharmaceutical formulation comprises a second compound with anti-hyperproliferative activity. Alternatively, or additionally, the kit may further comprise a third container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In certain other embodiments, wherein the kit comprises a VHL ligand and a second therapeutic agent, the kit may comprise a container for containing the separate compositions such as a divided bottle or a divided foil packet; however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

### VI. Examples

The following examples are offered by way of illustration and not by way of limitation. Some of the compounds used in the following examples are tautomers. Although the illustrations of these compounds provided below depict only a single tautomer, these illustrations should not be viewed in a limiting sense, but rather, the corresponding tautomers are also intended and embraced by the following examples, as if each and every one of the tautomers of the compound is individually depicted.

### Abbreviations

The following abbreviations are used in the examples:
ABPR - automated back pressure regulator
Ac₂O - acetic anhydride
ACN - acetonitrile
Boc - tert-butyloxycarbonyl
CDCl₃ - Deuterochloroform
Cy₃PHBF₄ - Tricyclohexylphosphine tetrafluoroborate
DBU - 1,8-Diazabicyclo[5.4.0]undec-7-ene
DCE - 1,2-Dichloroethane
DCM - dichloromethane
DEA - diethanolamine
DIPEA or DIEA - N,N-diisopropylethylamine
DME - dimethoxyethane
DMF - dimethylformamide
DMEM - Dulbecco's Modified Eagle's medium
DMSO - dimethyl sulfoxide
DTT - dithiothreitol
EDCI - N-(3-Dimethylaminopropyl)-N'-ethylcarbodiimide hydrochloride
EDTA - ethylenediaminetetraacetic acid
ESI - electrospray ionization
Et₃N - trimethylamine
EtOAc - ethyl acetate
EtOH - ethanol
FA - formic acid
Fmoc - Fluorenylmethyloxycarbonyl
HATU - 1-[Bis(dimethylamino)methylene]-1H-1,2,3-triazolo[4,5-b]pyridinium 3-oxide hexafluorophosphate
HEPES - 4-(2-Hydroxyethyl)piperazine-1-ethanesulfonic acid
Hex - hexane
HOAc - acetic acid
HOBt or HOBT - hydroxybenzotriazole
HPLC - high performance liquid chromatography
KOAc - potassium acetate
LC/MS or LCMS - liquid chromatography - mass spectrometry
LDA - Lithium diisopropylamide
LiHMDS - lithium bis(trimethylsilyl)amide
MeI - methyl iodide
MeOH - methanol or methyl alcohol
MeONa or NaOMe- sodium methoxide
MSD - mass selective detector
MeSO₂Na - sodium methanesulphinate
MTBE - methyl tert-butyl ether
NBS - N-bromosuccinimide
n-BuLi - butyllithium
(n-Bu)₃SnCl - tributylin chloride
NIS --N-iodosuccinimide
NMP - N-Methyl-2-pyrrolidone
NMR - nuclear magnetic resonance
PBS - phosphate buffered saline
Pd/C - palladium on carbon
Pd(dppf)Cl₂.CH₂Cl₂ - [1,1'-Bis(diphenylphosphino)ferrocene]dichloropalladium(II) dichloromethane
Pd(PPh₃)₄ - Palladium-tetrakis(triphenylphosphine)
PEG - polyethylene glycol
Ph₃P - triphenylphosphine
PPTS - Pyridinium p-toluenesulfonate
SFC - supercritical fluid chromatography
TAMRA - carboxytetramethylrhodamine
TBAF - tetrabutylammonium fluoride
TBS - tert-Butyldimethylsilyl
TBSCl - tert-Butyldimethylsilyl chloride
tBuOK - potassium tert-butoxide
TCEP - Tris(2-carboxyethyl)phosphine
TEA - triethylamine
TFA - trifluoroacetic acid
THF - tetrahydrofuran
TMSCN - Trimethylsilyl cyanide
TMSI - trimethylsilyl iodide
TMSOTf - Trimethylsilyl trifluoromethanesulfonate
TsCl - 4-toluenesulfonyl chloride
TsOH - toluenesulfonic acid
UV - ultraviolet

### LC/MS Methods

*Method A:* Experiments performed on an SHIMADZU 2020 HPLC with SHIMADZU MSD mass spectrometer using ESI as ionization source using an Shim-Pack XR-ODS C18 50 x 3.0 mm 2.2µm column and a 1.2 ml / minute flow rate. The solvent A is water with 0.05% TFA and solvent B is acetonitrile with 0.05% TFA, The gradient consisted with 20 - 80% solvent B over 3.6 minutes, 80 - 100% solvent B over 0.4 minutes and hold 100% B for 0.5 minutes. LC column temperature is 40 °C. UV absorbance was collected from 190 nm to 400 nm.

*Method B:* Experiments performed on an SHIMADZU 2020 HPLC with SHIMADZU MSD mass spectrometer using ESI as ionization source using a Shim-pack XR-ODS C18 50 x 3.0 mm column and a 1.2 ml / minute flow rate. The solvent system was a gradient starting with 95% water with 0.05% TFA (solvent A) and 5% acetonitrile with 0.05% TFA (solvent B), ramping up to 100% solvent B over 1.1 minutes. The final solvent system was held constant for a further 0.6 minutes. LC column temperature is 40 °C. UV absorbance was collected from 190 nm to 400 nm.

*Method C:* Experiments performed on an SHIMADZU 2020 HPLC with SHIMADZU MSD mass spectrometer using ESI as ionization source using an Ascentis Express C18 50 x 2.1 mm column and a 1.0 ml / minute flow rate. The solvent system was a gradient starting with 95% water with 0.05% TFA (solvent A) and 5% acetonitrile with 0.05% TFA (solvent B), ramping up to 100% solvent B over 1.1 minutes. The final solvent system was held constant for a further 0.5 minutes. LC column temperature is 40 °C. UV absorbance was collected from 190 nm to 400 nm.

*Method D:* Experiments performed on an SHIMADZU 2020 HPLC with SHIMADZU MSD mass spectrometer using ESI as ionization source using a Shim-pack XR-ODS 50 x 3.0 mm column and a 1.2 ml / minute flow rate. The solvent system was a gradient starting with 95% water with 0.05% TFA (solvent A) and 5% acetonitrile with 0.05% TFA (solvent B), ramping up to 95% solvent B over 2.0 minutes. The final solvent system was held constant for a further 0.7 minutes. LC column temperature is 40 °C. UV absorbance was collected from 190 nm to 400 nm.

*Method E:* Experiments performed on an SHIMADZU 2020 HPLC with SHIMADZU MSD mass spectrometer using ESI as ionization source using a CORTECS C18 50 x 3.1 mm column and a 1.0 ml / minute flow rate. The solvent system was a gradient starting with 95% water with 0.05% TFA (solvent A) and 5% acetonitrile with 0.05% TFA (solvent B), ramping up to 100% solvent B over 1.1 minutes. The final solvent system was held constant for a further 0.5 minutes. LC column temperature is 45 °C. UV absorbance was collected from 190 nm to 400 nm.

*Method F:* Experiments performed on a Shimadzu 2020 HPLC with Shimadzu MSD mass spectrometer using ESI as ionization source using a Poroshell HPH-C18 50 x 3.0 mm column and a 1.2 mL/minute flow rate. The solvent A is water with 0.05% NH₄HCO₃ and solvent B is acetonitrile. The gradient consisted with 10 - 50% solvent B over 3.5 minutes then 50 - 95% solvent B over 0.5 minutes and hold 95% B for 0.7 minutes. LC column temperature is 40 °C. UV absorbance was collected from 190 nm to 400 nm.

*Method G:* Experiments performed on an SHIMADZU 2020 HPLC with SHIMADZU MSD mass spectrometer using ESI as ionization source using an XSELECT CSH C18 50 x 3.0 mm column and a 1.5 ml / minute flow rate. The solvent system was a gradient starting with 90% water with 0.1% FA (solvent A) and 10% acetonitrile with 0.1% FA (solvent B), ramping up to 100% solvent B over 1.1 minutes. The final solvent system was held constant for a further 0.6 minutes. LC column temperature is 40 °C. UV absorbance was collected from 190 nm to 400 nm.

*Method H:* Experiments performed on an SHIMADZU 2020 HPLC with SHIMADZU MSD mass spectrometer using ESI as ionization source using an Accucore C18 50 x 2.1 mm column and a 1.0 ml / minute flow rate. The solvent system was a gradient starting with 90% water with 0.1% FA (solvent A) and 10% acetonitrile with 0.1% FA (solvent B), ramping up to 95% solvent B over 2 minutes. The final solvent system was held constant for a further 0.7 minutes. LC column temperature is 40 °C. UV absorbance was collected from 190 nm to 400 nm.

*Method I:* Experiments performed on a Shimadzu LCMS-2020 coupled with SHIMADZU MSD mass spectrometer using ESI as ionization source. The LC separation was using a CAPCELL CORE C18, 50 x 2.1 mm column with a 1 ml / minute flow rate. Solvent A is water with 0.05% TFA and solvent B is acetonitrile with 0.05% TFA. The gradient consisted with 5 - 95% solvent B over 2.0 minutes and hold 95% B for 0.7 minutes. LC column temperature is 40 °C. UV absorbance was collected from 190 nm to 400 nm.

*Method J:* Experiments performed on a Shimadzu LCMS-2020 coupled with SHIMADZU MSD mass spectrometer using ESI as ionization source. The LC separation was using a Shim-pack XR-ODS, 50 x 3.0 mm column with a 1.2 ml / minute flow rate. Solvent A is water with 0.05% TFA and solvent B is acetonitrile with 0.05% TFA. The gradient consisted with 5 - 70% solvent B over 3.7 minutes, 70 - 95% solvent B over 0.2 minutes and hold 95% B for 0.7 minutes. LC column temperature is 40 °C. UV absorbance was collected from 190 nm to 400 nm.

*Method K:* Experiments performed on a Shimadzu LCMS-2020. The LC separation was using a Ascentis Express C18, 100 x 4.6 mm column with a 1.2 ml / minute flow rate. Solvent A is water with 0.05% TFA and solvent B is methanol. The gradient consisted with 30 - 95% solvent B over 10 minutes and hold 95% B for 2 minutes. LC column temperature is 40 °C. UV absorbance was collected from 190 nm to 400 nm.

*Method L:* Experiments performed on a Shimadzu LCMS-2020 coupled with SHIMADZU MSD mass spectrometer using ESI as ionization source. The LC separation was using a Kinetex EVO C18, 50 x 2.1 mm column with a 1.0 ml / minute flow rate. Solvent A is water with 0.05% NH₄HCO₃ and solvent B is acetonitrile. The gradient consisted with 10 - 95% solvent B over 1.1 minutes, and hold 95% B for 0.5 minutes. LC column temperature is 35 °C. UV absorbance was collected from 190 nm to 400 nm.

*Method M:* Experiments were performed on a HPLC column coupled with a mass spectrometer using ESI as an ionization source. The LC separation was using MK RP18e, 25 x 2 mm column with a 1.5 mL/minute flow rate. Solvent A was 1.5 mL TFA in 4 L water, and solvent B was 0.75 mL TFA in 4 L acetonitrile. The gradient consisted of 5 - 95 % solvent B over 0.7 minutes, and holding at 95% for 0.4 minutes. LC column temperature was 50°C. UV absorbance was collected from 220 nm to 254 nm.

*Method N:* Experiments were performed on a HPLC column coupled with a mass spectrometer using ESI as an ionization source. The LC separation was using MK RP18e, 25 x 2 mm column with a 1.5 mL/minute flow rate. Solvent A was 1.5 mL TFA in 4 L water, and solvent B was 0.75 mL TFA in 4 L acetonitrile. The gradient consisted of 10 - 80% solvent B over 7 minutes, and holding at 95% for 0.4 minutes. LC column temperature was 50°C. UV absorbance was collected from 220 nm to 254 nm.

*Method O*: Experiments were performed on a HPLC column coupled with a mass spectrometer using ESI as an ionization source. The LC separation was using MK RP18e, 25 x 2 mm column with a 1.5 mL/minute flow rate. Solvent A was 1.5 mL TFA in 4 L water, and solvent B was 0.75 mL TFA in 4 L acetonitrile. The gradient consisted of 0 - 60% solvent B over 7 minutes, and holding at 95% for 0.4 minutes. LC column temperature was 50°C. UV absorbance was collected from 220 nm to 254 nm.

*Method P:* Experiments performed on SHIMADZU 2020 HPLC with SHIMADZU MSD mass spectrometer using ESI as ionization source using Shim-Pack XR-ODS C18 50 x 3.0 mm 2.2µm column and a 1.2 ml / minute flow rate. The solvent A is water with 0.05% TFA and solvent B is acetonitrile with 0.05% TFA. The gradient consisted with 5 - 95% solvent B over 2.0 minutes, hold 95% B for 0.7 minutes. LC column temperature is 40 °C. UV absorbance was collected from 190 nm to 400 nm.

*Method Q:* Experiments performed on SHIMADZU 2020 HPLC with SHIMADZU MSD mass spectrometer using ESI as ionization source using Shim-Pack XR-ODS C18 50 x 3.0 mm 2.2µm column and a 1.2 ml / minute flow rate. The solvent A is water with 0.05% TFA and solvent B is acetonitrile with 0.05% TFA. The gradient consisted with 5 - 60% solvent B over 3.2 minutes, 60 - 100% solvent B over 0.5 minutes, hold 100% B for 0.8 minutes. LC column temperature is 40 °C. UV absorbance was collected from 190 nm to 400 nm.

*Method R:* Experiments performed on SHIMADZU 2020 HPLC with SHIMADZU MSD mass spectrometer using ESI as ionization source using Shim-Pack XR-ODS C18 50 x 3.0 mm 2.2µm column and a 1.2 ml / minute flow rate. The solvent A is water with 0.05% TFA and solvent B is acetonitrile with 0.05% TFA. The gradient consisted with 20 - 60% solvent B over 3.6 minutes, 60 - 100% solvent B over 0.4 minutes, hold 100% B for 0.5 minutes. LC column temperature is 40 °C. UV absorbance was collected from 190 nm to 400 nm.

*Method S:* Experiments performed on Shimadzu LCMS-2020. The LC separation was using Ascentis Express C18, 100 x 4.6 mm column with a 1.5 ml / minute flow rate. Solvent A is water with 0.05% TFA and solvent B is ACN/0.05%TFA. The gradient consisted with 5% B hold 0.8 min, 5 - 40% solvent B over 7.2 minutes, 40 - 95% solvent B over 2.0 minutes and hold 95% B for 2.0 minutes. LC column temperature is 60 °C. UV absorbance was collected from 190 nm to 400 nm.

*Method T:* Experiments performed on Shimadzu LCMS-2020. The LC separation was using Ascentis Express C18, 100 x 4.6 mm column with a 1.5 ml / minute flow rate. Solvent A is water with 0.05% TFA and solvent B is ACN/0.05%TFA. The gradient consisted with 10 - 60% solvent B over 10 minutes, 60 - 95% solvent B over 1.0 minutes and hold 95% B for 2.0 minutes. LC column temperature is 60 °C. UV absorbance was collected from 190 nm to 400 nm.

*Method U:* Experiments performed on Shimadzu LCMS-2020. The LC separation was using Ascentis Express C18, 100 x 4.6 mm column with a 1.0 ml / minute flow rate. Solvent A is water with 0.05% TFA and solvent B is ACN/0.05%TFA. The gradient consisted with 10 - 60% solvent B over 10 minutes, 60 - 95% solvent B over 2.0 minutes and hold 95% B for 2.0 minutes. LC column temperature is 60 °C. UV absorbance was collected from 190 nm to 400 nm.

*Method V:* Experiments performed on Shimadzu LCMS-2020. The LC separation was using Ascentis Express C18, 100 x 4.6 mm column with a 1.0 ml / minute flow rate. Solvent A is water with 0.05% TFA and solvent B is ACN/0.05%TFA. The gradient consisted with 5 - 95% solvent B over 8 minutes, hold 95% B for 2.0 minutes. LC column temperature is 60 °C. UV absorbance was collected from 190 nm to 400 nm.

*Method W:* Experiments performed on Shimadzu 2020 HPLC with Shimadzu MSD mass spectrometer using ESI as ionization source using Poroshell HPH-C18 50 x 3.0 mm column and a 1.2mL/minute flow rate. The solvent A is water with 0.05% NH₄HCO₃ and solvent B is acetonitrile. The gradient consisted with 10 - 95% solvent B over 2.0 minutes and hold 95% B for 0.7 minutes. LC column temperature is 40 °C. UV absorbance was collected from 190 nm to 400 nm.

*Method X:* Experiments performed on Shimadzu 2020 HPLC with Shimadzu MSD mass spectrometer using ESI as ionization source using Poroshell HPH-C18 50 x 3.0 mm column and a 1.2mL/minute flow rate. The solvent A is water with 0.05% NH₄HCO₃ and solvent B is acetonitrile. The gradient consisted with 10 - 70% solvent B over 3.5 minutes, 70 - 95% solvent B over 0.5 minutes and hold 95% B for 0.7 minutes. LC column temperature is 40 °C. UV absorbance was collected from 190 nm to 400 nm.

*Method Y:* Experiments performed on Shimadzu 2020 HPLC with Shimadzu MSD mass spectrometer using ESI as ionization source using Poroshell HPH-C18 50 x 3.0 mm column and a 1.2mL/minute flow rate. The solvent A is water with 0.05% NH₄HCO₃ and solvent B is acetonitrile. The gradient consisted with 30 - 70% solvent B over 4.0 minutes, 70 - 95% solvent B over 0.5 minutes and hold 95% B for 0.3 minutes. LC column temperature is 40 °C. UV absorbance was collected from 190 nm to 400 nm.

*Method Z:* Experiments performed on Shimadzu 2020 HPLC with Shimadzu MSD mass spectrometer using ESI as ionization source using Poroshell HPH-C18 50 x 3.0 mm column and a 1.2mL/minute flow rate. The solvent A is water with 0.05% NH₄HCO₃ and solvent B is acetonitrile. The gradient consisted with 30 - 95% solvent B over 4.0 minutes and hold 95% B for 0.7 minutes. LC column temperature is 40 °C. UV absorbance was collected from 190 nm to 400 nm.

*Method AA:* Experiments performed on SHIMADZU 2020 HPLC with SHIMADZU MSD mass spectrometer using ESI as ionization source using Accucore C18 50 x 2.1 mm column and a 1.0 ml / minute flow rate. The solvent A is water with 0.1% FA and solvent B is acetonitrile with 0.1% FA. The gradient consisted with 10 - 95% solvent B over 3.0 minutes and hold 95% B for 0.7 minutes. LC column temperature is 40 °C. UV absorbance was collected from 190 nm to 400 nm.

*Method BB:* Experiments performed on SHIMADZU 2020 HPLC with SHIMADZU MSD mass spectrometer using ESI as ionization source using Accucore C18 50 x 2.1 mm column and a 1.0 ml / minute flow rate. The solvent A is water with 0.1% FA and solvent B is acetonitrile with 0.1% FA. The gradient consisted with 10 - 50% solvent B over 3.5, 50 - 95% solvent B over 0.5 minutes and hold 95% B for 0.7 minutes. LC column temperature is 40 °C. UV absorbance was collected from 190 nm to 400 nm.

*Method CC:* Experiments performed on Shimadzu LCMS-2020 coupled with SHIMADZU MSD mass spectrometer using ESI as ionization source. The LC separation was using Shim-pack XR-ODS, 50 x 3.0 mm column with a 1.2 ml / minute flow rate. Solvent A is water with 0.05% TFA and solvent B is acetonitrile with 0.05% TFA. The gradient consisted with 5 - 50% solvent B over 3.5 minutes, 50 - 100% solvent B over 0.2 minutes and hold 100% B for 1.0 minutes. LC column temperature is 40 °C. UV absorbance was collected from 190 nm to 400 nm.

*Method DD:* Experiments performed on Shimadzu LCMS-2020 coupled with SHIMADZU MSD mass spectrometer using ESI as ionization source. The LC separation was using Shim-pack XR-ODS, 50 x 3.0 mm column with a 1.2 ml / minute flow rate. Solvent A is water with 0.05% TFA and solvent B is acetonitrile with 0.05% TFA. The gradient consisted with 5 - 95% solvent B over 2.0 minutes and hold 95% B for 0.7 minutes. LC column temperature is 40 °C. UV absorbance was collected from 190 nm to 400 nm.

*Method EE:* Experiments performed on SHIMADZU 2020 HPLC with SHIMADZU MSD mass spectrometer using ESI as ionization source using Ascentis Express C18 50 x 2.1 mm column and a 1.2 ml / minute flow rate. Solvent A is water with 0.05% TFA and solvent B is MeOH. The gradient consisted with 30 - 85% solvent B over 10 minutes and hold 80% B for 3.2 minutes. LC column temperature is 40 °C. UV absorbance was collected from 190 nm to 400 nm.

*Method FF:* Experiments performed on MK RP18e 25-2 mm column with mass spectrometer using ESI as ionization source. Solvent A was 1.5 mL / 4 L of TFA in water and solvent B was 0.75 mL / 4 L of TFA in acetonitrile. The gradient consisted of 5 - 95% solvent B over 0.7 minutes, and holding at 95% for 0.4 minutes at a flow rate of 1.5 mL/min. LC column temperature was 50°C. UV absorbance was collected at 220 nm and 254 nm.

*Method GG:* Experiments performed on Xtimate C18 2.1*30 mm, 3 µm column, with mass spectrometer using ESI as ionization source. Solvent A was 1.5 mL / 4 L of TFA in water, and solvent B was 0.75 mL / 4 L of TFA in acetonitrile. The gradient consisted of 10 - 80% solvent B over 6 minutes, holding at 80% for 0.5 minutes at a flow rate of 0.8 mL/min. LC column temperature was 50°C. UV absorbance was collected at 220 nm and 254 nm.

### SFC Methods

*Method 1:* Column: Chiralpak AD-3 150 x4.6 mm I.D., 3 um; Mobile phase: A: CO₂; B: ethanol (0.05% DEA); Gradient: from 5% to 40% of B in 5 minutes and from 40% to 5% of B in 0.5 minutes hold 5% of B for 1.5 minutes; Flow rate: 2.5 mL/minute; Column temperature: 35 °C; ABPR: 1500 psi.

*Method 2:* Column: Chiralcel OD-3 100×4.6 mm I.D., 3um; Mobile phase: A: CO₂; B: ethanol (0.05% DEA); Gradient: from 5% to 40% of B in 4.5 minutes and hold 40% for 2.5 minutes, then 5% of B for 1 minute; Flow rate: 2.8 mL/minute; Column temperature: 40 °C.

*Method 3:* Column: Chiralcel OJ-3 100×4.6 mm I.D., 3 um; Mobile phase: A: CO₂; B: methanol (0.05% DEA); Gradient: from 5% to 40% of B in 4.5 minutes and hold 40% for 0.5 minutes, then 5% of B for 1 minute; Flow rate: 2.8 mL/minute; Column temperature: 40 °C.

*Method 4:* Column: ChiralCel OJ-H 150×4.6 mm I.D., 5um; Mobile phase: A: CO₂; B: ethanol (0.05% DEA); Gradient: from 5% to 40% of B in 5.5 minutes, then 5% of B for 1.5 minutes; Flow rate: 2.5 mL/minute; Column temperature: 40 °C.

*Method 5:* Column: Chiralcel OJ-H 150*4.6mm I.D., 5 um; Mobile phase: A: CO₂; B: ethanol (0.05% DEA); Gradient: hold 5% for 0.5 minutes, then from 5% to 40% of B in 3.5 minutes and hold 40% for 2.5 minutes, then 5% of B for 1.5 minutes; Flow rate: 3 mL/minute; Column temperature: 40 °C.

*Method 6:* Column: Chiralpak AD-3 150×4.6 mm I.D., 3um; Mobile phase: A: CO₂; B: iso-propanol (0.05% DEA); Gradient: from 5% to 40% of B in 5 mininutes and hold 40% for 2.5 minutes, then 5% of B for 2.5 minutes; Flow rate: 2.5 mL/minute; Column temperature: 35 °C; ABPR: 1500 psi.

*Method* 7: Column: Chiralcel OJ-3 100×4.6 mm I.D., 3 um; Mobile phase: A: CO₂; B: ethanol (0.05% DEA); Gradient: from 5% to 40% of B in 4.5 minutes and hold 40% for 2.5 minutes, then 5% of B for 1 minute; Flow rate: 2.8 mL/minute; Column temperature: 40 °C.

The following generalized schemes are used to prepare the disclosed compounds, intermediates, and pharmaceutically acceptable salts thereof. Disclosed compounds and intermediates may be prepared using standard organic synthetic techniques and from comerically available starting materials and reagents. It will be appreciated that synthetic procedures employed in the preparation of disclosed compounds and intermediates will depend on the particular substituents present in the compound or intermediate and that various protection, deprotection, and conversion steps that are standard in organic synthesis may be required, but may not be illustrated in the following general schemes. It is also to be understood that any of the steps shown in any of the following general schemes may be used in any combination and in any order that is chemically feasible to achieve a desired intermediate or disclosed compound. Note that, in the following generalized schemes, the various moieties are as defined elsewhere herein. In the following generalized schemes and examples, 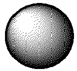 indicates a solid support-for example, a rink amide resin.

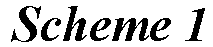

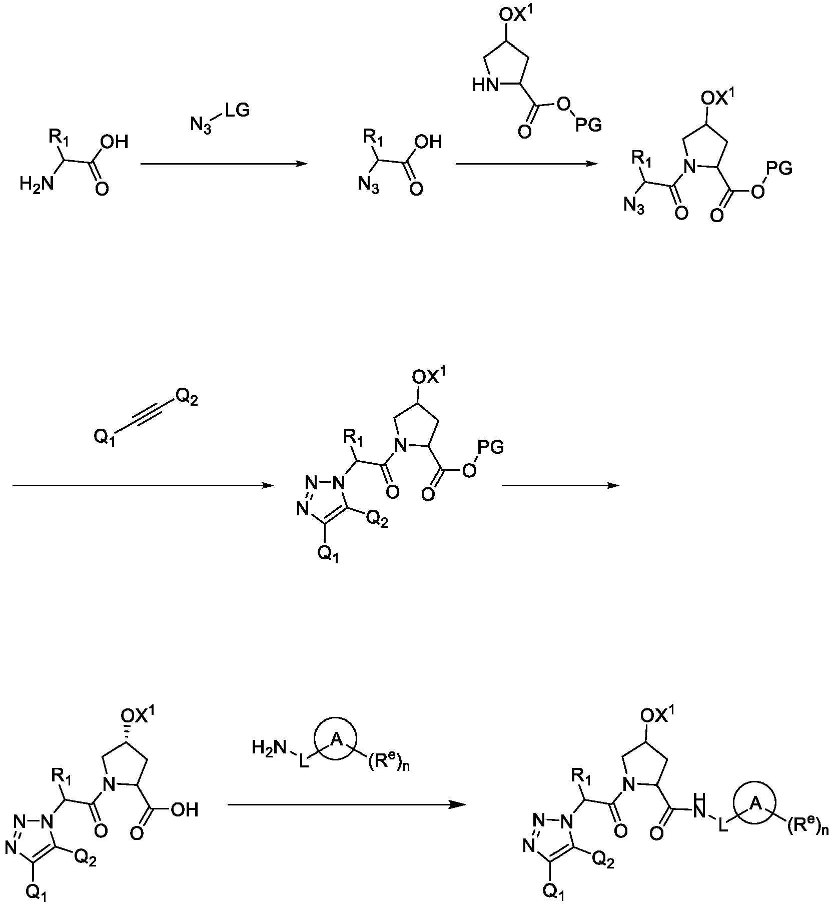

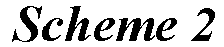

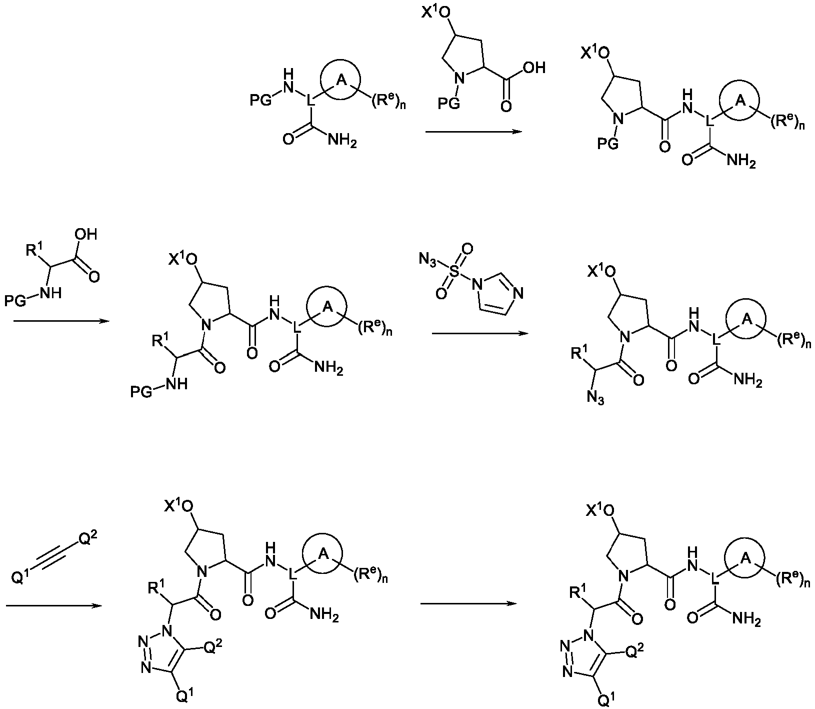

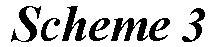

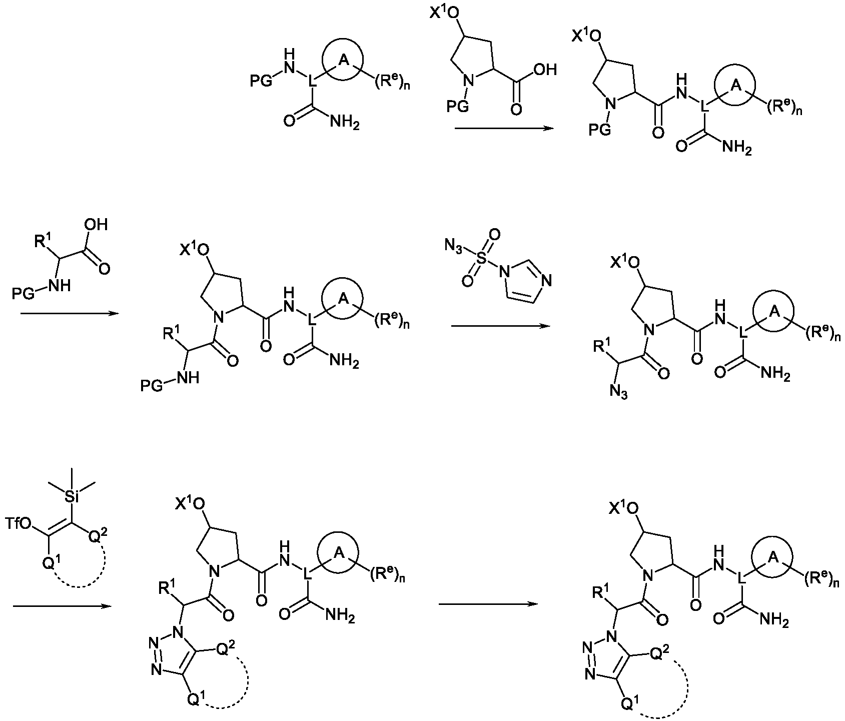

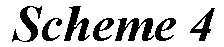

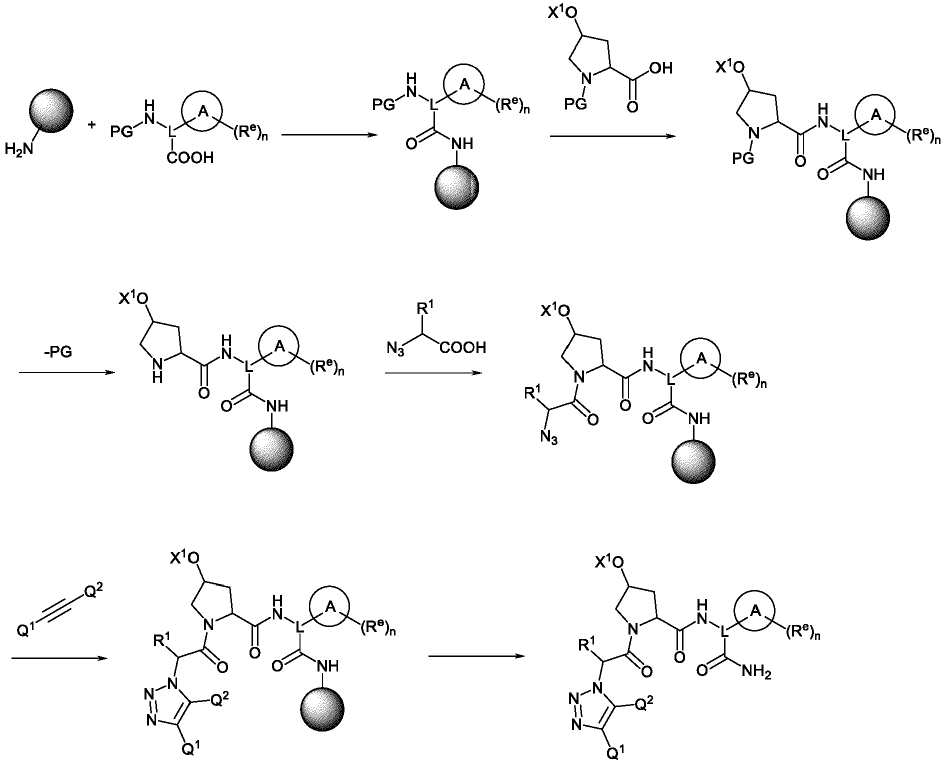

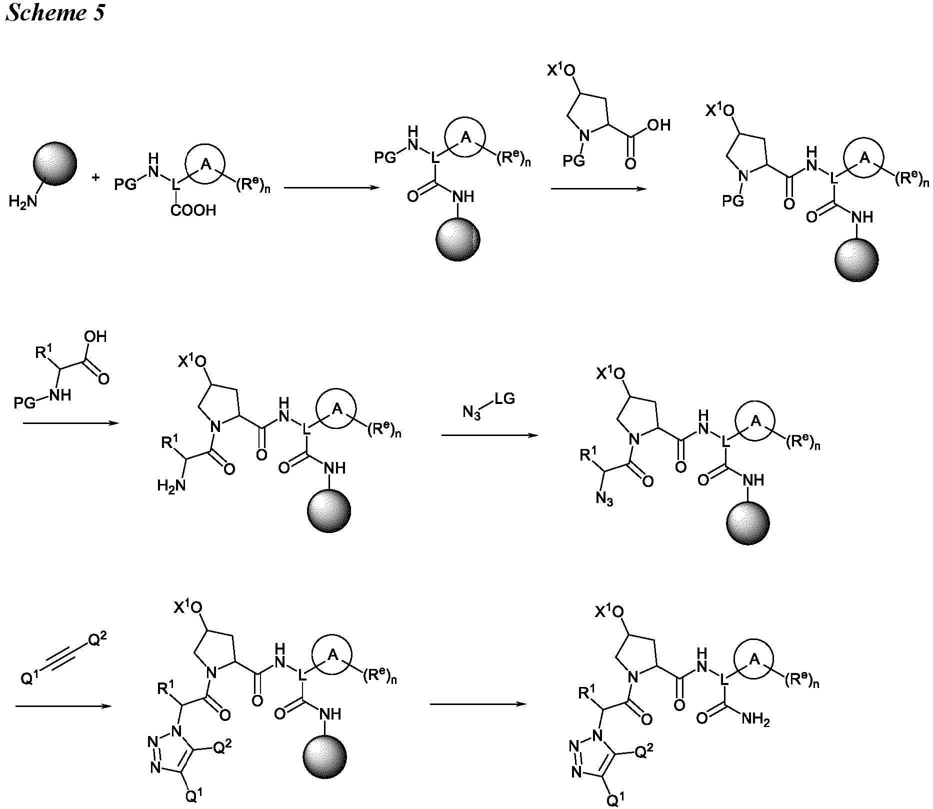

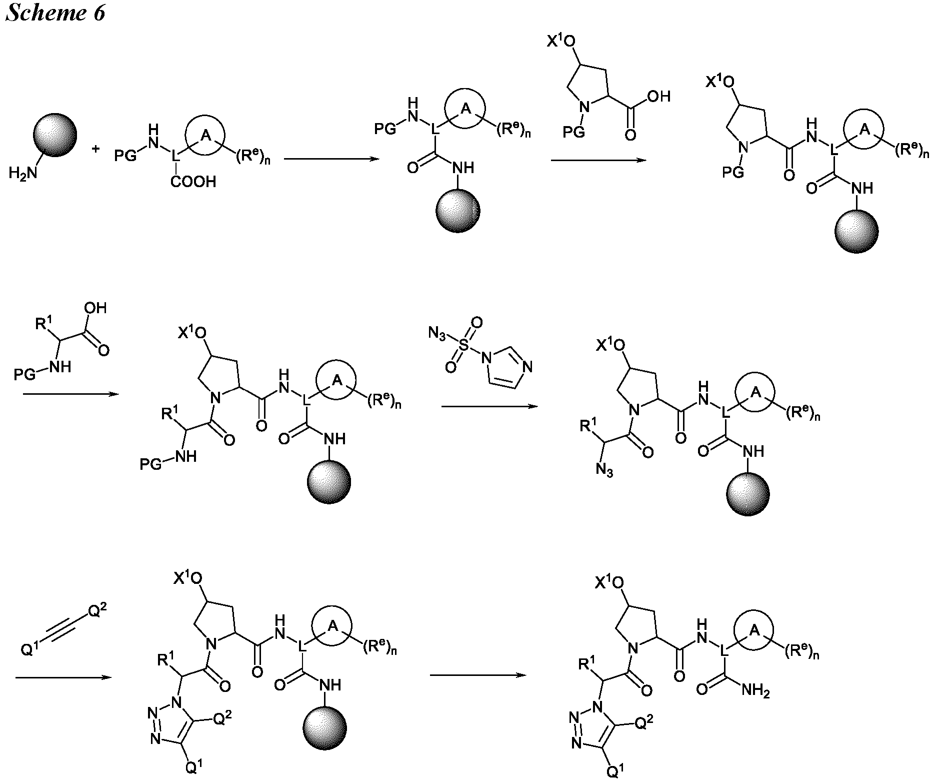

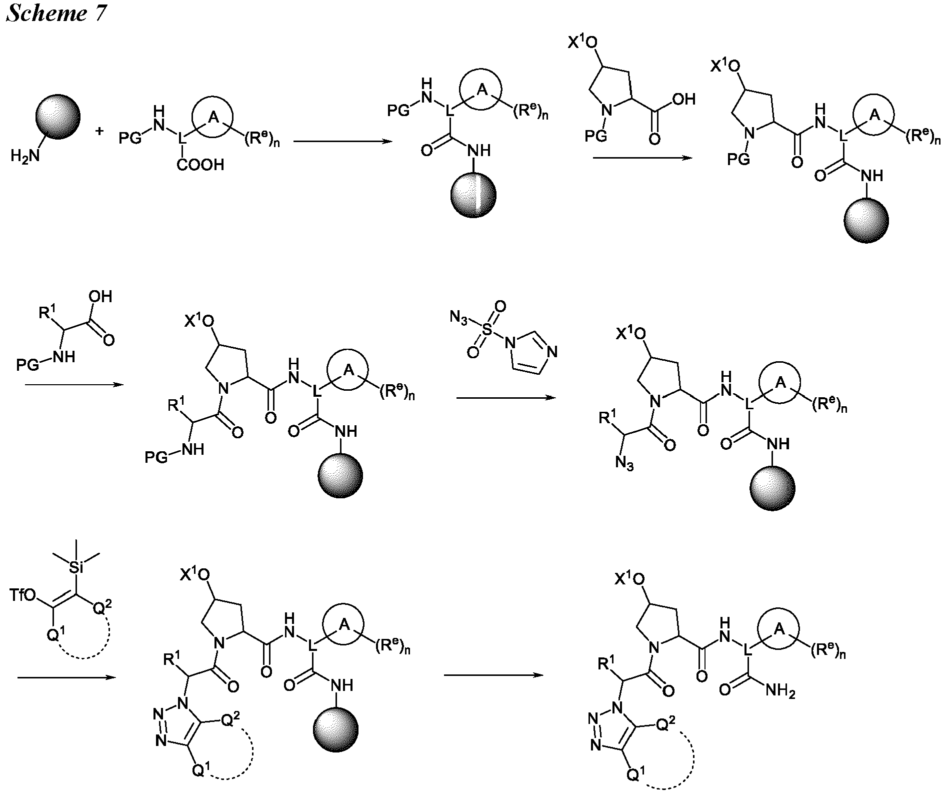

Some of the compounds used in the following examples may exists as tautomers. Although the illustrations of these compounds provided below depict only a single tautomer, these illustrations should not be viewed in a limiting sense; rather, the corresponding tautomers are also intended and embraced by the following examples, as if each and every one of the tautomers of the compound were individually depicted.

### Example S1: Synthesis of (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3-methylbutanoyl)-4-hydroxypyrrolidine-2-carboxamide (Compound 1)

Synthesis was carried out following the solid phase synthesis scheme given below:

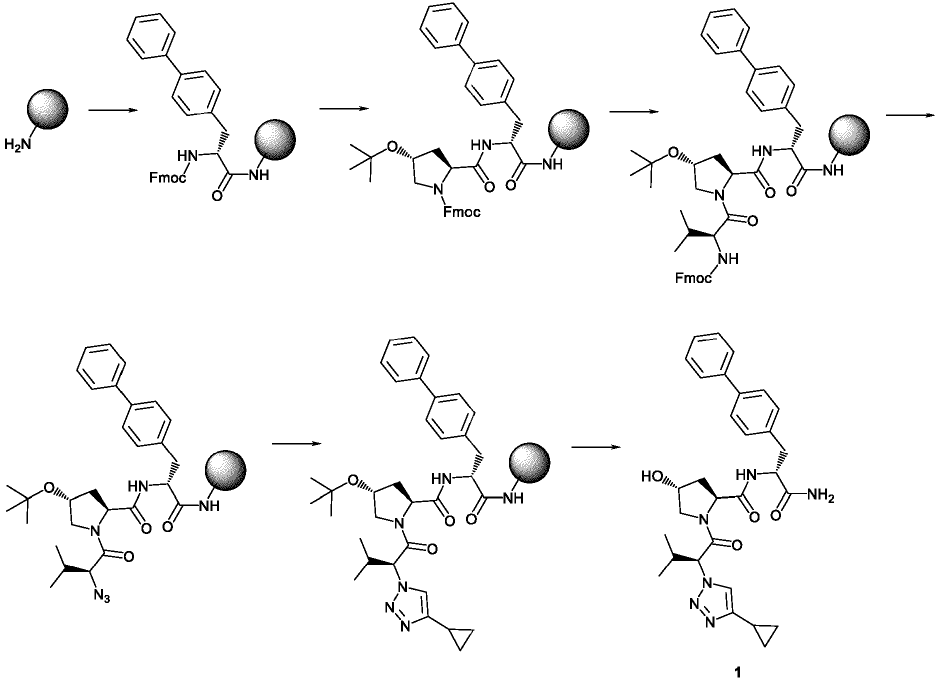

Rink Amide Resin (0.100 mmol) was added to to a plastic peptide synthesis vessel. 10 mL N,N-Dimethylformamide was added and the resin was allowed to swell for 30 min under nitrogen. The resin was drained under vacuum. 10 mL 20% 4-methylpiperidine in N,N-Dimethylformamide were drawn into reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added, then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide were added and then the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (S)-2-azido-3-methylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added, and the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was added into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 545.3, found: 545.3.

¹H NMR (400 MHz, DMSO-*d6*) δ 8.47 (d, J = 8.8 Hz, 1H), 7.83 (s, 1H), 7.70 - 7.59 (m, 2H), 7.56 (dd, J = 8.3, 2.1 Hz, 2H), 7.50 - 7.40 (m, 2H), 7.39 - 7.30 (m, 1H), 7.33 - 7.25 (m, 3H), 7.25 - 7.19 (m, 1H), 5.15 (dd, J = 10.3, 7.7 Hz, 1H), 4.45 (ddd, J = 10.6, 8.8, 4.0 Hz, 1H), 4.39 - 4.27 (m, 1H), 4.23 (dp, J = 4.3, 2.0 Hz, 1H), 3.70 (td, J = 9.9, 8.9, 4.2 Hz, 1H), 3.23 - 3.07 (m, 1H), 2.75 (dd, J = 13.9, 10.5 Hz, 1H), 2.42 - 2.28 (m, 1H), 1.94 (tt, J = 8.4, 5.0 Hz, 1H), 1.87 - 1.76 (m, 1H), 1.54 (ddd, J = 13.1, 8.8, 4.5 Hz, 1H), 0.98 (dd, J = 14.1, 6.6 Hz, 3H), 0.93 - 0.75 (m, 2H), 0.77 - 0.64 (m, 2H), 0.62 (dd, J = 6.6, 3.2 Hz, 3H).

### Example S2: Synthesis of (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)propanoyl)-4-hydroxypyrrolidine-2-carboxamide (Compound 2)

Synthesis was carried out following the solid phase synthesis scheme given below:

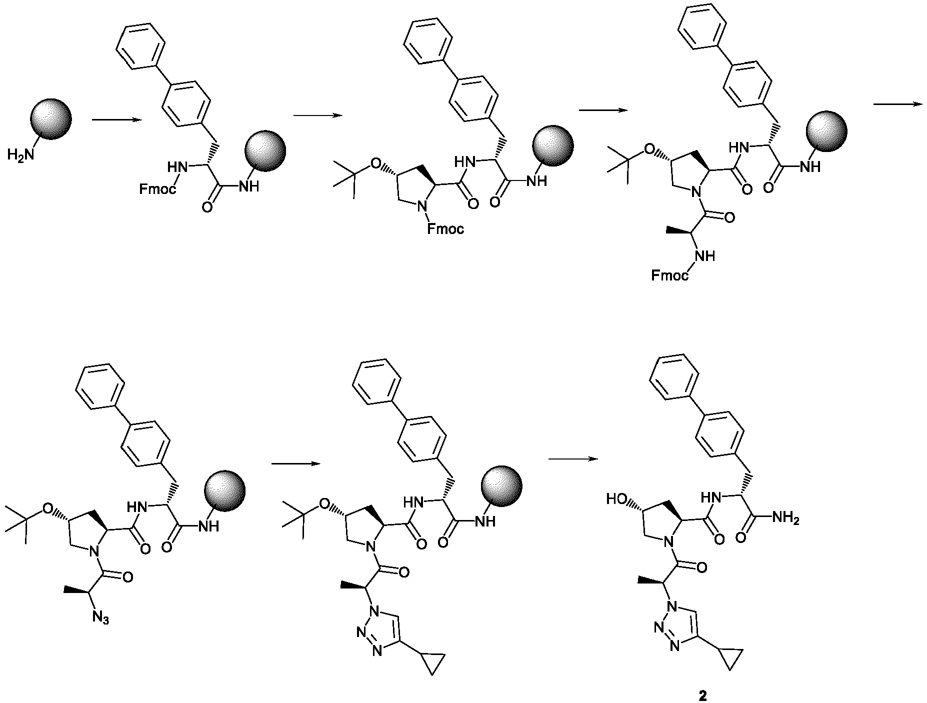

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added, and the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added, and the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (((9H-fluoren-9-yl)methoxy)carbonyl)-L-alanine (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel, and reacted under nitrogen for 1 hr to convert amine to azide. The solvent was drawn under vacuum, and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) were drawn into the reaction vessel and reacted overnight under nitrogen. The resin with washed 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was drained under vacuum. The remaining residue was mixed with 50 mL cold ether (-20 °C) to precipitate the compound. The precipitate was collected and separated by reverse phase HPLC (30%-60% Acetonitrile). The The desired product was freeze dried and characterized by LC-MS. ESI-MS: m/z [M+H]⁺ calculated: 517.3, found: 517.3.

¹H NMR (400 MHz, MeOH-*d*₄) δ 8.69 (d, *J =* 8.7 Hz, 1H), 7.75 (s, 1H), 7.66 - 7.51 (m, 4H), 7.46 - 7.37 (m, 2H), 7.37 - 7.27 (m, 3H), 5.67 (q, *J=* 7.1 Hz, 1H), 4.74 - 4.61 (m, 1H), 4.46 - 4.40 (m, 1H), 4.39 (dd, *J =* 6.1, 3.9 Hz, 1H), 3.76 (dd, *J =* 10.9, 4.1 Hz, 1H), 3.68 - 3.60 (m, 1H), 3.49 - 3.40 (m, 2H), 2.83 (dd, *J* = 14.2, 11.0 Hz, 1H), 1.94 (tt, *J =* 8.6, 5.2 Hz, 2H), 1.78 - 1.69 (m, 1H), 1.67 (d, *J =* 7.1 Hz, 3H), 0.99 - 0.88 (m, 2H), 0.82 - 0.71 (m, 2H).

### Example S3: Synthesis of (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3,3-dimethylbutanoyl)-4-hydroxypyrrolidine-2-carboxamide (Compound 3)

Synthesis was carried out following the solid phase synthesis scheme given below:

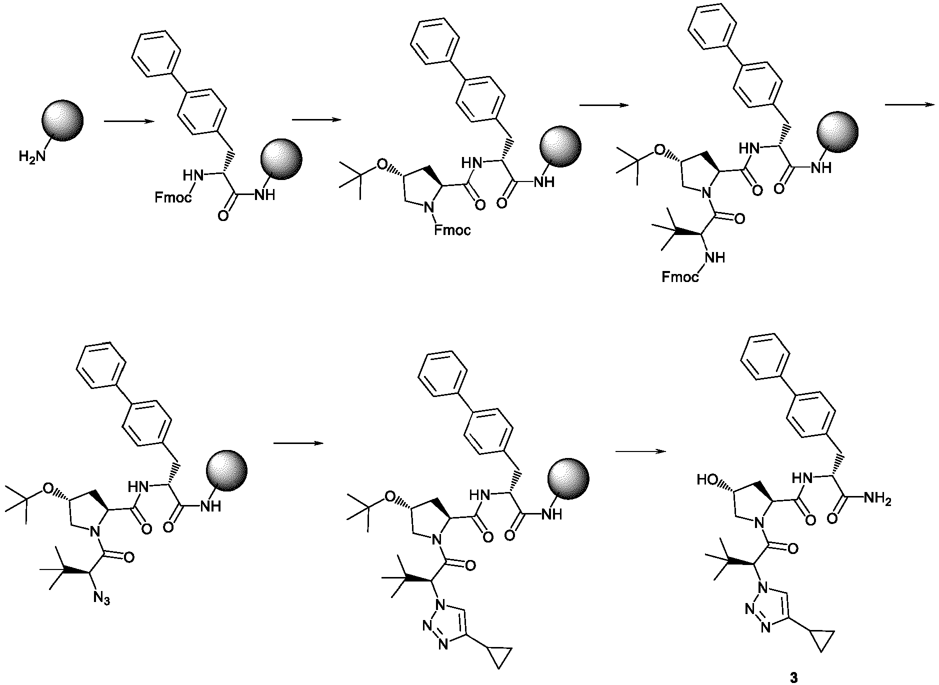

Rink Amide Resin (0.100 mmol) was added to a plastic peptide synthesis vessel. 10 mL of N,N-Dimethylformamide were added and the resin was allowed to swell for 30 min under nitrogen. The resin was drained under vacuum. 10 mL 20% 4-methylpiperidine in N,N-Dimethylformamide were drawn into the reaction vessel and reacted under nitrogen for 15 min to deprotect Fmoc group. The solvent was drained nder vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added and then the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tertbutoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into to the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 559.3, found: 559.3.

¹H NMR (400 MHz, MeOH-*d*₄) δ 8.78 (d, *J =* 8.8 Hz, 1H), 7.97 - 7.88 (m, 1H), 7.63 - 7.47 (m, 4H), 7.45 - 7.38 (m, 2H), 7.38 - 7.22 (m, 3H), 5.40 (s, 1H), 4.72 - 4.61 (m, 1H), 4.52 - 4.34 (m, 2H), 3.81 (dd, *J* = 11.0, 3.8 Hz, 1H), 3.72 - 3.65 (m, 1H), 3.51 - 3.38 (m, 1H), 2.84 (dd, *J =* 14.3, 11.2 Hz, 1H), 2.01 - 1.86 (m, 2H), 1.80 - 1.68 (m, 1H), 1.02 (s, 9H), 0.99 - 0.92 (m, 2H), 0.81 - 0.71 (m, 2H).

### Example S4: Synthesis of (2S,4R)-N-((R)-3-([1,1'-biphenyl]-4-yl)-1-amino-1-oxopropan-2-yl)-1-((S)-2-cyclobutyl-2-(4-cyclopropyl-1H-1,2,3-triazol-1-yl)acetyl)-4-hydroxypyrrolidine-2-carboxamide (Compound 4)

Synthesis was carried out following the solid phase synthesis scheme given below:

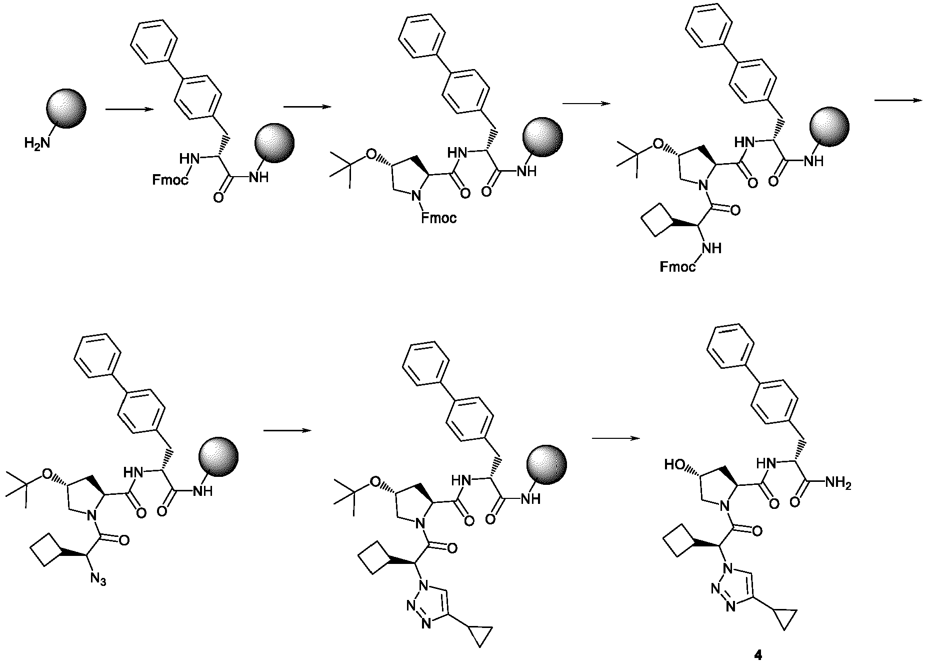

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect the Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (S)-2-((((9H-fluoren-9-yl)methoxy)carbonyl)amino)-2-cyclobutylacetic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and repeat the deprotection. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 557.3, found: 557.3.

¹H NMR (400 MHz, MeOH-*d*₄) δ 8.68 (d, *J =* 8.7 Hz, 1H), 7.74 (s, 1H), 7.61 - 7.51 (m, 4H), 7.45 - 7.37 (m, 2H), 7.35 - 7.26 (m, 3H), 5.52 (d, *J =* 10.0 Hz, 1H), 4.70 - 4.59 (m, 1H), 4.44 - 4.33 (m, 2H), 3.83 (dd, *J =* 11.0, 4.0 Hz, 1H), 3.66 (dt, *J =* 11.1, 1.7 Hz, 1H), 3.43 (dd, *J =* 14.3, 4.0 Hz, 2H), 3.10 (s, 1H), 2.97 - 2.79 (m, 1H), 2.10 (s, 1H), 1.98 - 1.84 (m, 6H), 1.83 - 1.76 (m, 1H), 1.72 (ddd, *J =* 13.4, 9.1, 4.4 Hz, 1H), 1.01 - 0.88 (m, 2H), 0.81 - 0.70 (m, 2H).

### Example S5: Synthesis of (2S,4R)-N-((R)-3-([1,1'-biphenyl]-4-yl)-1-amino-1-oxopropan-2-yl)-1-((2S,3S)-2-(4-cyclopropyl-1H-1,2,3-triazol-1-yl)-3-methylpentanoyl)-4-hydroxypyrrolidine-2-carboxamide (Compound 5)

Synthesis was carried out following the solid phase synthesis scheme given below:

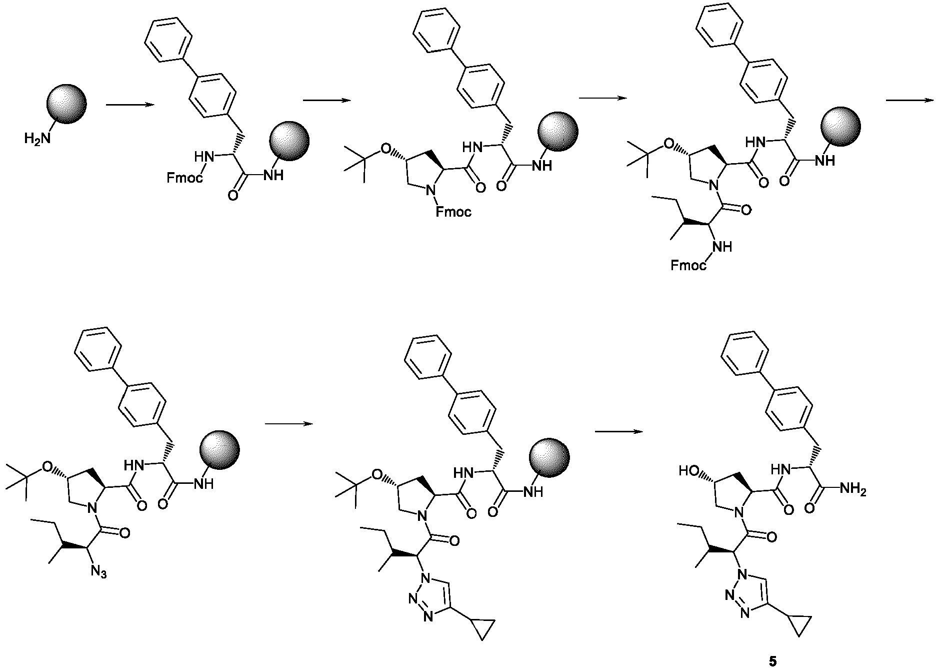

Rink Amide Resin (0.100 mmol) was added to a plastic peptide synthesis vessel. 10 mL N,N-Dimethylformamide was added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (((9H-fluoren-9-yl)methoxy)carbonyl)-L-isoleucine (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted and under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into to the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 559.3, found: 559.3.

¹H NMR (400 MHz, MeOH-*d*₄) δ 8.80 - 8.60 (m, 1H), 7.79 (s, 1H), 7.62 - 7.49 (m, 4H), 7.45 - 7.36 (m, 2H), 7.36 - 7.25 (m, 3H), 5.23 (d, *J =* 10.6 Hz, 1H), 4.70 - 4.61 (m, 1H), 4.42 - 4.34 (m, 2H), 3.83 (dd, *J =* 11.0, 3.9 Hz, 1H), 3.79 - 3.71 (m, 1H), 3.44 (dd, *J* = 14.3, 4.0 Hz, 1H), 2.84 (dd, *J* = 14.3, 11.1 Hz, 1H), 2.36 - 2.20 (m, 1H), 2.00 - 1.86 (m, 2H), 1.73 (ddd, *J =* 13.4, 9.5, 4.3 Hz, 1H), 1.02 (dd, *J =* 6.7, 3.5 Hz, 4H), 0.98 - 0.92 (m, 3H), 0.81 (t, *J =* 7.3 Hz, 3H), 0.78 - 0.71 (m, 2H).

### Example S6: Synthesis of (2S,4R)-N-((R)-3-([1,1'-biphenyl]4-yl)-1-amino-1-oxopropan-2-yl)-1-((S)-2-(4-cyclopropyl-1H-1,2,3-triazol-1-yl)-3,3-dimethylpentanoyl)-4-hydroxypyrrolidine-2-carboxamide (Compound 6)

Synthesis was carried out following the solid phase synthesis scheme given below:

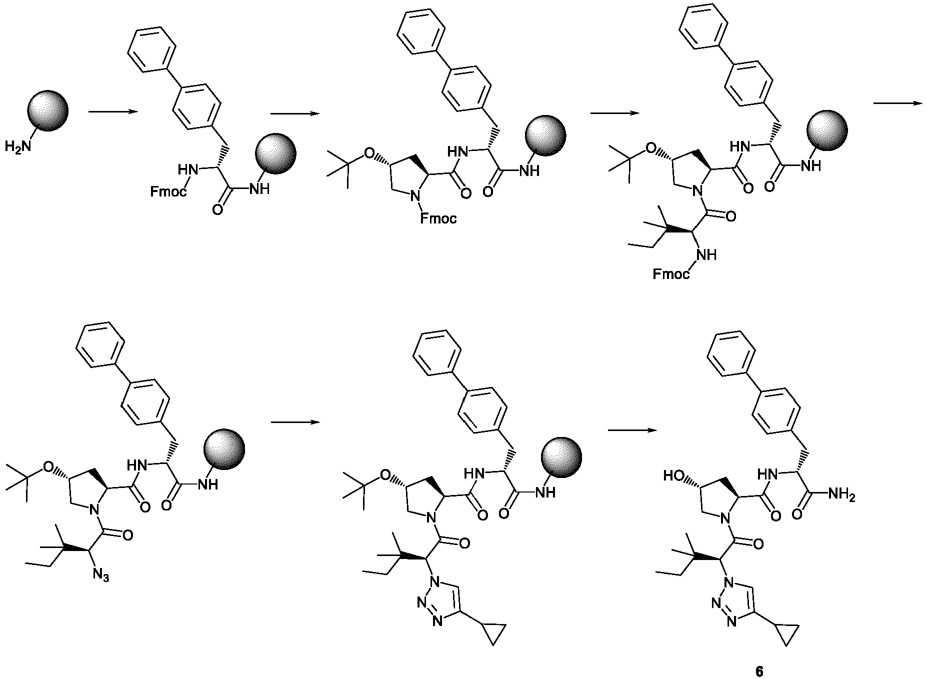

Rink Amide Resin (0.100 mmol) was added to a plastic peptide synthesis vessel. 10 mL N,N-Dimethylformamide was added and the resin was allowed to swell for 30 min under nitrogen. The resin was drained under vacuum. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-((((9H-fluoren-9-yl)methoxy)carbonyl)amino)-3,3-dimethylpentanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. RThe washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 573.3, found: 573.3.

¹H NMR (400 MHz, MeOH-*d*₄) δ 8.77 (d, *J =* 8.8 Hz, 1H), 7.93 (s, 1H), 7.61 - 7.47 (m, 4H), 7.41 (dd, *J =* 8.5, 6.8 Hz, 2H), 7.34 (s, 1H), 7.33 - 7.24 (m, 2H), 5.44 (s, 1H), 4.71 - 4.61 (m, 1H), 4.44 - 4.35 (m, 2H), 3.80 (dd, *J* = 10.9, 3.7 Hz, 1H), 3.67 (d, *J* = 11.1 Hz, 1H), 3.47 (dd, *J =* 14.2, 3.9 Hz, 1H), 2.84 (dd, *J* = 14.3, 11.2 Hz, 1H), 2.01 - 1.85 (m, 2H), 1.74 (ddd, *J =* 13.6, 9.8, 4.2 Hz, 1H), 1.27 (q, *J* = 7.4 Hz, 2H), 1.05 (s, 3H), 0.99 - 0.93 (m, 5H), 0.87 (t, *J =* 7.3 Hz, 3H), 0.76 (ddt, *J* = 7.1, 4.5, 1.3 Hz, 2H).

### Example S7: Synthesis of (2S,4R)-N-((R)-3-([1,1'-biphenyl]-4-yl)-1-amino-1-oxopropan-2-yl)-1-((S)-2-(4-cyclopropyl-1H-1,2,3-triazol-1-yl)-3,3-dimethylpent-4-enoyl)-4-hydroxypyrrolidine-2-carboxamide (Compound 7)

Synthesis was carried out following the solid phase synthesis scheme given below:

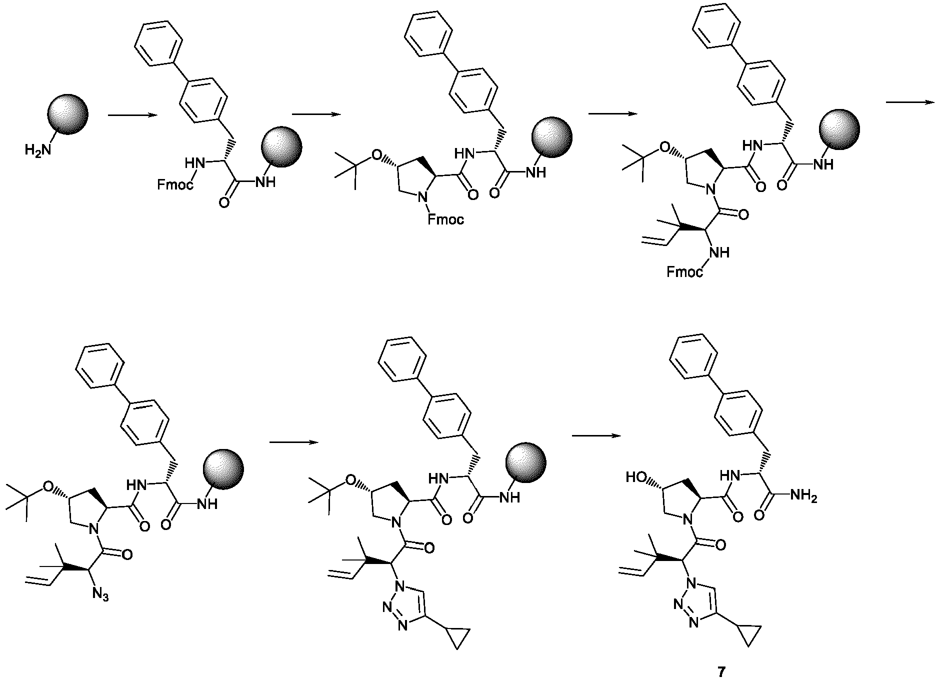

Rink Amide Resin (0.100 mmol) was added to a plastic peptide synthesis vessel. 10 mL N,N-Dimethylformamide was added and the resin was allowed to swell for 30 min under nitrogen. The resin was drained under vacuum. 10 mL of 20% 4-methylpiperidine in N,N-Dimethylformamide were drawn into the reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated for 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (S)-2-((((9H-fluoren-9-yl)methoxy)carbonyl)amino)-3,3-dimethylpent-4-enoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel, and reacted under nitrogen for 1 hr to convert amine to azide. The solvent was drained under vacuum, and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 571.3, found: 571.3.

¹H NMR (400 MHz, MeOH-*d*₄) δ 8.78 (d, *J =* 8.8 Hz, 1H), 7.87 (s, 1H), 7.56 (ddt, *J =* 16.2, 8.7, 1.8 Hz, 4H), 7.45 - 7.37 (m, 2H), 7.36 - 7.25 (m, 3H), 6.14 - 6.01 (m, 1H), 5.48 (s, 1H), 5.07 (dd, *J =* 10.8, 1.1 Hz, 1H), 4.94 (dd, *J* = 17.5, 1.1 Hz, 2H), 4.72 - 4.62 (m, 1H), 4.44 - 4.32 (m, 2H), 3.81 (dd, *J* = 11.1, 3.8 Hz, 1H), 3.69 (d, *J =* 11.1 Hz, 1H), 3.52 - 3.43 (m, 1H), 2.84 (dd, *J =* 14.3, 11.2 Hz, 1H), 1.98 - 1.86 (m, 2H), 1.74 (ddd, *J =* 13.4, 9.7, 4.2 Hz, 1H), 1.17 (s, 3H), 1.04 (s, 3H), 0.99 - 0.88 (m, 2H), 0.79 - 0.67 (m, 2H).

### Example S8: Synthesis of (2S,4R)-N-((R)-3-([1,1'-biphenyl]-4-yl)-1-amino-1-oxopropan-2-yl)-1-((2S)-2-(adamantan-1-yl)-2-(4-cyclopropyl-1H-1,2,3-triazol-1-yl)acetyl)-4-hydroxypyrrolidine-2-carboxamide (Compound 8)

Synthesis was carried out following the solid phase synthesis scheme given below:

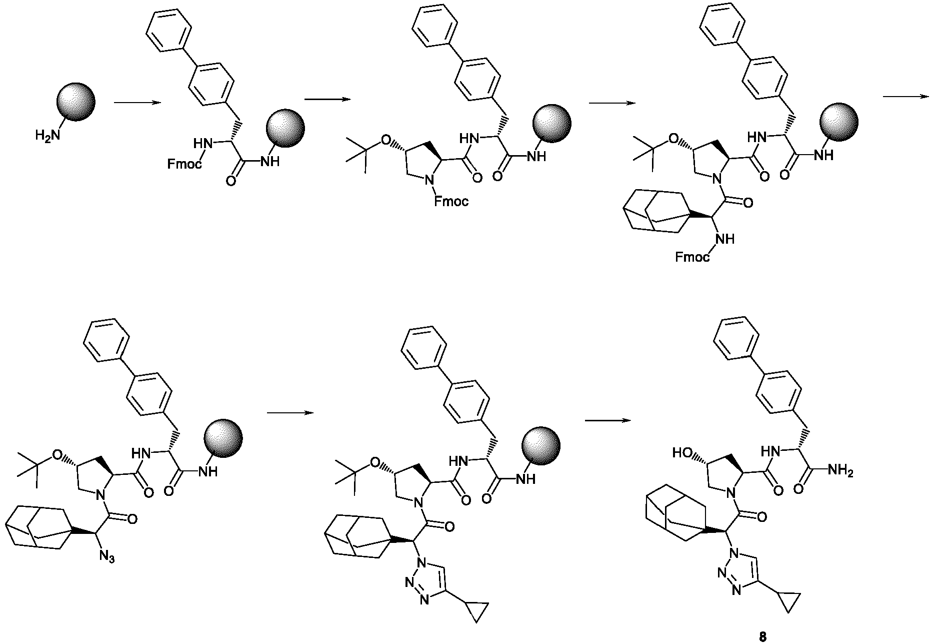

Rink Amide Resin (0.100 mmol) was added to a plastic peptide synthesis vessel. 10 mL N,N-Dimethylformamide was added and the resin was allowed to swell for 30 min under nitrogen. The resin was drained under vacuum. 10 mL of 20% 4-methylpiperidine in N,N-Dimethylformamide were drawn into the reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated for 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S)-2-((((9H-fluoren-9-yl)methoxy)carbonyl)amino)-2-(adamantan-1-yl)acetic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 637.3, found: 637.3.

¹H NMR (400 MHz, MeOH-*d*₄) δ 7.97 - 7.87 (m, 1H), 7.62 - 7.48 (m, 4H), 7.45 - 7.37 (m, 2H), 7.34 (s, 1H), 7.33 - 7.23 (m, 2H), 5.25 (s, 1H), 4.75 - 4.63 (m, 1H), 4.45 - 4.31 (m, 2H), 3.81 (dd, *J* = 11.1, 3.6 Hz, 1H), 3.74 - 3.60 (m, 1H), 3.47 (dd, *J =* 14.2, 4.0 Hz, 1H), 2.85 (dd, *J =* 14.2, 11.1 Hz, 1H), 2.01 - 1.87 (m, 5H), 1.80 - 1.65 (m, 7H), 1.59 (t, *J =* 14.7 Hz, 6H), 1.01 - 0.90 (m, 2H), 0.82 - 0.67 (m, 2H).

### Example S9: Synthesis of (2S,4R)-N-((R)-3-([1,1'-biphenyl]-4-yl)-1-amino-1-oxopropan-2-yl)-1-((S)-3,3-dimethyl-2-(1H-1,2,3-triazol-1-yl)butanoyl)-4-hydroxypyrrolidine-2-carboxamide (Compound 9)

Synthesis was carried out following the solid phase synthesis scheme given below:

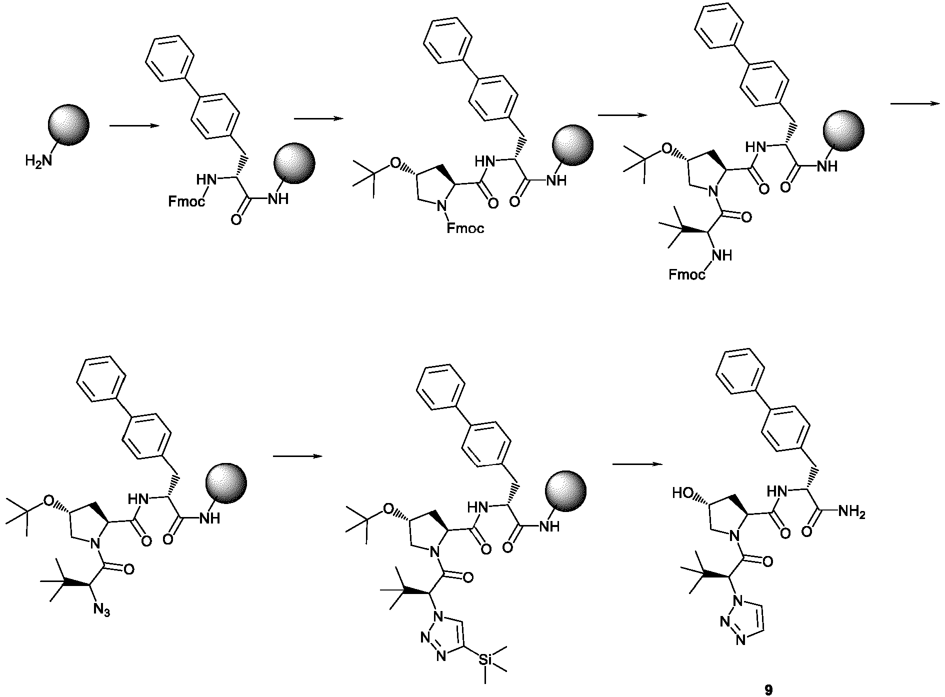

Rink Amide Resin (0.100 mmol) was added to a plastic peptide synthesis vessel. 10 mL N,N-Dimethylformamide was added and the resin was allowed to swell for 30 min under nitrogen. The resin was drained under vacuum. 10 mL of 20% 4-methylpiperidine in N,N-Dimethylformamide were drawn into the reaction vessel, and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the mixture into synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the mixture into synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum, and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynyltrimethylsilane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 519.3, found: 519.3.

¹H NMR (400 MHz, DMSO-d6) δ 8.57 (d, J = 8.8 Hz, 1H), 8.22 (d, J = 1.1 Hz, 1H), 7.77 - 7.69 (m, 1H), 7.68 - 7.60 (m, 2H), 7.60 - 7.51 (m, 2H), 7.50 - 7.40 (m, 2H), 7.34 (tt, J = 6.6, 1.3 Hz, 2H), 7.30 (dd, J = 8.5, 2.0 Hz, 3H), 5.51 (s, 1H), 4.49 - 4.31 (m, 2H), 4.24 (dq, J = 3.9, 2.0 Hz, 1H), 3.68 (dd, J = 11.1, 3.8 Hz, 1H), 3.58 (d, J = 11.1 Hz, 1H), 3.22 (dd, J = 14.0, 3.9 Hz, 1H), 2.76 (dd, J = 14.0, 10.7 Hz, 1H), 1.83 (ddt, J = 12.9, 7.4, 1.8 Hz, 1H), 1.56 (ddd, J = 13.2, 9.3, 4.3 Hz, 1H), 0.94 (s, 10H).

### Example S10: Synthesis of (2S,4R)-N-((R)-3-([1,1'-biphenyl]-4-yl)-1-amino-1-oxopropan-2-yl)-4-hydroxy-1-((S)-2-(4-(methoxymethyl)-1H-1,2,3-triazol-1-yl)-3,3-dimethylbutanoyl)pyrrolidine-2-carboxamide (Compound 10)

Synthesis was carried out following the solid phase synthesis scheme given below:

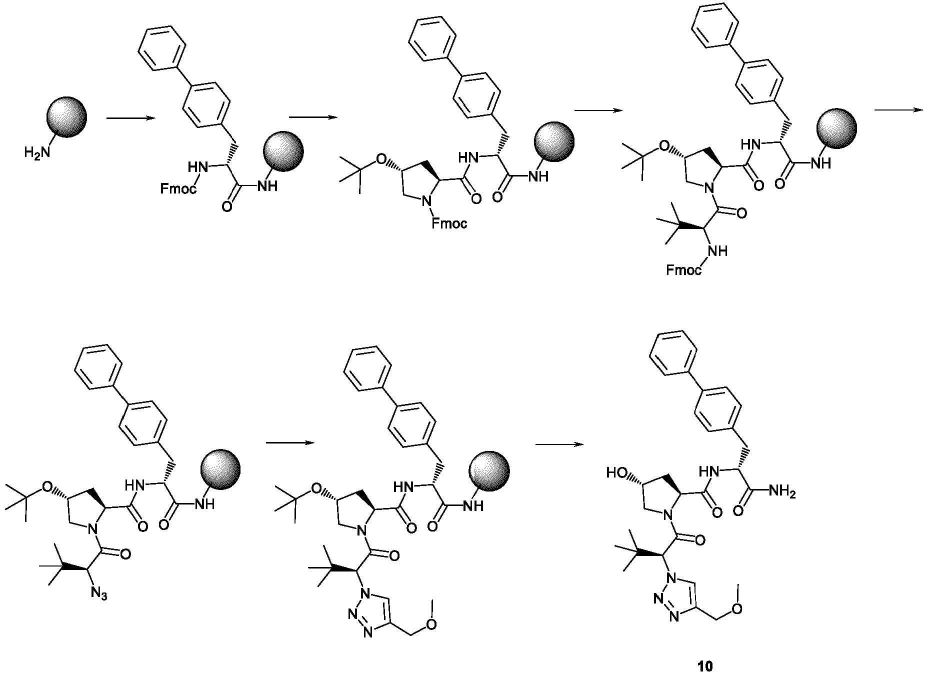

Rink Amide Resin (0.100 mmol) was added to a plastic peptide synthesis vessel. 10 mL of N,N-Dimethylformamide were added and the resin was allowed to swell for 30 min under nitrogen. The resin was drained under vacuum. 10 mL of 20% 4-methylpiperidine in N,N-Dimethylformamide was drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of 3-methoxyprop-1-yne (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 563.3, found: 563.3.

¹H NMR (400 MHz, DMSO-d6) δ 8.58 (d, J = 8.8 Hz, 1H), 8.18 (s, 1H), 7.68 - 7.59 (m, 2H), 7.59 - 7.50 (m, 2H), 7.50 - 7.40 (m, 2H), 7.39 - 7.18 (m, 5H), 5.46 (s, 1H), 4.52 - 4.38 (m, 3H), 4.43 - 4.31 (m, 1H), 4.25 (dt, J = 4.4, 2.3 Hz, 1H), 3.67 (dd, J = 11.1, 3.7 Hz, 1H), 3.59 (d, J = 11.2 Hz, 1H), 3.25 (d, J = 2.7 Hz, 4H), 2.76 (dd, J = 14.0, 10.8 Hz, 1H), 1.88 - 1.78 (m, 1H), 1.57 (ddd, J = 13.3, 9.4, 4.3 Hz, 1H), 0.95 (s, 10H).

### Example S11: Synthesis of (2S,4R)-N-((R)-3-([1,1'-biphenyl]-4-yl)-1-amino-1-oxopropan-2-yl)-1-((S)-2-(4-benzyl-1H-1,2,3-triazol-1-yl)-3,3-dimethylbutanoyl)-4-hydroxypyrrolidine-2-carboxamide (Compound 11)

Synthesis was carried out following the solid phase synthesis scheme given below:

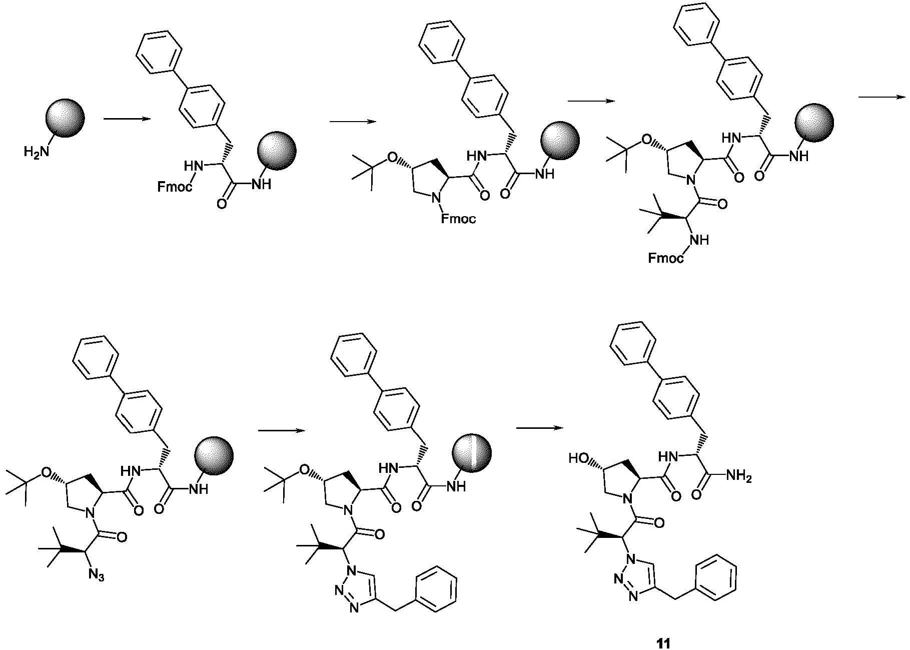

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL 20% 4-methylpiperidine in N,N-Dimethylformamide were drawn into the reaction vessel, and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide was drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of prop-2-yn-1-ylbenzene (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 609.3, found: 609.3.

¹H NMR (400 MHz, DMSO-d6) δ 8.55 (d, J = 8.8 Hz, 1H), 7.95 (s, 1H), 7.67 - 7.59 (m, 2H), 7.59 - 7.40 (m, 5H), 7.39 - 7.14 (m, 10H), 5.42 (s, 1H), 4.46 - 4.29 (m, 2H), 4.23 (dt, J = 5.5, 2.6 Hz, 1H), 4.00 (s, 2H), 3.98 (d, J = 7.2 Hz, 0H), 3.66 (dd, J = 11.0, 3.8 Hz, 1H), 3.57 (d, J = 11.1 Hz, 1H), 3.21 (dd, J = 13.9, 3.9 Hz, 1H), 2.75 (dd, J = 14.0, 10.6 Hz, 1H), 1.92 - 1.76 (m, 1H), 1.55 (ddd, J = 13.1, 9.2, 4.3 Hz, 1H), 0.92 (s, 8H), 0.92 (d, J = 7.9 Hz, 1H).

### Example S12: Synthesis of (2S,4R)-N-((R)-3-([1,1'-biphenyl]-4-yl)-1-amino-1-oxopropan-2-yl)-1-((R)-2-(4-(1-(acetamidomethyl)cyclopropyl)-1H-1,2,3-triazol-1-yl)-3,3-dimethylbutanoyl)-4-hydroxypyrrolidine-2-carboxamide (Compound 12)

Synthesis was carried out following the solid phase synthesis scheme given below:

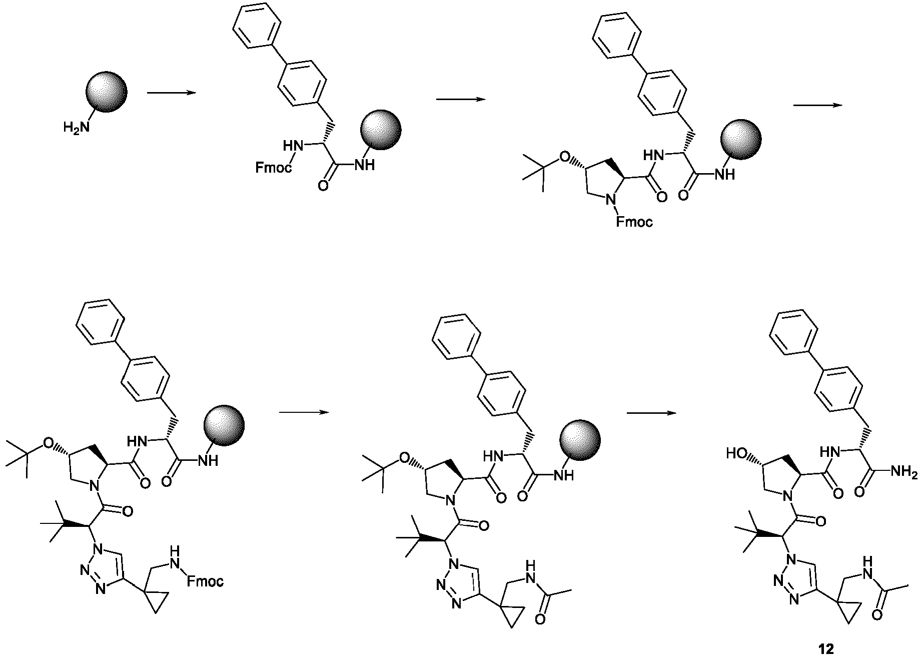

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. Add mixture of (8)-2-(4-(1-(((((9*H*-fluoren-9-yl)methoxy)carbonyl)amino)methyl)cyclopropyl)-1*H*-1,2,3-triazol-1-yl)-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of Acetic Anhydride (5.0 equiv.) and N,N-Diisopropylethylamine (10.0 equiv.) in 10 mL dichloromethane was added; then, the mixture was drawn into the synthesis vessel and reacted for 30 min under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 630.3, found: 630.3.

¹H NMR (400 MHz, DMSO-d6) δ 8.63 (d, J = 8.7 Hz, 1H), 8.00 - 7.88 (m, 1H), 7.79 (s, 1H), 7.67 - 7.58 (m, 2H), 7.61 - 7.51 (m, 2H), 7.49 - 7.16 (m, 8H), 5.37 (s, 1H), 5.09 (s, 1H), 4.42 (t, J = 7.9 Hz, 1H), 4.36 - 4.23 (m, 1H), 4.22 (dq, J = 6.5, 3.8 Hz, 1H), 3.72 - 3.59 (m, 2H), 3.27 - 3.17 (m, 2H), 3.12 (dd, J = 14.0, 4.8 Hz, 1H), 2.85 - 2.71 (m, 1H), 1.89 - 1.67 (m, 2H), 1.75 (s, 3H), 1.54 (ddd, J = 12.9, 8.4, 4.7 Hz, 1H), 1.05 - 0.97 (m, 1H), 0.95 (s, 9H), 0.94 - 0.84 (m, 4H).

### Example S13: Synthesis of (2S,4R)-N-((R)-3-([1,1'-biphenyl]-4-yl)-1-amino-1-oxopropan-2-yl)-1-((S)-2-(4-(1-(acetamidomethyl)cyclopropyl)-1H-1,2,3-triazol-1-yl)-3,3-dimethylbutanoyl)-4-hydroxypyrrolidine-2-carboxamide (Compound 13)

Synthesis was carried out following the solid phase synthesis scheme given below:

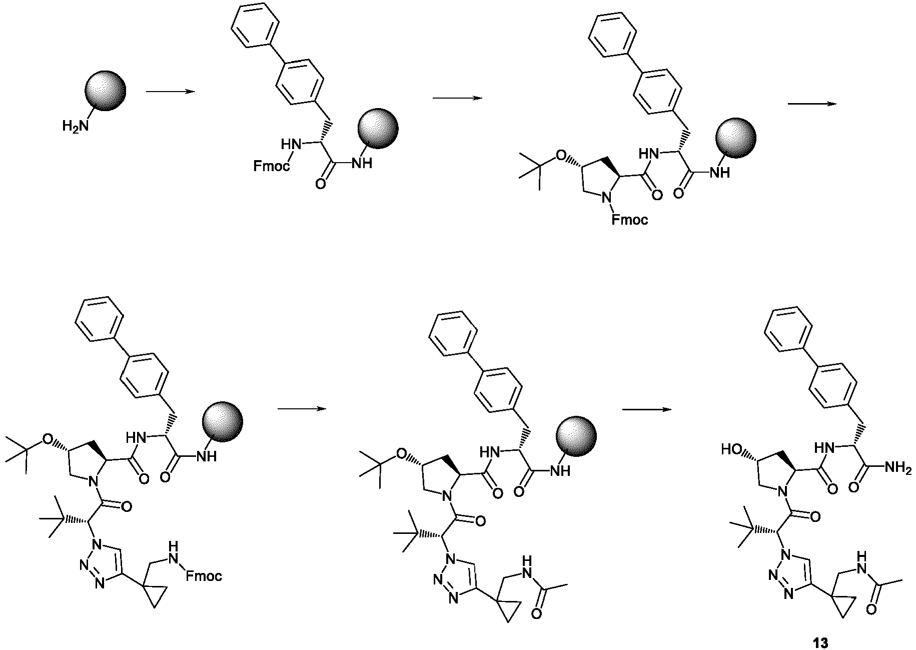

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (*R*)-2-(4-(1-(((((9*H*-fluoren-9-yl)methoxy)carbonyl)amino)methyl)cyclopropyl)-1*H*-1,2,3-triazol-1-yl)-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of Acetic Anhydride (5.0 equiv.) and N,N-Diisopropylethylamine (10.0 equiv.) in 10 mL dichloromethane was added; then, the mixture was drawn into the synthesis vessel and reacted for 30 min under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 630.3, found: 630.3.

¹H NMR (400 MHz, DMSO-d6) δ 8.54 (d, J = 8.8 Hz, 1H), 8.00 (s, 1H), 7.93 (t, J = 5.8 Hz, 1H), 7.68 - 7.51 (m, 5H), 7.45 (dd, J = 8.4, 7.0 Hz, 2H), 7.40 - 7.23 (m, 4H), 7.27 - 7.18 (m, 1H), 5.38 (s, 1H), 4.52 - 4.30 (m, 2H), 4.23 (dq, J = 4.0, 2.1 Hz, 1H), 3.66 (dd, J = 11.0, 3.8 Hz, 1H), 3.62 - 3.51 (m, 2H), 3.20 (ddd, J = 13.8, 7.3, 4.6 Hz, 2H), 2.77 (dd, J = 14.0, 10.6 Hz, 1H), 1.94 - 1.72 (m, 1H), 1.77 (s, 3H), 1.55 (ddd, J = 13.3, 9.3, 4.4 Hz, 1H), 1.02 (ddd, J = 7.3, 5.3, 2.7 Hz, 1H), 0.93 (s, 8H), 0.98 - 0.79 (m, 4H).

### Example S14: Synthesis of (2S,4R)-N-((R)-3-([1,1'-biphenyl]-4-yl)-1-amino-1-oxopropan-2-yl)-1-((S)-2-(4-((2-acetamidoethoxy)methyl)-1H-1,2,3-triazol-1-yl)-3,3-dimethylbutanoyl)-4-hydroxypyrrolidine-2-carboxamide (Compound 14)

Synthesis was carried out following the solid phase synthesis scheme given below:

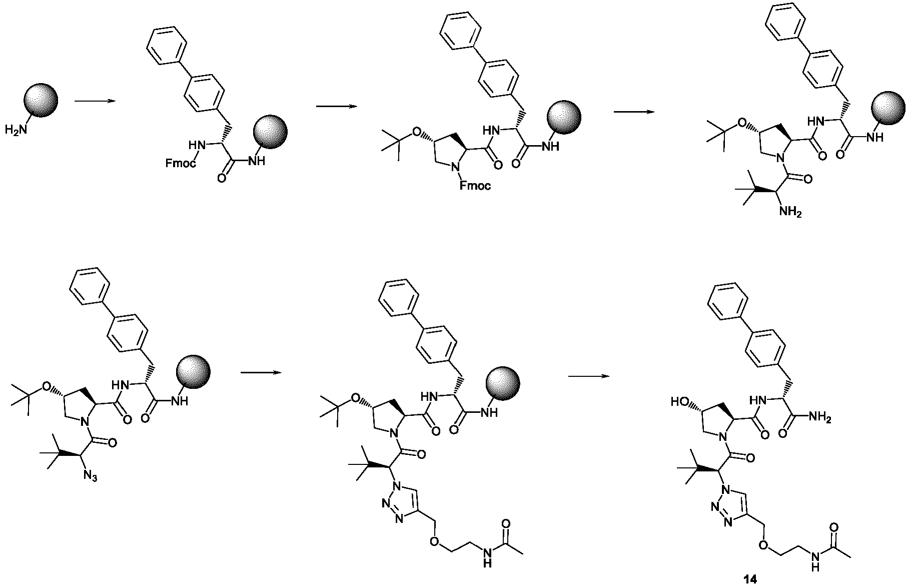

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of N-(2-(prop-2-yn-1-yloxy)ethyl)acetamide (5.0 equiv.), N,N-Diisopropylethylamine (10.0 equiv.) in 10 mL N,N-Dimethylformamide (nitrogen purged) was drawn into the reaction vessel reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 634.3, found: 634.3.

¹H NMR (400 MHz, DMSO-d6) δ 8.56 (d, J = 8.8 Hz, 1H), 8.20 (s, 1H), 7.92 (t, J = 5.8 Hz, 1H), 7.68 - 7.59 (m, 2H), 7.59 - 7.51 (m, 2H), 7.50 - 7.40 (m, 2H), 7.39 - 7.29 (m, 2H), 7.30 (dd, J = 8.6, 2.2 Hz, 3H), 6.51 (s, 0H), 5.46 (s, 1H), 5.10 (d, J = 3.5 Hz, 1H), 4.53 (s, 2H), 4.44 (ddd, J = 10.8, 8.8, 3.9 Hz, 1H), 4.36 (dd, J = 9.3, 7.4 Hz, 1H), 4.24 (s, 1H), 3.67 (dd, J = 11.1, 3.7 Hz, 1H), 3.60 (d, J = 11.2 Hz, 1H), 3.52 - 3.36 (m, 2H), 3.19 (s, 1H), 3.27 - 3.14 (m, 2H), 2.76 (dd, J = 14.0, 10.7 Hz, 1H), 1.89 - 1.76 (m, 1H), 1.79 (s, 3H), 1.56 (ddd, J = 13.2, 9.4, 4.3 Hz, 1H), 0.95 (d, J = 7.6 Hz, 2H), 0.95 (s, 8H).

### Example S15: Synthesis of 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-1H-1,2,3-triazole-4-carboxylic acid (Compound 15)

Synthesis was carried out following the solid phase synthesis scheme given below:

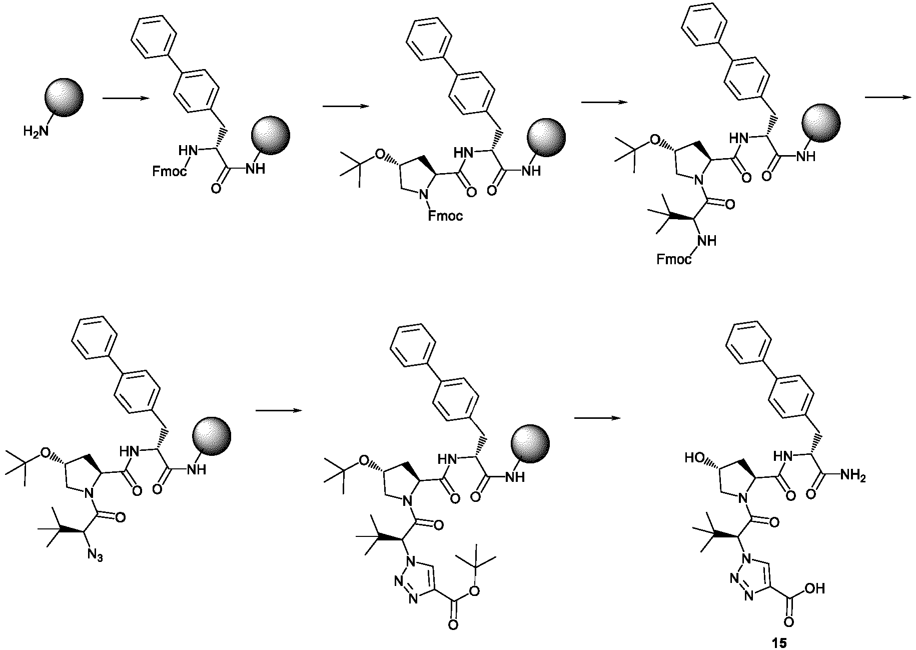

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of tert-butyl propiolate (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 563.3, found: 563.2.

¹H NMR (400 MHz, DMSO-d6) δ 13.19 (s, 1H), 8.66 - 8.58 (m, 2H), 7.68 - 7.49 (m, 4H), 7.45 (dd, J = 8.5, 6.9 Hz, 2H), 7.39 - 7.30 (m, 1H), 7.35 - 7.24 (m, 4H), 5.59 (s, 1H), 5.10 (d, J = 6.7 Hz, 1H), 4.48 - 4.33 (m, 2H), 4.25 (s, 1H), 3.66 (d, J = 3.2 Hz, 2H), 3.23 (dd, J = 14.0, 3.9 Hz, 1H), 2.76 (dd, J = 14.0, 10.8 Hz, 1H), 1.90 - 1.79 (m, 1H), 1.57 (ddd, J = 13.3, 9.4, 4.3 Hz, 1H), 0.96 (s, 9H).

### Example S16: Synthesis of 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-1H-1,2,3-triazole-4-carboxamide (Compound 16)

Synthesis was carried out following the solid phase synthesis scheme given below:

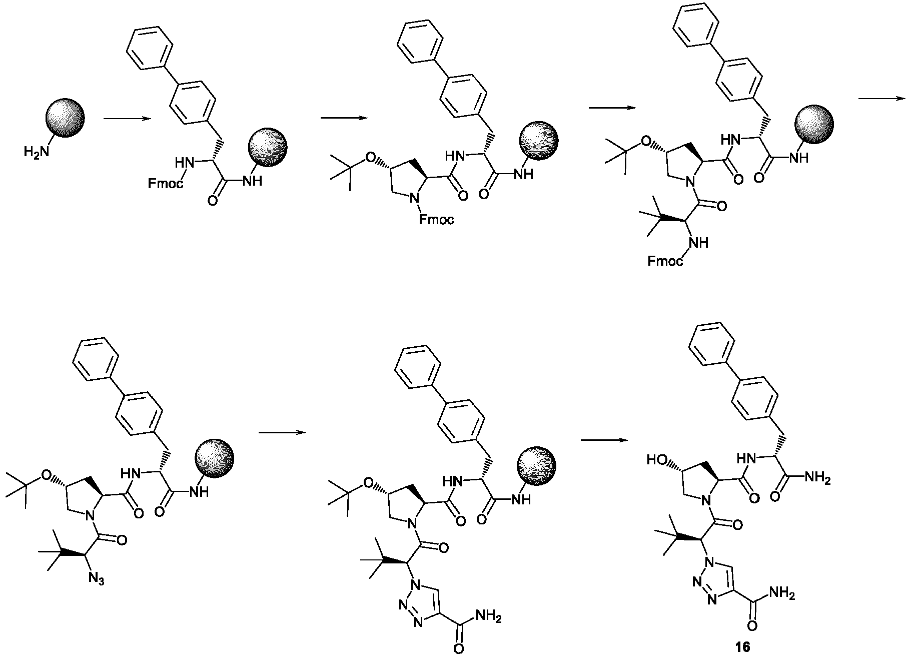

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added, then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of propiolamide (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 562.3, found: 562.2.

¹H NMR (400 MHz, DMSO-d6) δ 8.61 (d, J = 8.8 Hz, 1H), 8.59 (s, 1H), 7.91 (s, 1H), 7.68 - 7.51 (m, 5H), 7.51 - 7.40 (m, 4H), 7.39 - 7.30 (m, 1H), 7.35 - 7.27 (m, 4H), 5.57 (s, 1H), 4.49 - 4.33 (m, 2H), 4.25 (dq, J = 4.7, 2.3 Hz, 1H), 3.66 (d, J = 2.6 Hz, 2H), 3.23 (dd, J = 14.0, 3.9 Hz, 1H), 2.76 (dd, J = 14.0, 10.8 Hz, 1H), 1.90 - 1.79 (m, 1H), 1.57 (ddd, J = 13.3, 9.4, 4.2 Hz, 1H), 0.96 (s, 10H).

### Example S17: Synthesis of 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3,3-dimethyl-1-oxobutan-2-yl)-N-methyl-1H-1,2,3-triazole-4-carboxamide (Compound 17)

Synthesis was carried out following the solid phase synthesis scheme given below:

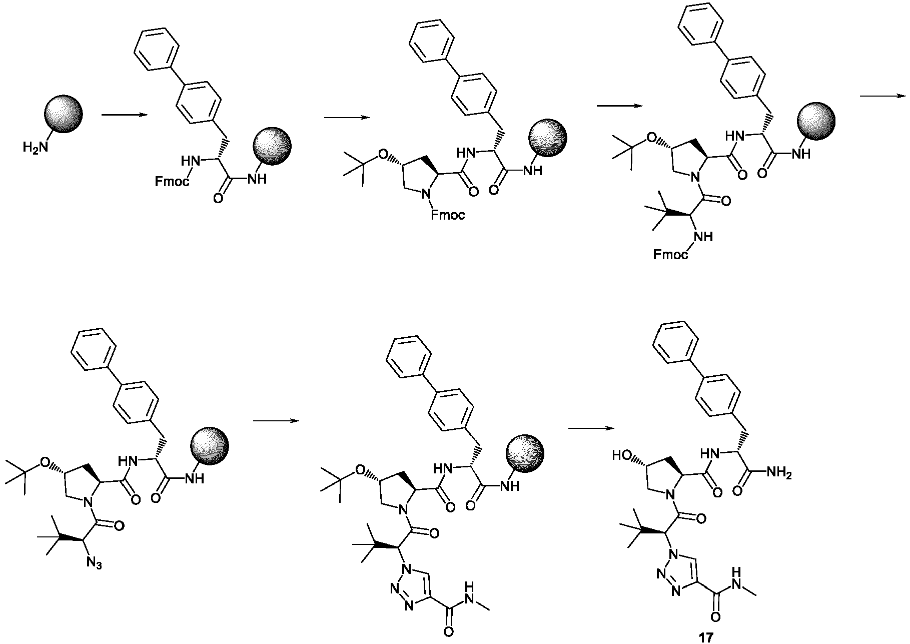

Rink Amide Resin (0.100 mmol) was added to a plastic peptide synthesis vessel. 10 mL N,N-Dimethylformamide were added and the resin was allowed to swell for 30 min under nitrogen. The resin was drained under vacuum. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (R)-2-((((9H-fluoren-9-yl)methoxy)carbonyl)amino)-3-([1,1'-biphenyl]-4-yl)propanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added, then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture ofN-methylpropiolamide (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 576.3, found: 576.3.

¹H NMR (400 MHz, DMSO*-d*₆) δ 8.66 - 8.55 (m, 1H), 8.55 - 8.45 (m, 1H), 8.45 - 8.34 (m, 1H), 7.63 (ddt, *J* = 7.1, 6.1, 1.3 Hz, 2H), 7.60 - 7.50 (m, 2H), 7.50 - 7.39 (m, 2H), 7.37 - 7.20 (m, 5H), 5.55 (d, *J =* 14.3 Hz, 1H), 4.41 (dddd, *J =* 18.5, 9.5, 8.2, 3.7 Hz, 2H), 4.29 - 4.14 (m, 1H), 3.73 - 3.63 (m, 2H), 3.32 - 3.06 (m, 2H), 2.76 (dd, *J =* 4.7, 1.7 Hz, 3H), 1.91 - 1.79 (m, 1H), 1.53 (dddd, *J =* 32.8, 12.8, 8.7, 4.5 Hz, 1H), 0.97 (d, *J =* 9.2 Hz, 9H).

### Example S18: Synthesis of (2S,4R)-1-((S)-2-(4-cyclopropyl-1H-1,2,3-triazol-1-yl)-3,3-dimethylbutanoyl)-4-hydroxy-N-((S)-1-(4-(4-methylthiazol-5-yl)phenyl)ethyl)pyrrolidine-2-carboxamide (Compound 18)

Synthesis was carried out following the solid phase synthesis scheme given below:

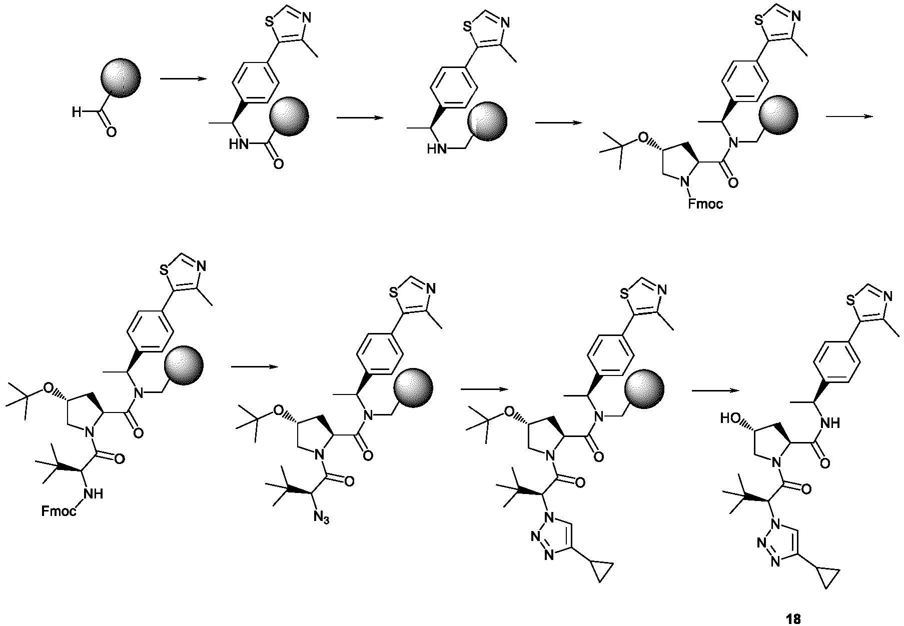

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. (S)-1-(4-(4-methylthiazol-5-yl)phenyl)ethan-1-amine hydrochloride (3.0 equiv.) was added to the plastic reactor. A solution of triethylamine (10.0 equiv.) solution in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 537.3, found: 537.3.

¹H NMR (400 MHz, MeOH-*d*₄) δ 9.12 (d, *J =* 1.7 Hz, 1H), 7.99 (d, *J =* 2.4 Hz, 1H), 7.51 - 7.37 (m, 4H), 5.46 (s, 1H), 5.09 - 4.96 (m, 1H), 4.54 (dd, *J* = 9.2, 7.6 Hz, 1H), 4.48 - 4.36 (m, 1H), 3.84 (dd, *J* = 11.0, 3.8 Hz, 1H), 3.77 - 3.66 (m, 1H), 2.50 (d, *J =* 3.3 Hz, 3H), 2.20 (ddt, *J* = 13.2, 7.7, 1.9 Hz, 1H), 2.05 - 1.90 (m, 2H), 1.58 (dd, *J =* 35.9, 7.0 Hz, 3H), 1.06 (d, *J* = 3.0 Hz, 9H), 1.02 - 0.90 (m, 2H), 0.84 - 0.72 (m, 2H).

### Example S19: Synthesis of (2S,4R)-1-((S)-2-cyclohexyl-2-(4-cyclopropyl-1H-1,2,3-triazol-1-yl)acetyl)-4-hydroxy-N-((S)-1-(4-(4-methylthiazol-5-yl)phenyl)ethyl)pyrrolidine-2-carboxamide (Compound 19)

Synthesis was carried out following the solid phase synthesis scheme given below:

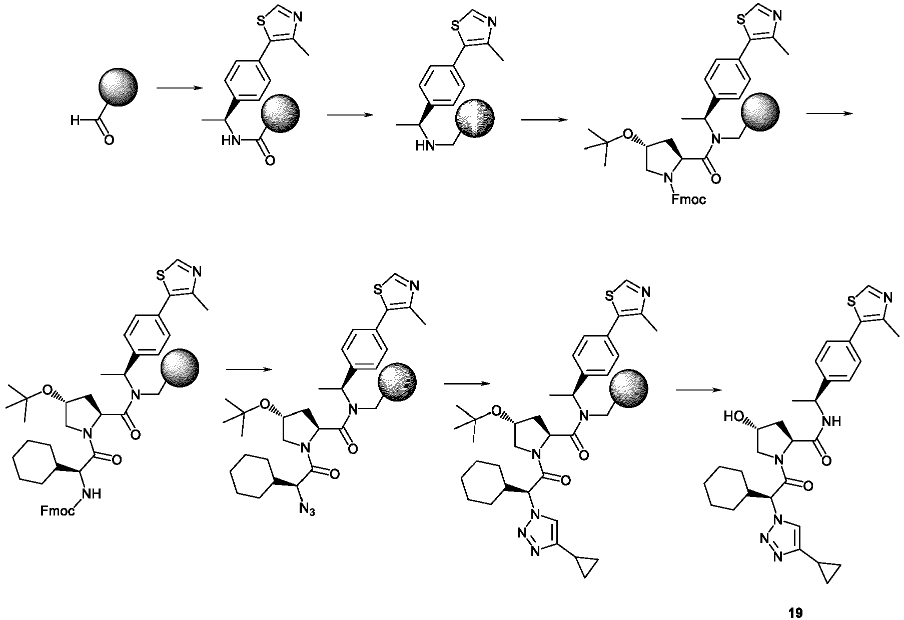

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. (S)-1-(4-(4-methylthiazol-5-yl)phenyl)ethan-1-amine hydrochloride (3.0 equiv.) was added to the plastic reactor. A solution of triethylamine (10.0 equiv.) solution in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10 mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (*S*)-2-((((9*H*-fluoren-9-yl)methoxy)carbonyl)amino)-2-cyclohexylacetic acid (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 563.3, found: 563.3.

¹H NMR (400 MHz, DMSO-d6) δ 8.99 (d, J = 2.6 Hz, 1H), 8.46 (d, J = 7.7 Hz, 1H), 7.90 (d, J = 3.4 Hz, 1H), 7.51 - 7.33 (m, 4H), 5.22 (d, J = 10.4 Hz, 1H), 4.92 (p, J = 7.1 Hz, 1H), 4.36 (t, J = 8.1 Hz, 1H), 4.30 (dq, J = 4.7, 2.3 Hz, 1H), 3.74 (dd, J = 10.7, 4.2 Hz, 1H), 3.61 (d, J = 10.7 Hz, 1H), 2.46 (s, 0H), 2.46 (s, 3H), 2.15 - 1.88 (m, 2H), 1.78 (ddd, J = 12.9, 8.6, 4.6 Hz, 1H), 1.62 (dd, J = 26.1, 10.0 Hz, 3H), 1.38 (d, J = 7.0 Hz, 3H), 1.12 (s, 4H), 1.02 (d, J = 12.0 Hz, 0H), 0.96 - 0.82 (m, 4H), 0.79 - 0.65 (m, 2H).

### Example S20: Synthesis of (2S,4R)-1-((2S)-2-(adamantan-1-yl)-2-(4-cyclopropyl-1H-1,2,3-triazol-1-yl)acetyl)-4-hydroxy-N-((S)-1-(4-(4-methylthiazol-5-yl)phenyl)ethyl)pyrrolidine-2-carboxamide (Compound 20)

Synthesis was carried out following the solid phase synthesis scheme given below:

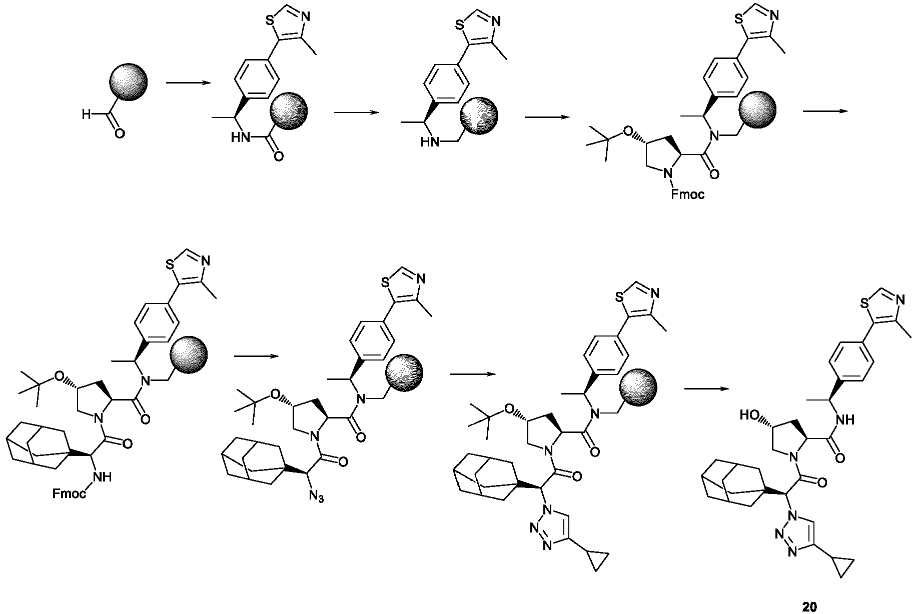

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. (S)-1-(4-(4-methylthiazol-5-yl)phenyl)ethan-1-amine hydrochloride (3.0 equiv.) was added to the plastic reactor. A solution of triethylamine (10.0 equiv.) solution in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (2S)-2-((((9H-fluoren-9-yl)methoxy)carbonyl)amino)-2-(adamantan-1-yl)acetic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 615.3, found: 615.3.

¹H NMR (400 MHz, MeOH-*d*₄) δ 8.98 (s, 1H), 7.97 (d, *J =* 6.7 Hz, 1H), 7.50 - 7.34 (m, 4H), 5.30 (s, 1H), 5.12 - 4.96 (m, 2H), 4.54 (dd, *J =* 9.3, 7.6 Hz, 1H), 4.47 - 4.37 (m, 1H), 3.85 (dd, *J =* 11.1, 3.8 Hz, 1H), 3.79 - 3.66 (m, 1H), 2.49 (d, *J =* 3.6 Hz, 3H), 2.20 (ddt, *J* = 11.3, 7.6, 2.0 Hz, 1H), 2.04 - 1.91 (m, 5H), 1.85 - 1.58 (m, 14H), 1.55 (d, *J* = 7.0 Hz, 3H), 1.02 - 0.93 (m, 2H), 0.79 (ddt, *J =* 6.4, 4.9, 2.2 Hz, 2H).

### Example S21: Synthesis of (2S,4R)-1-((S)-2-(4-cyclopropyl-1H-1,2,3-triazol-1-yl)-3,3-dimethylpentanoyl)-4-hydroxy-N-((S)-1-(4-(4-methylthiazol-5-yl)phenyl)ethyl)pyrrolidine-2-carboxamide (Compound 21)

Synthesis was carried out following the solid phase synthesis scheme given below:

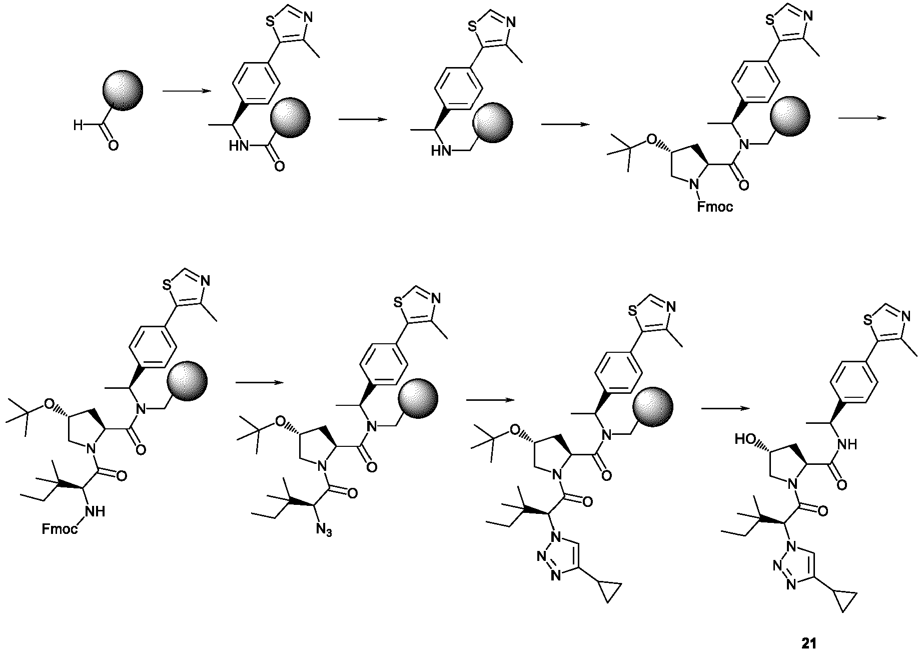

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. (S)-1-(4-(4-methylthiazol-5-yl)phenyl)ethan-1-amine hydrochloride (3.0 equiv.) was added to the plastic reactor. A solution of triethylamine (10.0 equiv.) solution in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-((((9H-fluoren-9-yl)methoxy)carbonyl)amino)-3,3-dimethylpentanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 551.3, found: 551.3.

¹H NMR (400 MHz, DMSO-d6) δ 8.99 (s, 1H), 8.48 (d, J = 7.7 Hz, 1H), 7.97 (d, J = 12.3 Hz, 1H), 7.49 - 7.41 (m, 2H), 7.41 - 7.32 (m, 2H), 5.41 (s, 1H), 4.92 (h, J = 7.0 Hz, 1H), 4.41 (dd, J = 8.9, 7.7 Hz, 1H), 4.29 (dt, J = 4.8, 2.7 Hz, 1H), 3.69 (dd, J = 10.8, 3.9 Hz, 1H), 2.46 (s, 3H), 2.07 (ddt, J = 11.4, 7.7, 2.1 Hz, 1H), 1.96 (tt, J = 8.5, 5.1 Hz, 1H), 1.77 (ddd, J = 13.1, 9.0, 4.5 Hz, 1H), 1.51 (d, J = 7.0 Hz, 1H), 1.39 (d, J = 7.0 Hz, 3H), 1.30 - 1.11 (m, 2H), 1.00 (d, J = 11.7 Hz, 3H), 0.90 (s, 3H), 0.94 - 0.84 (m, 3H), 0.88 - 0.67 (m, 6H).

### Example S22: Synthesis of (2S,4R)-N-(1-(2-chloro-4-(4-methylthiazol-5-yl)phenyl)ethyl)-1-((S)-2-(4-cyclopropyl-1H-1,2,3-triazol-1-yl)-3,3-dimethylbutanoyl)-4-hydroxypyrrolidine-2-carboxamide (Compound 22)

Synthesis was carried out following the solid phase synthesis scheme given below:

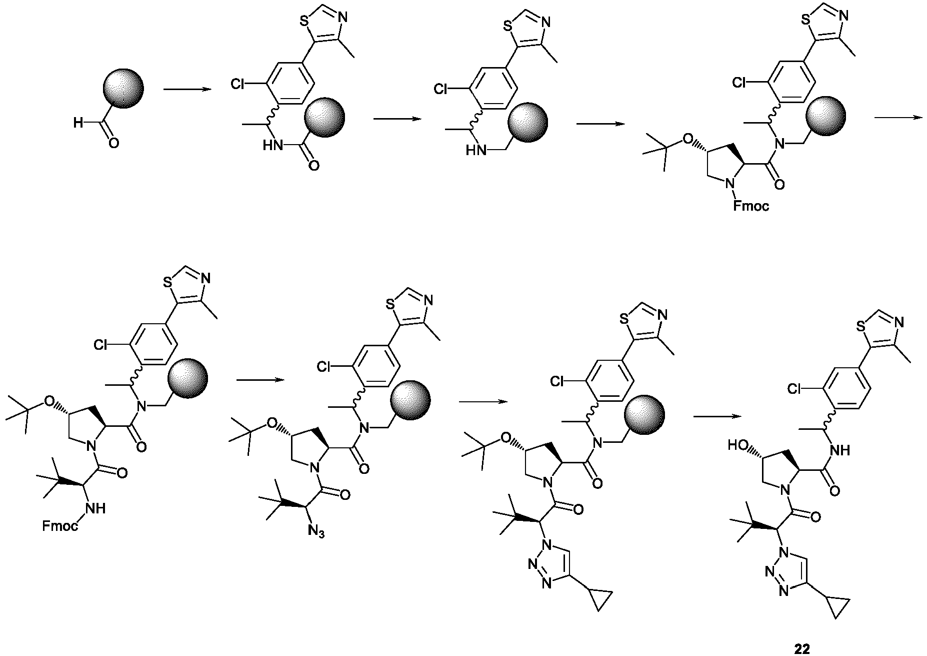

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. 1-[2-chloro-4-(4-methyl-1,3-thiazol-5-yl)phenyl]ethan-1-amine dihydrochloride (3.0 equiv.) was added to the plastic reactor. A solution of triethylamine (10.0 equiv.) solution in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 571.2, found: 571.2.

¹H NMR (400 MHz, MeOH-*d*₄) δ 8.92 (d, *J =* 2.0 Hz, 1H), 7.98 (s, 1H), 7.53 - 7.46 (m, 2H), 7.45 - 7.39 (m, 1H), 5.45 (s, 1H), 5.42 - 5.32 (m, 1H), 4.55 (dd, *J* = 9.2*,* 7.6 Hz, 1H), 4.44 (p, *J =* 2.5 Hz, 1H), 3.83 (dd, *J =* 11.0, 3.8 Hz, 1H), 3.76 - 3.65 (m, 1H), 2.48 (d, *J =* 3.4 Hz, 3H), 2.22 (ddt, *J =* 13.3, 7.7, 1.9 Hz, 1H), 2.07 - 1.91 (m, 2H), 1.57 (dd, *J* = 34.4, 7.0 Hz, 3H), 1.06 (d, *J =* 4.6 Hz, 9H), 1.02 - 0.90 (m, 2H), 0.84 - 0.72 (m, 2H).

### Example S23: Synthesis of (2S,4R)-1-((2S)-2-(adamantan-1-yl)-2-(4-cyclopropyl-1H-1,2,3-triazol-1-yl)acetyl)-N-(1-(2-chloro-4-(4-methylthiazol-5-yl)phenyl)ethyl)-4-hydroxypyrrolidine-2-carboxamide (Compound 23)

Synthesis was carried out following the solid phase synthesis scheme given below:

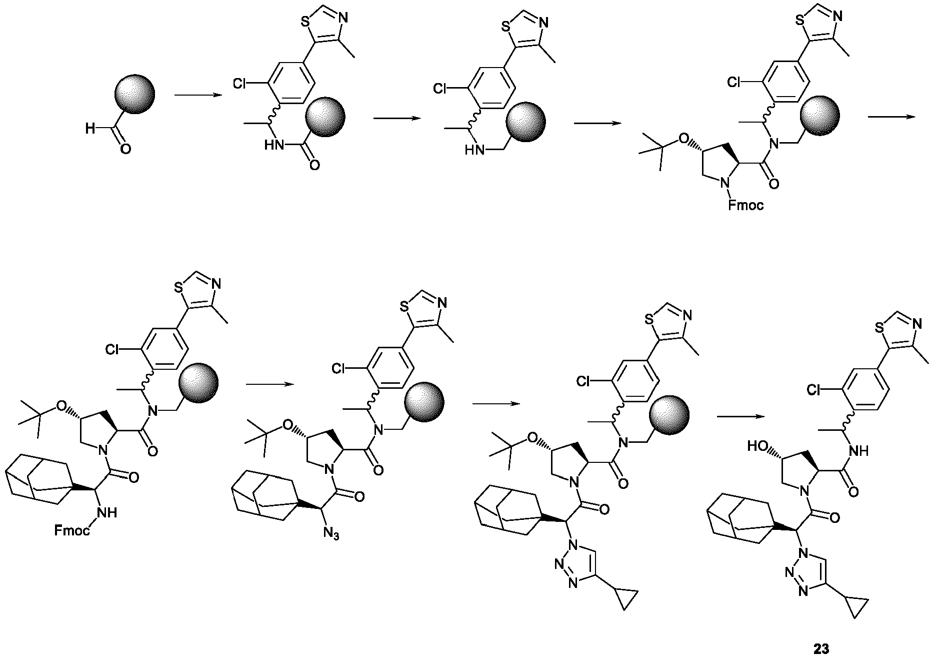

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. 1-[2-chloro-4-(4-methyl-1,3-thiazol-5-yl)phenyl]ethan-1-amine dihydrochloride (3.0 equiv.) was added to the plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture (2S)-2-((((9H-fluoren-9-yl)methoxy)carbonyl)amino)-2-(adamantan-1-yl)acetic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 649.3, found: 649.3.

¹H NMR (400 MHz, DMSO-d₆) δ 9.04 (s, 1H), 8.62 (d, *J =* 7.3 Hz, 1H), 7.99 (s, 1H), 7.53 (d, *J* = 1.2 Hz, 1H), 7.51 - 7.45 (m, 2H), 5.25 (s, 1H), 5.17 (h, *J =* 6.9 Hz, 1H), 4.28 (dt, *J =* 5.6, 2.7 Hz, 2H), 3.71 - 3.51 (m, 2H), 2.47 (s, 3H), 2.11 (dd, *J =* 12.9, 7.9 Hz, 1H), 2.03 - 1.88 (m, 4H), 1.80 - 1.56 (m, 8H), 1.55 - 1.42 (m, 7H), 1.39 (d, *J =* 7.0 Hz, 3H), 0.89 (dd, *J =* 8.4, 2.4 Hz, 2H), 0.80 - 0.69 (m, 2H).

### Example S24: Synthesis of (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)benzyl)pyrrolidine-2-carboxamide (Compound 24)

Synthesis was carried out following the solid phase synthesis scheme given below:

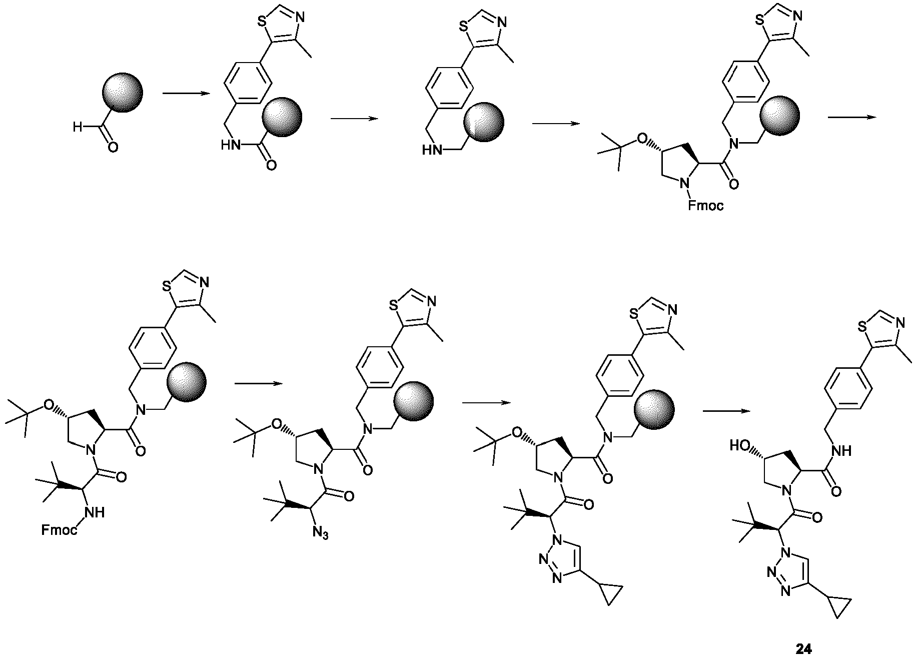

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. [4-(4-methyl-1,3-thiazol-5-yl)phenyl]methanamine dihydrochloride (3.0 equiv.) was added into the plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30% - 60% Acetonitrile). The desired product was freeze dried and characterized by LC-MS. ESI-MS: m/z [M+H]⁺ calculated: 523.2, found: 523.2.

¹H NMR (400 MHz, DMSO-d6) δ 8.64 (t, J = 6.0 Hz, 0H), 8.00 (s, 0H), 7.50 - 7.36 (m, 2H), 5.41 (s, 0H), 4.47 - 4.26 (m, 2H), 3.75 (dd, J = 10.8, 3.9 Hz, 1H), 3.74 (s, 19H), 3.64 - 3.54 (m, 0H), 2.46 (s, 1H), 2.12 - 1.85 (m, 1H), 0.97 (d, J = 8.5 Hz, 5H), 0.93 - 0.83 (m, 1H), 0.80 - 0.67 (m, 1H).

### Example S25: Synthesis of (2S,4R)-1-((S)-2-cyclohexyl-2-(4-cyclopropyl-1H-1,2,3-triazol-1-yl)acetyl)-4-hydroxy-N-(4-(4-methylthiazol-5-yl)benzyl)pyrrolidine-2-carboxamide (Compound 25)

Synthesis was carried out following the solid phase synthesis scheme given below:

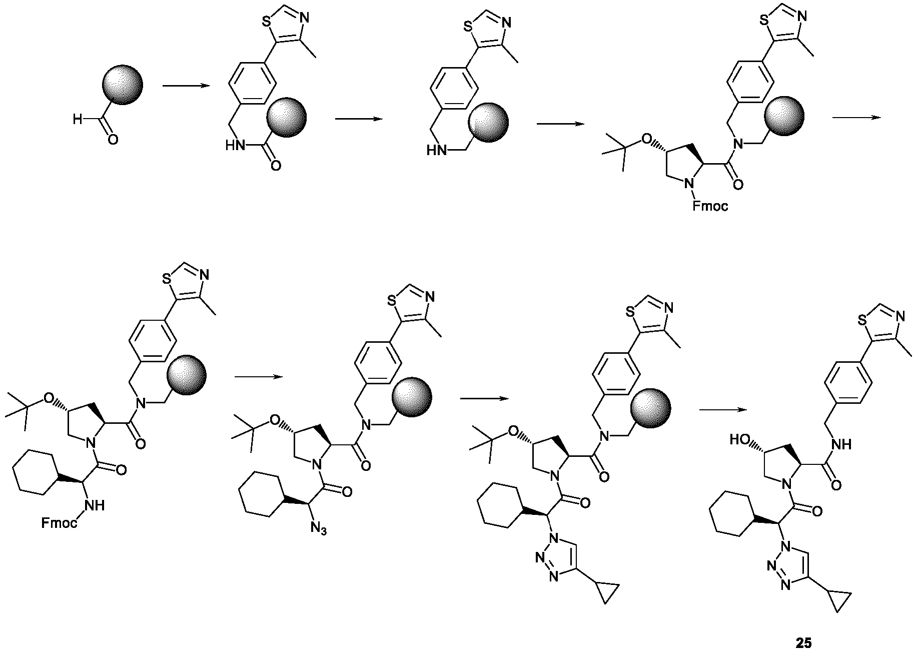

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. [4-(4-methyl-1,3-thiazol-5-yl)phenyl]methanamine dihydrochloride (3.0 equiv.) into plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture (*S*)-2-((((9*H*-fluoren-9-yl)methoxy)carbonyl)amino)-2-cyclohexylacetic acid (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 549.3, found: 549.3.

¹H NMR (400 MHz, DMSO-d6) δ 8.99 (s, 1H), 8.56 (t, J = 5.9 Hz, 1H), 7.89 (s, 1H), 7.49 - 7.34 (m, 4H), 5.23 (d, J = 10.4 Hz, 1H), 4.43 - 4.21 (m, 4H), 3.81 (dd, J = 10.7, 4.2 Hz, 1H), 3.65 - 3.57 (m, 1H), 2.46 (s, 3H), 2.44 (d, J = 3.7 Hz, 1H), 2.18 - 2.01 (m, 1H), 2.01 - 1.85 (m, 3H), 1.68 - 1.55 (m, 4H), 1.12 (s, 4H), 1.12 - 0.98 (m, 1H), 0.96 - 0.79 (m, 5H), 0.79 - 0.64 (m, 2H).

### Example S26: Synthesis of (2S,4R)-1-((S)-2-(4-cyclopropyl-1H-1,2,3-triazol-1-yl)-3-methyl-3-phenylbutanoyl)-4-hydroxy-N-(4-(4-methylthiazol-5-yl)benzyl)pyrrolidine-2-carboxamide (Compound 26)

Synthesis was carried out following the solid phase synthesis scheme given below:

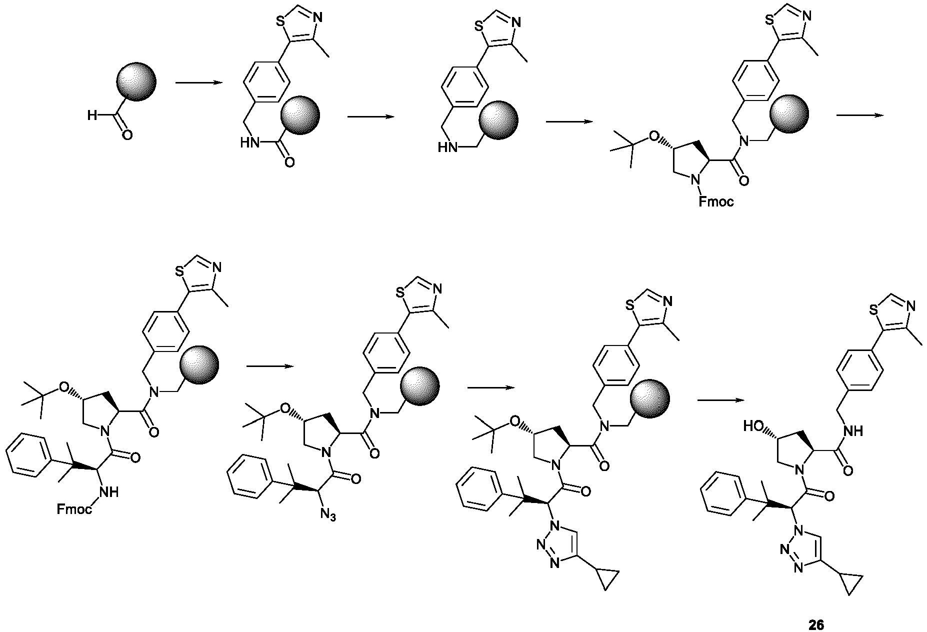

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. [4-(4-methyl-1,3-thiazol-5-yl)phenyl]methanamine dihydrochloride (3.0 equiv.) was added to the plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (*S*)-2-((((9*H*-fluoren-9-yl)methoxy)carbonyl)amino)-3-methyl-3-phenylbutanoic acid (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. 1H-imidazole-1-sulfonyl azide hydrochloride (3.0 equiv.) and N,N-Diisopropylethylamine (6.0 equiv.) were mixed in dichloromethane. The mixture was drawn into the reaction vessel and reacted under nitrogen for 1 hr to convert amine to azide. The solvent was drained under vacuum and the deprotection was repeated. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of ethynylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 585.3, found: 585.3.

¹H NMR (400 MHz, DMSO-d6) δ 9.00 (d, J = 2.2 Hz, 0H), 8.53 (t, J = 6.0 Hz, 1H), 7.61 (s, 1H), 7.50 - 7.39 (m, 2H), 7.38 - 7.23 (m, 2H), 7.23 - 7.12 (m, 2H), 5.74 (s, 1H), 4.48 - 4.22 (m, 2H), 3.81 (s, 17H), 3.54 (d, J = 10.6 Hz, 1H), 3.41 (dd, J = 10.9, 3.9 Hz, 1H), 2.47 (d, J = 7.1 Hz, 2H), 2.10 - 2.00 (m, 1H), 1.94 - 1.81 (m, 1H), 1.46 (d, J = 10.6 Hz, 3H), 0.84 (dd, J = 8.4, 2.4 Hz, 1H), 0.68 - 0.54 (m, 1H).

### Example S27: Synthesis of (2S,4R)-1-((S)-2-(4-benzyl-1H-1,2,3-triazol-1-yl)-3,3-dimethylbutanoyl)-4-hydroxy-N-(4-(4-methylthiazol-5-yl)benzyl)pyrrolidine-2-carboxamide (Compound 27)

Synthesis was carried out following the solid phase synthesis scheme given below:

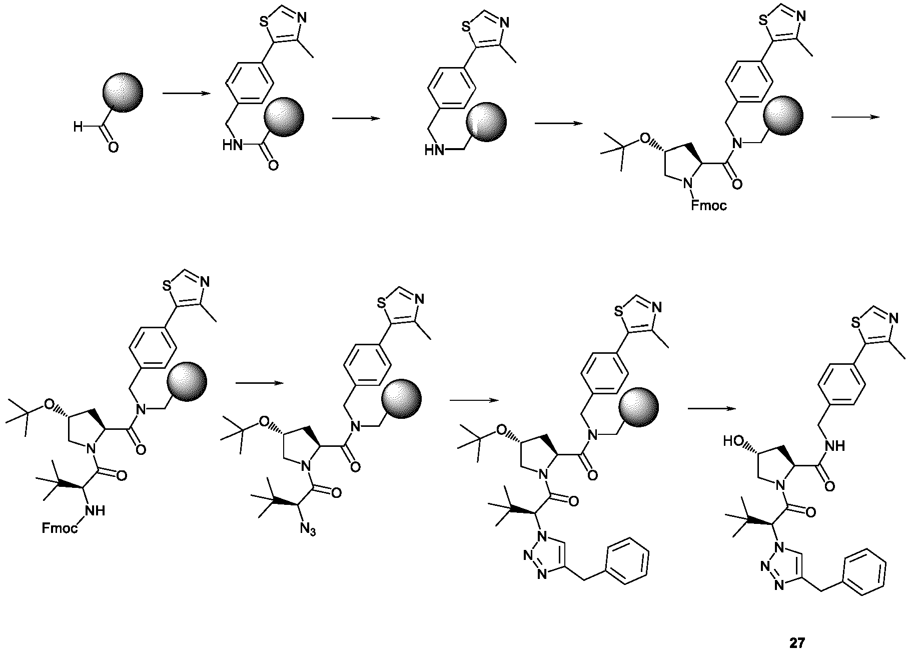

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. [4-(4-methyl-1,3-thiazol-5-yl)phenyl]methanamine dihydrochloride (3.0 equiv.) was added to the plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. Add mixture (S)-2-azido-3,3-dimethylbutanoic acid (3.0 equiv.), Ethyl cyano(hydroxyimino)acetate (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. Draw the mixture of prop-2-yn-1-ylbenzene (5.0 equiv.), N,N-Diisopropylethylamine (10.0 equiv.) in 10 mL N,N-Dimethylformamide (nitrogen purged) into reaction vessel, react for overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 573.3, found: 573.3.

¹H NMR (400 MHz, DMSO-d6) δ 8.99 (d, *J* = 2.4 Hz, 1H), 8.64 (t, *J* = 6.0 Hz, 1H), 8.02 (s, 1H), 7.41 (s, 4H), 7.35 - 7.11 (m, 6H), 5.45 (d, *J* = 9.3 Hz, 1H), 4.48 - 4.22 (m, 5H), 3.98 (d, *J* = 21.9 Hz, 2H), 3.79 - 3.69 (m, 1H), 3.69 - 3.53 (m, 1H), 3.51 - 3.38 (m, 1H), 2.44 (d, *J* = 6.4 Hz, 3H), 2.14 - 2.00 (m, 1H), 1.96 - 1.82 (m, 1H), 0.97 (d, *J* = 5.8 Hz, 9H).

### Example S28: Synthesis of (2S,4R)-1-((S)-3,3-dimethyl-2-(4-(1-(trifluoromethyl)cyclopropyl)-1H-1,2,3-triazol-1-yl)butanoyl)-4-hydroxy-N-(4-(4-methylthiazol-5-yl)benzyl)pyrrolidine-2-carboxamide (Compound 28)

Synthesis was carried out following the solid phase synthesis scheme given below:

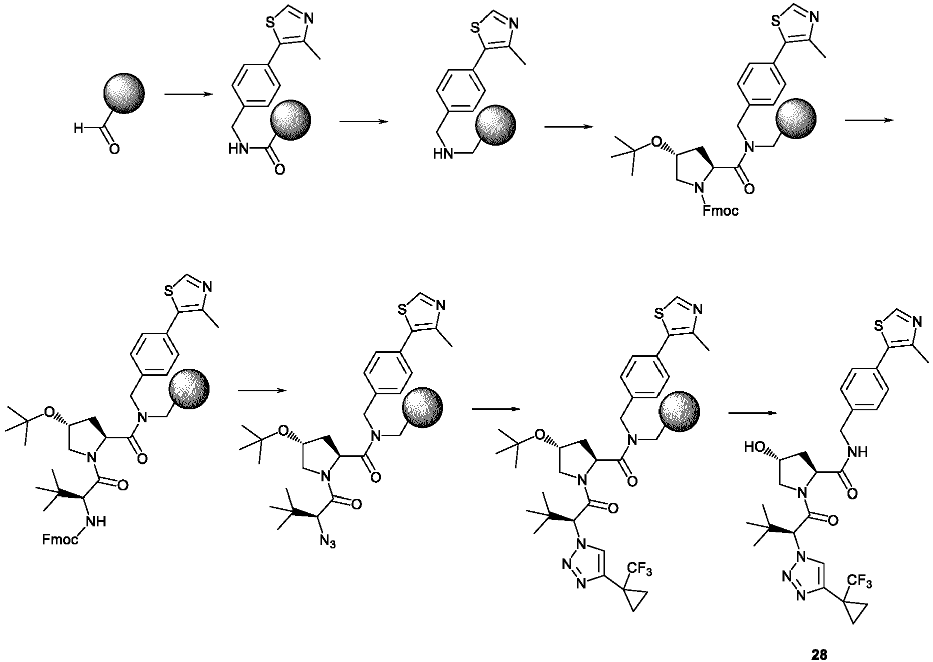

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. Add [4-(4-methyl-1,3-thiazol-5-yl)phenyl]methanamine dihydrochloride (3.0 equiv.) into plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture (S)-2-azido-3,3-dimethylbutanoic acid (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of 1-ethynyl-1-(trifluoromethyl)cyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 591.2, found: 591.2.

¹H NMR (400 MHz, DMSO-d6) δ 9.00 (d, J = 1.6 Hz, 1H), 8.67 (t, J = 6.0 Hz, 1H), 8.29 (s, 1H), 7.50 - 7.36 (m, 4H), 5.54 (s, 1H), 4.49 - 4.38 (m, 1H), 4.41 - 4.22 (m, 3H), 3.79 - 3.65 (m, 2H), 2.45 (s, 3H), 2.08 (ddd, J = 9.7, 7.7, 3.9 Hz, 1H), 1.92 (ddd, J = 13.1, 9.0, 4.4 Hz, 1H), 1.46 - 1.26 (m, 4H), 0.97 (d, J = 9.4 Hz, 10H).

### Example S29: Synthesis of (2S,4R)-1-((S)-3,3-dimethyl-2-(4-(1-methylcyclopropyl)-1H-1,2,3-triazol-1-yl)butanoyl)-4-hydroxy-N-(4-(4-methylthiazol-5-yl)benzyl)pyrrolidine-2-carboxamide (Compound 29)

Synthesis was carried out following the solid phase synthesis scheme given below:

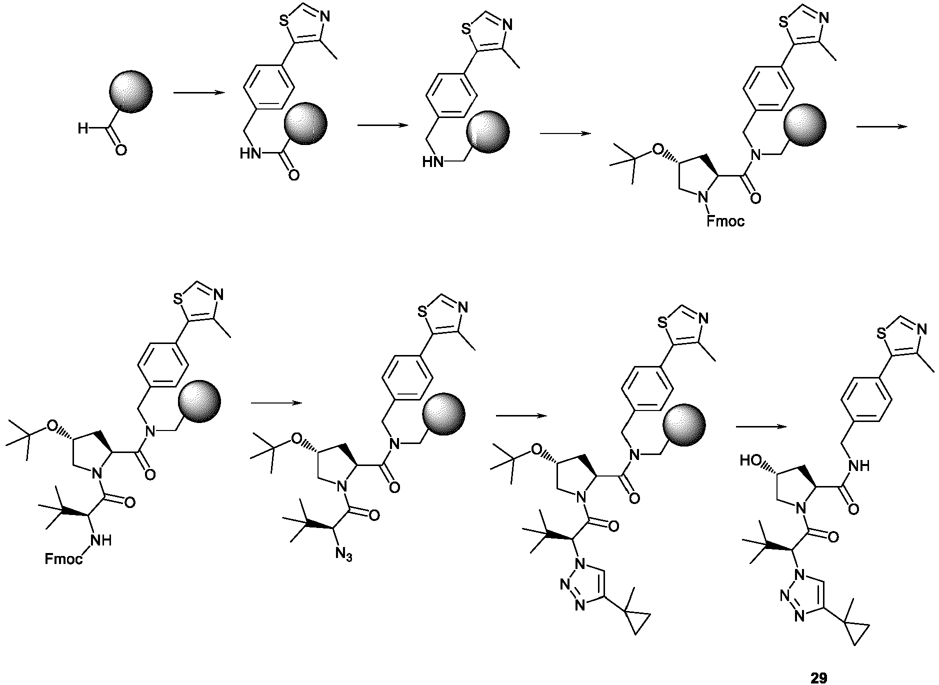

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. [4-(4-methyl-1,3-thiazol-5-yl)phenyl]methanamine dihydrochloride (3.0 equiv.) was added to the plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture of (S)-2-azido-3,3-dimethylbutanoic acid (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of 1-ethynyl-1-methylcyclopropane (5.0 equiv.), N,N-Diisopropylethylamine (10.0 equiv.) in 10 mL N,N-Dimethylformamide (nitrogen purged) was drawn into the reaction vessel and reacted overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. The precipitate was collected and separated by reverse phase HPLC (30%-60% Acetonitrile). The desired product was freeze dried and characterized by LC-MS. ESI-MS: m/z [M+H]⁺ calculated: 537.3, found: 537.3.

¹H NMR (400 MHz, DMSO-d6) δ 9.00 (s, 0H), 8.64 (t, J = 6.0 Hz, 0H), 7.95 (s, 0H), 7.49 - 7.35 (m, 2H), 5.43 (s, 0H), 4.47 - 4.27 (m, 2H), 3.85 (s, 21H), 3.76 (dd, J = 10.9, 3.9 Hz, 0H), 3.61 (d, J = 11.2 Hz, 0H), 2.45 (s, 1H), 2.07 (ddd, J = 12.4, 5.8, 4.0 Hz, 0H), 1.40 (s, 1H), 0.98 (s, 4H), 0.96 (s, 1H), 0.74 (t, J = 1.4 Hz, 1H).

### Example S30: Synthesis of (2S,4R)-1-((S)-2-(4-(1-ethynylcyclopropyl)-1H-1,2,3-triazol-1-yl)-3,3-dimethylbutanoyl)-4-hydroxy-N-(4-(4-methylthiazol-5-yl)benzyl)pyrrolidine-2-carboxamide (Compound 30) and (2S,2'S,4R,4'R)-1,1'-((2S,2'S)-2,2'-(cyclopropane-1,1-diylbis(1H-1,2,3-triazole-4,1-diyl))bis(3,3-dimethylbutanoyl))bis(4-hydroxy-N-(4-(4-methylthiazol-5-yl)benzyl)pyrrolidine-2-carboxamide) (Compound 31)

Synthesis was carried out following the solid phase synthesis scheme given below:

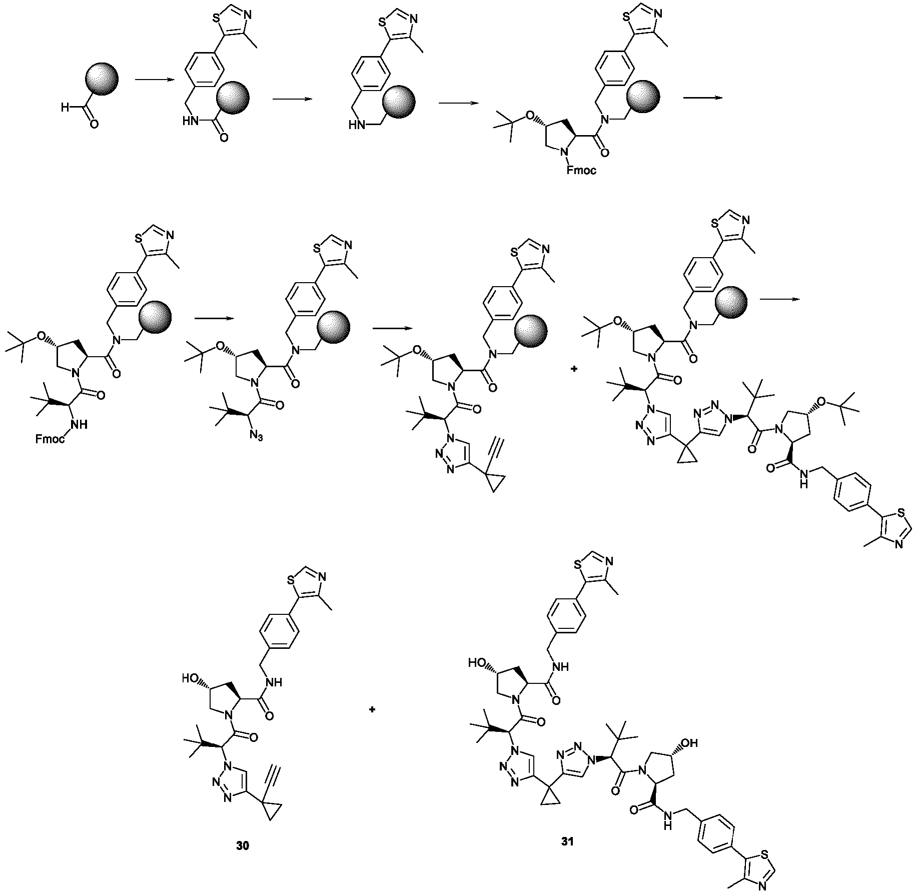

4-Formyl-3-methoxy-phenyloxymethyl polystyrene resin (0.100 mmol) was added to a plastic peptide synthesis vessel. 10 mL 1,2-Dichloroethane were added and the resin was allowed to swell for 30 min under nitrogen. The resin was drained under vacuum. [4-(4-methyl-1,3-thiazol-5-yl)phenyl]methanamine dihydrochloride (3.0 equiv.) was added to the plastic reactor. A solution of triethylamine (10.0 equiv.) in 1,2-Dichloroethane (1 mL) was prepared. The solution was drawn into the plastic reactor and reacted for 2 hr at room temperature. The reactor was opened, and sodium cyanoborohydride (10.0 equiv.) and acetic acid (2 equiv.) were added to the reactor. The reactor was left opened on manifold, mixed by pipetting, and reacted overnight at RT. The resin was washed by 10mL methanol, 10 mL N,N-Dimethylformamide, then 10 mL dichloromethane, and drained under vacuum. The washing procedure was repeated 3 times. A mixture of (2S,4R)-1-(((9H-fluoren-9-yl)methoxy)carbonyl)-4-(tert-butoxy)pyrrolidine-2-carboxylic acid (3.0 equiv.), 1-[Bis(dimethylamino)methylene]-1H-1,2,3-triazolo[4,5-b]pyridinium 3-oxid hexafluorophosphate (3.0 equiv.), 1-hydroxy-7-azabenzotriazole (3.0 equiv.), and N,N-Diisopropylethylamine (6.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. 10 mL of 20% 4-methylpiperidine in N,N-Dimethylformamide were drawn into the reaction vessel and reacted under nitrogen for 15 min to deprotect Fmoc group. The solvent was drained under vacuum and the deprotection was repeated. The resin was washed with 10 mL N,N-Dimethylformamide, then 10 mL dichloromethane, and drained under vacuum. The washing procedure was repeated 3 times. A mixture (S)-2-azido-3,3-dimethylbutanoic acid (3.0 equiv.) and N,N'-Diisopropylcarbodiimide (3.0 equiv.) in 10 mL N,N-Dimethylformamide was added; then, the mixture was drawn into the synthesis vessel and reacted for 2 hr under nitrogen. The resin was washed with 10 mL N,N-Dimethylformamide, then 10 mL dichloromethane, and drained under vacuum. The washing procedure was repeated 3 times. Tetrakis(acetonitrile)copper(I) hexafluorophosphate (0.2 equiv.) was added directly into the peptide synthesis vessel to perform on-resin "click" reaction. The mixture of 1,1-diethynylcyclopropane (5.0 equiv.), N,N-Diisopropylethylamine (10.0 equiv.) in 10 mL N,N-Dimethylformamide (nitrogen purged) was drawn into the reaction vessel, react for overnight under nitrogen. The resin was washed with 10 mL N,N-Dimethylformamide, then 10 mL dichloromethane, and drained under vacuum. A cleavage solution was prepared by mixing 5% Triisopropylsilane in 95% Trifluoroacetic acid. The solution was drawn into the reaction vessel and reacted for 1 hr. The Trifluoroacetic acid was removed under vacuum. The remaining residue was mixed with 50 mL cold ether (-20 °C) to precipitate the compound. Collect the precipitation, and separate the mixture by reverse phase HPLC (30% - 60% Acetonitrile) to yield **compound 30** and **compound 31.** Desired product **compound 30** was freeze dried and characterized by LC-MS. ESI-MS: m/z [M+H]⁺ calculated: 547.2, found: 547.2.

¹H NMR (400 MHz, DMSO-d6) δ 8.99 (s, 0H), 8.66 (t, J = 6.0 Hz, 0H), 8.11 (d, J = 8.9 Hz, 0H), 7.43 (s, 1H), 7.45 - 7.35 (m, 1H), 5.46 (s, 0H), 4.43 (q, J = 8.3 Hz, 0H), 4.35 (dt, J = 6.0, 3.2 Hz, 1H), 3.87 (s, 20H), 3.75 (dd, J = 11.0, 3.9 Hz, 0H), 3.63 (d, J = 11.1 Hz, 0H), 2.46 (s, 2H), 2.13 - 2.03 (m, 0H), 1.92 (ddd, J = 13.0, 9.0, 4.4 Hz, 0H), 1.44 - 1.29 (m, 2H), 0.98 (d, J = 8.0 Hz, 5H).

Desired product **compound 31** was freeze dried and characterized by LC-MS. ESI-MS: m/z [M+H]⁺ calculated: 1003.4, found: 1003.4.

¹H NMR (400 MHz, DMSO-d6) δ 8.99 (s, 2H), 8.67 (t, J = 6.0 Hz, 2H), 8.04 (s, 2H), 7.49 - 7.34 (m, 9H), 5.45 (s, 2H), 4.48 - 4.36 (m, 3H), 4.36 (dd, J = 5.8, 3.2 Hz, 3H), 4.27 (dd, J = 15.7, 5.7 Hz, 2H), 3.75 (dd, J = 10.9, 4.0 Hz, 2H), 3.65 (d, J = 10.9 Hz, 2H), 2.44 (d, J = 7.3 Hz, 1H), 2.44 (s, 5H), 2.07 (dd, J = 13.0, 7.8 Hz, 2H), 1.91 (ddd, J = 13.0, 9.0, 4.4 Hz, 2H), 1.53 (q, J = 4.0, 3.6 Hz, 2H), 1.31 (p, J = 3.8 Hz, 2H), 0.97 (s, 2H), 0.93 (s, 16H), 0.91 (s, 2H).

### Example S31: Synthesis of (2S,4R)-N-((R)-3-([1,1'-biphenyl]-4-yl)-1-amino-1-oxopropan-2-yl)-4-hydroxy-1-((S)-3-methyl-2-(4-methyl-1H-1,2,3-triazol-1-yl)butanoyl)pyrrolidine-2-carboxamide (Compound 32)

Synthesis was carried out following the scheme given below:

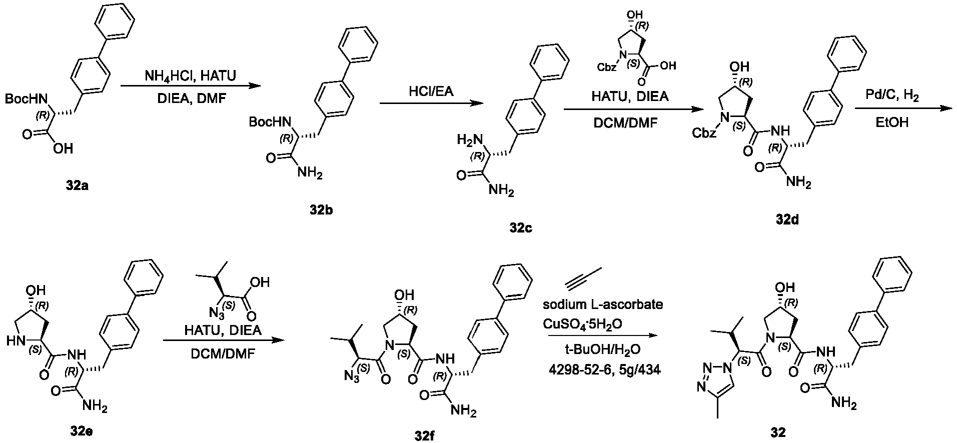

### Preparation of intermediate 32b

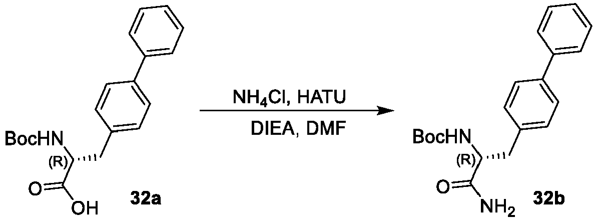

To a solution of (*R*)-3-([1,1'-biphenyl]-4-yl)-2-((tert-butoxycarbonyl)amino)propanoic acid (9.60 g, 28.1 mmol) and *N*,*N*-diisopropylethylamine (9.30 mL, 56.2 mmol) in anhydrous *N*,*N*-dimethylformamide (100 mL) was added (1-[Bis(dimethylamino)methylene]-1H-1,2,3-triazolo[4,5-b] pyridinium 3-oxid hexafluorophosphate (12.8 g, 33.7 mmol) at 0 °C. The solution was stirred at 0 °C for 5 minutes, then ammonium chloride (1.80 g, 33.7 mmol) was added. The reaction mixture was stirred at 25 °C for 8 h and diluted with water (400 mL). The solid was collected by filtration and dried under reduced pressure to afford (*R*)-tert-butyl (3-([1,1'-biphenyl]-4-yl)-1-amino-1-oxopropan-2-yl)carbamate (9.50 g, 99.2% yield) as a white solid.

### Preparation of intermediate 32c

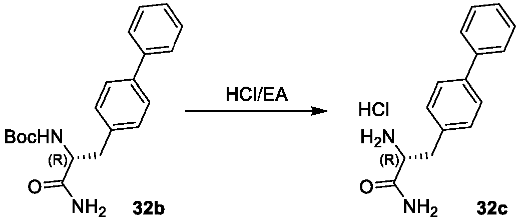

A solution of (R)-tert-butyl (3-([1,1'-biphenyl]-4-yl)-1-amino-1-oxopropan-2-yl)carbamate (9.50 g, 27.8 mmol) in HCl (4.0 M in ethyl acetate, 100 mL, 400 mmol) was stirred at 25 °C for 8 h and concentrated under reduce pressure to afford (R)-3-([1,1'-biphenyl]-4-yl)-2-aminopropanamide hydrochloride (6.80 g, 88.3% yield) as a white solid.

### Preparation of intermediate 32d

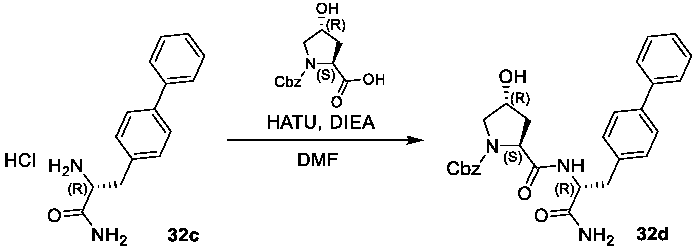

To a solution of (*R*)-3-([1,1'-biphenyl]-4-yl)-2-aminopropanamide hydrochloride (6.80 g, 24.6 mmol) and *N*,*N*-diisopropylethylamine (14mL, 84.9 mmol) and (2*S*, 4*R*)-1-((benzyloxy)carbonyl)-4-hydroxypyrrolidine-2-carboxylic acid (6.52 g, 24.6 mmol) in anhydrous *N*,*N*-dimethylformamide (100 mL) was added (1-[Bis(dimethylamino)methylene]-1H-1,2,3-triazolo[4,5-b]pyridinium 3-oxid hexafluorophosphate (12.9 g, 34.0 mmol) at 20 °C. The solution was stirred at 20 °C for 2 h and then partitioned between water (600 mL) and ethyl acetate (600 mL). The separated organic layer was washed with brine (400 mL), dried over sodium sulfate and concentrated to dryness. The residue was purified by RP-HPLC (water (0.1% TFA)-ACN) to afford (2*S*,4*R*)-benzyl 2-(((*R*)-3-([1,1'-biphenyl]-4-yl)-1-amino-1-oxopropan-2-yl)carbamoyl)-4-hydroxypyrrolidine-1-carboxylate (8.00 g, 67.8 % yield) as a white solid.

### Preparation of intermediate 32e

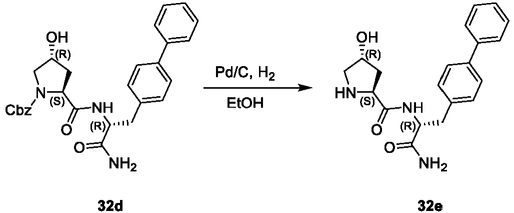

A mixture of (2*S*,4*R*)-benzyl 2-(((*R*)-3-([1,1'-biphenyl]-4-yl)-1-amino-1-oxopropan -2-yl)carbamoyl)-4-hydroxypyrrolidine-1-carboxylate (7.00 g, 14.4 mmol) and Pd (10% on carbon, 1.51 g, 1.44 mmol) in methanol (70 mL) was hydrogenated (15 psi) at 40 °C for 8 h and filtered. The filtrate was concentrated under reduce pressure to give crude (2S,4R)-N-((*R*)-3-([1,1'-biphenyl]-4-yl)-1-amino-1-oxopropan-2-yl) -4-hydroxypyrrolidine-2-carboxamide (3.50 g, 69% yield) as a white solid.

### Preparation of intermediate 32f

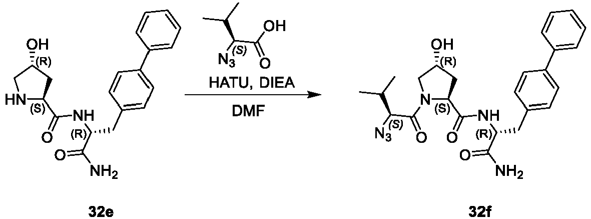

A mixture of (2*S*,4*R*)-N-((*R*)-3-([1,1'-biphenyl]-4-yl)-1-amino-1-oxopropan-2-yl) -4-hydroxypyrrolidine-2-carboxamide (2.20 g, 6.23 mmol), (2S)-2-azido-3-methylbutanoic acid (1.07 g, 7.47 mmol), *N*,*N*-diisopropylethylamine (3.10 mL, 18.7 mmol) and (1-[Bis(dimethylamino)methylene]-1H-1,2,3-triazolo[4,5-b] pyridinium 3-oxid hexafluorophosphate (2.84 g, 7.47 mmol) in *N*,*N*-dimethylformamide (25 mL) was stirred at 20 °C for 2 h and diluted with water (50 mL). The solid was collected by filtration and dried to afford crude (2*S*, 4*R*)-N-((*R*)-3-([1,1'-biphenyl]-4-yl)-1-amino-1-oxopropan-2-yl)-1-((S)-2-azido-3-methylbutanoyl)-4-hydroxypyrrolidine-2-carboxamide (2.40 g, 80.6% yield) as a white solid.

### Preparation of intermediate 32

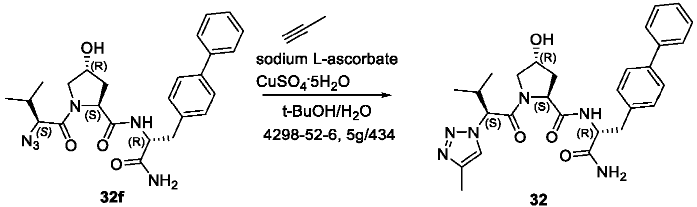

To a solution of sodium L-ascorbate (165.6mg, 0.8400mmol) in water (4 mL) and tert-Butyl alcohol (4 mL) were added L-propyne (0.25 mL, 0.25 mmol), Copper sulfate pentahydrate (67.8 mg, 0.27mmol) and (2*S*, 4*R*)-N-((*R*)-3-([1,1'-biphenyl]-4-yl)-1-amino-1-oxopropan-2-yl)-1-((*S*)-2-azido-3-methylbutanoyl)-4-hydroxypyrrolidine-2-carboxamide (100 mg, 0.21 mmol). The reaction was stirred at 25 °C for 16 h and extracted with ethyl acetate (2 x 40 mL). The combined organic layers were washed with brine (2 x 20 mL), dried and concentrated under reduced pressure. The residue was purified by RP-HPLC (acetonitrile 25-65/0.075% in water) to afford (2*S*, 4*R*)-N-((*R*)-3-([1,1'-biphenyl]-4-yl) -1-amino-1-oxopropan-2-yl)-4-hydroxy-1-((*S*)-3-methyl-2-(4-methyl-1H-1,2,3-triazol-1-yl)butanoyl)pyrrolidine-2-carboxamide (38.0 mg, 34.7% yield) as white solid. ¹H NMR (400 MHz, DMSO-*d*₆) δ 7.61 (s, 1H), 7.58 - 7.51 (m, 4H), 7.44 (t, *J=* 7.6 Hz, 2H), 7.38 - 7.32 (m, 2H), 7.25 (s, 1H), 6.88 (d, *J* = 8.4 Hz, 1H), 6.18 (br s, 1H), 5.08 (d, *J=* 10.4 Hz, 1H), 4.88 - 4.74 (m, 1H), 4.52 (br s, 1H), 4.28 (t, *J=* 8.4 Hz, 1H), 3.98 (d, *J* = 10.8 Hz, 1H), 3.87 - 3.75 (m, 1H), 3.30 - 3.10 (m, 2H), 2.50 - 2.37 (m, 1H), 2.29 (s, 3H), 2.10 - 1.96 (m, 2H), 1.04 (d, *J=* 6.5 Hz, 3H), 0.75 (d, *J =* 6.5 Hz, 3H). LCMS (Method 5-95 AB, ESI): R_{T} = 0.764 min, [M+H]⁺ = 519.2.

### Example S32: Synthesis of (2S,4R)-N-((R)-3-([1,1'-biphenyl]-4-yl)-1-amino-1-oxopropan-2-yl)-4-hydroxy-1-((S)-3-methyl-2-(5-methyl-1H-1,2,3-triazol-1-yl)butanoyl)pyrrolidine-2-carboxamide (Compound 33)

Synthesis was carried out following the scheme given below:

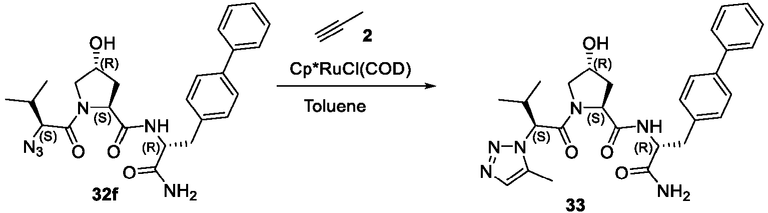

To a solution of (2*S*, 4*R*)-N-((*R*)-3-([1,1'-biphenyl]-4-yl)-1-amino-1-oxopropan -2-yl)-1-((*S*)-2-azido-3-methylbutanoyl)-4-hydroxypyrrolidine-2-carboxamide (200 mg, 0.42 mmol)) in toluene (10 mL) were added 1-propyne (0.50 mL, 0.50 mmol) and chloro(1,5-cyclooctadiene)(pentamethylcyclopentadienyl)ruthenium(II) (6.35 mg, 0.02 mmol). The reaction mixture was stirred at 25 °C for 16 h and filtered. The filtrate was concentrated under reduced pressure and the residue was purified by RP-HPLC (acetonitrile 28-58/0.075%TFA in water) to afford (2*S,* 4*R*)-N-((*R*)-3-([1,1'-biphenyl]-4-yl)-1-amino-1-oxopropan-2-yl)-4-hydroxy-1-((*S*)-3-methyl-2-(5-methyl-1H-1,2,3-triazol-1-yl)butanoyl)pyrrolidine-2-carboxamide (42.0 mg, 19% yield) as a white solid. ¹H NMR (400 MHz, DMSO-*d*₆) δ 7.57 - 7.50 (m, 4H), 7.47 - 7.40 (m, 3H), 7.38 - 7.32 (m, 2H), 7.29 (d, *J* = 8.4 Hz, 2H), 6.93 (br s, 1H), 6.19 (br s, 1H), 4.92 (d, *J* = 10.8 Hz, 1H), 4.87 - 4.75 (m, 1H), 4.48 (br s, 1H), 4.32 (br t, *J* = 8.4 Hz, 1H), 3.78 - 3.66 (m, 2H), 3.30 - 3.15 (m, 3H), 2.85 - 2.72 (m, 1H), 2.38 (s, 3H), 2.12 - 1.94 (m, 2H), 1.06 (d, *J* = 6.5 Hz, 3H), 0.74 (d, *J* = 6.5 Hz, 3H). LCMS (Method 5-95 AB, ESI): R_{T} = 0.764 min, [M+H]⁺ = 519.2.

### Example S33: Synthesis of methyl 1-((R)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate (Compound 34) and methyl 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate (Compound 35)

Synthesis was carried out following the scheme given below:

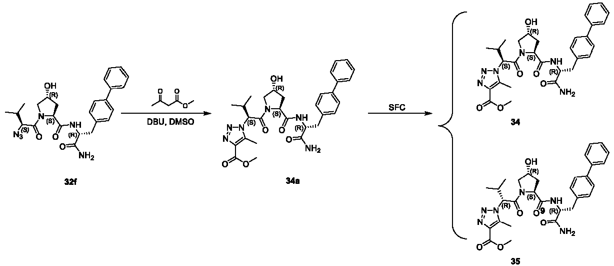

### Preparation of intermediate 34a

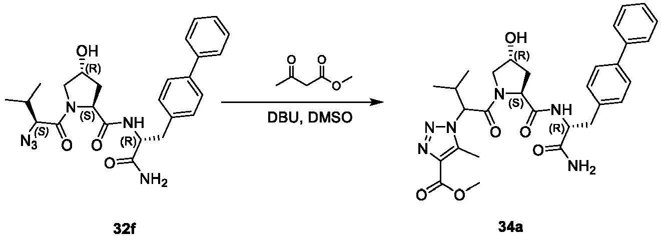

To a solution of (2*S*, 4*R*)-N-((*R*)-3-([1,1'-biphenyl]-4-yl)-1-amino-1-oxopropan-2-yl)-1-((*S*)-2-azido-3-methylbutanoyl)-4-hydroxypyrrolidine-2-carboxamide (450 mg, 0.94 mmol) in dimethyl sulfoxide (5mL) was added 1,8-diazabicyclo[5.4.0]undec-7-ene (0.70 mL, 4.70 mmol) and methyl 3-oxobutanoate (0.30 mL, 2.82 mmol). The reaction mixture was heated at 80 °C for 2 h under microwave conditions and cooled. The mixture was diluted with water (20 mL) and extracted with ethyl acetate (3 x 30 mL). The combined organic layers were dried and concentrated under reduced pressure. The residue was purified by RP-HPLC (water (0.2% FA) - ACN) to afford methyl 1-(1-((2*S*,4*R*)-2-(((*R*)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate (200 mg, 36.9% yield) as a white solid.

### Preparation of compound 34 and compound 35

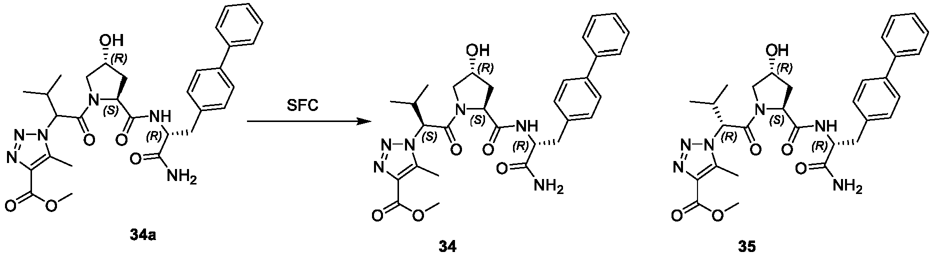

The above diastereomeric mixture was further separated by chiral SFC to give a first-eluting isomer A and a second-eluting isomer B:
**Isomer A:** (Peak 1, retention time = 1.872 min) (100 mg, 49% yield) was obtained as a yellow solid. ¹H NMR (400 MHz, MeOH-*d*₄) δ 7.58 - 7.51 (m, 4H), 7.43 - 7.39 (m, 2H), 7.34 - 7.30 (m, 3H), 5.25 (d, *J* = 10.4 Hz, 1H), 4.59 - 4.57 (m, 1H), 4.48 - 4.46 (m, 1H), 4.31 (s, 1H), 3.92 - 3.90 (m, 3H), 3.76 - 3.72 (m, 1H), 3.42 - 3.40 (m, 1H), 3.24 - 3.22 (m, 1H), 2.89 - 2.79 (m, 2H), 2.57 (s, 3H), 1.94 - 1.89 (m, 1H), 1.63 - 1.56 (m, 1H), 1.15 (d, *J* = 6.8 Hz, 3H), 0.74 (d, *J =* 6.8 Hz, 3H). LCMS (Method 5-95 AB, ESI): R_{T} = 0.767 min, [M+H]⁺ = 577.3.
**Isomer B:** (Peak 2, retention time = 2.195 min) (100 mg, 49% yield) was obtained a yellow solid. ¹H NMR (400 MHz, MeOH-*d*₄) δ 7.59 - 7.53 (m, 4H), 7.43 - 7.39 (m, 2H), 7.34 - 7.29 (m, 3H), 5.17 (d, *J* = 10.0 Hz, 1H), 4.66 - 4.63 (m, 2H), 4.44 - 4.40 (m, 1H), 3.91 (s, 3H), 3.77 - 3.73 (m, 1H), 3.58 - 3.55 (m, 1H), 3.47 - 3.42 (m, 1H), 2.93 - 2.80 (m, 2H), 2.65 (s, 3H), 1.93 - 1.88 (m, 1H), 1.72 - 1.66 (m, 1H), 1.13 (d, *J=* 6.8 Hz, 3H), 0.81 (d, *J* = 6.8 Hz, 3H). LCMS (Method 5-95 AB, ESI): R_{T} = 0.766 min, [M+H]⁺ = 577.3.

### Example S34: Synthesis of 1-((R)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid (Compound 36)

Synthesis was carried out following the scheme given below:

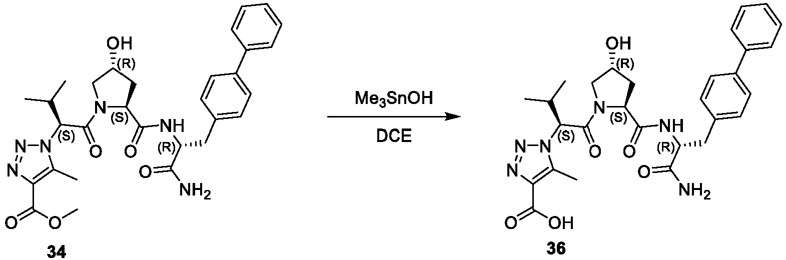

To a solution of methyl 1-((*S*)-1-((2*S*, 4*R*)-2-(((*R*)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate (89.0 mg, 0.15 mmol) in 1,2-dichloroethane (10mL) was added trimethyltin hydroxide (419 mg, 2.32 mmol). The reaction stirred was at 25 °C for 8 h and concentrated under reduced pressure. The mixture was purified by RP-HPLC (water (0.2% FA)-ACN) to afford 1-((*S*)-1-((2*S*, 4*R*)-2-(((*R*)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid (31.3 mg, 35.3% yield) as a white solid. ¹H NMR (400 MHz, MeOH-*d*₄) δ 7.58 - 7.51 (m, 4H), 7.42 - 7.37 (m, 2H), 7.32 - 7.29 (m, 3H), 5.24 (d, *J=* 10.0 Hz, 1H), 4.61 - 4.47 (m, 2H), 4.31 (s, 1H), 3.76 - 3.73 (m, 1H), 3.43 - 3.39 (m, 1H), 3.23 - 3.19 (m, 1H), 2.89 - 2.80 (m, 2H), 2.57 (s, 3H), 1.94 - 1.89 (m, 1H), 1.64 - 1.58 (m, 1H), 1.14 (d, *J=* 6.8 Hz, 3H), 0.74 (d, *J=* 6.8 Hz, 3H). LCMS (Method 5-95 AB, ESI): R_{T} = 0.703 min, [M+H]⁺ = 563.3.

### Example S35: Synthesis of 1-((S)-1-((2S,4R)-2-(((R)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid (Compound 37)

Synthesis was carried out following the scheme given below:

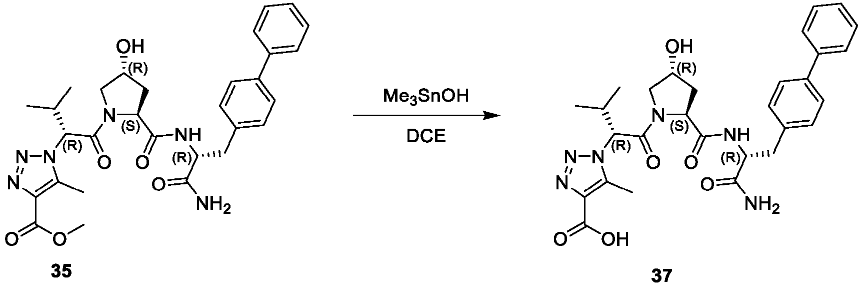

To a solution of methyl 1-((*R*)-1-((2*S*, *R*)-2-(((*R*)-3-([1,1'-biphenyl]-4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylate (67.0 mg, 0.12 mmol) in 1,2-dichloroethane (10mL) was added trimethyltin hydroxide (315 mg, 1.74 mmol). The reaction stirred was at 25 °C for 8 h and concentrated under reduced pressure. The residue was purified by RP-HPLC (water (0.2% FA)-ACN) to afford 1-((*R*)-1-((2*S*, 4*R*)-2-(((*R*)-3-([1,1'-biphenyl] -4-yl)-1-amino-1-oxopropan-2-yl)carbamoyl)-4-hydroxypyrrolidin-1-yl)-3-methyl-1-oxobutan-2-yl)-5-methyl-1H-1,2,3-triazole-4-carboxylic acid (33.9 mg, 50.3% yield) as a white solid. ¹H NMR (400 MHz, MeOH-*d*₄) δ 7.59 - 7.53 (m, 4H), 7.43 - 7.39 (m, 2H), 7.33 - 7.29 (m, 3H), 5.14 (d, *J* = 10.0 Hz, 1H), 4.66 - 4.62 (m, 1H), 4.43 - 4.36 (m, 2H), 3.77 - 3.73 (m, 1H), 3.57 - 3.54 (m, 1H), 3.47 - 3.42 (m, 1H), 2.92 - 2.80 (m, 2H), 2.64 (s, 3H), 1.92 - 1.87 (m, 1H), 1.72 - 1.66 (m, 1H), 1.11 (d, *J =* 6.8 Hz, 3H), 0.81 (d, *J =* 6.8 Hz, 3H). LCMS (Method 5-95 AB, ESI): R_{T} = 0.710 min, [M+H]⁺ = 563.2.

### Example S36: Synthesis of (2S,4R)-1-((S)-2-(1H-benzo[d][1,2,3]triazol-1-yl)-3-methylbutanoyl)-N-((R)-3-([1,1'-biphenyl]-4-yl)-1-amino-1-oxopropan-2-yl)-4-hydroxypyrrolidine-2-carboxamide (Compound 38) and (2S,4R)-1-((R)-2-(1H-benzo[d][1,2,3]triazol-1-yl)-3-methylbutanoyl)-N-((R)-3-([1,1'-biphenyl]-4-yl)-1-amino-1-oxopropan-2-yl)-4-hydroxypyrrolidine-2-carboxamide (Compound 39)

Synthesis was carried out following the scheme given below:

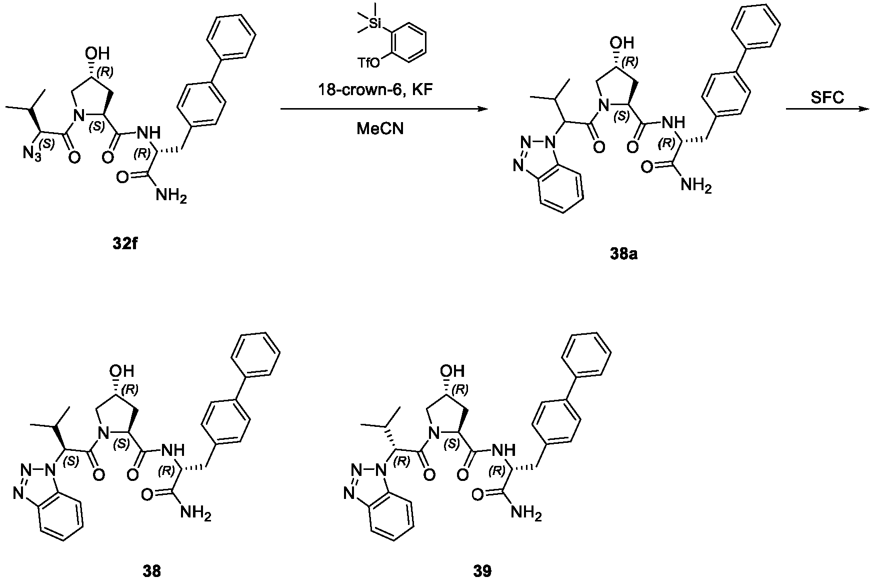

### Preparation of intermediate 38a

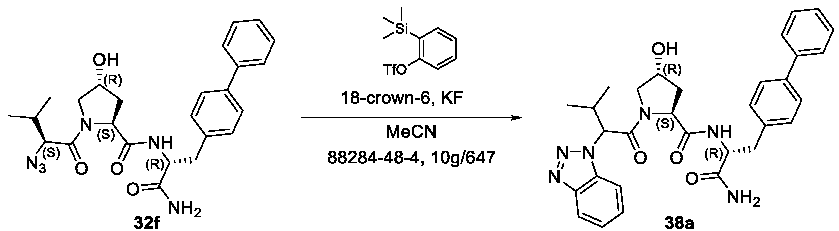

A mixture of (2*S*, 4*R*)-N-((*R*)-3-([1,1'-biphenyl]-4-yl)-1-amino-1-oxopropan-2-yl) -1-((*S*)-2-azido-3-methylbutanoyl)-4-hydroxypyrrolidine-2-carboxamide (200 mg, 0.42 mmol), 18-crown-6 (221 mg, 0.84 mmol), 2-(trimethylsilyl)phenyl trifluoromethanesulfonate (187 mg, 0.63 mmol) and potassium fluoride (97.1 mg, 1.67 mmol) in acetonitrile (4 mL) was stirred at 125 °C for 30 minutes under microwave conditions and filtered. The filtrate was concentrated under reduced pressure. The residue was purified by RP-HPLC (acetonitrile 35 - 65/ 0.075% TFA in water) to afford (2*S*, 4*R*)-1-(2-(1H-benzo[d][1,2,3]triazol-1-yl) -3-methylbutanoyl)-N-((*R*)-3-([1,1'-biphenyl]-4-yl)-1-amino-1-oxopropan-2-yl)-4-hydroxypyrrolidine-2-carboxamide (85.0 mg, 36.7% yield) as a white solid.

### Preparation of compound 38 and compound 39

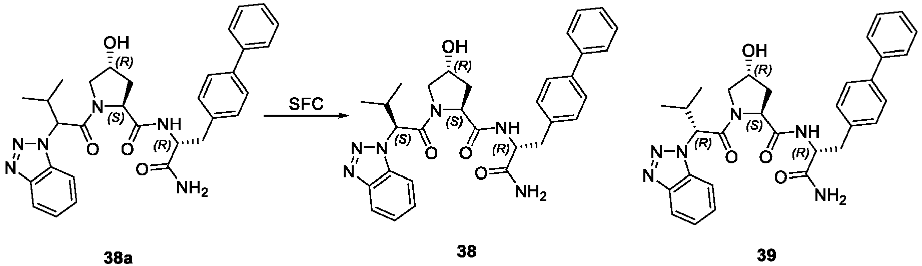

The above diastereomeric mixture was further separated by chiral SFC to give a first-eluting Isomer A and a second-eluting Isomer B:
**Isomer A:** (Peak 1, retention time = 4.646 min) (24.4 mg, 27.9% yield) as a white solid. ¹H NMR (400 MHz, DMSO-*d*₆) δ 8.53 (d, *J =* 8.8 Hz, 1H), 8.06 - 8.00 (m, 2H), 7.63 (d, *J=* 7.6 Hz, 2H), 7.58 - 7.51 (m, 3H), 7.49 - 7.38 (m, 4H), 7.36 - 7.28 (m, 4H), 5.59 (d, *J=* 10.4 Hz, 1H), 5.04 (d, *J=* 3.6 Hz, 1H), 4.39 - 4.53 (m, 1 H), 4.20 - 4.33 (m, 2H), 3.83 (dd, *J =* 10.8, 4.0 Hz, 1H), 3.48 (br d, *J=* 10.8 Hz, 1H), 3.22 - (m, 1H), 2.61 - 2.94 (m, 2 H), 1.70 - 1.85 (m, 1 H), 1.50 - 1.60 (m, 1H), 1.08 (d, *J=* 6.8 Hz, 3H), 0.54 (d, *J* = 6.8 Hz, 3H). LCMS (Method 5-95 AB, ESI): R_{T} = 0.818 min, [M+H]⁺ = 555.1.
**Isomer B:** (Peak 2, retention time = 5.434 min) (47.0 mg, 54.2% yield) as a white solid. ¹H NMR (400 MHz, DMSO-*d*₆) δ 8.51 (d, *J =* 8.8 Hz, 1H), 7.97 - 8.06 (m, 1H), 7.85 (d, *J=* 8.4 Hz, 1H), 7.60 (d, *J =* 7.6 Hz, 2H), 7.53 - 7.47 (m, 3H), 7.46 - 7.38 (m, 4H), 7.31 - 7.22 (m, 4H), 5.66 (d, *J=* 10.2 Hz, 1H) 5.26 - 5.09 (m, 1H), 4.68 - 4.51 (m, 1H), 4.42 (t, *J=* 8.0 Hz, 1H), 4.28 - 4.19 (m, 1H), 4.15 (br s, 1H), 3.78 (br d, *J* =11.2 Hz, 1H), 3.21 - 3.05 (m, 2H), 2.87 - 2.79 (m, 1H), 2.71 - 2.70 (m, 1H), 1.85 - 1.72 (m, 1H), 1.43-1.42 (m, 1H), 1.08 (d, *J=* 6.6 Hz, 3H), 0.46 (d, *J* = 6.8 Hz, 2H). LCMS (Method 5-95 AB, ESI): R_{T} = 0.824 min, [M+H]⁺ = 555.1.

### Example S37: Synthesis of (2S,4R)-N-((R)-3-([1,1'-biphenyl]-4-yl)-1-amino-1-oxopropan-2-yl)-1-((S)-2-(4,5-dimethyl-1H-1,2,3-triazol-1-yl)-3-methylbutanoyl)-4-hydroxypyrrolidine-2-carboxamide (Compound 40)

Synthesis was carried out following the scheme given below:

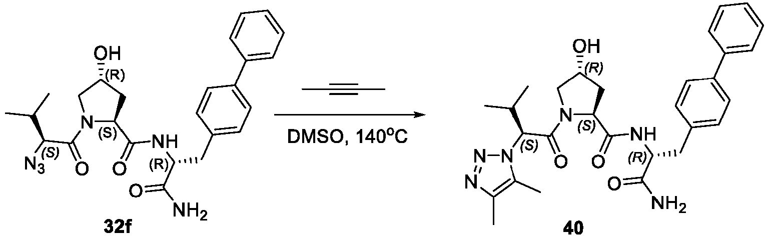

To a solution of (2*S*, 4*R*)-N-((*R*)-3-([1,1'-biphenyl]-4-yl)-1-amino -1-oxopropan-2-yl)-1-((*S*)-2-azido-3-methylbutanoyl)-4-hydroxypyrrolidine-2-carboxamide (200 mg, 0.42 mmol) in dimethyl sulfoxide (2mL) was added but-2-yne (0.07mL, 0.84 mmol). The reaction was stirred at 140 °C for 6 h and filtered. The filtrate was concentrated under reduced pressure. The residue was purified by RP-HPLC (acetonitrile 30-60/0.075% in water) to afford (2*S*, 4*R*)-N-((*R*)-3-([1,1'-biphenyl]-4-yl)-1-amino-1-oxopropan-2-yl)-1-((*S*)-2-(4,5-dimethyl-1H-1,2,3-triazol-1-yl)-3-methylbutanoyl)-4-hydroxypyrrolidine-2-carboxamide (75.0 mg, 32.3% yield) as white solid. ¹H NMR (400 MHz, CDCl₃) δ 7.57 - 7.49 (m, 4H), 7.42 (t, *J* = 7.5 Hz, 2H), 7.37 - 7.28 (s, 4H), 7.26 (s, 1H), 6.24 (br s, 1H), 4.90 (br d, *J=* 10.8 Hz, 1H), 4.84 - 4.74 (m, 1H), 4.45 (br s, 1H), 4.37 (br t, *J=* 8.0 Hz, 1H), 3.78 - 3.73 (m, 2H), 3.31 - 3.26 (m, 1H), 3.12 - 3.06 (m, 1H), 2.83 - 2.70 (m, 1H), 2.31 (s, 3H), 2.21 (s, 3H), 2.08 - 2.03 (m, 1H), 1.96 - 1.84 (m, 1H), 1.07 (d, *J* = 6.6 Hz, 3H), 0.74 (d, *J=* 6.6 Hz, 3H). LCMS (Method 5-95 AB, ESI): R_{T} = 0.760 min, [M+H]⁺ = 533.3.

### Example S38: Synthesis of (2S,4R)-N-((R)-3-([1,1'-biphenyl]-4-yl)-1-amino-1-oxopropan-2-yl)-4-hydroxy-1-((S)-3-methyl-2-(4-(thiophen-2-yl)-1H-1,2,3-triazol-1-yl)butanoyl)pyrrolidine-2-carboxamide (Compound 42)

Synthesis was carried out following the scheme given below:

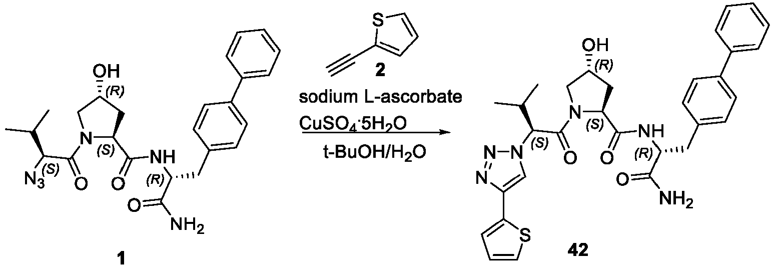

To a solution of sodium L-ascorbate (166 mg, 0.84 mmol) in water (4 mL) and tert-Butyl alcohol (4 mL) were added 2-ethynylthiophene (22.6 mg, 0.21 mmol), copper sulfate pentahydrate (67.8 mg, 0.27 mmol) and (2*S*, 4*R*)-N-((*R*)-3-([1,1'-biphenyl]-4-yl)-1-amino-1-oxopropan-2-yl)-1-((*S*)-2-azido-3-methylbutanoyl)-4-hydroxypyrrolidine-2-carboxamide (100 mg, 0.21 mmol). The reaction mixture was stirred at 25 °C for 12 h and concentrated under reduced pressure. The residue was purified by RP-HPLC (acetonitrile 35-75/0.075% in water) to afford (2S,4R)-N-((R)-3-([1,1'-biphenyl]-4-yl)-1-amino-1-oxopropan-2-yl) -4-hydroxy-1-((S)-3-methyl-2-(4-(thiophen-2-yl)-1H-1,2,3-triazol-1-yl)butanoyl)pyrrolidine-2-carboxamide (48.8 mg, 39% yield) as a white solid. ¹H NMR (400 MHz, CDCl₃) δ 8.01 (s, 1H), 7.47 - 7.57 (m, 4H), 7.39 - 7.46 (m, 3H), 7.29 - 7.38 (m, 2H), 7.18 - 7.26 (m, 3H), 7.02 - 6.87 (m, 1H), 6.90 (br d, *J =* 8.4 Hz, 1H), 6.43 (br s, 1H), 5.14 (d, *J =* 10.2 Hz, 1H), 4.74 - 4.87 (m, 1H), 4.53 (br s, 1H), 4.26 - 4.38 (m, 1H), 3.99 (d, *J=* 10.8 Hz, 1H), 3.75 - 3.87 (m, 1H), 3.17 - 3.27 (m, 1H), 3.07 - 3.16 (m, 1H), 2.41 - 2.54 (m, 1H), 1.96 - 2.13 (m, 2H), 1.05 (d, *J =* 6.4 Hz, 3H), 0.79 (d, *J =* 6.4 Hz, 3H). LCMS (Method 5-95 AB, ESI): R_{T} = 0.816 min, [M+H]⁺ = 587.2.

### Example S39: Synthesis of (2S,4R)-N-((R)-3-([1,1 '-biphenyl]-4-yl)-1-amino-1-oxopropan-2-yl)-1-((S)-2-(4-(furan-2-yl)-1H-1,2,3-triazol-1-yl)-3-methylbutanoyl)-4-hydroxypyrrolidine-2-carboxamide (Compound 43)

Synthesis was carried out following the scheme given below:

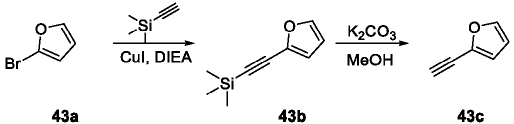

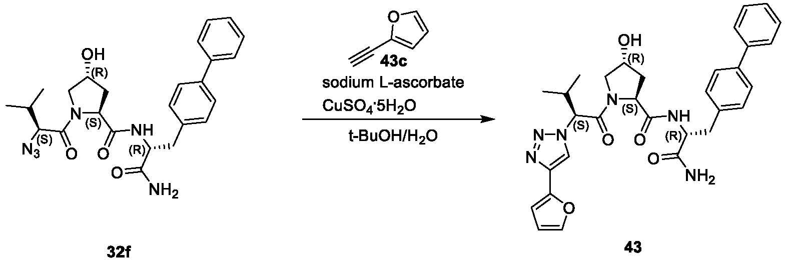

### Preparation of intermediate 43b

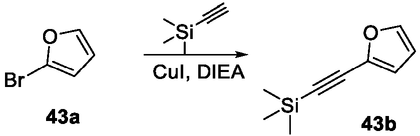

To a solution of 2-bromofuran (1.50 g, 10.2 mmol), bis-triphenylphosphine-palladium(II) chloride (227 mg, 0.32 mmol), copper(I) iodide (120 mg, 0.63 mmol) and diisopropylamine (2.67 mL, 19.2 mmol) in tetrahydrofuaran (12 mL) was degassed thoroughly with argon, trimethylsilyl acetylene (1.94 mL, 14.1 mmol) was added at 25 oC. Then the reaction mixture was stirred at 25 °C for 16 h and concentrated under reduced pressure. The residue was purified by column chromatography (silica gel, 100-200 mesh, 0 - 2% ethyl acetate in petroleum ether) to afford (furan-2-ylethynyl)trimethylsilane (0.60 g, 35.8% yield) as a color oil.

### Preparation of compound 43c

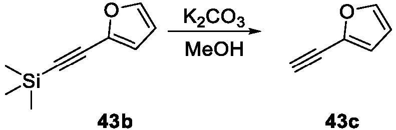

To a solution of (furan-2-ylethynyl)trimethylsilane (250 mg, 1.52 mmol) in methanol (3mL) was added potassium carbonate (0.48 g, 3.50 mmol). The reaction mixture was stirred at room temperature for 18 h and diluted with water (30 mL). The mixture was extracted with dichloromethane (3 x 30 mL). The combined organic phase was washed with water (20 mL), brine (3 x 20 mL), dried and concentrated to give crude 2-ethynylfuran (140 mg, 99.9% yield) as a yellow oil.

### Preparation of compound 43

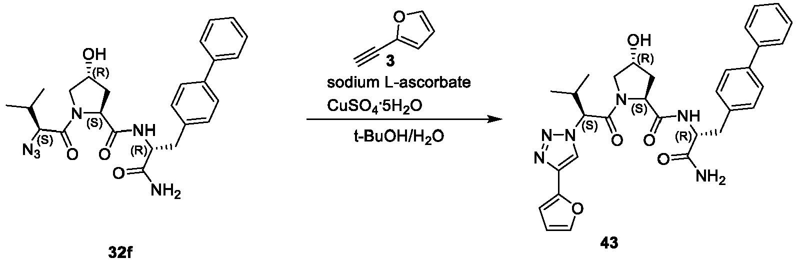

To a solution of (2*S*, 4*R*)-N-((*R*)-3-([1,1'-biphenyl]-4-yl)-1-amino -1-oxopropan-2-yl)-1-((*S*)-2-azido-3-methylbutanoyl)-4-hydroxypyrrolidine-2-carboxamide (110 mg, 0.23 mmol) in water (4 mL) and tert-butyl alcohol (4 mL) was added 2-ethynylfuran (0.28 mL, 0.28 mmol), copper sulfate pentahydrate (74.6 mg, 0.30 mmol) and sodium L-ascorbate (182 mg, 0.92 mmol). The reaction was stirred at 25 °C for 16 h and filtered. The filtrate was concentrated under reduced pressure and the residue was purified by RP-HPLC (acetonitrile 33 - 63/0.075 % in water) to afford (2*S*, 4*R*)-N-((*R*)-3-([1,1'-biphenyl]-4-yl)-1-amino-1-oxopropan-2-yl) -1-((*S*)-2-(4-(furan-2-yl)-1H-1,2,3-triazol-1-yl)-3-methylbutanoyl)-4-hydroxypyrrolidine-2-carboxamide (35.0 mg, 26.4% yield) as white solid. ¹H NMR (400 MHz, CDCl₃) δ 8.06 (s, 1H), 7.56 - 7.35 (m, 10H), 7.35 - 7.30 (m, 1H), 7.27 (s, 1H), 7.20 (d, *J =* 8.0 Hz, 2H), 6.70 (d, *J =* 3.2 Hz, 1H), 6.62 (br s, 1H), 6.40 - (m, 1H), 5.22 - 5.10 (m, 1H), 4.84 - 4.75 (m, 1H), 4.46 (br s, 1H), 4.37 - 4.33 (m, 1H), 3.95 (d, *J=* 10.8 Hz, 1H), 3.82 - 3.71 (m, 1H), 3.28 -3.25 (m, 1H), 3.01 - 2.98 (m, 1H), 2.52 - 2.39 (m, 1H), 2.09 - 2.00 (m, 1H), 1.97 - 1.86 (m, 1H), 1.03 (d, *J=* 6.4 Hz, 3H), 0.76 (d, *J=* 6.6 Hz, 3H). LCMS (Method 5-95 AB, ESI): R_{T} = 0.796 min, [M+H]⁺ = 571.2.

### Example S40: Synthesis of (2S, 4R)-1-((S)-2-(4-cyclopropyl-1H-1,2,3-triazol-1-yl)-3,3-dimethylbutanoyl)-4-hydroxy-N-((R)-2,2,2-trifluoro-1-(4-(4-methylthiazol-5-yl)phenyl)ethyl)pyrrolidine-2-carboxamide (Compound 46)

Synthesis was carried out following the scheme given below:

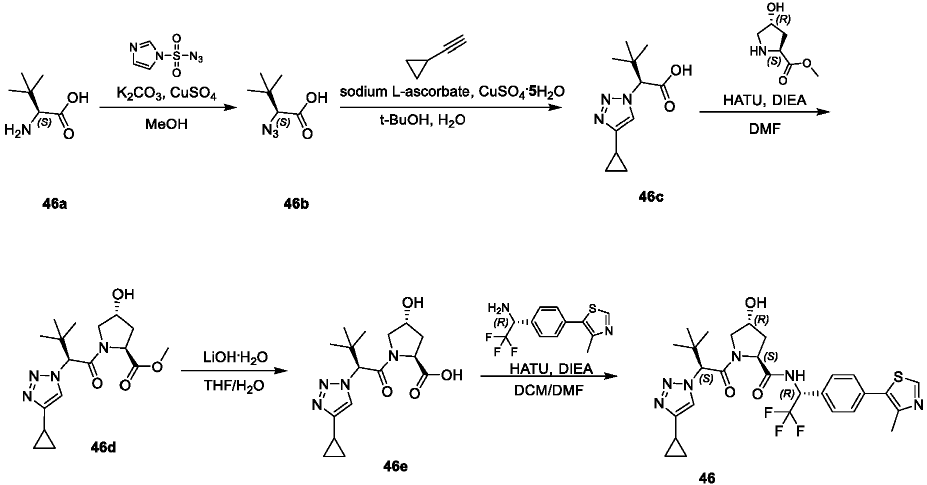

### Preparation of intermediate 46b

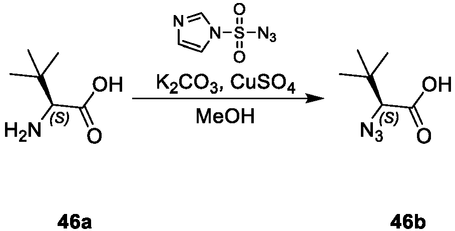

To a mixture of (*S*)-2-amino-3,3-dimethylbutanoic acid (8.00 g, 61.0 mmol), potassium carbonate (21.2 g, 152 mmol) and cupric sulfate (976 mg, 6.10 mmol) in methanol (100 mL) was added 1H-imidazole-1-sulfonyl azide (12.8 g, 61.0 mmol) at 25 °C. The reaction mixture was stirred at 25 °C for 16 h. The reaction mixture was diluted with water (60 mL) and concentrated in vacuum to remove methanol. The aqueous phase was adjust to pH = 3 with potassium bisulfate and extracted with ethyl acetate (100 mL). The organic layer was separated and concentrated to give crude (S)-2-azido-3,3-dimethylbutanoic acid (8.00 g, 83.5% yield) as yellow oil.

### Preparation of intermediate 46c

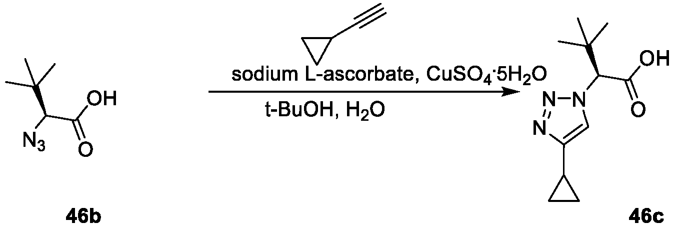

To a solution of (*S*)-2-azido-3,3-dimethylbutanoic acid (500 mg, 3.18 mmol) and ethynylcyclopropane (0.40 mL, 4.77 mmol) in tert-butyl alcohol (5 mL) and water (10 mL) mixture (1:2), copper (II) sulfate pentahydrate (397 mg, 1.59 mmol) and sodium L-ascorbate (63.0 mg, 0.32 mmol) was added 25 °C. The reaction mixture was stirred vigorously at 25 °C for 4 h. After the reaction was completed, the reaction mixture was quenched with water (30 mL) and the residue was extracted with ethyl acetate (3 x 40 mL). The organic layers were combined and washed with water (30.0 mL) and brine (30 mL). The organic layer was separated and concentrated to dryness to give (*S*)-2-(4-cyclopropyl-1H-1,2,3-triazol-1-yl)-3,3-dimethylbutanoic acid (560 mg, 78.8% yield) as a blue oil.

### Preparation of intermediate 46d

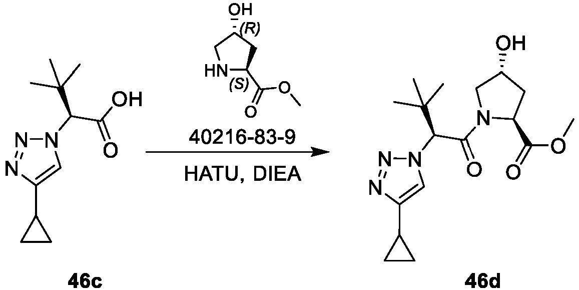

A solution of (8)-2-(4-cyclopropyl-1H-1,2,3-triazol-1-yl)-3,3-dimethylbutanoic acid (554 mg, 2.48 mmol), methyl (2S, 4R)-methyl 4-hydroxypyrrolidine-2-carboxylate (300 mg, 2.07 mmol) and N,N-diisopropylethylamine (1.02 mL, 6.20 mmol) in anhydrous N,N-dimethylformamide (25 mL), 1-[Bis(dimethylamino)methylene]-1H-1,2,3-triazolo[4,5-b]pyridinium 3-Oxide Hexafluorophosphate (943 mg, 2.48 mmol) was added at 0 °C. The reaction mixture was stirred at 25 °C for 2 h. The reaction mixture was quenched with water (30 mL) and extracted with ethyl acetate (3 x 30 mL). The combined organic layers were washed with water (30 mL), brine (30 mL), dried and concentrated to dryness. The residue was purified by pre-HPLC (water (0.2% FA) - ACN) to afford (2*S*, 4*R*)-methyl-((*S*)-2-(4-cyclopropyl-1H-1, 2, 3-triazol-1-yl)-3,3 -dimethylbutanoyl)-4-hydroxypyrrolidine-2-carboxylate (320 mg, 44.2% yield) as a white solid.

### Preparation of intermediate 46e

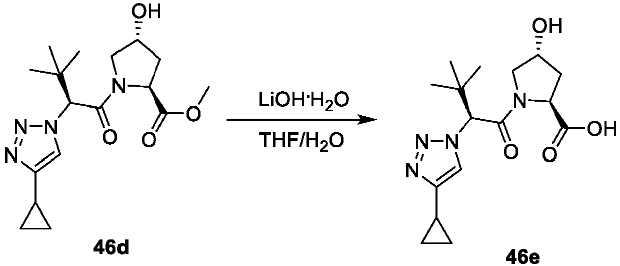

To a solution of (2S, 4R)-methyl 1-((S)-2-(4-cyclopropyl-1H-1,2,3-triazol-1-yl)-3,3-dimethylbutanoyl)-4-hydroxypyrrolidine-2-carboxylate (100 mg, 0.29 mmol) in tetrahydrofuran (10 mL) and water (10 mL) was added lithium hydroxide monohydrate (12.0 mg, 0.29 mmol) at 25 °C. The reaction mixture was stirred at 25 °C for 2 h. The reaction mixture was concentrated in vacuum to remove tetrahydrofuran. 5% potassium bisulfate was added to the reaction mixture until pH = 5. The residue was extracted with ethyl acetate (3 x 30 mL). The combined organic layers were washed with water (30 mL), brine (30 mL), dried and concentrated to afford crude (2*S*, 4*R*)-1-((S)-2-(4-cyclopropyl-1H-1,2,3-triazol-1-yl)-3,3-dimethylbutanoyl)-4-hydroxypyrrolidine-2-carboxylic acid (80.0 mg, 83.3% yield) as a white solid.

### Preparation of 46

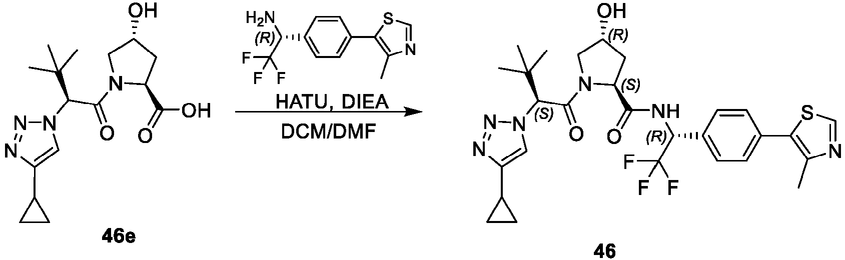

A solution of (2*S*, 4*R*)-1-((S)-2-(4-cyclopropyl-1H-1, 2, 3-triazol-1-yl)-3, 3-dimethylbutanoyl)-4-hydroxypyrrolidine-2-carboxylic acid (89.0 mg, 0.26 mmol) (*R*)-2,2,2-trifluoro-1-(4-(4-methylthiazol-5-yl)phenyl)ethanamine (60.0 mg, 0.22 mmol) and N,N-diisopropylethylamine (0.11 mL, 0.66 mmol) in anhydrous N,N-dimethylformamide (25 mL) was added 1-[Bis(dimethylamino)methylene]-1H-1,2,3-triazolo[4,5-b]pyridinium 3-Oxide Hexafluorophosphate (100 mg, 0.26 mmol) was added at 0 °C. The reaction mixture was stirred at 25°C for 8 h. The reaction mixture was quenched with water (30 mL) and extracted with ethyl acetate (3 x 30 mL). The combined organic layers were washed with water (30 mL), brine (30 mL), dried and concentrated to dryness. The residue was purified by pre-HPLC (water (0.2% FA) - ACN) to afford **compound 46** (2*S,* 4*R*)-1-((*S*)-2-(4-cyclopropyl-1H-1,2,3-triazol-1-yl)-3,3-dimethylbutanoyl)-4-hydroxy-N-((*R*)-2,2,2-trifluoro-1-(4-(4-methylthiazol-5-yl)phenyl)ethyl)pyrrolidine-2-carboxamide (29.2 mg, 21.3% yield) as a white solid.
¹H NMR (400 MHz, MeOH-*d*₄) δ 8.92 (m, 1H), 8.01 (s, 1H), 7.60 - 7.52 (m, 4H), 5.77 - 5.71 (m, 1H), 5.47 (s, 1H), 4.87 - 4.60 (m, 2H), 4.44 (s, 1H), 3.88 - 3.72 (m, 2H), 2.51 - 2.50 (m, 3H), 2.21 - 2.16 (m, 1H), 2.00 - 1.93 (m, 2H), 1.01 - 0.95 (m, 9H), 0.80 - 0.79 (m, 2H), 0.78 - 0.77 (m, 2H). LCMS (Method 5-95 AB, ESI): R_{T} = 0.760 min, [M+H]⁺ = 591.1.

### Example S41: Synthesis of (2S,4R)-1-((S)-2-(4-cyclopropyl-1H-1,2,3-triazol-1-yl)-3,3-dimethylbutanoyl)-N-((S)-1-(2'-fluoro-[1,1'-biphenyl]-4-yl)ethyl)-4-hydroxypyrrolidine-2-carboxamide (Compound 47)

Synthesis was carried out following the scheme given below:

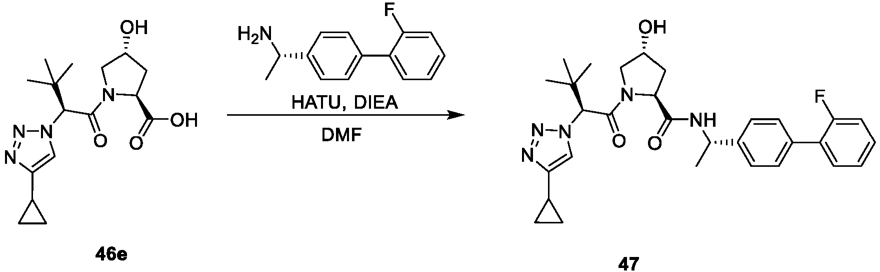

### Preparation of intermediate 47b

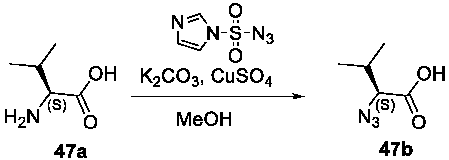

To a mixture of (S)-2-amino-3-methylbutanoic acid (1.00 g, 8.54 mmol), potassium carbonate (2.97 g, 21.3 mmol) and copper sulfate (136.6 mg, 0.85 mmol) in methanol (20 mL) was added 1H-imidazole-1-sulfonyl azide (1.79 g, 8.54 mmol) at 25 °C. The reaction was stirred for 16 h and diluted with water (60 mL). The methanol was removed under reduced pressure and the aqueous residue was washed with ethyl acetate (100 mL). The aqueous was then adjusted to pH = 5 by addition of potassium bisulfate and extracted with ethyl acetate (2 x 150 mL). The combined organic layers were dried and concentrated in vacuum to give crude (S)-2-azido-3-methylbutanoic acid (1.20 g, 98.2% yield) as a yellow oil.

### Preparation of intermediate 47c

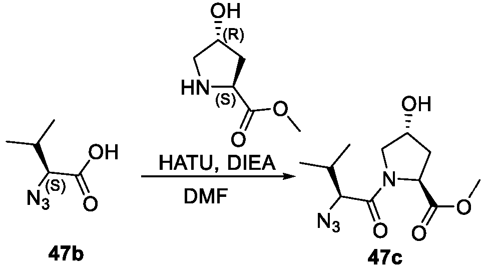

A mixture of (*S*)-2-azido-3-methylbutanoic acid (1.00 g, 6.99 mmol), (2*S*, 4*R*)-methyl 4-hydroxypyrrolidine-2-carboxylate (1.01 g, 6.99 mmol), N,N-diisopropylethylamine (5.77 mL, 34.9 mmol) and (1-[Bis(dimethylamino)methylene]-1H-1,2,3-triazolo[4,5-b] pyridinium 3-oxid hexafluorophosphate (2.66 g, 6.99 mmol) in N,N-dimethylformamide (10 mL) was stirred at 25 °C for 3 h and diluted with water (50 mL). The mixture was extracted with ethyl acetate (3 x 50 mL). The combined organic layers were washed with brine (50 mL), dried and concentrated to afford crude (2*S*, 4*R*)-methyl 1-((*S*)-2-azido-3-methylbutanoyl)-4-hydroxypyrrolidine -2-carboxylate (1.00 g, 53% yield) as a blue oil.

### Preparation of intermediate 47d

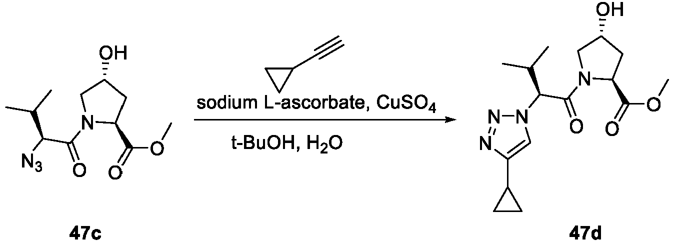

To a solution of sodium L-ascorbate (2.93 g, 14.8 mmol) in water (20 mL) and tert-butyl alcohol (20 mL) was added ethynylcyclopropane (0.31 mL, 3.7mmol), copper sulfate pentahydrate (1.51 g, 4.81 mmol) and (2*S*,4*R*)-methyl 1-((*S*)-2-azido-3-methylbutanoyl)-4-hydroxypyrrolidine-2-carboxylate (1.00 g, 3.70 mmol). The reaction was stirred at 25 °C for 16 h and concentrated under reduced pressure. The residue was purified by column chromatography (silica gel, 100-200 mesh, 0-4% ethyl acetate in petroleum ether) to afford (2*S*,4*R*)-methyl 1-((*S*)-2-(4-cyclopropyl-1H-1,2,3-triazol-1-yl)-3-methylbutanoyl)-4-hydroxypyrrolidine-2-carboxylate (1.10 g, 88.4% yield) as a yellow solid.

### Preparation of intermediate 47e

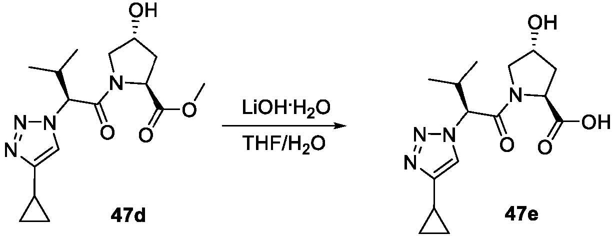

To a solution of (2*S*,4*R*)-methyl 1-((*S*)-2-(4-cyclopropyl-1H-1,2,3-triazol-1-yl) -3-methylbutanoyl)-4-hydroxypyrrolidine-2-carboxylate (300 mg, 0.89 mmol) in water (5 mL) and tetrahydrofuran (10 mL) were added lithium hydroxide monohydrate (37.42 mg, 0.89 mmol). The reaction was stirred at 25 °C for 16 h. The reaction mixture was partitioned between ethyl acetate (20 mL) and water (15 mL). The aqueous layer was adjusted to pH = 4 by addition of hydrochloric acid (2 M) and extracted with ethyl acetate (3 x 50 mL). The combined organic layers were dried and concentrated under reduced pressure to give crude (2*S*,4*R*)-1-((*S*)-2-(4-cyclopropyl -1H-1,2,3-triazol-1-yl)-3-methylbutanoyl)-4-hydroxypyrrolidine-2-carboxylic acid (200 mg, 69.6% yield) as a light yellow oil.

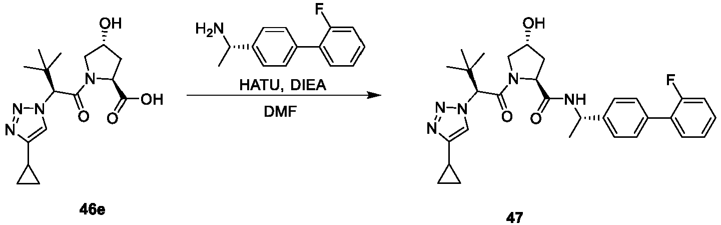

A mixture of (2*S*,4*R*)-1-((*S*)-2-(4-cyclopropyl-1H-1,2,3-triazol-1-yl) -3,3-dimethylbutanoyl)-4-hydroxypyrrolidine-2-carboxylic acid (113 mg, 0.33 mmol), (*S*)-1-(2'-fluoro-[1,1'-biphenyl]-4-yl)ethanamine (60.0 mg, 0.28 mmol), N,N-diisopropylethylamine (0.14 mL, 0.84 mmol) and (1-[Bis(dimethylamino)methylene]-1H-1,2,3-triazolo[4,5-b] pyridinium 3-oxid hexafluorophosphate (127 mg, 0.33 mmol) in N,N-dimethylformamide (5mL) was stirred at 20 °C for 2 h and concentrated under reduced pressure. The residue was purified by RP-HPLC (water (0.2%FA)-ACN) to afford (2*S*,4*R*)-1-((*S*)-2-(4-cyclopropyl-1H-1,2,3-triazol-1-yl)-3,3-dimethylbutanoyl)-N-((*S*)-1-(2'-fluoro-[1,1'-biphenyl]-4-yl)ethyl)-4-hydroxypyrrolidine-2-carboxamide (47.1 mg, 31.4% yield) as a white solid. ¹H NMR (400 MHz, MeOH-*d*₄) δ 7.89 (s, 1H), 7.55 - 7.53 (m, 2H), 7.53 - 7.42 (m, 3H), 7.42 - 7.40 (m, 1H), 7.27 - 7.23 (m, 1H), 7.21 - 7.15 (m, 2H), 5.49 (s, 1H), 5.19 - 5.13 (m, 1H), 4.59 - 4.53 (m, 2H), 4.01 (d, *J =* 6.8 Hz, 1H), 3.74 (d, *J =* 6.8 Hz, 1H), 2.38 - 2.34 (m, 1H), 2.03 - 1.92 (m, 3H), 1.57 (d, *J=* 6.8 Hz, 3H), 1.08 (s, 9H), 0.94 - 0.80 (m, 4H). LCMS (Method 5-95 AB, ESI): R_{T} = 0.862 min, [M+H]⁺ = 534.7.

### Example S42: Synthesis of (2S,4R)-1-((S)-2-(4-cyclopropyl-1H-1,2,3-triazol-1-yl)-3,3-dimethylbutanoyl)-N-((R)-1-(2'-fluoro-[1,1'-biphenyl]-4-yl)ethyl)-4-hydroxypyrrolidine-2-carboxamide (Compound 48)

Synthesis was carried out following the scheme given below:

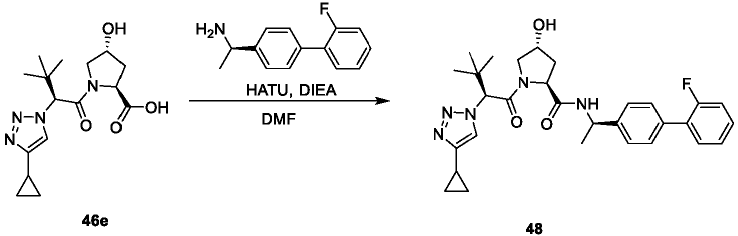

A mixture of (2S, 4R)-1-((S)-2-(4-cyclopropyl-1H-1,2,3-triazol-1-yl) -3,3-dimethylbutanoyl)-4-hydroxypyrrolidine-2-carboxylic acid (113 mg, 0.33 mmol), (R)-1-(2'-fluoro-[1,1'-biphenyl]-4-yl)ethanamine (60.0 mg, 0.28 mmol), N,N-diisopropylethylamine (0.14 mL, 0.84 mmol) and (1-[Bis(dimethylamino)methylene]-1H-1,2,3-triazolo[4,5-b] pyridinium 3-oxid hexafluorophosphate (127 mg, 0.33 mmol) in N,N-dimethylformamide (5 mL) was stirred at 20 °C for 2 h and concentrated under reduced pressure. The residue was purified by RP-HPLC (water (0.2% FA)-ACN) to afford (2*S*,4*R*)-1-((*S*)-2-(4-cyclopropyl-1H-1,2,3-triazol-1-yl)-3,3-dimethylbutanoyl)-N-((*R*)-1-(2'-fluoro-[1,1'-biphenyl]-4-yl)ethyl)-4-hydroxypyrrolidine-2-carboxamide (36.9 mg, 24.6% yield) as a white solid. ¹H NMR (400 MHz, CDCl₃) δ 7.89 (s, 1H), 7.60 - 7.41 (m, 5H), 7.31 (s, 1H), 7.24 - 7.08 (m, 3H), 5.43 (s, 1H), 5.20 - 5.08 (m, 1H), 4.76 - 4.51 (m, 2H), 4.01 (br d, *J* = 12 Hz, 1H), 3.67 (br d, *J =* 8 Hz, 1H), 2.68 - 2.46 (m, 1H), 2.09 (br d, *J =* 9.1 Hz, 2H), 1.91 (br s, 1H), 1.56 (br d, *J=* 4 Hz, 3H), 1.00 - 0.80 (m, 13H). LCMS (Method 5-95 AB, ESI): R_{T} = 0.861 min, [M+H]⁺ = 534.7.

### Example S43: Synthesis of (2S, 4R)-1-((S)-2-(4-cyclopropyl-1H-1,2,3-triazol-1-yl)-3,3-dimethylbutanoyl)-N-((2'-fluoro-[1,1'-biphenyl]-4-yl)methyl)-4-hydroxypyrrolidine-2-carboxamide (Compound 49)

Synthesis was carried out following the scheme given below:

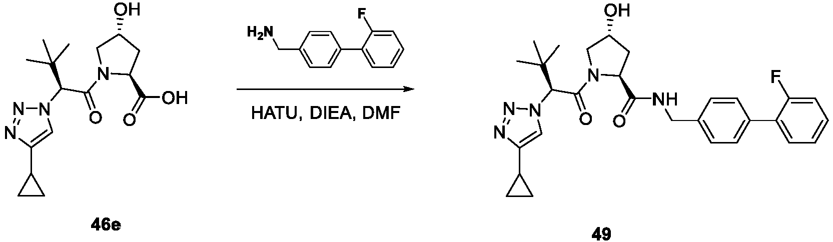

A solution of (2*S*, 4*R*)-1-((*S*)-2-(4-cyclopropyl-1H-1,2,3-triazol-1-yl)-3,3-dimethylbutanoyl)-4-hydroxypyrrolidine-2-carboxylic acid (162 mg, 0.48 mmol), (2'-fluoro-[1,1'-biphenyl]-4-yl)methanamine (80.78 mg, 0.40 mmol) and N,N-diisopropylethylamine (0.11 mL, 0.66 mmol) in anhydrous N,N-dimethylformamide (5 mL) was added 1-[Bis(dimethylamino)methylene]-1H-1,2,3-triazolo[4,5-b]pyridinium 3-Oxide Hexafluorophosphate (200 mg, 0.52 mmol) at 25 °C. The reaction mixture was stirred at 25 °C for 2 h. The reaction mixture was quenched with water (20 mL) and extracted with ethyl acetate (3 x 20 mL). The combined organic layers were dried and concentrated to dryness. The residue was purified by pre-HPLC (water (0.2% FA) - ACN 45% ~ 75%) to afford **compound 49** (2*S*, 4*R*)-1-((*S*)-2-(4-cyclopropyl-1H-1,2,3-triazol-1-yl)-3,3-dimethylbutanoyl)-N-((2'-fluoro-[1,1'-biphenyl]-4-yl)methyl)-4-hydroxypyrrolidine-2-carboxamide (100 mg, 47% yield) as a white solid.

¹H NMR (400 MHz, CDCl₃) δ 7.87 (s, 1H), 7.52 - 7.50 (m, 2H), 7.49 - 7.40 (m, 3H), 7.38 - 7.26 (m, 1H), 7.22 - 7.20 (m, 2H), 7.14 - 6.94 (m, 1H), 5.48 (s, 1H), 4.62 - 4.58 (m, 3H), 4.55 - 4.54 (m, 1H), 4.48 - 4.46 (m, 1H), 4.08 (s, 1H), 4.05 - 3.75 (m, 1H), 2.45 - 2.44 (m, 1H), 2.11 - 1.89 (m, 1H), 1.89 - 1.88 (m, 1H), 1.04 - 0.99 (m, 9H), 0.93 - 0.90 (m, 2H), 0.80 - 0.78 (m, 3H). LCMS (Method 5-95 AB, ESI): R_{T} = 0.868 min, [M+H]⁺ = 520.1.

### Example S44: Synthesis of (2S,4R)-1-((S)-2-(4-cyclopropyl-1H-1,2,3-triazol-1-yl)-3,3-dimethylbutanoyl)-4-hydroxy-N-((R)-2-(4-methylthiazol-5-yl)-6,7,8,9-tetrahydro-5H-benzo[7]annulen-5-yl)pyrrolidine-2-carboxamide (Compound 52)

Synthesis was carried out following the scheme given below:

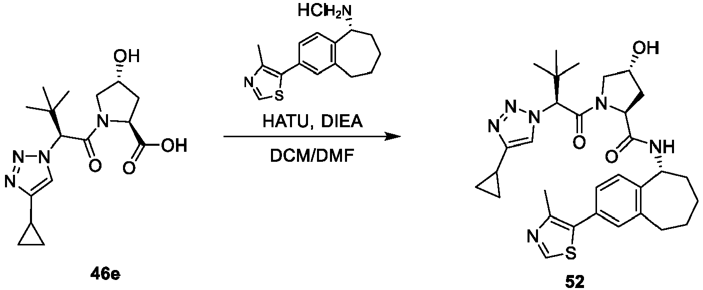

A mixture of (R)-2-(4-methylthiazol-5-yl)-6,7,8,9-tetrahydro-5H-benzo [7]annulen-5-amine hydrochloride (65.0 mg, 0.25 mmol), (25,4R)-1-((S)-2-(4-cyclopropyl-1H-1,2,3-triazol-1-yl)-3,3-dimethylbutanoyl)-4-hydroxypyrrolidine-2-carboxylic acid (70.0 mg, 0.21 mmol), N,N-diisopropylethylamine (0.09 mL, 0.52 mmol) and (1-[Bis(dimethylamino)methylene]-1H-1,2,3-triazolo[4,5-b] pyridinium 3-oxid hexafluorophosphate (95.0 mg, 0.25 mmol) in N,N-dimethylformamide (3 mL) was stirred at 20 °C for 2 h and concentrated under reduced pressure. The residue was purified by RP-HPLC (acetonitrile 42-72/0.225% FA in water) to afford (2*S*,4*R*)-1-((*S*)-2-(4-cyclopropyl-1H-1,2,3-triazol-1-yl) -3,3-dimethylbutanoyl)-4-hydroxy-N-((R)-2-(4-methylthiazol-5-yl)-6,7,8,9-tetrahydro-5H-benzo[7]annulen-5-yl)pyrrolidine-2-carboxamide (38.8 mg, 31.7% yield) as a white solid. ¹H NMR (400 MHz, MeOH-*d*₄) δ 8.86 - 8.82 (m, 1H), 8.00 - 7.96 (m, 1H), 7.29 - 7.15 (m, 3H), 5.48 (s, 1H), 5.19 - 5.12 (m, 1H), 4.67 - 4.63 (m, 1H), 4.50 (br s, 1H), 3.91 - 3.85 (m, 1H), 3.79 - 3.70 (m, 1H), 3.04 - 2.89 (m, 2H), 2.48 - 2.47 (m, 3H), 2.37 - 2.31 (m, 1H), 2.14 - 2.04 (m, 3H), 2.01 - 1.89 (m, 3H), 1.81 - 1.76 (m, 1H), 1.48 - 1.43 (m, 1H), 1.08 - 1.07 (m, 9H), 1.00 - 0.95 (m, 2H), 0.80 - 0.77 (m, 2H). LCMS (Method 5-95 AB, ESI): R_{T} = 1.105 min, [M+H]⁺ = 577.2.

### Example S45: Synthesis of (2S,4R)-1-((S)-2-(4-cyclopropyl-1H-1,2,3-triazol-1-yl)-3,3-dimethylbutanoyl)-4-hydroxy-N-((S)-2-(4-methylthiazol-5-yl)-6,7,8,9-tetrahydro-5H-benzo[7]annulen-5-yl)pyrrolidine-2-carboxamide (Compound 53)

Synthesis was carried out following the scheme given below:

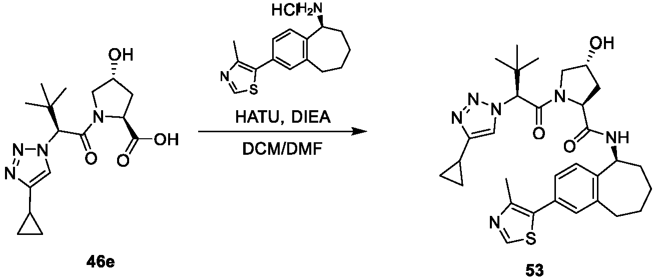

A mixture of (*S*)-2-(4-methylthiazol-5-yl)-6,7,8,9-tetrahydro-5H-benzo[7] annulen-5-amine hydrochloride (64.5 mg, 0.25 mmol), (2*S*, 4*R*)-1-((S)-2-(4-cyclopropyl-1H-1,2,3-triazol-1-yl)-3,3-dimethylbutanoyl)-4-hydroxypyrrolidine-2-carboxylic acid (70.0 mg, 0.21 mmol), N,N-diisopropylethylamine (0.09 mL, 0.52 mmol) and (1-[Bis(dimethylamino)methylene]-1H-1,2,3-triazolo[4,5-b] pyridinium 3-oxid hexafluorophosphate (95.0 mg, 0.25 mmol) in N,N-dimethylformamide (25mL) was stirred at 20 °C for 2 h and concentrated under reduced pressure. The residue was purified by RP-HPLC (acetonitrile 39- 69/0.2% FA in water) to afford (2*S*,4*R*)-1-((*S*)-2-(4-cyclopropyl-1H-1,2,3-triazol-1-yl) -3,3-dimethylbutanoyl)-4-hydroxy-N-((*S*)-2-(4-methylthiazol-5-yl)-6,7,8,9-tetrahydro-5H-benzo[7]annulen-5-yl)pyrrolidine-2-carboxamide (43.8 mg, 35.8% yield) as a white solid. 1 ¹H NMR (400 MHz, MeOH-*d*₄) δ 8.96 (s, 1H), 8.06 (s, 1H), 7.81 (d, *J=* 8.0 Hz, 1H), 7.31 - 7.29 (m, 1H), 7.24 (s, 1H), 5.45 (s, 1H), 5.04 - 5.00 (m, 1H), 4.74 - 4.68 (m, 1H), 4.53 (br s, 1H), 3.91 - 3.87 (m, 1H), 3.77 - 3.72 (m, 1H), 3.02 - 2.87 (m, 2H), 2.49 (s, 3H), 2.28 - 2.23 (m, 1H), 2.18 - 2.11 (m, 1H), 2.04 - 1.96 (m, 4H), 1.90 - 1.68 (m, 2H), 1.45 - 1.36 (m, 1H), 1.08-1.03 (m, 1H), 1.01 - 0.95 (m, 10H), 0.85 - 0.76 (m, 2H). LCMS (Method 5-95 AB, ESI): R_{T} = 1.105 min, [M+H]⁺ = 577.2.

### Example S46: Synthesis of (2S, 4R)-N-((S)-1-(2'-chloro-[1,1'-biphenyl]-4-yl)ethyl)-1-((S)-2-(4-cyclopropyl-1H-1,2,3-triazol-1-yl)-3,3-dimethylbutanoyl)-4-hydroxypyrrolidine-2-carboxamide (Compound 54)

Synthesis was carried out following the scheme given below:

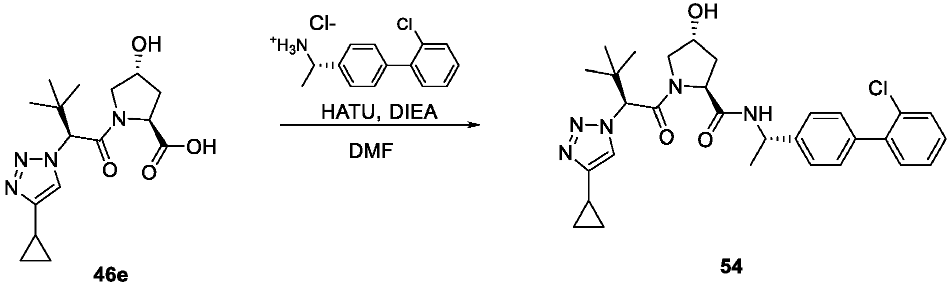

To a solution of (*S*)-1-(2'-chloro-[1,1'-biphenyl]-4-yl)ethanaminium chloride (69.9 mg, 0.26 mmol) and (2*S*, 4*R*)-1-((*S*)-2-(4-cyclopropyl-1H-1,2,3-triazol-1-yl)-3,3-dimethylbutanoyl)-4-hydroxypyrrolidine-2-carboxylic acid (80.0 mg, 0.24 mmol) in anhydrous N,N-dimethylformamide (2 mL) was added N,N-diisopropylethylamine (0.17 mL, 0.95 mmol), 1-[Bis(dimethylamino)methylene]-1H-1,2,3-triazolo[4,5-b]pyridinium 3-Oxide Hexafluorophosphate (0.14 mL, 0.29 mmol) was added at 0 °C. The reaction mixture was stirred at 25 °C for 3 h and concentrated. The residue was purified by prep-HPLC (water (0.225 % FA) - ACN 31% ~ 61%) to afford **compound 54** *(2S,* 4*R*)-N-((*S*)-1-(2'-chloro-[1,1'-biphenyl]-4-yl)ethyl)-1-((S)-2-(4-cyclopropyl-1H-1,2,3-triazol-1-yl)-3,3-dimethylbutanoyl)-4-hydroxypyrrolidine-2-carboxamide (95 mg, 71.9% yield) as a white solid. ¹H NMR (400 MHz, MeOH-*d*₄) δ 7.99 (s, 1H), 7.48 (d, *J =* 8.0 Hz, 1H), 7.42 - 7.30 (m, 7H), 5.47 - 5.42 (m, 1H), 5.09 - 4.98 (m, 1H), 4.60 - 4.53 (m, 1H), 4.45 - 4.40 (m, 1H), 3.87 - 3.83 (m, 1H), 3.75 - 3.69 (m, 1H), 2.24 - 2.19 (m, 1H), 2.01 - 1.95 (m, 2H), 1.55 (d, *J=* 8.0 Hz, 3H), 1.07 (m, 9H), 1.00 - 0.95 (m, 2H), 0.83 - 0.75 (m, 2H). LCMS (Method 5-95 AB, ESI): R_{T} = 0.892 min, [M+H]⁺ = 550.1.

### Biological Assays

### Example A: Fluorescence Polarization (FP) VHL Binding Assay

The binding of test compounds to the VHL Elongin B/C complex was measured using a fluorescence polarization tracer competition assay. The VHL / Elongin B/C protein complex used in the assay was generated as follows. The coding region for amino acids E55-D213 of human VHL with N-terminal His6 tag with a TEV -protease cleavage site was co-expressed with Elongin B (residues M1-Q118) and Elongin C (ResiduesM17-C112) in *E*. *coli.* The VHL / Elongin B/C complex was purified using an affinity nickel column, anion exchange HiTrap QP HP column chromatography, and gel filtration using a Superdex 75 26/60 column. The purified VHL / Elongin B/C complex was dialyzed into formulation buffer: 20mM Bis-Tris pH7.0, 150mM NaCl, 1mM DTT. A VHL fluorescence polarization probe consisted of a VHL ligand coupled to carboxytetramethylrhodamine (TAMRA); (2S,4R)-N-(2-(2-(3',6'-bis(dimethylamino)-3-oxo-3H-spiro[isobenzofuran-1,9'-xanthene]-5-carboxamido)ethoxy)-4-(4-methylthiazol-5-yl)benzyl)-4-hydroxy-1-((R)-3-methyl-2-(3-methylisoxazol-5-yl)butanoyl)pyrrolidine-2-carboxamide. Compounds were prepared as a serial dilution in DMSO at a concentration 25-fold higher than the final desired concentration and acoustically dispensed (400 nl) into a ProxiPlate-384 Plus F, Black 384-shallow well Microplate (Part Number 6008260). DMSO was dispensed into wells designated for "VHL control" (without compound) wells. The "Assay Buffer" consisted of 50 mM Tris pH 8.0, 120 mM NaCl, 0.005% Nonidet P-40, and 1% DMSO (v/v). Assay Buffer containing 5.28 µM VHL Elongin B/C complex was prepared and 5µl dispensed using a BioRapTR (Beckman Coulter) into each well of the assay plate. Assay Buffer was also dispensed into "no VHL control" wells using the same method. A "pre-assay" fluorescence measurement was made using an Infinite^{®} M1000 (Tecan) plate reader (Excitation 530 nm, Emission 574 nm, Bandwidth 10 nm). Assay Buffer containing 3.34 nM of the VHL FP probe was prepared in Assay Buffer and 5µl dispensed into each well of the assay plate using a BioRapTR (Beckman Coulter). The final VHL / Elongin B/C protein concentration is 2.64 nM and the final probe concentration is 1.67 nM. Assay plates were briefly centrifuged and incubated for 1 hour at room temperature. "Post-assay" fluorescence polarization measurements were made as described for the "pre-assay" fluorescence measurement. Fluorescence polarization was calculated for each sample; taking into account the "pre-assay" fluorescence measurements and subtracting the fluorescence signal of the compound/VHL only ("pre-assay") measurements from the "post-assay" fluorescence polarization measurements, for each plane of polarization. The data were analyzed using Genedata Screener software and normalized to the "no VHL control" and "VHL control" (without compound). IC₅₀ values were calculated using a four parameter curve fit (Robust method).

### Example B: Surface Plasmon Resonance Assay

Using a Biacore T200, Avidin tagged VHL co-expressed with Elongins B and C are immobilized to a Biacore SA chip in running buffer without DMSO. Compounds are tested individually at varying concentrations in running buffer (50 mM HEPES pH 7.2, 150 mM NaCl, 0.5 mM TCEP, 0.001% Tween 20, 0.2% PEG3350, 2% DMSO) at 20°C. Sensorgrams are run in order from low to high concentration using a flow rate of 80 µL/min. Association and disassociation times are varied depending on the estimated potency of the compound tested. Analysis of the binding curves and determination of the kinetic parameters is done using evaluation software (Version 2.0, Biacore).

### Example C: VHL HEK-293 BRET Assay

The VHL NanoBRET^{™} Target Engagement Assay analyzes the apparent affinity of test compounds for VHL in cells by competitive displacement of a VHL NanoBRET^{™} tracer reversibly bound to a NanoLuc^{®} VHL fusion protein stably expressed in the cells.

Test compounds were transferred to the assay plate (384 Well White Non-Binding Corning Assay Plates (Corning-3574)) using an Echo 555 Liquid Handler (Labcyte) in 2.5 nL increments and, as appropriate, intermediate stock concentrations of compounds, in order to prepare a titration series. 50 nL of control compound (10mM; parental unlabeled VHL antagonist; see structure below) and 50 nL of DMSO (negative control) were dispensed into the appropriate control wells. DMSO was backfilled to a final volume of 50 nL as required. 50nl per well of 1 mM VHL NanoBRET^{™} Tracer in DMSO (NanoBRET^{™} Tracer-PEG2-590 (see structure below)) was transferred into each well using an Echo 555 (ultimately yielding a final concentration of 1uM). HEK 293 RT VHL-NanoLuc^{®} stable cells were cultured in DMEM High Glucose with Pyruvate, 10% fetal bovine serum, 2 mg/mL of Geneticin Selective Antibiotic (50 mg/ml) and 2 mM HEPES (1 M). Cells were seeded in Opti-MEM (Life Technologies-11058-021), 1.7 × 10⁵ cells/mL, 40 µl per well into the assay plate, centrifuged at 500 rpm for 30 seconds and incubated for 2 hours. Max Signal control wells consisted of DMSO only treated wells. Minimum Signal control wells contained of 10 uM parental unlabeled VHL antagonist (control compound - see structure below). 3X Complete Substrate plus Inhibitor Solution was prepared in Opti-MEM (consists of a 1:166 dilution of NanoBRET^{™} Nano-Glo^{®} Substrate plus a 1:500 dilution of Extracellular NanoLuc^{®} Inhibitor in Opti-MEM), and 20 ul was dispensed into each well of the 384-well plate and centrifuged at 1000 rpm for 1 minute, then incubated for 2 minutes at room temperature. Background Signal control wells were prepared without tracer for background correction steps.

Plates were read using a PerkinElmer Envision Reader (model 2104-0020) equipped with Luminescence option (Mirror: BRET2 Enh (PE Barcode 659), Emission Filter: Omega 610LP (Barcode 504), 2nd Emission Filter: Umbelliferone 460 (Barcode 207), Measurement height: 6.5 mm, Measurement time: 1s). The raw BRET ratio values were calculated by dividing the acceptor emission value (610 nm) by the donor emission value (460nm) for each sample. To correct for background, the BRET ratio in the absence of tracer (average of no-tracer control samples) was subtracted from the BRET ratio of each sample. Raw BRET units were converted to milliBRET units (mBU) by multiplying each raw BRET value by 1,000. The normalized NanoBRET^{™} signal was calculated relative to the Max Signal control wells (DMSO treated control wells) and the Minimum Signal control wells. Percentage inhibition was calculated relative to the Minimum Signal control and Maximum Signal control wells. IC₅₀ values were derived by four parameter curve fitting using the Robust method.

### NanoBRET^{™} Tracer-PEG2-590:

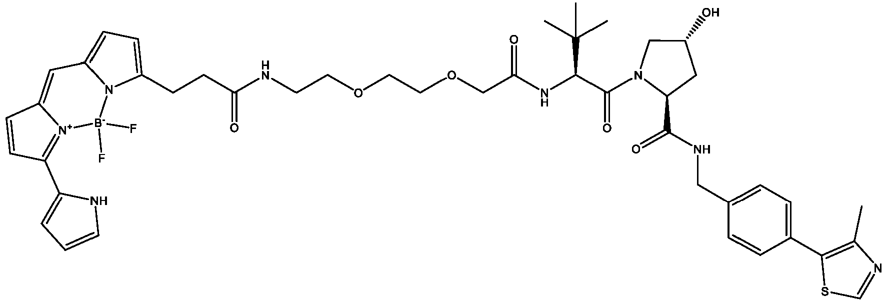

### Parental unlabeled VHL antagonist (control compound):

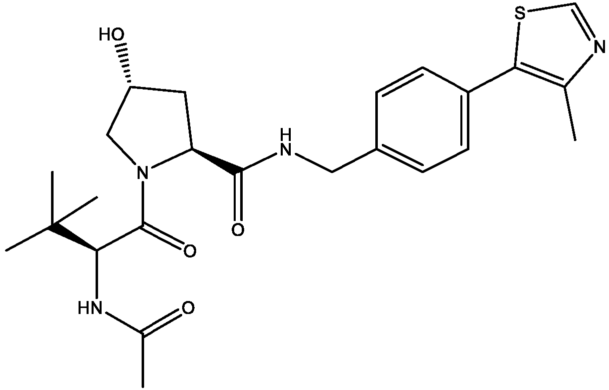

The results for VHL binding IC₅₀ values from the FP assay and the HEK-293 BRET assay and K_{d} values measured in the SPR assay are shown in Table 2. Where more than one measurement was performed for the same assay, the value reported is the geometric mean of all values.

**Table 2**

| **Final product from synthetic example** | **VHL binding FP (µM)** | **VHL binding in cells (HEK293 nanoBRET, µM)** | **VHL nanoBRET (+ Digitonin) EC50 (µM)** | **Ratio cell permeability shift** |
|---|---|---|---|---|
| Final product from Example S1 | 0.11 | 0.22 | 0.19 | 2.09 |
| Final product from Example S2 | - | 15.10 | 6.53 | 2.31 |
| Final product from Example S3 | 0.15 | 0.15 | 0.14 | 0.97 |
| Final product from Example S4 | - | 0.19 | 0.20 | 0.96 |
| Final product from Example S5 | - | 0.13 | 0.17 | 0.77 |
| Final product from Example S6 | - | 0.10 | 0.14 | 0.76 |
| Final product from Example S7 | - | 0.11 | 0.16 | 0.68 |
| Final product from Example S8 | - | 0.12 | 0.16 | 0.73 |
| Final product from Example S9 | - | 0.45 | 0.32 | 1.39 |
| Final product from Example S10 | - | 0.28 | 0.21 | 1.36 |
| Final product from Example S11 | - | 0.15 | 0.23 | 0.63 |
| Final product from Example S12 | - | >100 | 24.48 | - |
| Final product from Example S13 | - | 2.27 | 0.22 | 10.22 |
| Final product from Example S14 | 0.08 | 20.70 | - | 264.37 |
| Final product from Example S15 | - | 69.80 | 0.61 | 115.18 |
| Final product from Example S16 | - | 3.31 | 0.40 | 8.30 |
| Final product from Example S17 | - | 2.21 | 0.48 | 4.65 |
| Final product from Example S18 | - | 0.01 | 0.01 | 0.85 |
| Final product from Example S19 | - | <0.005 | <0.005 | ~ 1 |
| Final product from Example S20 | - | 0.00 | 0.01 | 0.54 |
| Final product from Example S21 | - | 0.00 | 0.01 | 0.67 |
| Final product from Example S22 | - | 0.01 | 0.01 | 0.72 |
| Final product from Example S23 | - | 0.02 | 0.04 | 0.42 |
| Final product from Example S24 | 0.01 | 0.01 | 0.01 | 1.22 |
| Final product from Example S25 | - | 0.00 | 0.00 | 0.67 |
| Final product from Example S26 | - | 0.02 | 0.02 | 1.02 |
| Final product from Example S27 | 0.02 | 0.02 | 0.02 | 0.88 |
| Final product from Example S28 | - | 0.04 | 0.04 | 0.98 |
| Final product from Example S29 | - | 0.01 | 0.01 | 1.04 |
| Compound 30 from Example S30 | - | 0.02 | 0.02 | 1.15 |
| Compound 31 from Example S30 | - | 0.31 | 0.02 | 19.75 |
| Final product from Example S31 | - | 0.46 | 0.31 | 1.48 |
| Final product from Example S32 | - | 5.40 | 3.64 | 1.48 |
| Isomer A from Example S33 | - | 62.50 | 43.70 | 1.43 |
| Isomer B from Example S33 | - | 1.62 | 0.99 | 1.64 |
| Final product from Example S34 | - | >100 | >100 | - |
| Final product from Example S35 | - | >100 | 13.00 | - |
| Isomer A from Example S36 | - | 0.30 | 0.39 | 0.77 |
| Isomer B from Example S36 | - | 68.50 | 68.00 | 1.01 |
| Final product from Example S37 | - | 1.66 | 1.16 | 1.43 |
| Final product from Example S38 | - | 0.19 | 0.17 | 1.12 |
| Final product from Example S39 | - | 0.11 | 0.11 | 0.93 |
| Final product from Example S40 | - | 0.08 | 0.11 | 0.74 |
| Final product from Example S41 | - | 0.01 | 0.01 | 0.72 |
| Final product from Example S42 | - | 1.10 | 1.54 | 0.71 |
| Final product from Example S43 | - | 0.03 | 0.03 | 1.03 |
| Final product from Example S44 | - | <0.005 | <0.005 | - |
| Final product from Example S45 | - | 0.20 | 0.23 | 0.86 |
| Final product from Example S46 | - | 0.01 | 0.01 | 0.84 |

## Claims

1. A compound of formula (I): 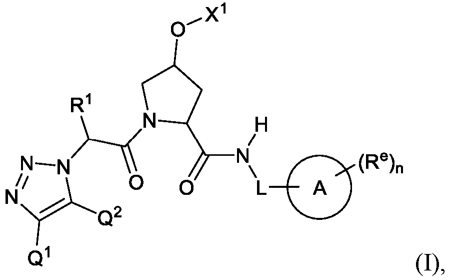 or a stereoisomer or tautomer thereof, or a pharmaceutically acceptable salt of any of the foregoing, wherein:
X¹ is, independently at each occurrence, H, C₁₋₁₂alkyl, or -C(O)-C₁₋₁₂alkyl;
R¹ is, independently at each occurrence, C₁₋₁₂alkyl, C₂₋₁₂alkenyl, C₂₋₁₂alkynyl, C₃₋₁₅cycloalkyl, or 3-15 membered heterocyclyl,
wherein the C₁₋₁₂alkyl, C₂₋₁₂alkenyl, C₂₋₁₂alkynyl, C₃₋₁₅cycloalkyl, or 3-15 membered heterocyclyl of R¹ is independently optionally substituted with one or more C₁₋₁₂alkyl, C₆₋₂₀aryl, -S(O)₂-C₁₋₁₂alkyl, or -C(O)-C₁₋₁₂alkyl;
L is, independently at each occurrence, absent or is C₁₋₁₂alkylene, wherein the C₁₋₁₂alkylene of L is independently optionally substituted with one or more R^{t}, wherein R^{t} is C₁₋₁₂alkyl or - C(O)NH₂, wherein the C₁₋₁₂alkyl of R^{t} is further optionally substituted with one or more halo;
ring A is, independently at each occurrence, C₆₋₂₀aryl or C₇₋₁₅cycloalkyl;
R^{e} is, independently at each occurrence, halo, C₆₋₂₀aryl, or 5-20 membered heteroaryl, provided that at least one R^{e} is C₆₋₂₀aryl or 5-20 membered heteroaryl comprising one or more annular sulfur atoms, wherein the C₆₋₂₀aryl or 5-20 membered heteroaryl of R^{e} is independently optionally substituted with one or more C₁₋₁₂alkyl or halo;
n is, independently at each occurrence, 1, 2, 3, 4, or 5; and
Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
wherein the C₁₋₁₂alkyl or C₃₋₁₅cycloalkyl of Q¹ or Q² is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 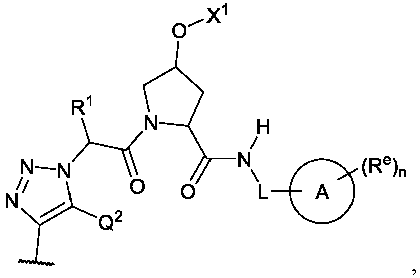 wherein the C₁₋₁₂alkyl or C₁₋₁₂alkoxy of R^{q} is independently further optionally substituted with one or more halo or - NHC(O)-C₁₋₁₂alkyl,
or Q¹ and Q² are taken, together with the atoms to which they are attached, to form a C₃₋₁₅cycloalkyl, 3-15 membered heterocyclyl, C₆₋₂₀aryl, or 5-20 membered heteroaryl,
wherein the C₃₋₁₅cycloalkyl, 3-15 membered heterocyclyl, C₆₋₂₀aryl, or 5-20 membered heteroaryl formed by Q¹ and Q² is independently optionally substituted with one or more R^{s}, wherein R^{s} is OH, cyano, halogen, oxo, -NH₂, -NO₂, -CHO, -C(O)OH, - C(O)NH₂, -SH, -SO₂C₁₋₁₂alkyl, -SO₂NH₂, or C₁₋₁₂alkyl, wherein the C₁₋₁₂alkyl of R^{s} is further optionally substituted with one or more halo, cyano, or OH,
provided that the compound of formula (I), or a pharmaceutically acceptable salt thereof, is not (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)benzyl)pyrrolidine-2-carboxamide, or a pharmaceutically acceptable salt thereof.

2. The compound of claim 1, or a stereoisomer or tautomer thereof, or a pharmaceutically acceptable salt of any of the foregoing, wherein L is, independently at each occurrence, C₁₋₆alkylene, wherein the C₁₋₆alkylene of L is independently optionally substituted with one or more R^{t}, wherein R^{t} is C₁₋₆alkyl or -C(O)NH₂, wherein the C₁₋₆alkyl of R^{t} is further optionally substituted with one or more halo.

3. The compound of claim 1 or claim 2, or a stereoisomer or tautomer thereof, or a pharmaceutically acceptable salt of any of the foregoing, wherein ring A is, independently at each occurrence, C₆₋₂₀aryl.

4. The compound of any one of claims 1-3, or a stereoisomer or tautomer thereof, or a pharmaceutically acceptable salt of any of the foregoing, wherein
n is 1, and
R^{e} is, independently at each occurrence, 5-20 membered heteroaryl, wherein the 5-20 membered heteroaryl of R^{e} comprises one or more annular sulfur atoms and is independently optionally substituted with one or more C₁₋₁₂alkyl.

5. The compound of any one of claims 1-4, or a stereoisomer or tautomer thereof, or a pharmaceutically acceptable salt of any of the foregoing, wherein the compound of formula (I) is a compound of formula (IA): 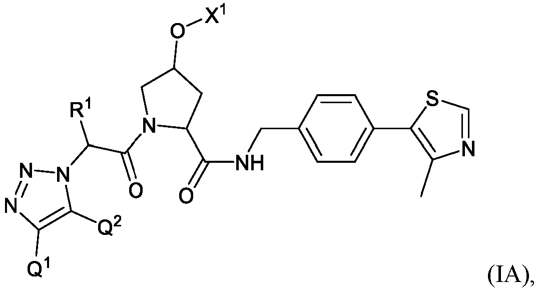 or a stereoisomer or tautomer thereof, or a pharmaceutically acceptable salt of any of the foregoing.

6. The compound of any one of claims 1-4, or a stereoisomer or tautomer thereof, or a pharmaceutically acceptable salt of any of the foregoing, wherein the compound of formula (I) is a compound of formula (IB): 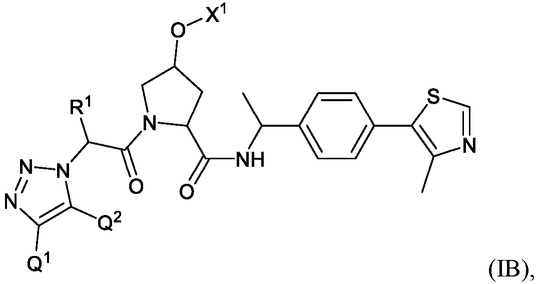 or a stereoisomer or tautomer thereof, or a pharmaceutically acceptable salt of any of the foregoing.

7. The compound of any one of claims 1-4, or a stereoisomer or tautomer thereof, or a pharmaceutically acceptable salt of any of the foregoing, wherein the compound of formula (I) is a compound of formula (IC): 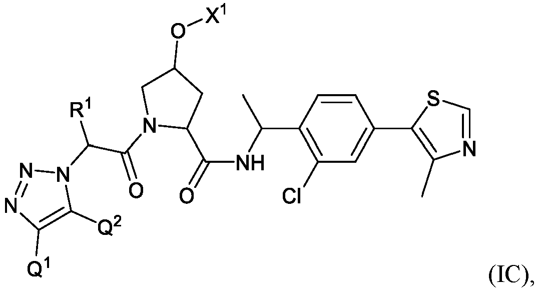 or a stereoisomer or tautomer thereof, or a pharmaceutically acceptable salt of any of the foregoing.

8. The compound of any one of claims 1-3, or a stereoisomer or tautomer thereof, or a pharmaceutically acceptable salt of any of the foregoing, wherein
n is 1, and
R^{e} is, independently at each occurrence, C₆₋₂₀aryl, wherein the C₆₋₂₀aryl of R^{e} is independently optionally substituted with one or more C₁₋₁₂alkyl or halo.

9. The compound of any one of claims 1-3, and 8, or a stereoisomer or tautomer thereof, or a pharmaceutically acceptable salt of any of the foregoing, wherein the compound of formula (I) is a compound of formula (ID): 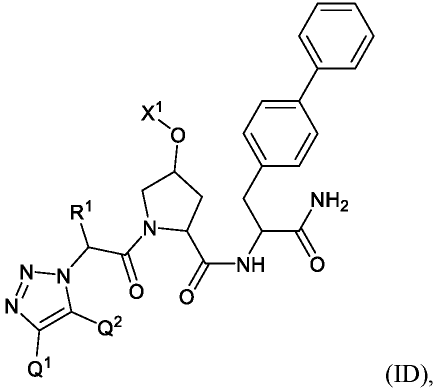 or a stereoisomer or tautomer thereof, or a pharmaceutically acceptable salt of any of the foregoing.

10. The compound of claim 9, or a stereoisomer or tautomer thereof, or a pharmaceutically acceptable salt of any of the foregoing, wherein the compound of formula (ID) is a compound selected from the group consisting of 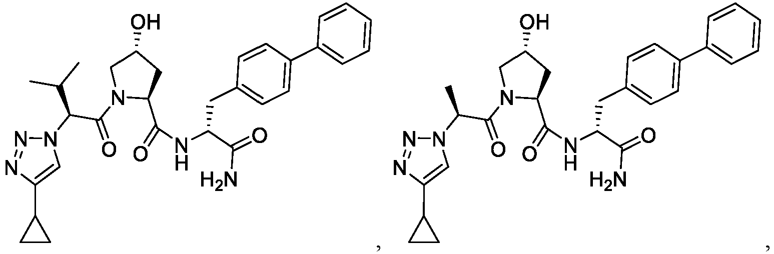 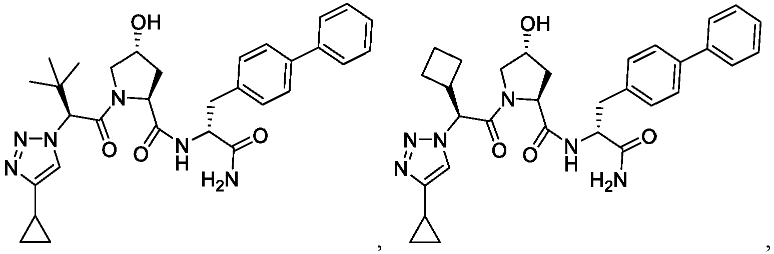 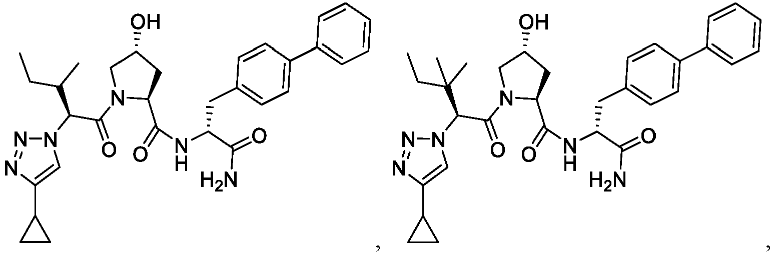 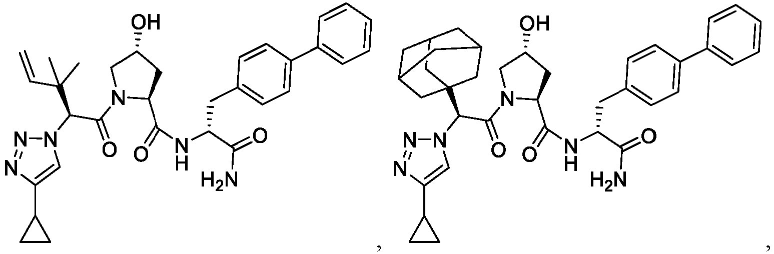 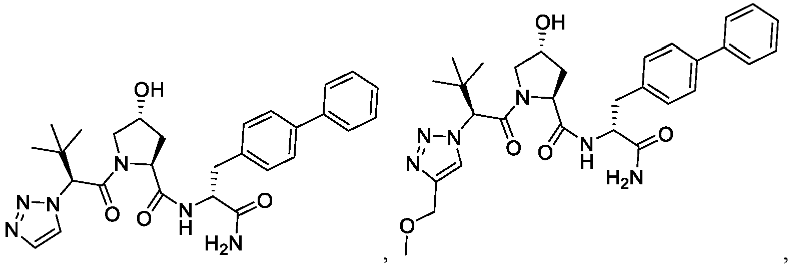 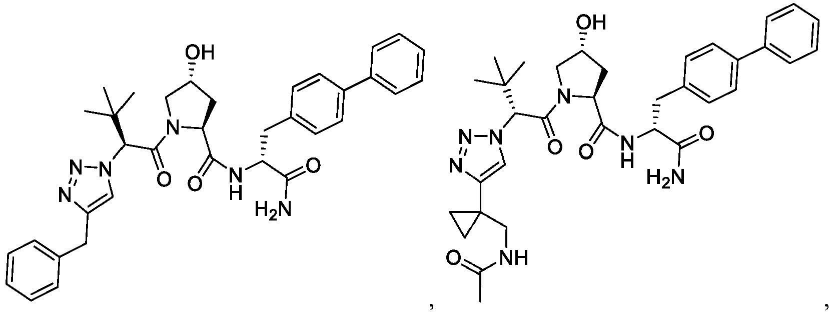 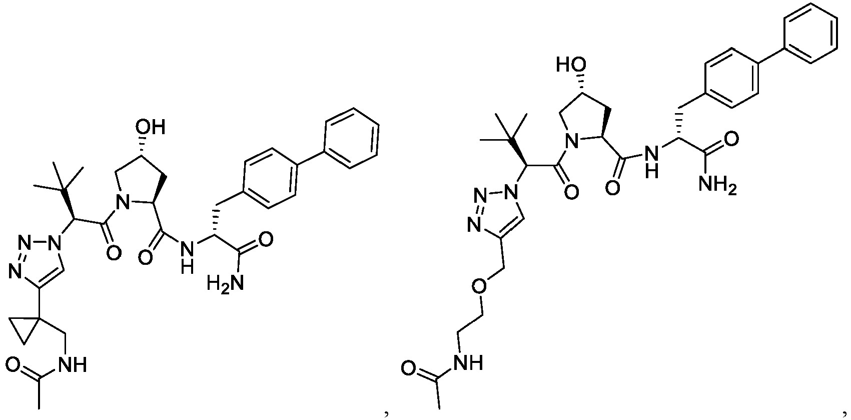 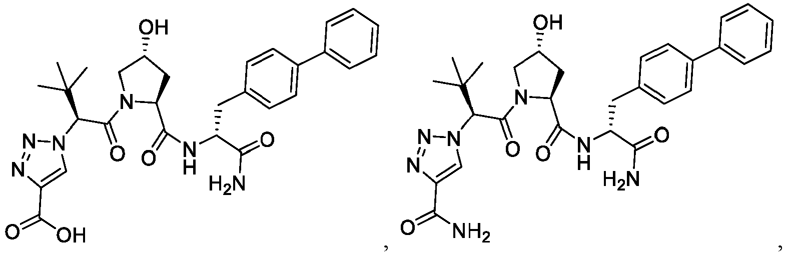 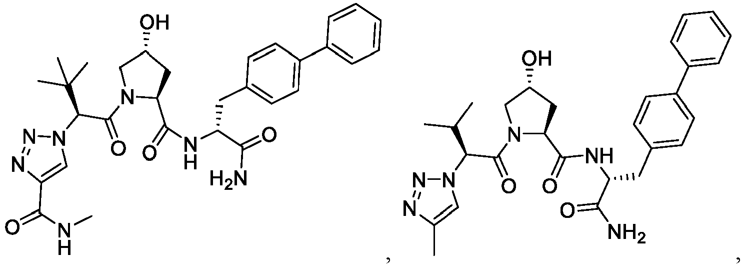 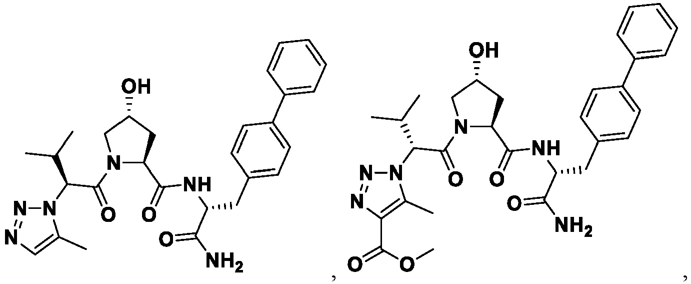 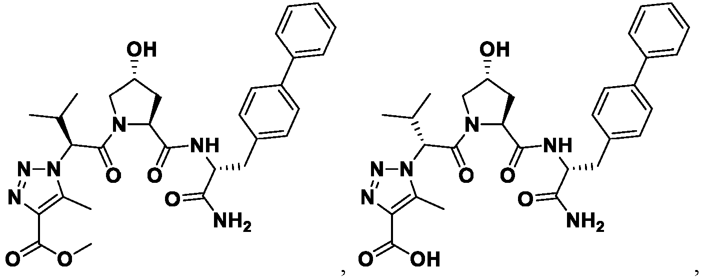 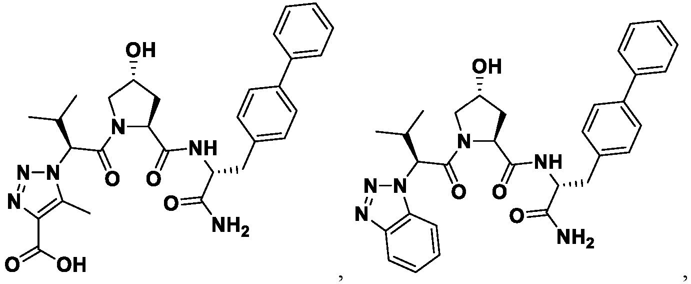 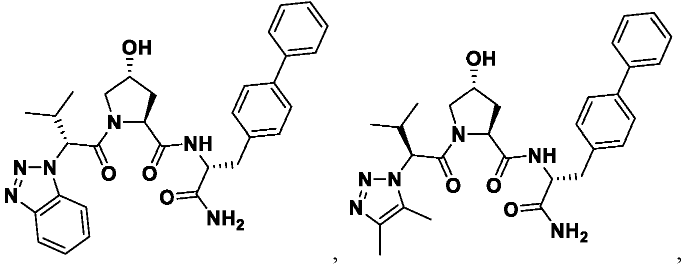 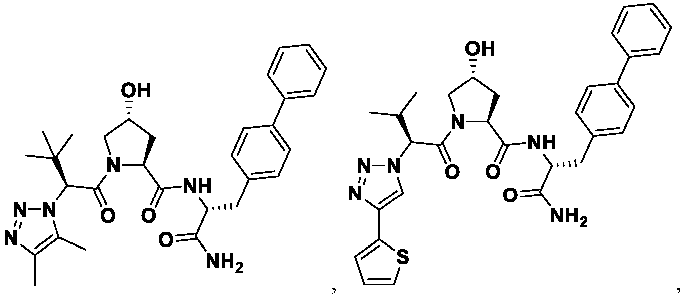 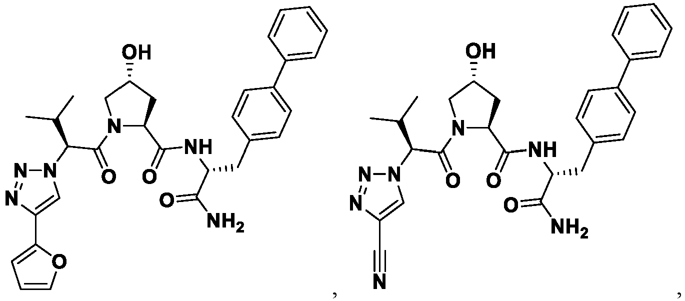 and 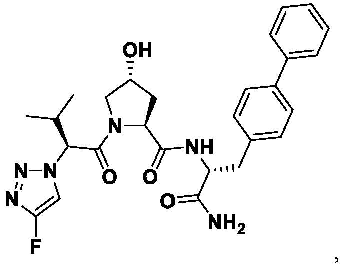 or a stereoisomer or tautomer thereof, or a pharmaceutically acceptable salt of any of the foregoing.

11. The compound of claim 1, or a stereoisomer or tautomer thereof, or a pharmaceutically acceptable salt of any of the foregoing, wherein the compound of formula (I) is a compound of formula (IF): 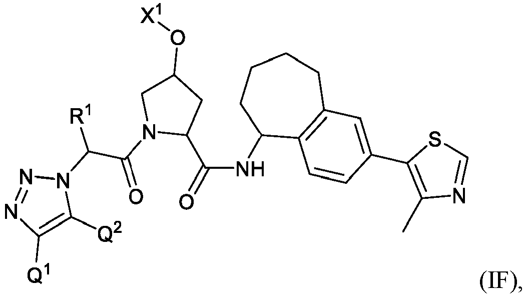 or a stereoisomer or tautomer thereof, or a pharmaceutically acceptable salt of any of the foregoing.

12. The compound of any one of claims 1-9 and 11, or a stereoisomer or tautomer thereof, or a pharmaceutically acceptable salt of any of the foregoing, wherein Q¹ and Q² are, independently of each other and independently at each occurrence, H, halo, cyano, C₁₋₁₂alkyl, C₃₋₁₅cycloalkyl, 3-15 membered heterocyclyl, C₆₋₂₀aryl, 5-20 membered heteroaryl, -C(O)-O(R^{a}), or -C(O)-N(R^{b})(R^{c}), wherein R^{a}, R^{b}, and R^{c} are each independently H or C₁₋₁₂alkyl,
wherein the C₁₋₁₂alkyl or C₃₋₁₅cycloalkyl of Q¹ or Q² is independently optionally substituted with one or more R^{q}, wherein R^{q} is C₁₋₁₂alkyl, C₂₋₁₂alkenyl, C₂₋₁₂alkynyl, C₆₋₂₀aryl, C₁₋₁₂alkoxy, or 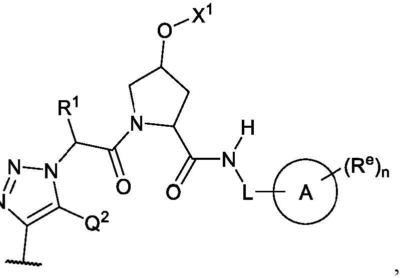 wherein the C₁₋₁₂alkyl or C₁₋₁₂alkoxy of R^{q} is independently further optionally substituted with one or more halo or - NHC(O)-C₁₋₁₂alkyl.

13. The compound of any one of claims 1-9 and 11-12, or a stereoisomer or tautomer thereof, or a pharmaceutically acceptable salt of any of the foregoing, wherein Q¹ is C₃₋₁₅cycloalkyl, wherein the C₃₋₁₅cycloalkyl of Q¹ is optionally substituted with one or more R^{q}, wherein R^{q} is independently C₁₋₁₂alkyl, C₂₋₁₂alkenyl, C₂₋₁₂alkynyl, C₆₋₂₀aryl, C₁₋₁₂alkoxy, or 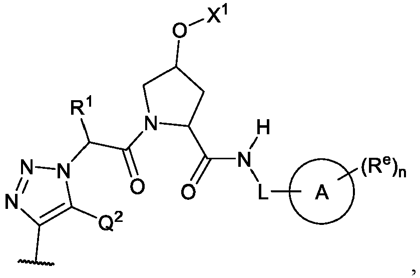 wherein the C₁₋₁₂alkyl or C₁₋₁₂alkoxy of R^{q} is independently further optionally substituted with one or more halo or -NHC(O)-C₁₋₁₂alkyl.

14. The compound of any one of claims 1-13, or a stereoisomer or tautomer thereof, or a pharmaceutically acceptable salt of any of the foregoing, wherein Q¹ is unsubstituted C₃₋₁₅cycloalkyl.

15. The compound of any one of claims 1-14, or a stereoisomer or tautomer thereof, or a pharmaceutically acceptable salt of any of the foregoing, wherein R¹ is, independently at each occurrence, C₁₋₁₂alkyl, wherein the C₁₋₁₂alkyl of R¹ is independently optionally substituted with one or more C₆₋₂₀aryl, -S(O)₂-C₁₋₁₂alkyl, or -C(O)-C₁₋₁₂alkyl.

16. The compound of any one of claims 1-14, or a stereoisomer or tautomer thereof, or a pharmaceutically acceptable salt of any of the foregoing, wherein R¹ is, independently at each occurrence, C₃₋₁₅cycloalkyl, wherein the C₃₋₁₅cycloalkyl of R¹ is optionally substituted with one or more C₁₋₁₂alkyl, C₆₋₂₀aryl, -S(O)₂-C₁₋₁₂alkyl, or -C(O)-C₁₋₁₂alkyl.

17. The compound of claim 16, or a stereoisomer or tautomer thereof, or a pharmaceutically acceptable salt of any of the foregoing, wherein R¹ is, independently at each occurrence, C₃₋₆cycloalkyl, wherein the C₃₋₆cycloalkyl of R¹ is optionally substituted with one or more C₁₋₁₂alkyl, C₆₋₂₀aryl, -S(O)₂-C₁₋₁₂alkyl, or -C(O)-C₁₋₁₂alkyl.

18. The compound of claim 1, or a stereoisomer or tautomer thereof, or a pharmaceutically acceptable salt of any of the foregoing, wherein the compound is selected from the group consisting of 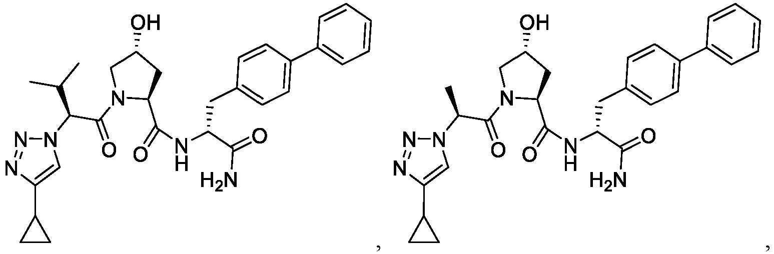 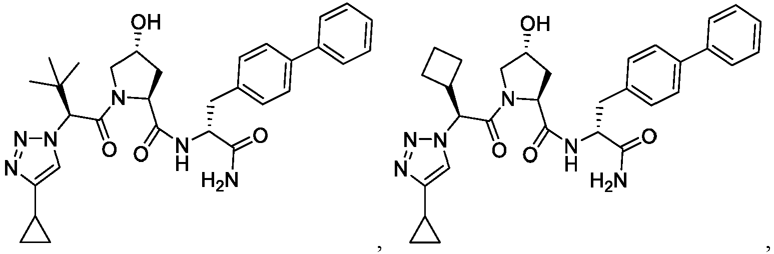 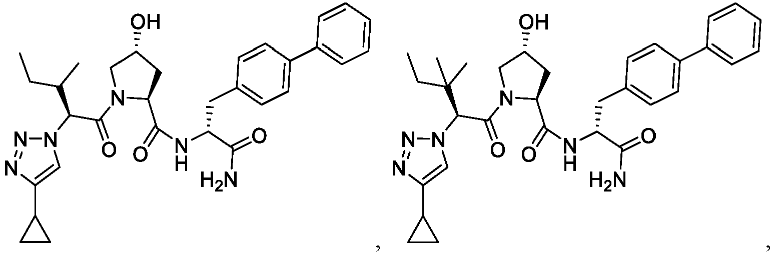 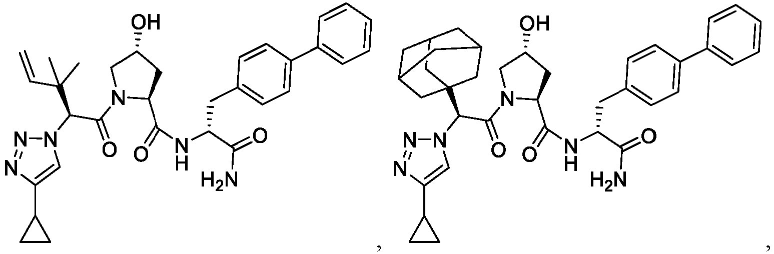 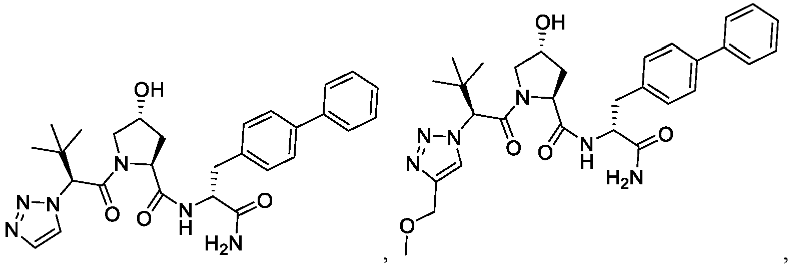 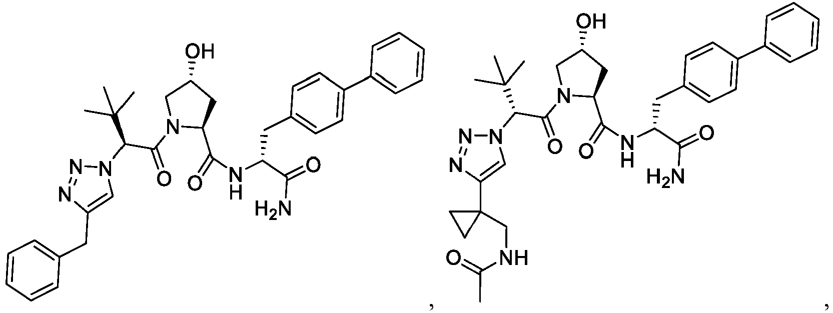 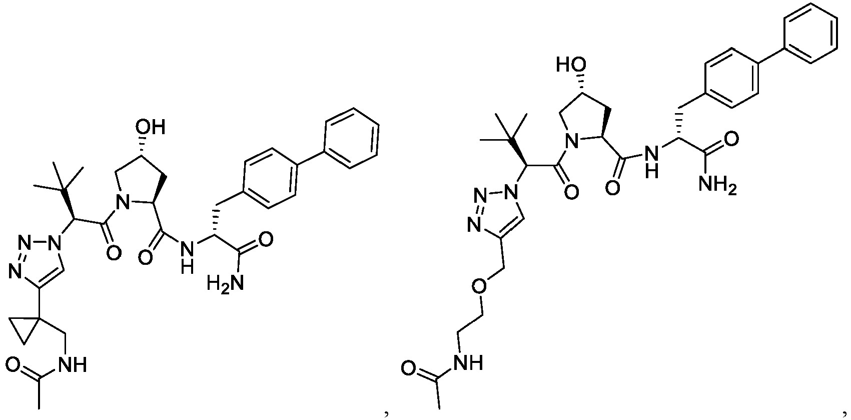 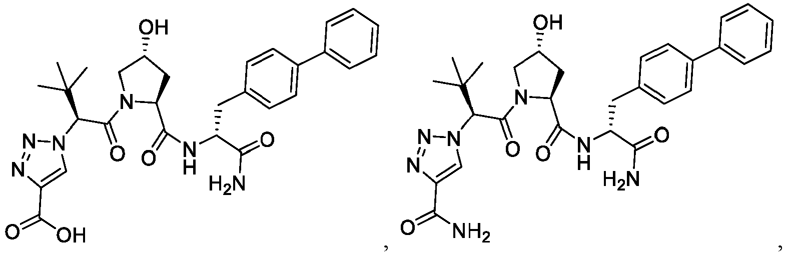 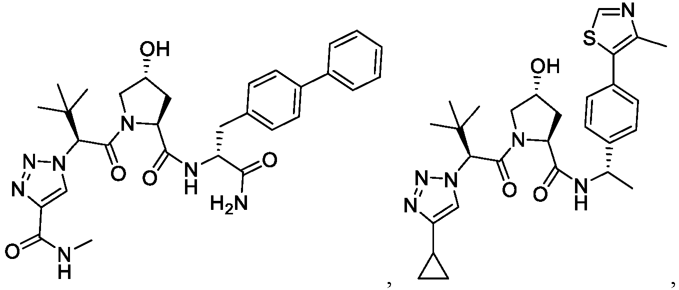 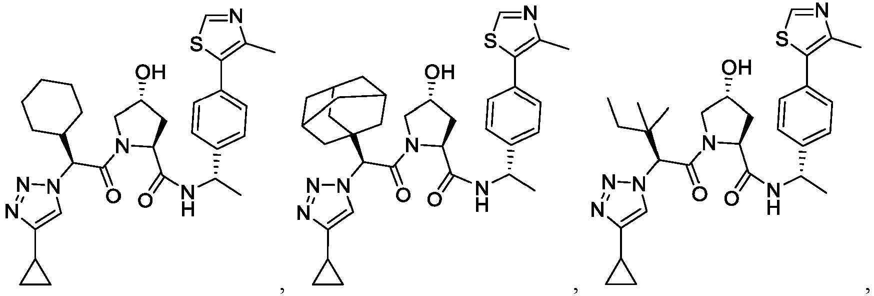 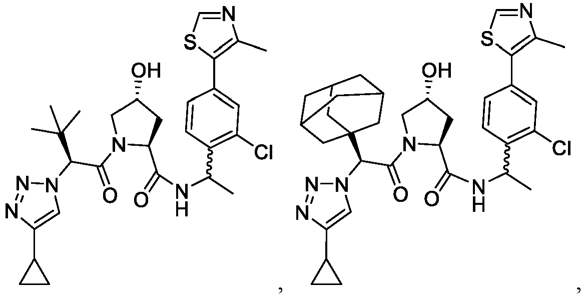 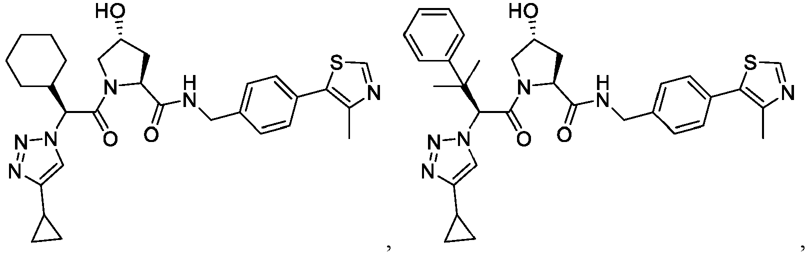 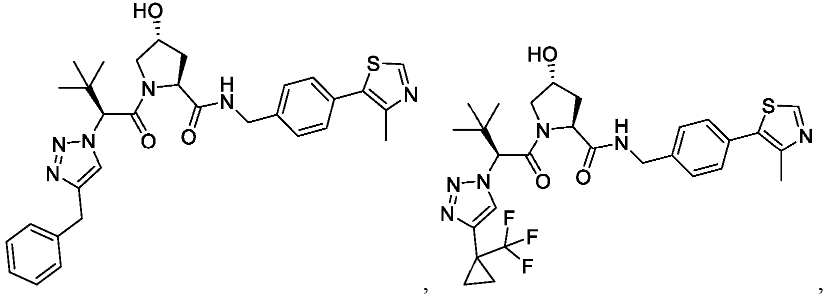 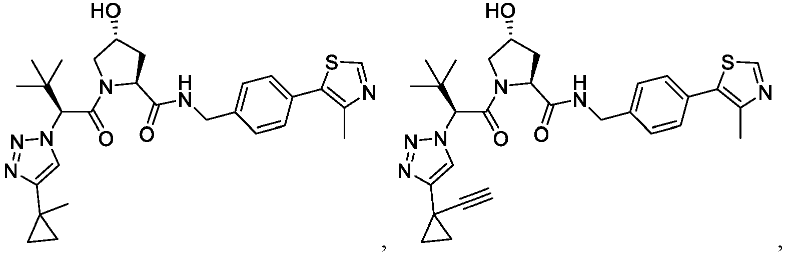 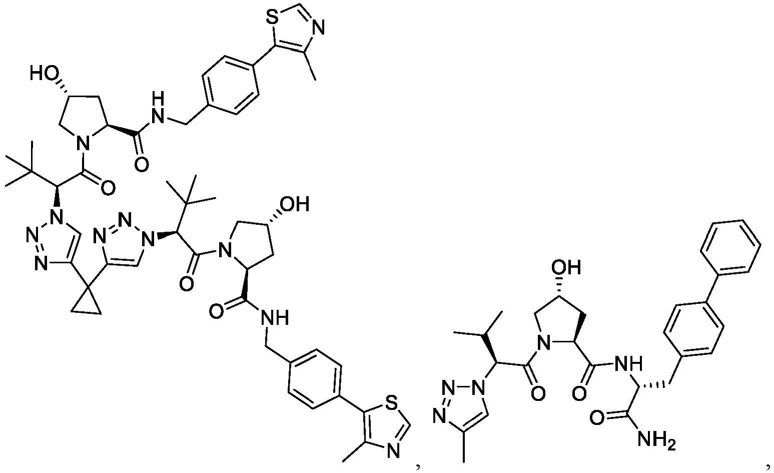 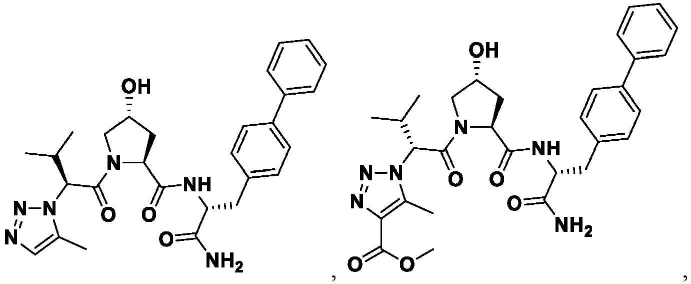 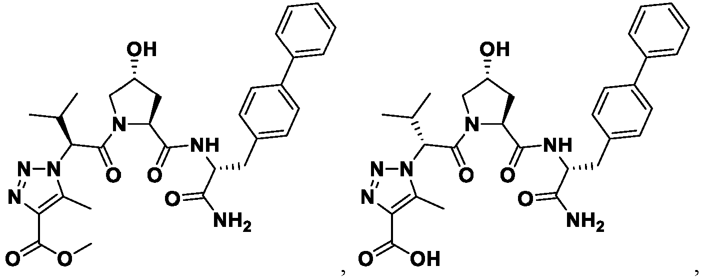 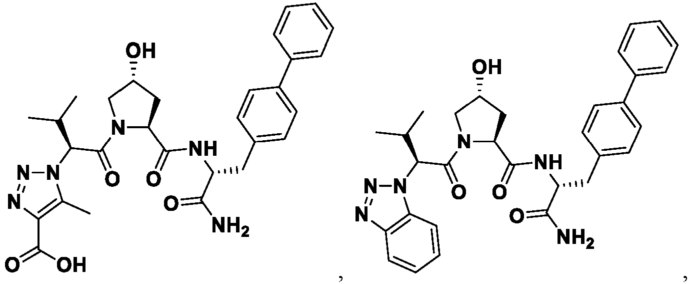 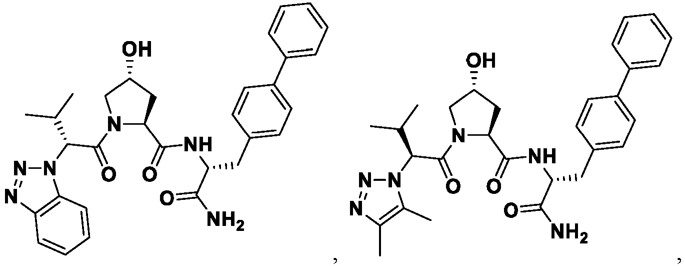 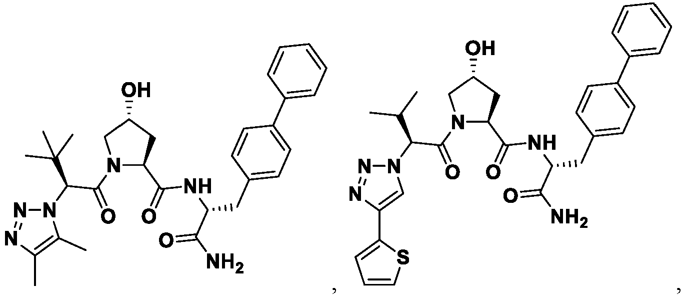 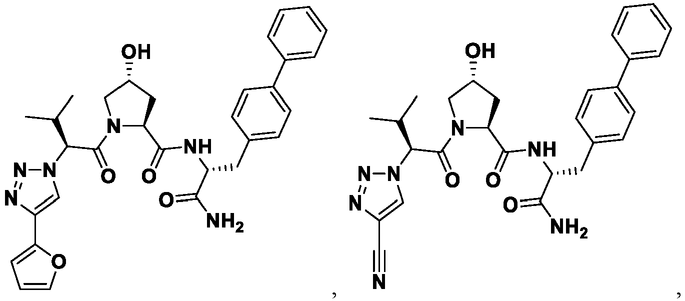 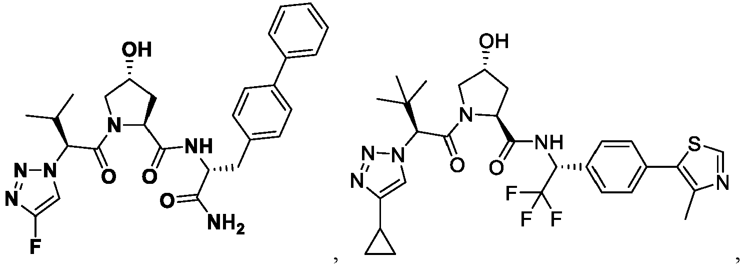 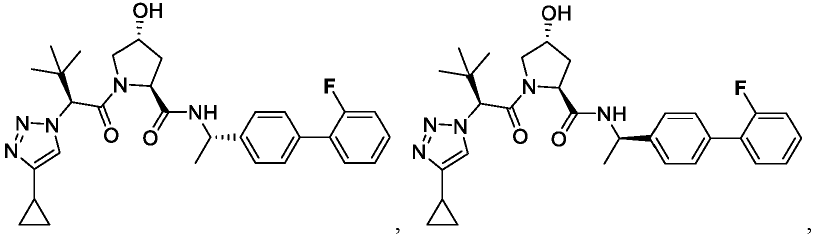 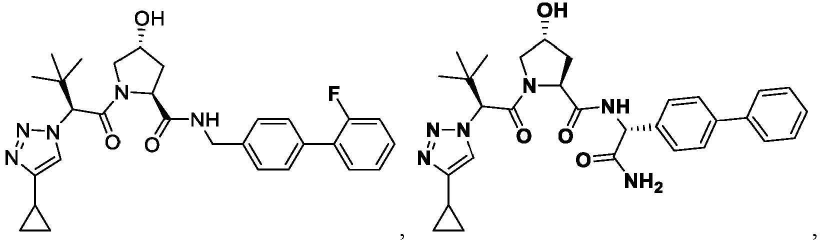 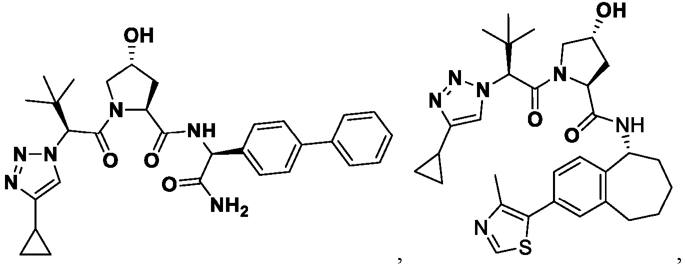 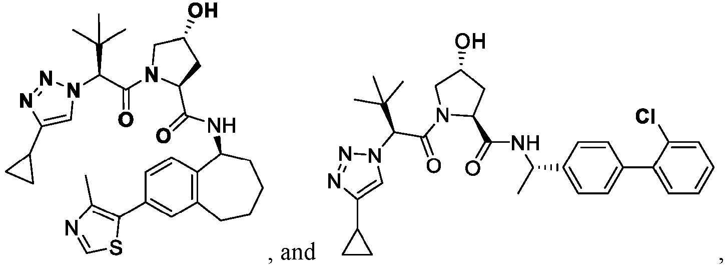 or a stereoisomer or tautomer thereof, or a pharmaceutically acceptable salt of any of the foregoing.

19. The compound of claim 1, or a stereoisomer or tautomer thereof, or a pharmaceutically acceptable salt of any of the foregoing, wherein the compound is selected from the group consisting of 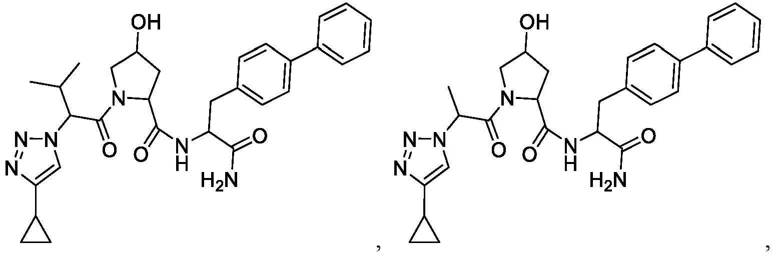 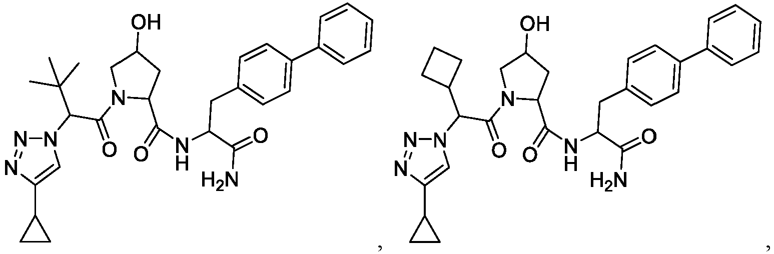 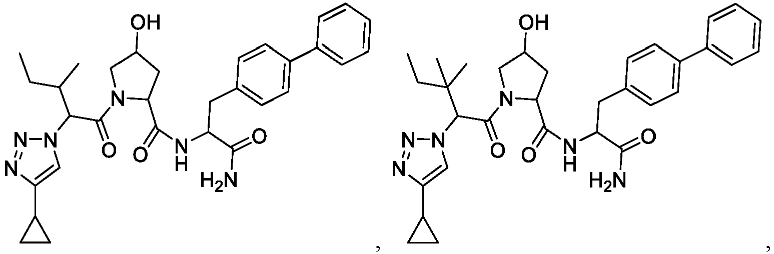 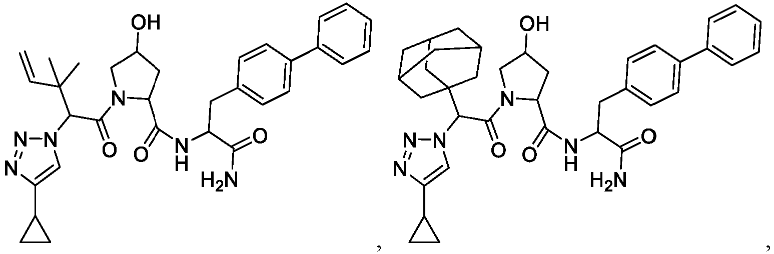 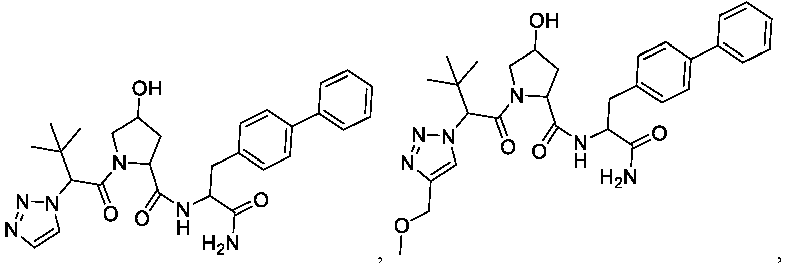 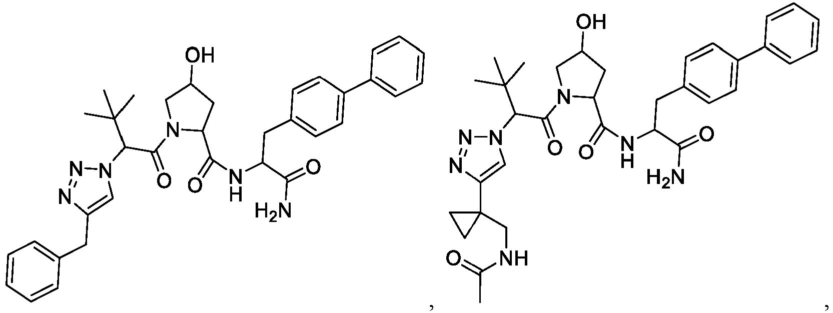 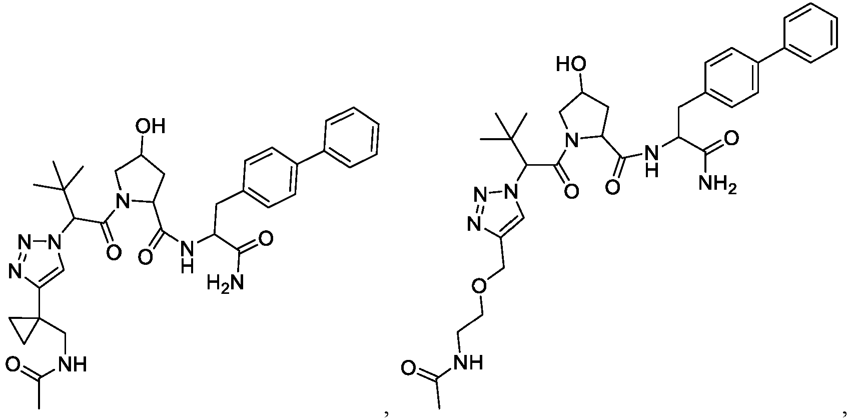 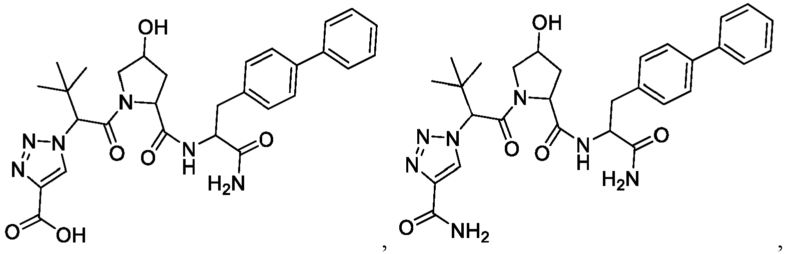 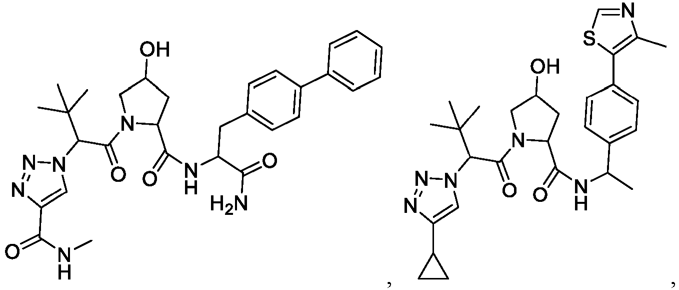 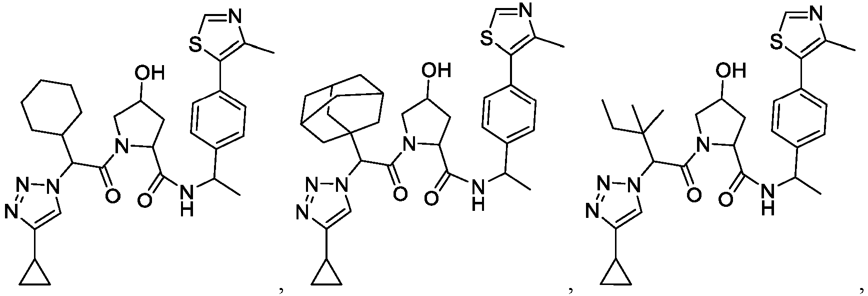 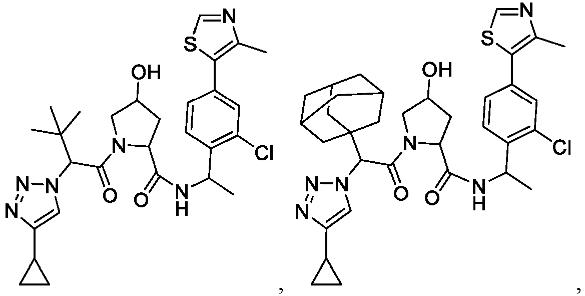 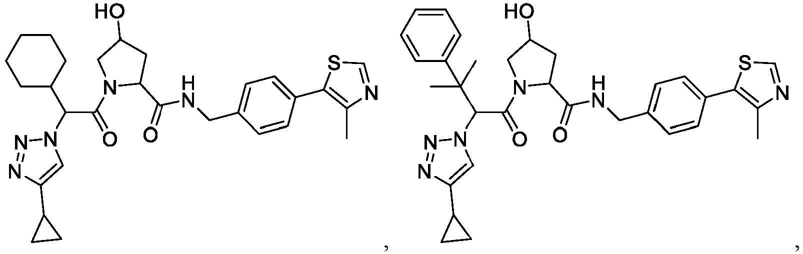 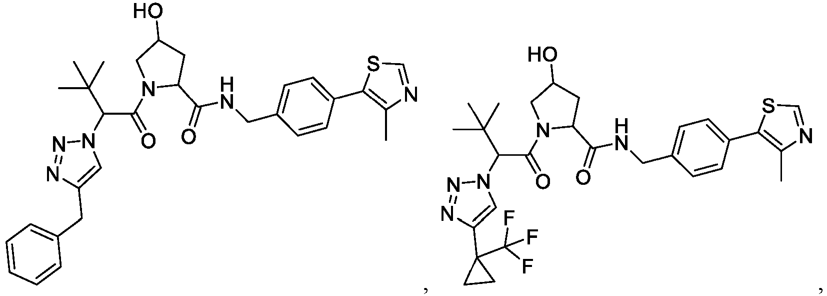 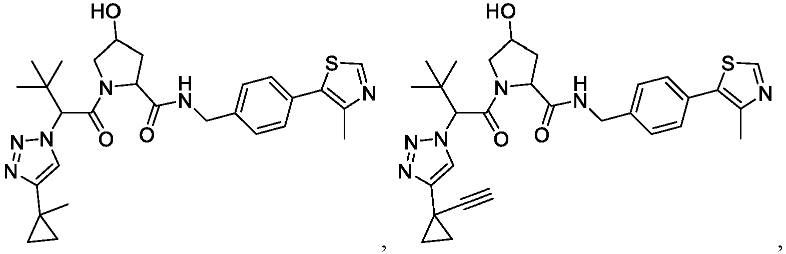 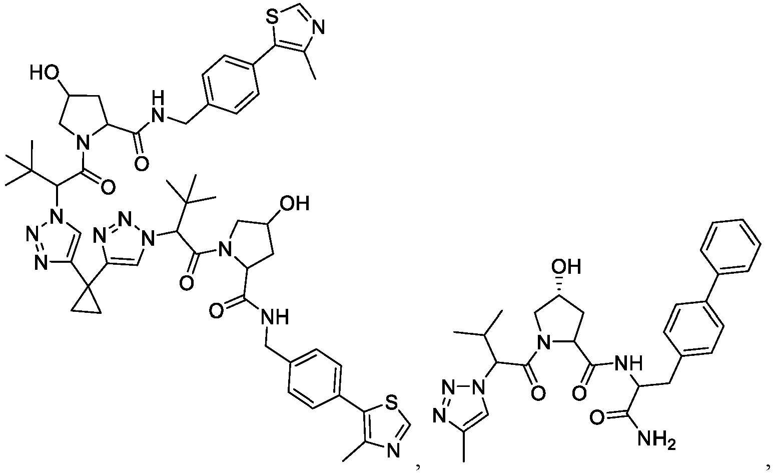 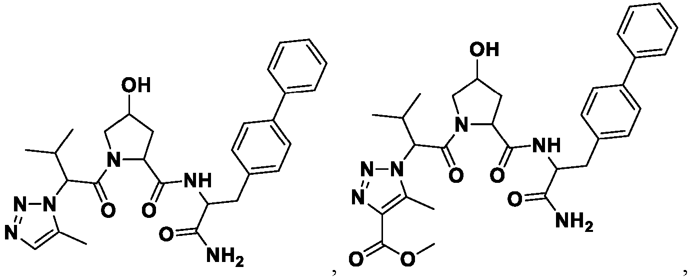 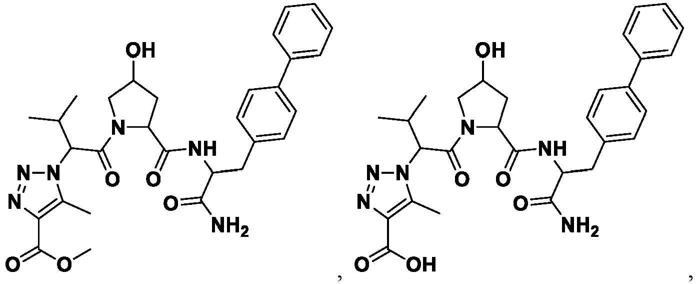 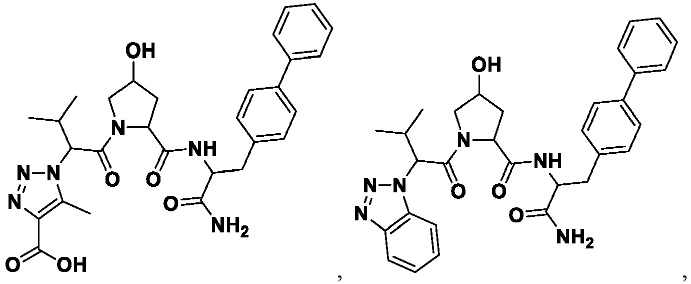 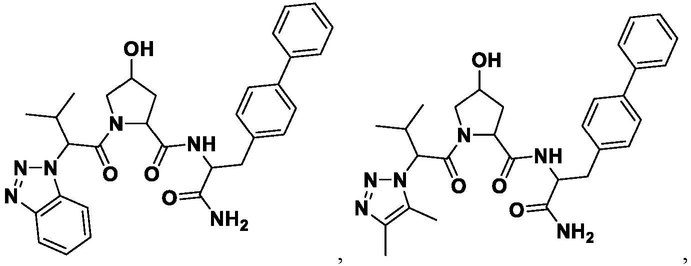 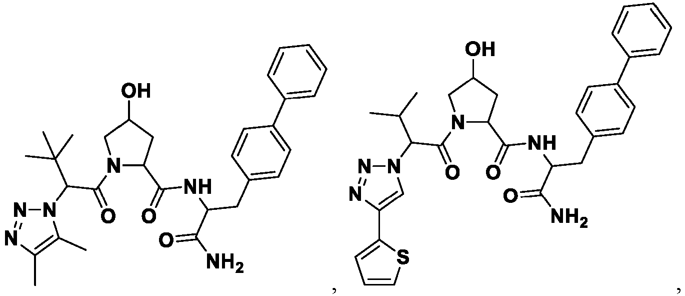 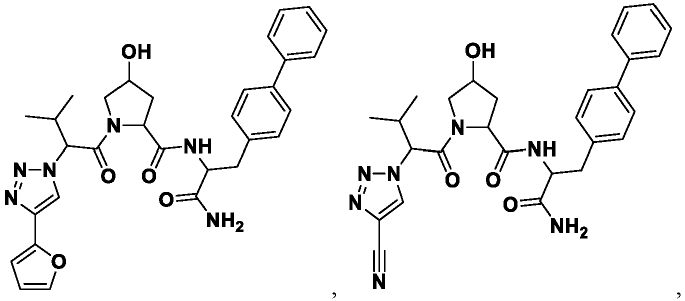 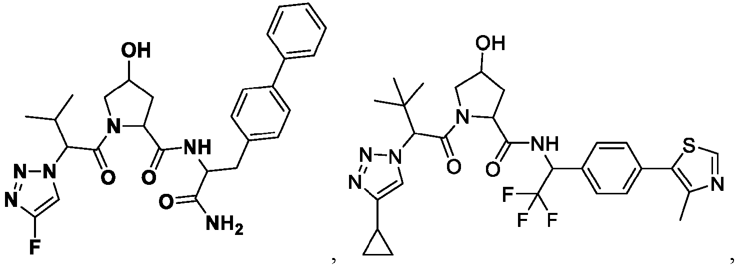 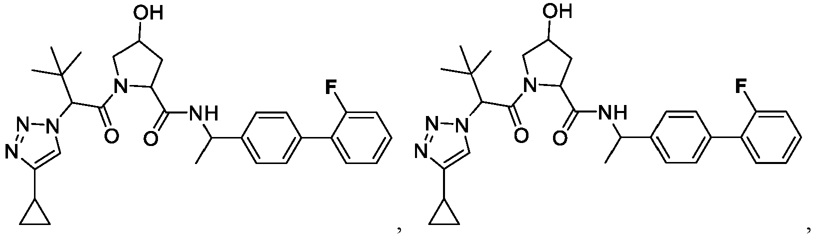 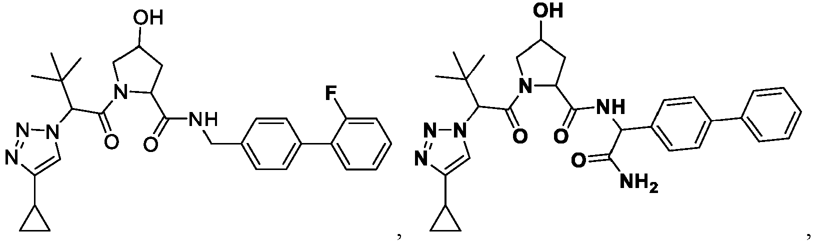 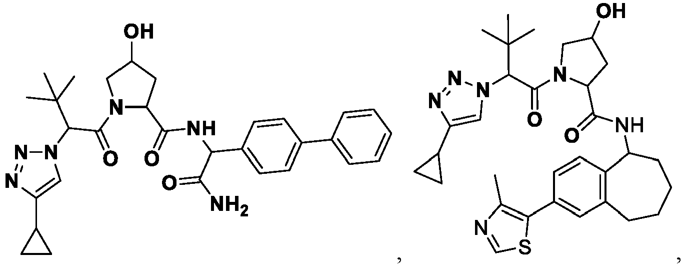 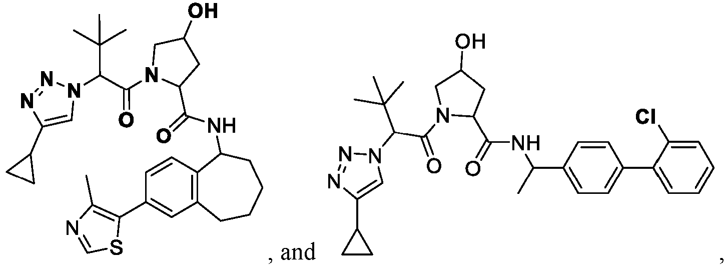 or a stereoisomer or tautomer thereof, or a pharmaceutically acceptable salt of any of the foregoing.

20. A compound of any one of claims 1-19, or a stereoisomer or tautomer thereof, or a pharmaceutically acceptable salt of any of the foregoing, for use in
(a) the treatment of anemia;
(b) the treatment of chronic anemia or anemia associated with chronic kidney disease, dialysis, or cancer chemotherapy, or any combination thereof; or
(c) the treatment of ischemia, stroke, or damage to the cardiovascular system during ischemia, or any combination thereof;
(d) the enhancement of wound healing in a human in need thereof;
(e) the reduction of scarring secondary to wound healing in a human in need thereof;
(f) the enhancement of angiogenesis or arteriogenesis, or both, in a human in need thereof;
(g) the enhancement of angiogenesis or arteriogenesis, or both, in a human, wherein the enhancement of angiogenesis or arteriogenesis, or both, occurs locally in the human; or
(h) reducing the likelihood of stent occlusion in a human in need thereof.

## Patentansprüche

1. Verbindung der Formel (I): 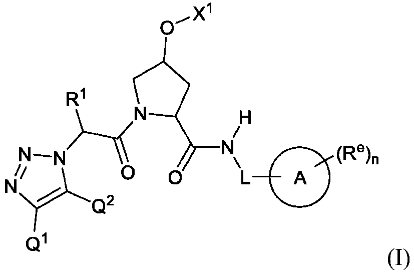 oder ein Stereoisomer oder Tautomer davon oder ein pharmazeutisch verträgliches Salz von einem der Vorstehenden, wobei:
X¹ unabhängig bei jedem Vorkommen H, C₁₋₁₂-Alkyl oder -C(O)-C₁₋₁₂-Alkyl ist;
R¹ unabhängig bei jedem Vorkommen C₁₋₁₂-Alkyl, C₂₋₁₂-Alkenyl, C₂₋₁₂-Alkinyl,
C₃₋₁₅-Cycloalkyl oder 3-15-gliedriges Heterocyclyl ist,
wobei das C₁₋₁₂-Alkyl, C₂₋₁₂-Alkenyl, C₂₋₁₂-Alkinyl, C₃₋₁₅-Cycloalkyl oder 3-15-gliedrige Heterocyclyl von R¹ unabhängig gegebenenfalls mit einem oder mehreren C₁₋₁₂-Alkyl, C₆₋₂₀-Aryl, -S(O)₂-C₁₋₁₂-Alkyl oder -C(O)-C₁₋₁₂-Alkyl substituiert ist;
L unabhängig bei jedem Vorkommen abwesend ist oder C₁₋₁₂-Alkylen ist, wobei das C₁₋₁₂-Alkylen von L unabhängig gegebenenfalls mit einem oder mehreren R^{t} substituiert ist, wobei R^{t} C₁₋₁₂-Alkyl oder -C(O)NH₂ ist, wobei das C₁₋₁₂-Alkyl von R^{t} ferner gegebenenfalls mit einem oder mehreren Halogenatomen substituiert ist;
Ring A unabhängig bei jedem Vorkommen C₆₋₂₀-Aryl oder C₇₋₁₅-Cycloalkyl ist;
R^{e}, unabhängig bei jedem Vorkommen, Halogen, C₆₋₂₀-Aryl oder 5-20-gliedriges Heteroaryl ist, mit der Maßgabe, dass mindestens ein R^{e} C₆₋₂₀-Aryl oder 5-20-gliedriges Heteroaryl ist, das ein oder mehrere Ring-Schwefelatome umfasst, wobei das C₆₋₂₀-Aryl oder das 5-20-gliedrige Heteroaryl von R^{e} unabhängig gegebenenfalls mit einem oder mehreren C₁₋₁₂-Alkyl oder Halogenatomen substituiert ist;
n unabhängig bei jedem Vorkommen 1, 2, 3, 4 oder 5 ist; und
Q¹ und Q², unabhängig voneinander und unabhängig bei jedem Vorkommen, H, Halogen, Cyano, C₁₋₁₂-Alkyl, C₃₋₁₅-Cycloalkyl, 3-15-gliedriges Heterocyclyl, C₆₋₂₀-Aryl, 5-20-gliedriges Heteroaryl, -C(O)-O(R^{a}) oder -C(O)-N(R^{b})(R^{c}) sind, wobei R^{a}, R^{b} und R^{c} jeweils unabhängig H oder C₁₋₁₂-Alkyl sind,
wobei das C₁₋₁₂-Alkyl oder C₃₋₁₅-Cycloalkyl von Q¹ oder Q² unabhängig gegebenenfalls mit einem oder mehreren R^{q} substituiert ist, wobei R^{q} C₁₋₁₂-Alkyl, C₂₋₁₂-Alkenyl, C₂₋₁₂-Alkinyl, C₆₋₂₀-Aryl, C₁₋₁₂-Alkoxy oder 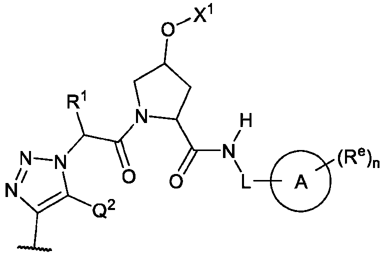 ist, wobei das C₁₋₁₂-Alkyl oder C₁₋₁₂-Alkoxy von R^{q} unabhängig ferner gegebenenfalls mit einem oder mehreren Halogenatomen oder -NHC(O)-C₁₋₁₂-Alkyl substituiert ist,
oder Q¹ und Q² zusammen mit den Atomen, an die sie gebunden sind, ein C₃₋₁₅-Cycloalkyl, 3-15-gliedriges Heterocyclyl, C₆₋₂₀-Aryl oder 5-20-gliedriges Heteroaryl bilden,
wobei das durch Q¹ und Q² gebildete C₃₋₁₅-Cycloalkyl, 3-15-gliedrige Heterocyclyl, C₆₋₂₀-Aryl oder 5-20-gliedrige Heteroaryl unabhängig gegebenenfalls mit einem oder mehreren R^{s} substituiert ist, wobei R^{s} OH, Cyano, Halogen, Oxo, -NH₂, -NO₂, -CHO, - C(O)OH, -C(O)NH₂, -SH, -SO₂C₁₋₁₂-Alkyl, -SO₂NH₂ oder C₁₋₁₂-Alkyl ist, wobei das C₁₋₁₂-Alkyl von R^{s} ferner gegebenenfalls mit einem oder mehreren Halogenatomen, Cyano oder OH substituiert ist,
mit der Maßgabe, dass die Verbindung der Formel (I) oder ein pharmazeutisch verträgliches Salz davon nicht (2S,4R)-1-((S)-2-(4-Cyclopropyl-1H-1,2,3-triazol-1-yl)-3,3-dimethylbutanoyl)-4-hydroxy-N-(4-(4-methylthiazol-5-yl)benzyl)pyrrolidin-2-carboxamid oder ein pharmazeutisch verträgliches Salz davon ist.

2. Verbindung nach Anspruch 1 oder ein Stereoisomer oder Tautomer davon oder ein pharmazeutisch verträgliches Salz von einem der Vorstehenden, wobei L unabhängig bei jedem Vorkommen C₁₋₆-Alkylen ist, wobei das C₁₋₆-Alkylen von L unabhängig gegebenenfalls mit einem oder mehreren R^{t} substituiert ist, wobei R C₁₋₆-Alkyl oder -C(O)NH₂ ist, wobei das C₁₋₆-Alkyl von R^{t} ferner gegebenenfalls mit einem oder mehreren Halogenatomen substituiert ist.

3. Verbindung nach Anspruch 1 oder Anspruch 2 oder ein Stereoisomer oder Tautomer davon oder ein pharmazeutisch verträgliches Salz von einem der Vorstehenden, wobei Ring A unabhängig bei jedem Vorkommen C₆₋₂₀-Aryl ist.

4. Verbindung nach einem der Ansprüche 1-3 oder ein Stereoisomer oder Tautomer davon oder ein pharmazeutisch verträgliches Salz von einem der Vorstehenden, wobei
n 1 ist und
R^{e} unabhängig bei jedem Vorkommen 5-20-gliedriges Heteroaryl ist, wobei das 5-20-gliedrige Heteroaryl von R^{e} ein oder mehrere Ring-Schwefelatome umfasst und unabhängig gegebenenfalls mit einem oder mehreren C₁₋₁₂-Alkyl substituiert ist.

5. Verbindung nach einem der Ansprüche 1-4 oder ein Stereoisomer oder Tautomer davon oder ein pharmazeutisch verträgliches Salz von einem der Vorstehenden, wobei die Verbindung der Formel (I) eine Verbindung der Formel (IA) ist: 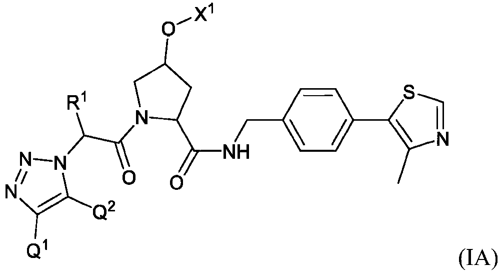 oder ein Stereoisomer oder Tautomer davon oder ein pharmazeutisch verträgliches Salz von einem der Vorstehenden.

6. Verbindung nach einem der Ansprüche 1-4 oder ein Stereoisomer oder Tautomer davon oder ein pharmazeutisch verträgliches Salz von einem der Vorstehenden, wobei die Verbindung der Formel (I) eine Verbindung der Formel (IB) ist: 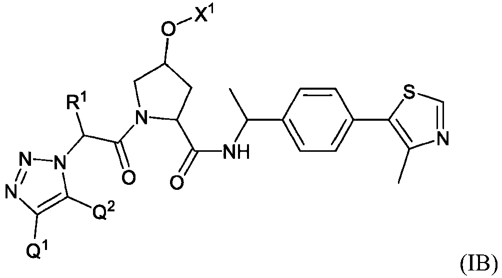 oder ein Stereoisomer oder Tautomer davon oder ein pharmazeutisch verträgliches Salz von einem der Vorstehenden.

7. Verbindung nach einem der Ansprüche 1-4 oder ein Stereoisomer oder Tautomer davon oder ein pharmazeutisch verträgliches Salz von einem der Vorstehenden, wobei die Verbindung der Formel (I) eine Verbindung der Formel (IC) ist: 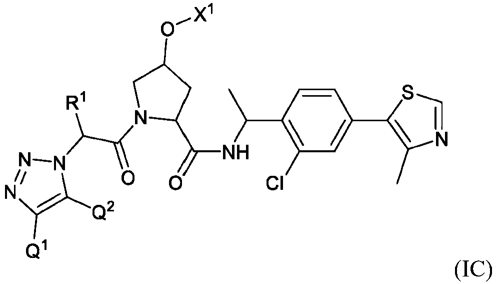 oder ein Stereoisomer oder Tautomer davon oder ein pharmazeutisch verträgliches Salz von einem der Vorstehenden.

8. Verbindung nach einem der Ansprüche 1-3 oder ein Stereoisomer oder Tautomer davon oder ein pharmazeutisch verträgliches Salz von einem der Vorstehenden, wobei
n 1 ist und
R^{e} unabhängig bei jedem Vorkommen C₆₋₂₀-Aryl ist, wobei das C₆₋₂₀-Aryl von R^{e} unabhängig gegebenenfalls mit einem oder mehreren C₁₋₁₂-Alkyl oder Halogenatomen substituiert ist.

9. Verbindung nach einem der Ansprüche 1-3 und 8 oder ein Stereoisomer oder Tautomer davon oder ein pharmazeutisch verträgliches Salz von einem der Vorstehenden, wobei die Verbindung der Formel (I) eine Verbindung der Formel (ID) ist: 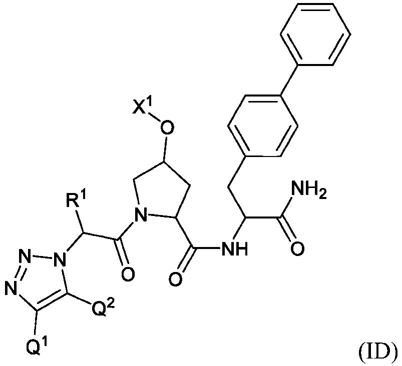 oder ein Stereoisomer oder Tautomer davon oder ein pharmazeutisch verträgliches Salz von einem der Vorstehenden.

10. Verbindung nach Anspruch 9 oder ein Stereoisomer oder Tautomer davon oder ein pharmazeutisch verträgliches Salz von einem der Vorstehenden, wobei die Verbindung der Formel (ID) eine Verbindung ist, ausgewählt aus der Gruppe, bestehend aus: 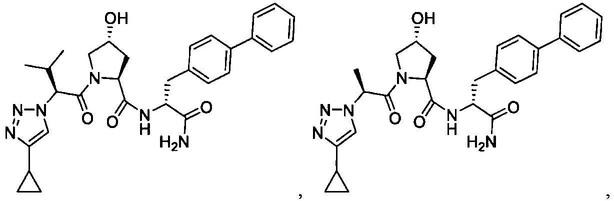 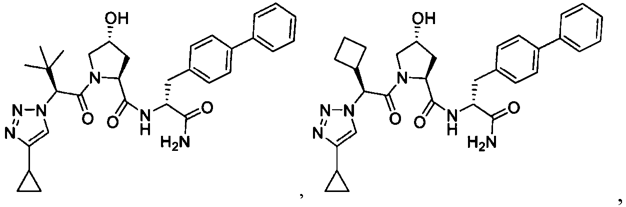 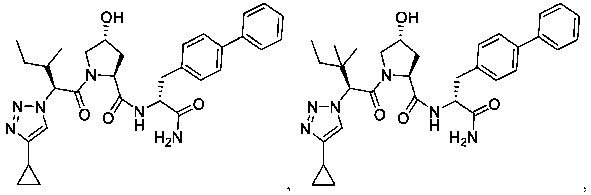 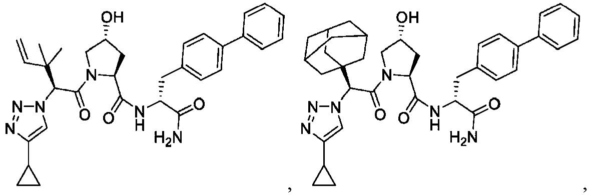 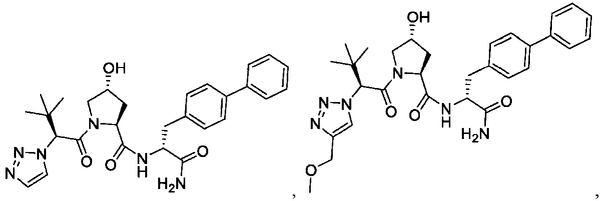 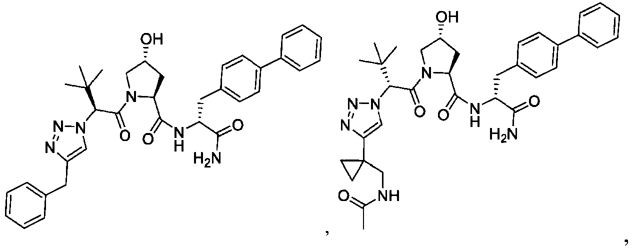 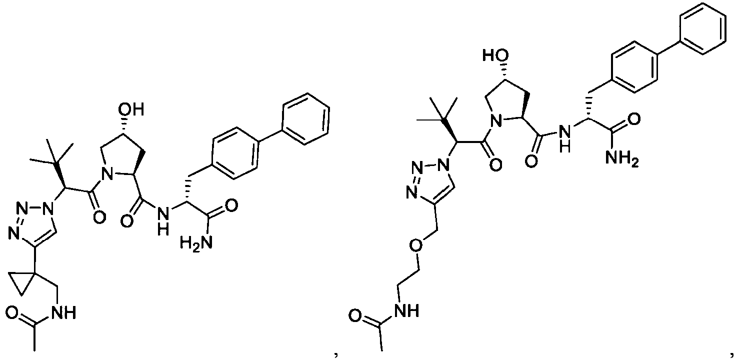 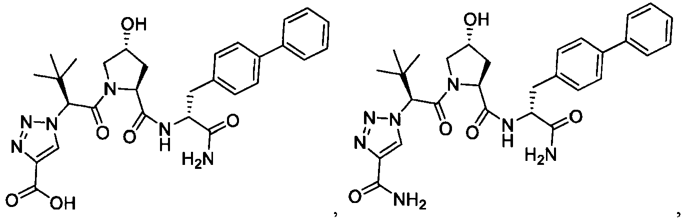 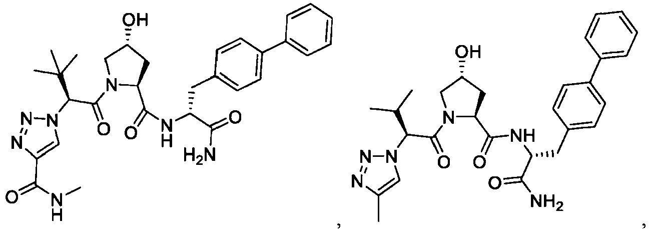 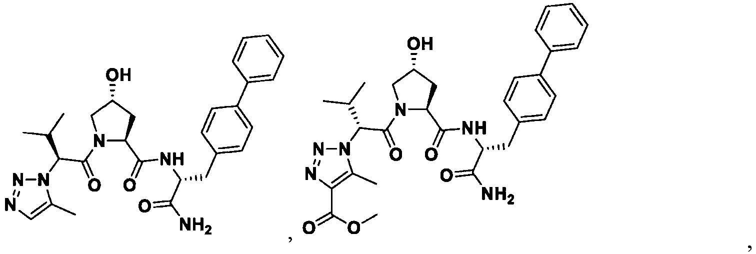 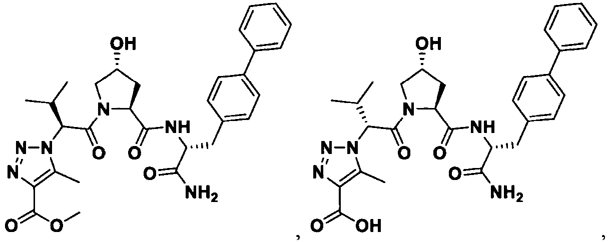 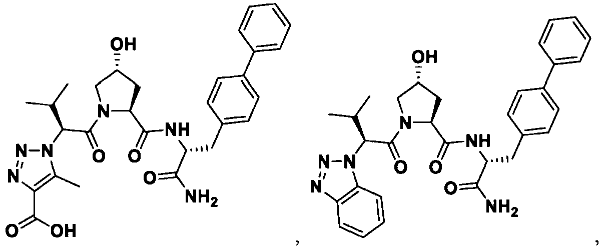 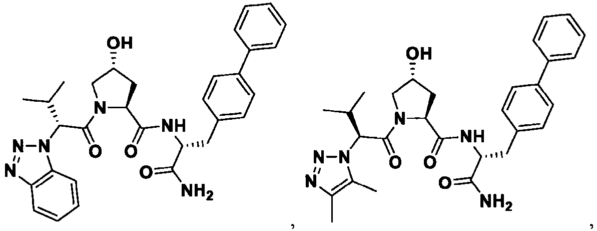 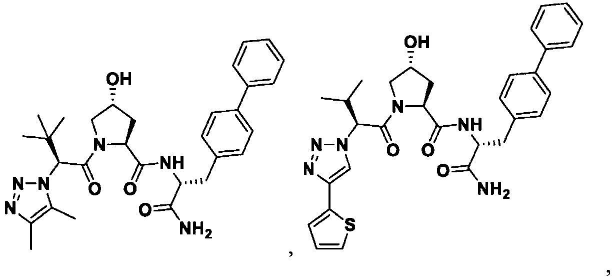 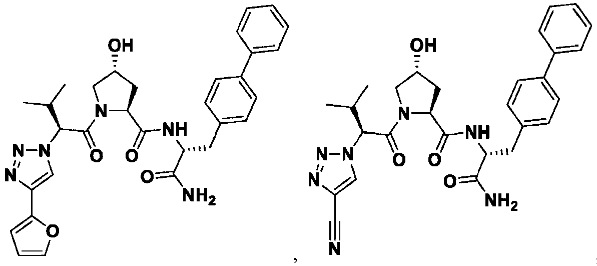 und 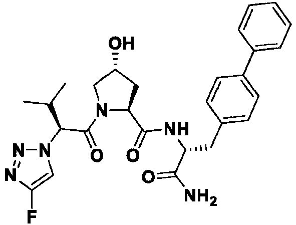 oder ein Stereoisomer oder Tautomer davon, oder ein pharmazeutisch verträgliches Salz von einem der Vorstehenden.

11. Verbindung nach Anspruch 1 oder ein Stereoisomer oder Tautomer davon oder ein pharmazeutisch verträgliches Salz von einem der Vorstehenden, wobei die Verbindung der Formel (I) eine Verbindung der Formel (IF) ist: 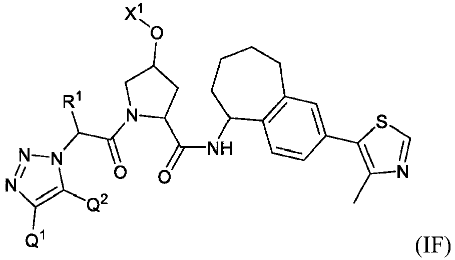 oder ein Stereoisomer oder Tautomer davon oder ein pharmazeutisch verträgliches Salz von einem der Vorstehenden.

12. Verbindung nach einem der Ansprüche 1-9 und 11 oder ein Stereoisomer oder Tautomer davon oder ein pharmazeutisch verträgliches Salz von einem der Vorstehenden, wobei Q¹ und Q² unabhängig voneinander und unabhängig bei jedem Vorkommen H, Halogen, Cyano, C₁₋₁₂-Alkyl, C₃₋₁₅-Cycloalkyl, 3-15-gliedriges Heterocyclyl, C₆₋₂₀-Aryl, 5-20-gliedriges Heteroaryl, -C(O)-O(R^{a}) oder -C(O)-N(R^{b})(R^{c}) sind, wobei R^{a}, R^{b} und R^{c} jeweils unabhängig H oder C₁₋₁₂-Alkyl sind,
wobei das C₁₋₁₂-Alkyl oder C₃₋₁₅-Cycloalkyl von Q¹ oder Q² unabhängig gegebenenfalls mit einem oder mehreren R^{q} substituiert ist, wobei R^{q} C₁₋₁₂-Alkyl, C₂₋₁₂-Alkenyl, C₂₋₁₂-Alkinyl, C₆₋₂₀-Aryl, C₁₋₁₂-Alkoxy oder 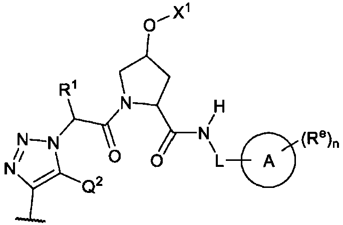 ist, wobei das C₁₋₁₂-Alkyl oder C₁₋₁₂-Alkoxy von R^{q} unabhängig ferner gegebenenfalls mit einem oder mehreren Halogenatomen oder -NHC(O)-C₁₋₁₂-Alkyl substituiert ist.

13. Verbindung nach einem der Ansprüche 1-9 und 11-12 oder ein Stereoisomer oder Tautomer davon oder ein pharmazeutisch verträgliches Salz von einem der Vorstehenden, wobei Q¹ C₃₋₁₅-Cycloalkyl ist, wobei das C₃₋₁₅-Cycloalkyl von Q¹ gegebenenfalls mit einem oder mehreren R^{q} substituiert ist, wobei R^{q} unabhängig C₁₋₁₂-Alkyl, C₂₋₁₂-Alkenyl, C₂₋₁₂-Alkinyl, C₆₋₂₀-Aryl, C₁₋₁₂-Alkoxy oder 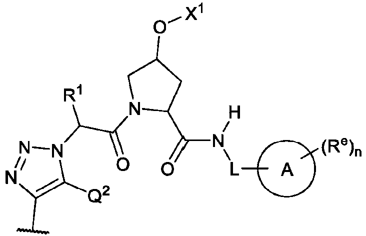 ist, wobei das C₁₋₁₂-Alkyl oder C₁₋₁₂-Alkoxy von R^{q} unabhängig ferner gegebenenfalls mit einem oder mehreren Halogenatomen oder -NHC(O)-C₁₋₁₂-Alkyl substituiert ist.

14. Verbindung nach einem der Ansprüche 1-13 oder ein Stereoisomer oder Tautomer davon oder ein pharmazeutisch verträgliches Salz von einem der Vorstehenden, wobei Q¹ unsubstituiertes C₃₋₁₅-Cycloalkyl ist.

15. Verbindung nach einem der Ansprüche 1-14 oder ein Stereoisomer oder Tautomer davon oder ein pharmazeutisch verträgliches Salz von einem der Vorstehenden, wobei R¹ unabhängig bei jedem Vorkommen C₁₋₁₂-Alkyl ist, wobei das C₁₋₁₂-Alkyl von R¹ unabhängig gegebenenfalls mit einem oder mehreren C₆₋₂₀-Aryl, -S(O)₂-C₁₋₁₂-Alkyl oder -C(O)-C₁₋₁₂-Alkyl substituiert ist.

16. Verbindung nach einem der Ansprüche 1-14 oder ein Stereoisomer oder Tautomer davon oder ein pharmazeutisch verträgliches Salz von einem der Vorstehenden, wobei R¹ unabhängig bei jedem Vorkommen C₃₋₁₅-Cycloalkyl ist, wobei das C₃₋₁₅-Cycloalkyl von R¹ gegebenenfalls mit einem oder mehreren C₁₋₁₂-Alkyl, C₆₋₂₀-Aryl, -S(O)₂-C₁₋₁₂-Alkyl oder - C(O)-C₁₋₁₂-Alkyl substituiert ist.

17. Verbindung nach Anspruch 16 oder ein Stereoisomer oder Tautomer davon oder ein pharmazeutisch verträgliches Salz von einem der Vorstehenden, wobei R¹ unabhängig bei jedem Vorkommen C₃₋₆-Cycloalkyl ist, wobei das C₃₋₆-Cycloalkyl von R¹ gegebenenfalls mit einem oder mehreren C₁₋₁₂-Alkyl, C₆₋₂₀-Aryl, -S(O)₂-C₁₋₁₂-Alkyl oder -C(O)-C₁₋₁₂-Alkyl substituiert ist.

18. Verbindung nach Anspruch 1 oder ein Stereoisomer oder Tautomer davon oder ein pharmazeutisch verträgliches Salz von einem der Vorstehenden, wobei die Verbindung ausgewählt ist aus der Gruppe, bestehend aus 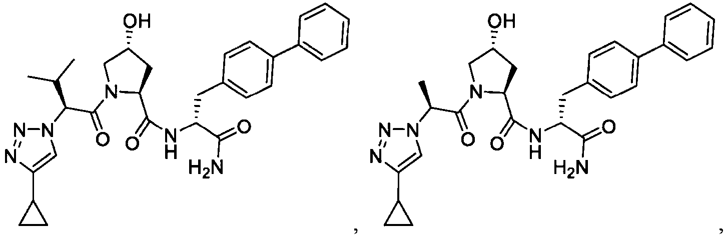 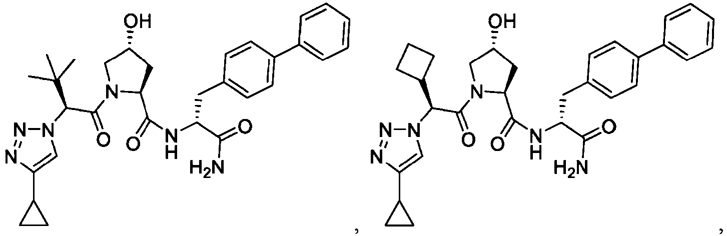 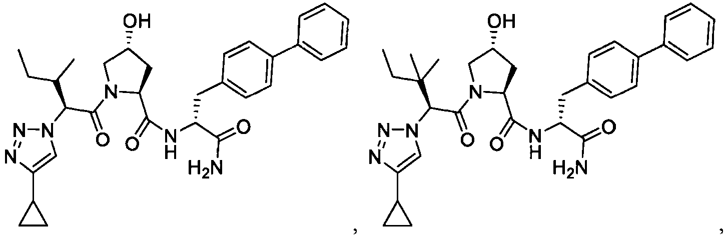 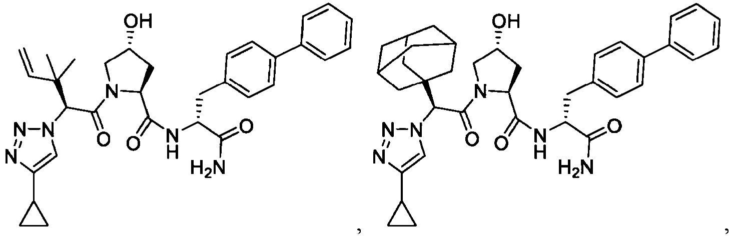 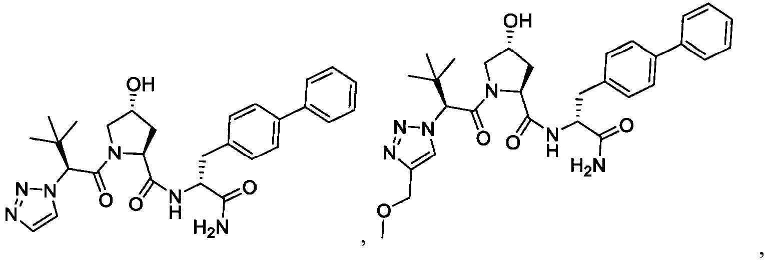 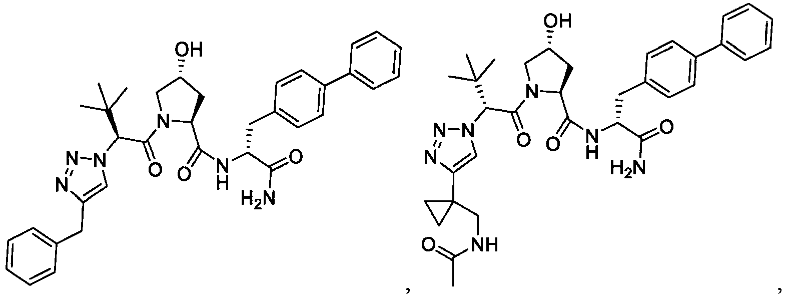 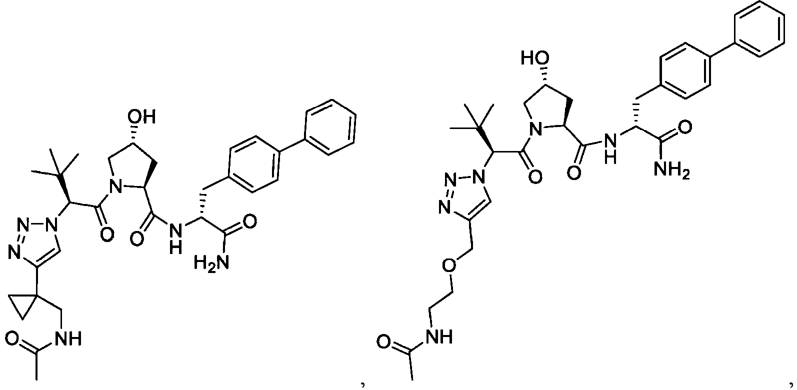 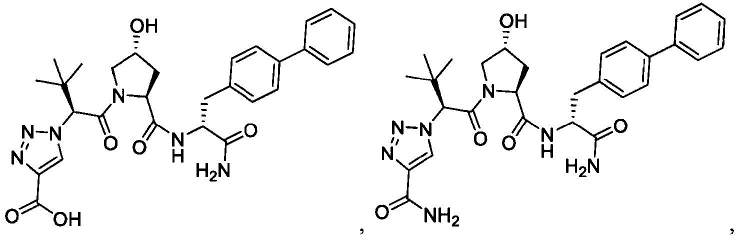 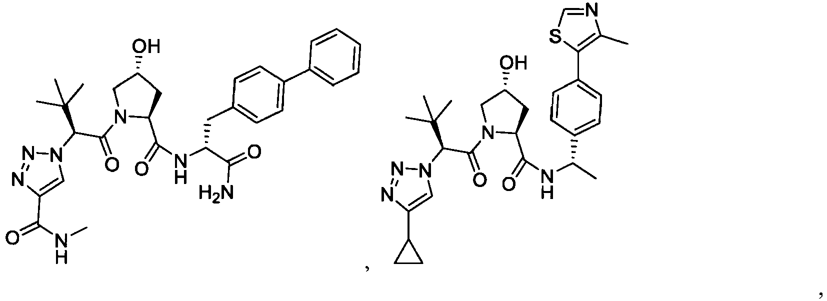 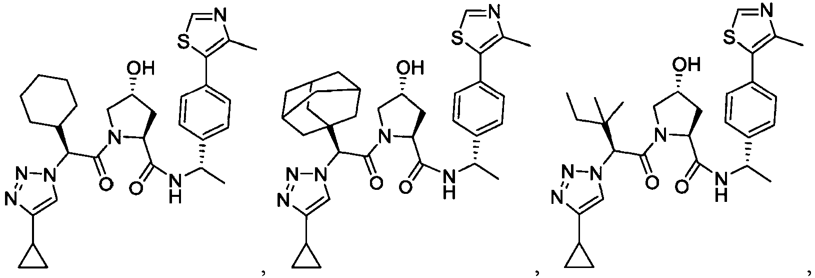 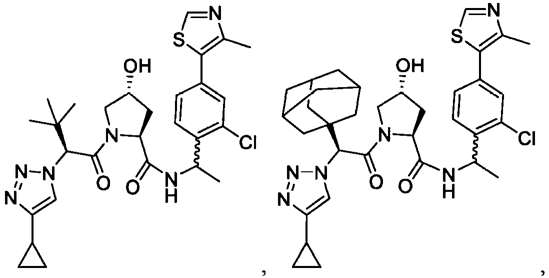 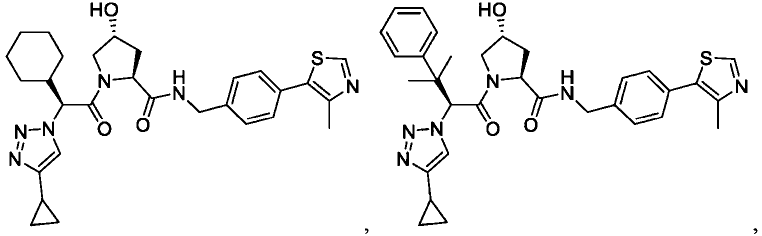 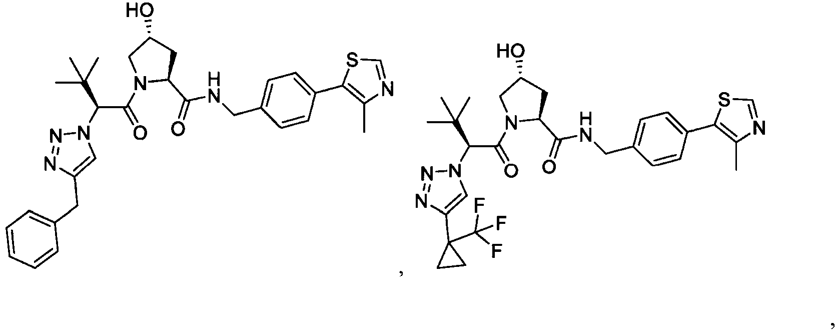 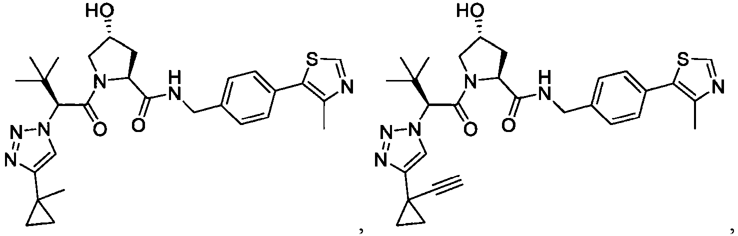 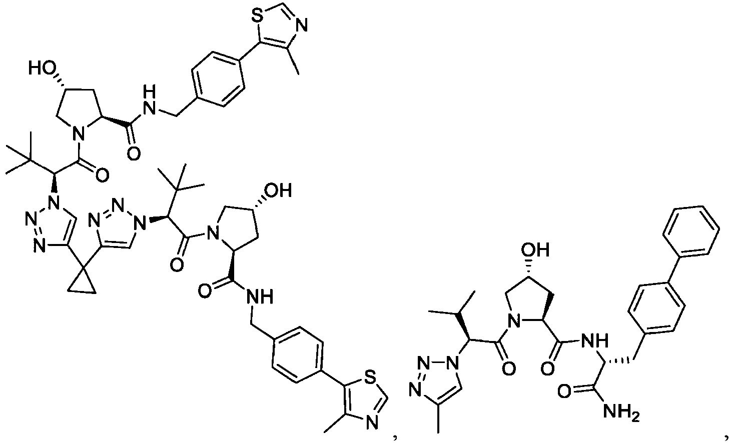 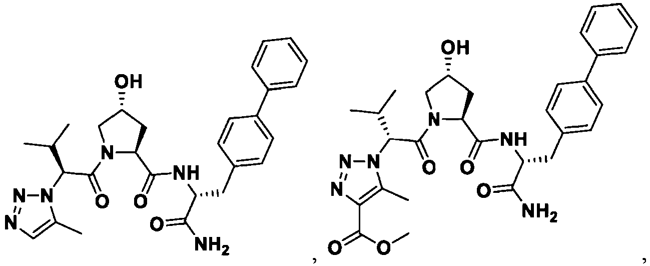 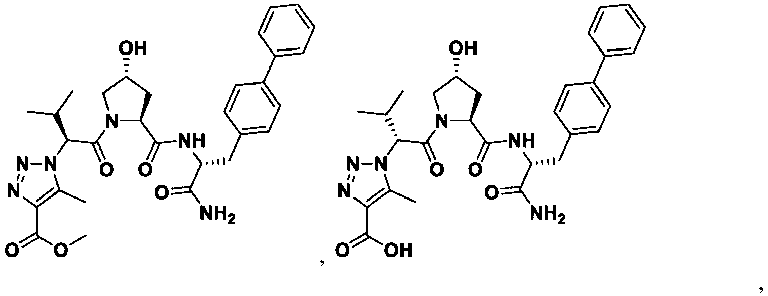 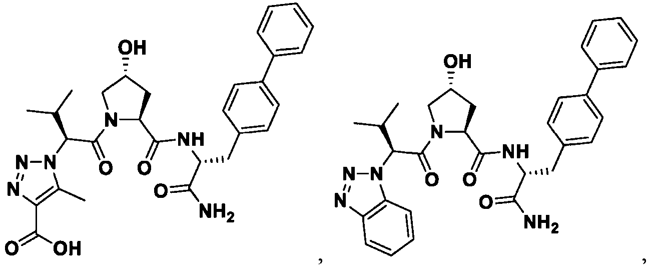 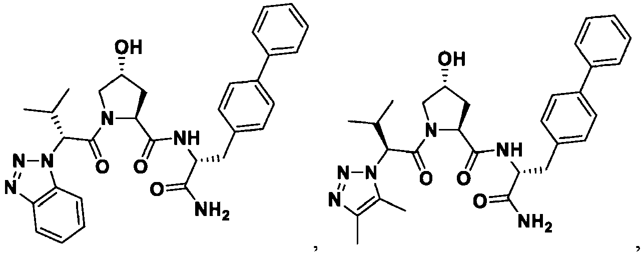 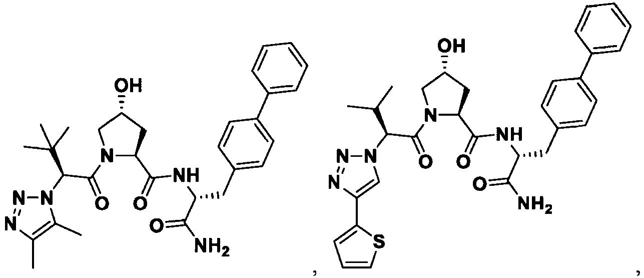 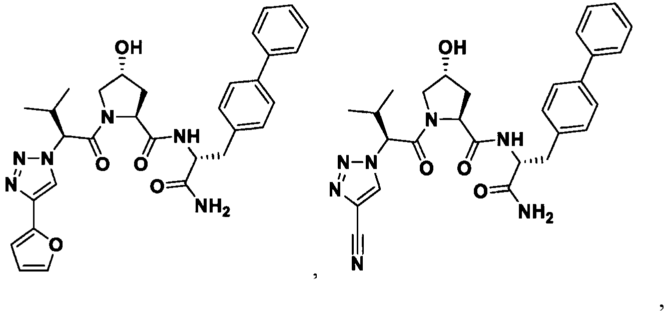 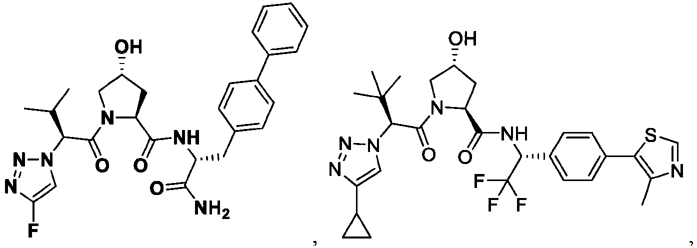 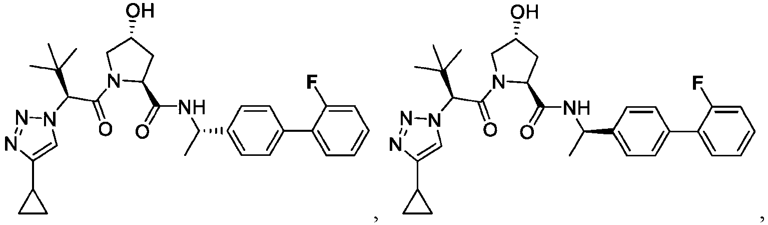 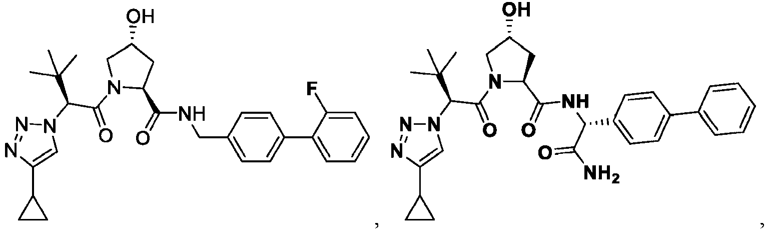 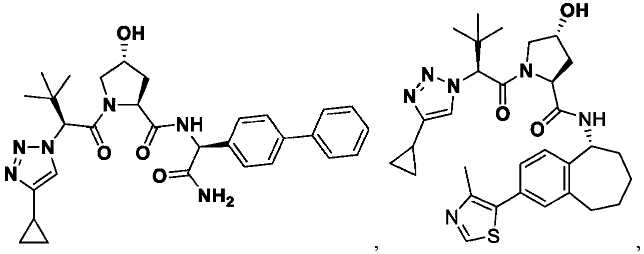 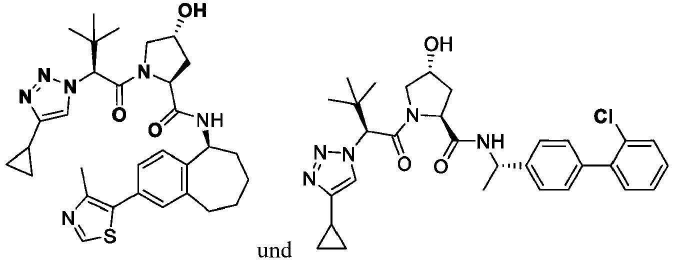 oder ein Stereoisomer oder Tautomer davon oder ein pharmazeutisch verträgliches Salz von einem der Vorstehenden.

19. Verbindung nach Anspruch 1 oder ein Stereoisomer oder Tautomer davon oder ein pharmazeutisch verträgliches Salz von einem der Vorstehenden, wobei die Verbindung ausgewählt ist aus der Gruppe, bestehend aus 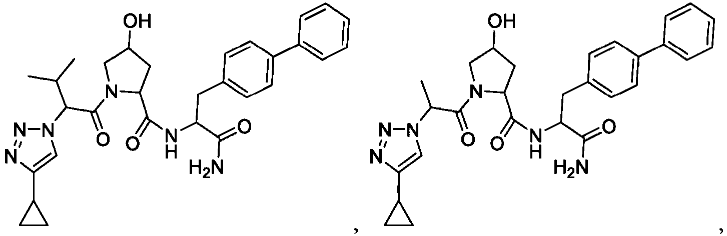 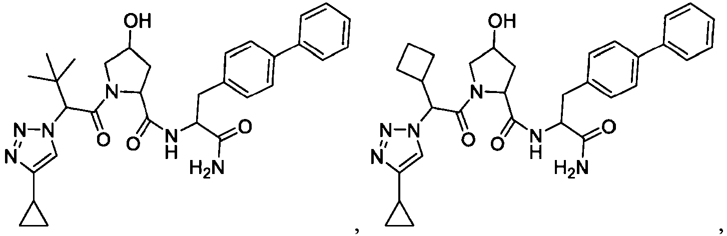 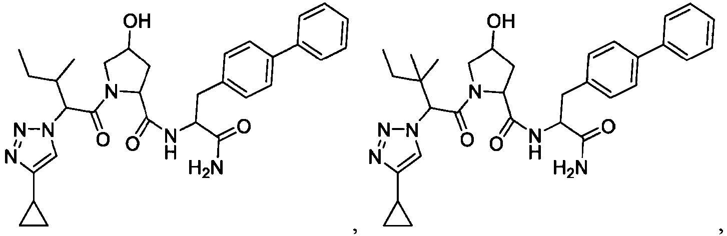 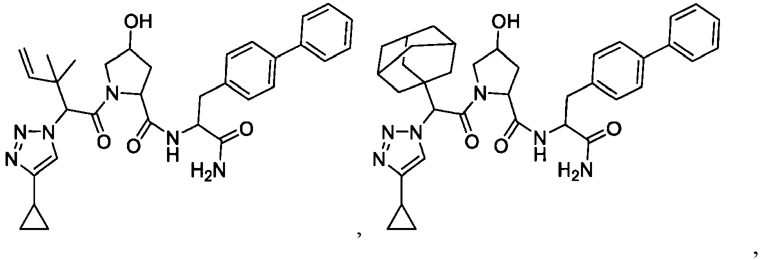 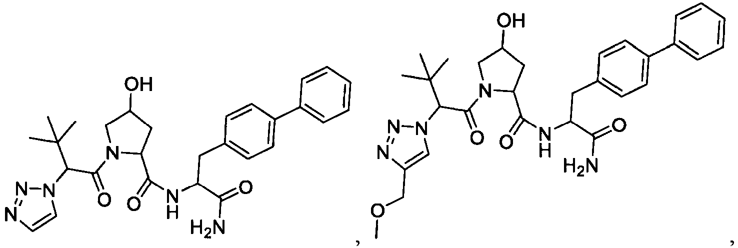 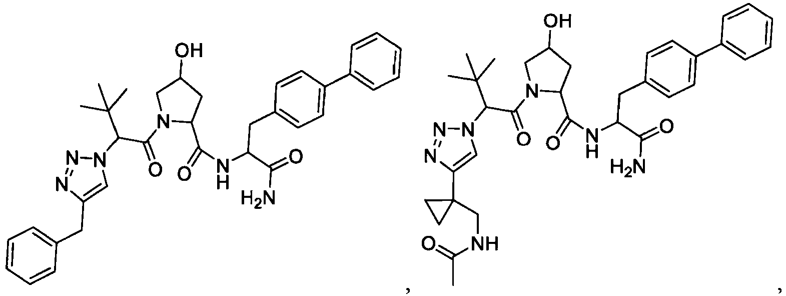 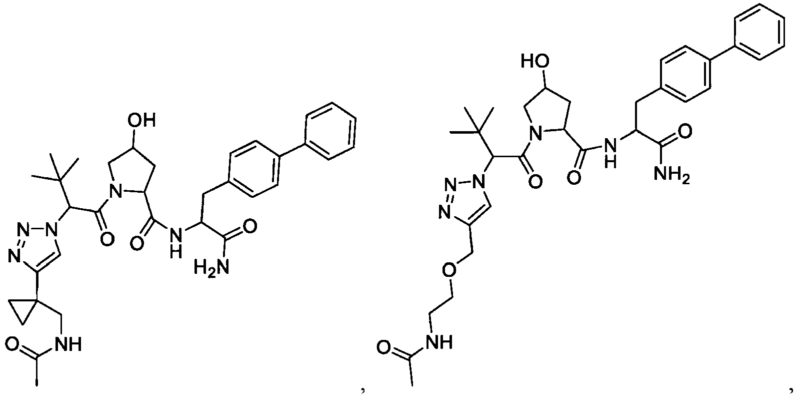 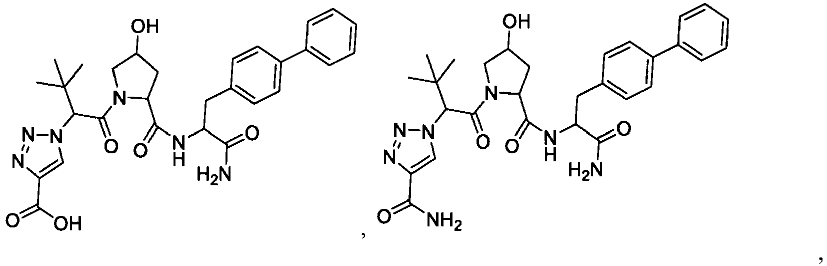 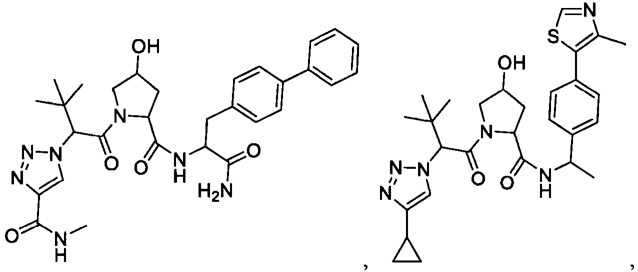 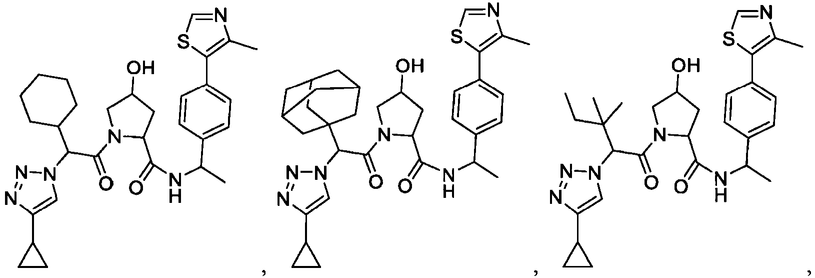 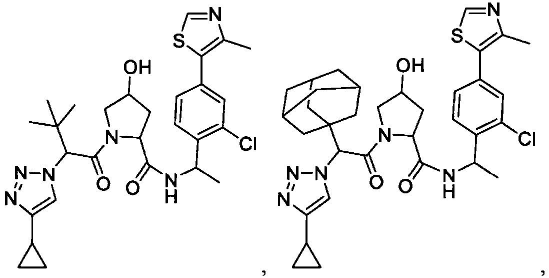 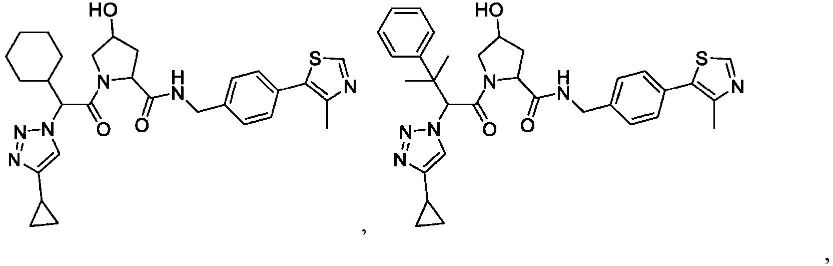 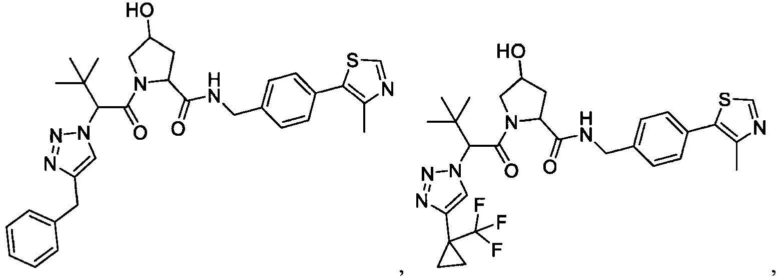 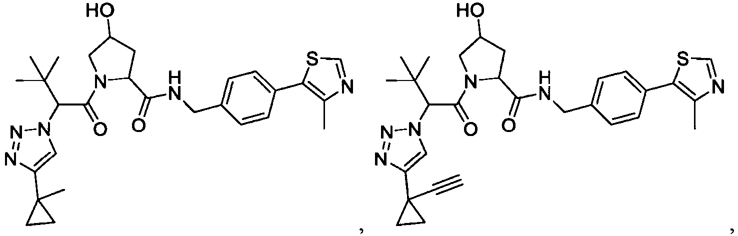 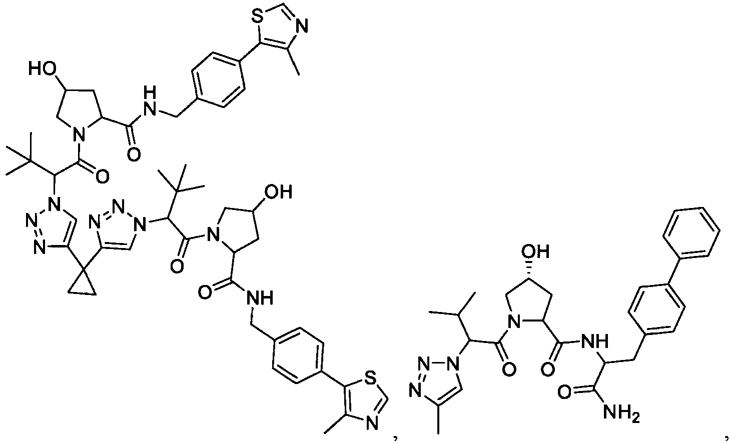 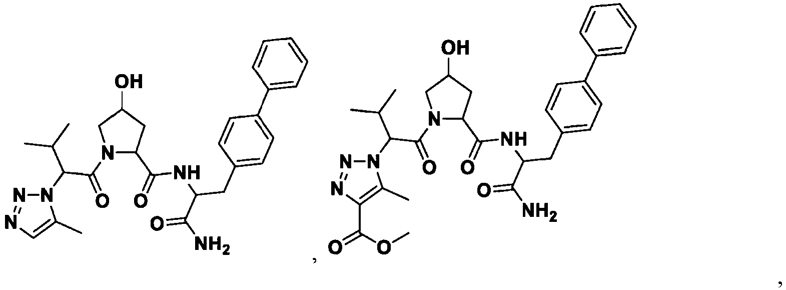 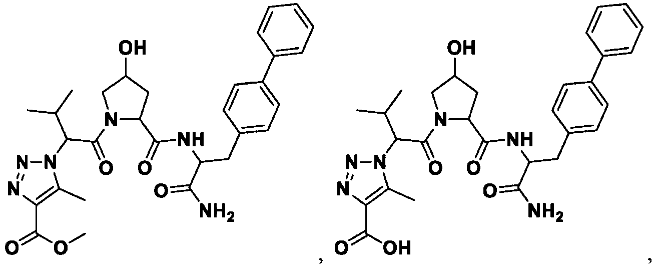 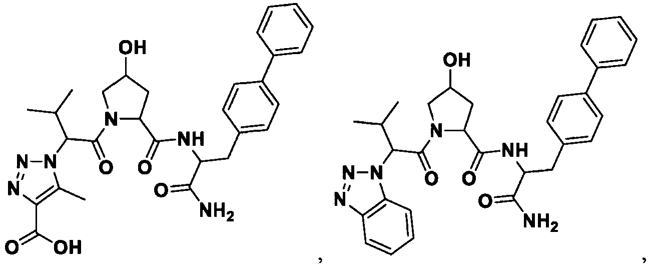 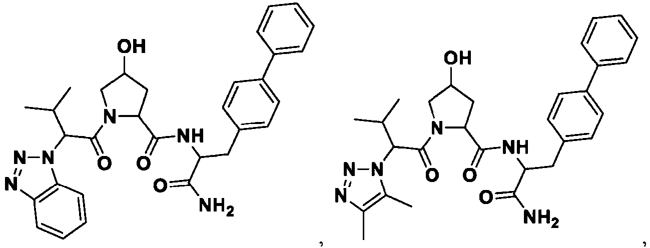 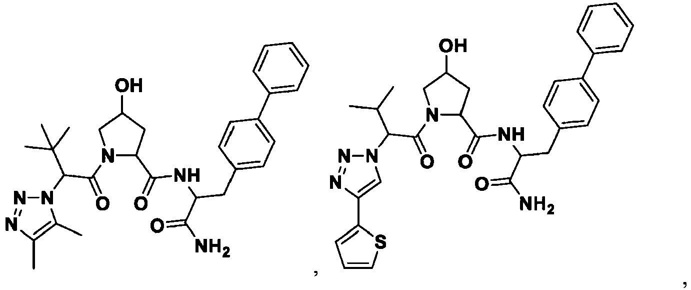 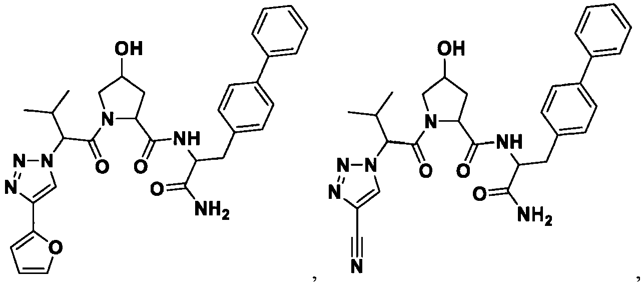 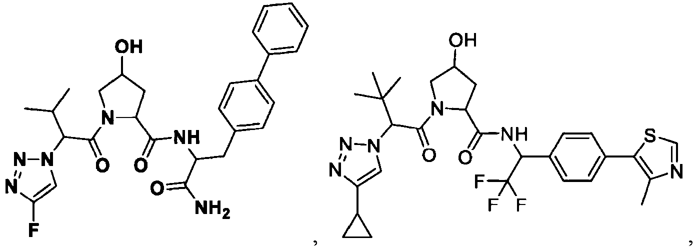 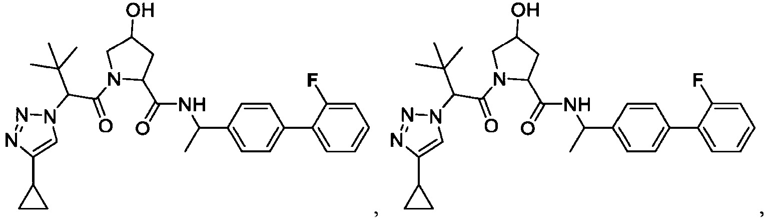 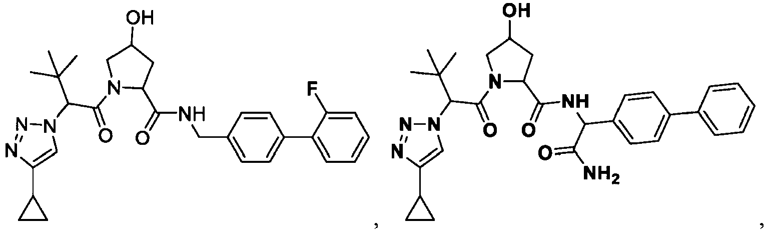 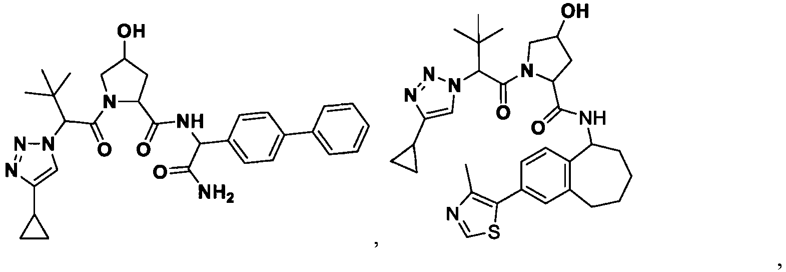 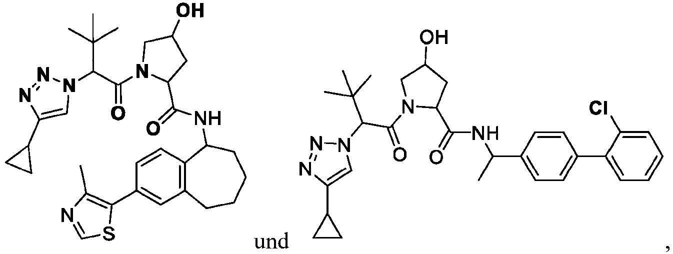 oder ein Stereoisomer oder Tautomer davon, oder ein pharmazeutisch verträgliches Salz von einem der Vorstehenden.

20. Verbindung nach einem der Ansprüche 1-19 oder ein Stereoisomer oder Tautomer davon oder ein pharmazeutisch verträgliches Salz von einem der Vorstehenden zur Verwendung bei
(a) der Behandlung von Anämie;
(b) der Behandlung von chronischer Anämie oder Anämie in Verbindung mit chronischer Nierenerkrankung, Dialyse oder Krebschemotherapie oder einer beliebigen Kombination davon; oder
(c) der Behandlung von Ischämie, Schlaganfall oder Schädigung des Herz-KreislaufSystems während Ischämie, oder einer beliebigen Kombination davon;
(d) der Verbesserung der Wundheilung bei einem Menschen, der Bedarf daran hat;
(e) der Verringerung der Narbenbildung sekundär zur Wundheilung bei einem Menschen, der Bedarf daran hat;
(f) der Förderung der Angiogenese oder Arteriogenese oder beidem bei einem Menschen, der Bedarf daran hat;
(g) der Förderung der Angiogenese oder Arteriogenese oder beidem bei einem Menschen, wobei die Förderung der Angiogenese oder Arteriogenese oder beidem lokal beim Menschen auftritt; oder
(h) der Verringerung der Wahrscheinlichkeit eines Stentverschlusses bei einem Menschen, der Bedarf daran hat.

## Revendications

1. Composé de formule (I) : 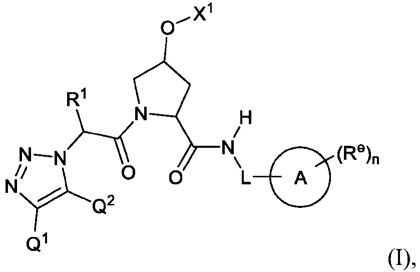 ou un stéréoisomère ou un tautomère de celui-ci, ou un sel pharmaceutiquement acceptable d'un quelconque des précédents, dans lequel :
X¹ représente, indépendamment à chaque occurrence, H, un alkyle en C₁₋₁₂ ou -C(O)-alkyle en C₁₋₁₂ ;
R¹ représente, indépendamment à chaque occurrence, un alkyle en C₁₋₁₂, un alcényle en C₂₋₁₂, un alcynyle en C₂₋₁₂, un cycloalkyle en C₃₋₁₅ ou un hétérocyclyle à 3 à 15 chaînons,
dans lequel l'alkyle en C₁₋₁₂, l'alcényle en C₂₋₁₂, l'alcynyle en C₂₋₁₂, le cycloalkyle en C₃₋₁₅ ou l'hétérocyclyle à 3 à 15 chaînons de R¹ est indépendamment éventuellement substitué par un ou plusieurs alkyles en C₁₋₁₂, aryles en C₆₋₂₀, -S(O)₂-alkyle en C₁₋₁₂ ou -C(O)-alkyle en C₁₋₁₂ ;
L est, indépendamment à chaque occurrence, absent ou représente un alkylène en C₁₋₁₂, dans lequel l'alkylène en C₁₋₁₂ de L est éventuellement indépendamment substitué par un ou plusieurs R^{t}, dans lequel R^{t} représente un alkyle en C₁₋₁₂ ou -C(O)NH₂, dans lequel l'alkyle en C₁₋₁₂ de R^{t} est en outre éventuellement substitué par un ou plusieurs halogènes ;
le cycle A représente, indépendamment à chaque occurrence, un aryle en C₆₋₂₀ ou un cycloalkyle en C₇₋₁₅ ;
R^{e} représente, indépendamment à chaque occurrence, un halogène, un aryle en C₆₋₂₀ ou un hétéroaryle à 5 à 20 chaînons, à condition qu'au moins un R° représente un aryle en C₆₋₂₀ ou un hétéroaryle à 5 à 20 chaînons comprenant un ou plusieurs atomes de soufre du cycle, dans lequel l'aryle en C₆₋₂₀ ou l'hétéroaryle à 5 à 20 chaînons de R^{e} est indépendamment éventuellement substitué par un ou plusieurs alkyles en C₁₋₁₂ ou halogènes ;
n représente, indépendamment à chaque occurrence, 1, 2, 3, 4 ou 5 ; et
Q¹ et Q² représentent, indépendamment l'un de l'autre et indépendamment à chaque occurrence, H, un halogène, un cyano, un alkyle en C₁₋₁₂, un cycloalkyle en C₃₋₁₅, un hétérocyclyle à 3 à 15 chaînons, un aryle en C₆₋₂₀, un hétéroaryle à 5 à 20 chaînons, -C(O)-O(R^{a}) ou -C(O)-N(R^{b})(R^{c}), dans lequel R^{a}, R^{b} et R^{c} représentent chacun indépendamment H ou un alkyle en C₁₋₁₂,
dans lequel l'alkyle en C₁₋₁₂ ou le cycloalkyle en C₃₋₁₅ de Q¹ ou Q² est indépendamment éventuellement substitué par un ou plusieurs R^{q}, dans lequel R^{q} représente un alkyle en C₁₋₁₂, un alcényle en C₂₋₁₂, un alcynyle en C₂₋₁₂, un aryle en C₆₋₂₀, un alcoxy en C₁₋₁₂ ou 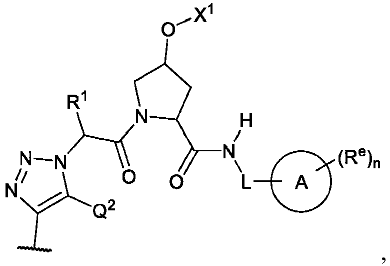 dans lequel l'alkyle en C₁₋₁₂ ou l'alcoxy en C₁₋₁₂ de R^{q} est indépendamment en outre éventuellement substitué par un ou plusieurs halogènes ou - NHC(O)-alkyle en C₁₋₁₂,
ou Q¹ et Q² sont pris, conjointement avec les atomes auxquels ils sont liés, pour former un cycloalkyle en C₃₋₁₅, un hétérocyclyle à 3 à 15 chaînons, un aryle en C₆₋₂₀ ou un hétéroaryle à 5 à 20 chaînons,
dans lequel le cycloalkyle en C₃₋₁₅, l'hétérocyclyle à 3 à 15 chaînons, l'aryle en C₆₋₂₀ ou l'hétéroaryle à 5 à 20 chaînons formé par Q¹ et Q² est indépendamment éventuellement substitué par un ou plusieurs R^{s}, dans lequel R^{s} représente OH, un cyano, un halogène, un oxo, -NH₂, -NO₂, -CHO, -C(O)OH, -C(O)NH₂, -SH, -SO₂-alkyle en C₁₋₁₂, -SO₂NH₂ ou un alkyle en C₁₋₁₂ dans lequel l'alkyle en C₁₋₁₂ de R^{s} est en outre éventuellement substitué par un ou plusieurs halogènes, cyano ou OH,
à condition que le composé de formule (I), ou un sel pharmaceutiquement acceptable de celui-ci, ne soit pas le (2S,4R)-1-((S)-2-(4-cyclopropyl-1H-1,2,3-triazol-1-yl)-3,3-diméthylbutanoyl)-4-hydroxy-N-(4-(4-méthylthiazol-5-yl)benzyl)pyrrolidine-2-carboxamide, ou un sel pharmaceutiquement acceptable de celui-ci.

2. Composé selon la revendication 1, ou un stéréoisomère ou tautomère de celui-ci, ou un sel pharmaceutiquement acceptable d'un quelconque des précédents, dans lequel L représente, indépendamment à chaque occurrence, un alkylène en C₁₋₆, dans lequel l'alkylène en C₁₋₆ de L est indépendamment éventuellement substitué par un ou plusieurs R^{t}, dans lequel R^{t} représente un alkyle en C₁₋₆ ou -C(O)NH₂, dans lequel l'alkyle en C₁₋₆ de R^{t} est en outre éventuellement substitué par un ou plusieurs halogènes.

3. Composé selon la revendication 1 ou la revendication 2, ou un stéréoisomère ou un tautomère de celui-ci, ou un sel pharmaceutiquement acceptable d'un quelconque des précédents, dans lequel le cycle A représente, indépendamment à chaque occurrence, un aryle en C₆₋₂₀.

4. Composé selon l'une quelconque des revendications 1 à 3, ou un stéréoisomère ou un tautomère de celui-ci, ou un sel pharmaceutiquement acceptable d'un quelconque des précédents, dans lequel
n représente 1, et
R^{e} représente, indépendamment à chaque occurrence, un hétéroaryle à 5 à 20 chaînons, dans lequel l'hétéroaryle à 5 à 20 chaînons de R^{e} comprend un ou plusieurs atomes de soufre du cycle et est indépendamment éventuellement substitué par un ou plusieurs alkyles en C₁₋₁₂.

5. Composé selon l'une quelconque des revendications 1 à 4, ou un stéréoisomère ou un tautomère de celui-ci, ou un sel pharmaceutiquement acceptable d'un quelconque des précédents, dans lequel le composé de formule (I) est un composé de formule (IA) : 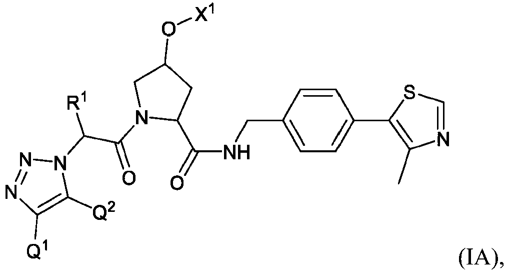 ou un stéréoisomère ou un tautomère de celui-ci, ou un sel pharmaceutiquement acceptable d'un quelconque des précédents.

6. Composé selon l'une quelconque des revendications 1 à 4, ou un stéréoisomère ou un tautomère de celui-ci, ou un sel pharmaceutiquement acceptable d'un quelconque des précédents, dans lequel le composé de formule (I) est un composé de formule (IB) : 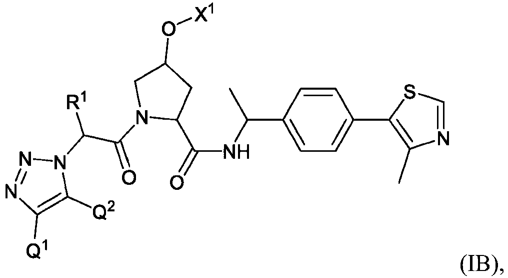 ou un stéréoisomère ou un tautomère de celui-ci, ou un sel pharmaceutiquement acceptable d'un quelconque des précédents.

7. Composé selon l'une quelconque des revendications 1 à 4, ou un stéréoisomère ou un tautomère de celui-ci, ou un sel pharmaceutiquement acceptable d'un quelconque des précédents, dans lequel le composé de formule (I) est un composé de formule (IC) : 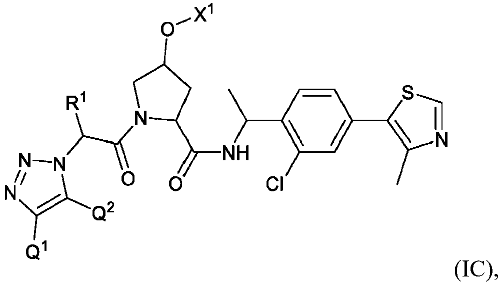 ou un stéréoisomère ou un tautomère de celui-ci, ou un sel pharmaceutiquement acceptable d'un quelconque des précédents.

8. Composé selon l'une quelconque des revendications 1 à 3, ou un stéréoisomère ou un tautomère de celui-ci, ou un sel pharmaceutiquement acceptable d'un quelconque des précédents, dans lequel
n représente 1, et
R^{e} représente, indépendamment à chaque occurrence, un aryle en C₆₋₂₀, dans lequel l'aryle en C₆₋₂₀ de R^{e} est indépendamment éventuellement substitué par un ou plusieurs alkyles en C₁₋₁₂ ou halogènes.

9. Composé selon l'une quelconque des revendications 1 à 3 et 8, ou un stéréoisomère ou un tautomère de celui-ci, ou un sel pharmaceutiquement acceptable d'un quelconque des précédents, dans lequel le composé de formule (I) est un composé de formule (ID) : 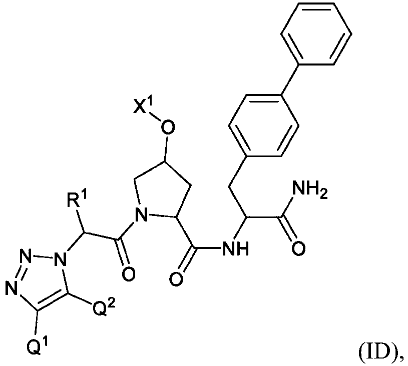 ou un stéréoisomère ou un tautomère de celui-ci, ou un sel pharmaceutiquement acceptable d'un quelconque des précédents.

10. Composé selon la revendication 9, ou un stéréoisomère ou un tautomère de celui-ci, ou un sel pharmaceutiquement acceptable d'un quelconque des précédents, dans lequel le composé de formule (ID) est un composé choisi dans le groupe constitué par 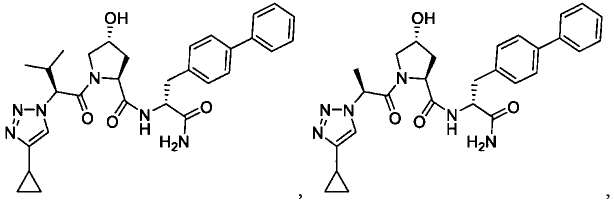 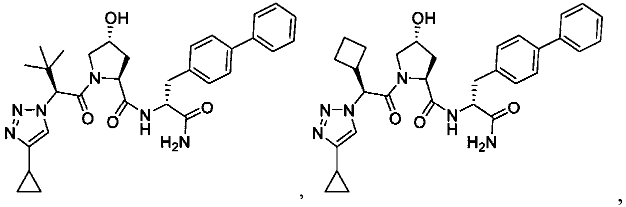 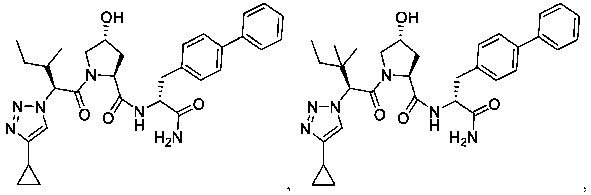 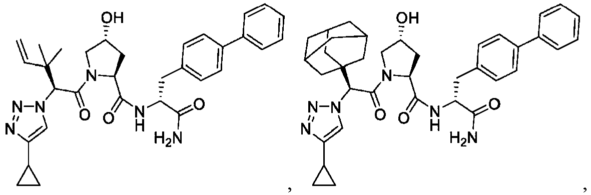 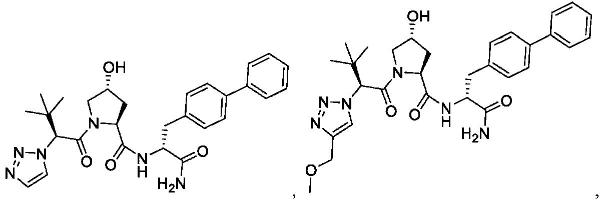 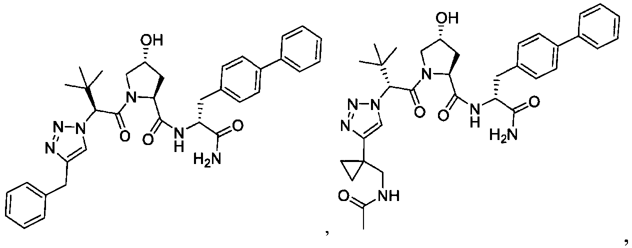 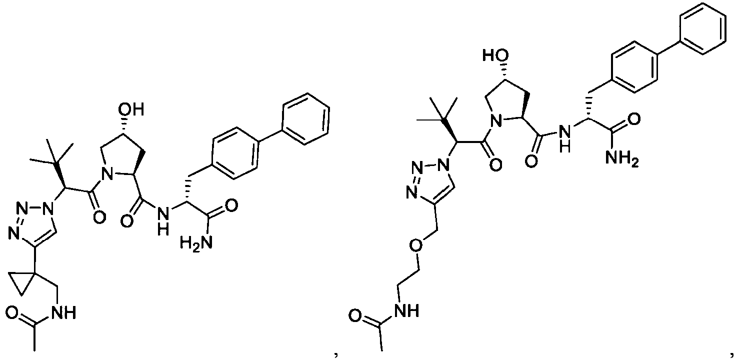 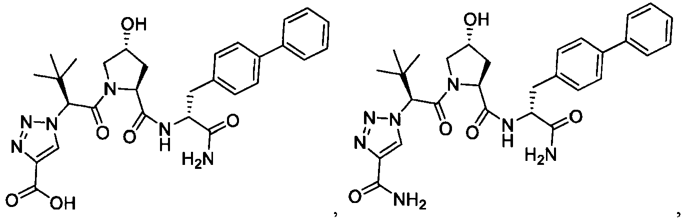 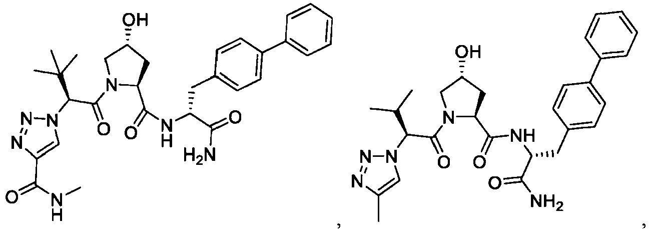 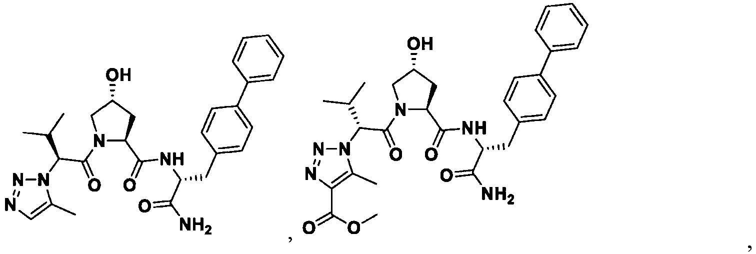 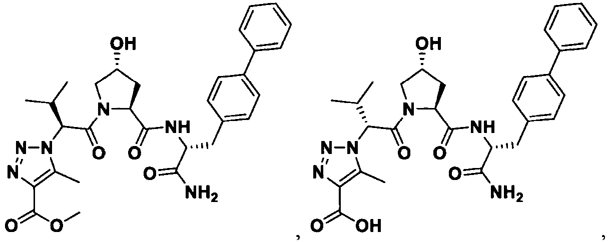 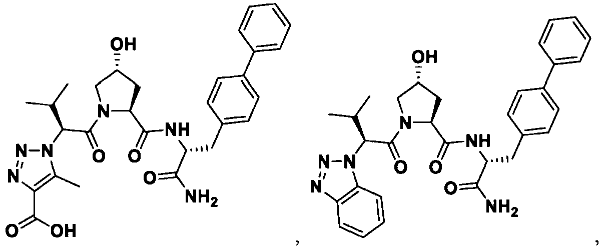 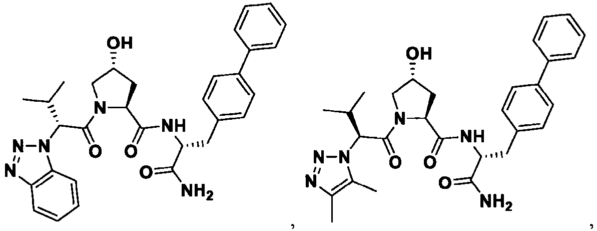 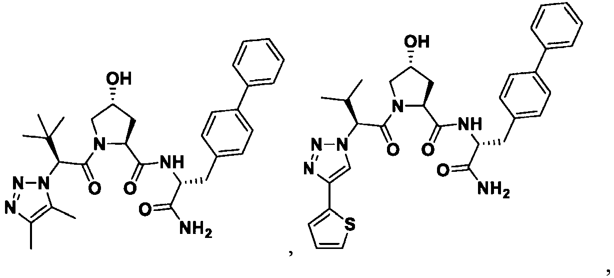 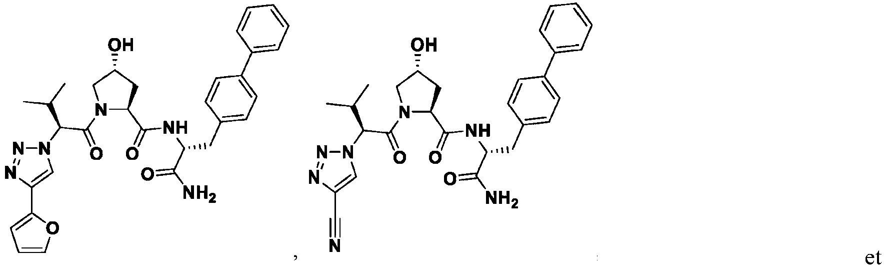 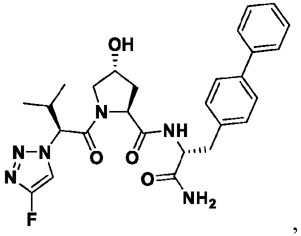 ou un stéréoisomère ou un tautomère de ceux-ci, ou un sel pharmaceutiquement acceptable d'un quelconque des précédents.

11. Composé selon la revendication 1, ou un stéréoisomère ou un tautomère de celui-ci, ou un sel pharmaceutiquement acceptable d'un quelconque des précédents, dans lequel le composé de formule (I) est un composé de formule (IF) : 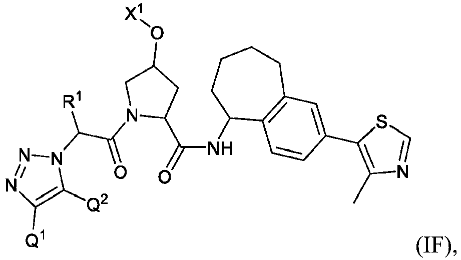 ou un stéréoisomère ou un tautomère de celui-ci, ou un sel pharmaceutiquement acceptable d'un quelconque des précédents.

12. Composé selon l'une quelconque des revendications 1 à 9 et 11, ou un stéréoisomère ou un tautomère de celui-ci, ou un sel pharmaceutiquement acceptable d'un quelconque des précédents, dans lequel Q¹ et Q² représentent, indépendamment l'un de l'autre et indépendamment à chaque occurrence, H, un halogène, un cyano, un alkyle en C₁₋₁₂, un cycloalkyle en C₃₋₁₅, un hétérocyclyle à 3 à 15 chaînons, un aryle en C₆₋₂₀, un hétéroaryle à 5 à 20 chaînons, -C(O)-O(R^{a}) ou -C(O)-N(R^{b})(R^{c}), dans lequel R^{a}, R^{b} et R^{c} représentent chacun indépendamment H ou un alkyle en C₁₋₁₂,
dans lequel l'alkyle en C₁₋₁₂ ou le cycloalkyle en C₃₋₁₅ de Q¹ ou Q² est indépendamment éventuellement substitué par un ou plusieurs R^{q}, dans lequel R^{q} représente un alkyle en C₁₋₁₂, un alcényle en C₂₋₁₂, un alcynyle en C₂₋₁₂, un aryle en C₆₋₂₀, un alcoxy en C₁₋₁₂ ou 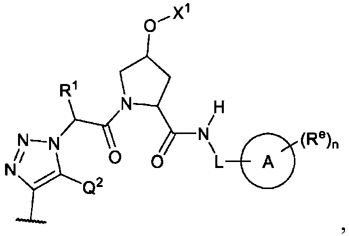 dans lequel l'alkyle en C₁₋₁₂ ou l'alcoxy en C₁₋₁₂ de R^{q} est indépendamment en outre éventuellement substitué par un ou plusieurs halogènes ou - NHC(O)-alkyle en C₁₋₁₂.

13. Composé selon l'une quelconque des revendications 1 à 9 et 11 à 12, ou un stéréoisomère ou un tautomère de celui-ci, ou un sel pharmaceutiquement acceptable d'un quelconque des précédents, dans lequel Q¹ représente un cycloalkyle en C₃₋₁₅, dans lequel le cycloalkyle en C₃₋₁₅ de Q¹ est éventuellement substitué par un ou plusieurs R^{q}, dans lequel R^{q} représente indépendamment un alkyle en C₁₋₁₂, un alcényle en C₂₋₁₂, un alcynyle en C₂₋₁₂, un aryle en C₆₋₂₀, un alcoxy en C₁₋₁₂ ou 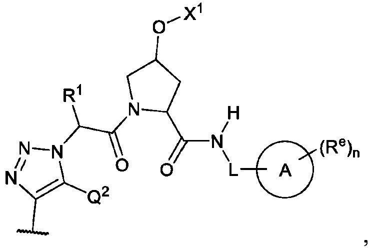 dans lequel l'alkyle en C₁₋₁₂ ou l'alcoxy en C₁₋₁₂ de R^{q} est indépendamment en outre éventuellement substitué par un ou plusieurs halogènes ou -NHC(O)-alkyle en C₁₋₁₂.

14. Composé selon l'une quelconque des revendications 1 à 13, ou un stéréoisomère ou un tautomère de celui-ci, ou un sel pharmaceutiquement acceptable d'un quelconque des précédents, dans lequel Q¹ représente un cycloalkyle en C₃₋₁₅ non substitué.

15. Composé selon l'une quelconque des revendications 1 à 14, ou un stéréoisomère ou un tautomère de celui-ci, ou un sel pharmaceutiquement acceptable d'un quelconque des précédents, dans lequel R¹ représente, indépendamment à chaque occurrence, un alkyle en C₁₋₁₂, dans lequel l'alkyle en C₁₋₁₂ de R¹ est indépendamment éventuellement substitué par un ou plusieurs aryles en C₆₋₂₀, -S(O)₂-alkyle en C₁₋₁₂ ou -C(O)-alkyle en C₁₋₁₂.

16. Composé selon l'une quelconque des revendications 1 à 14, ou un stéréoisomère ou un tautomère de celui-ci, ou un sel pharmaceutiquement acceptable d'un quelconque des précédents, dans lequel R¹ représente, indépendamment à chaque occurrence, un cycloalkyle en C₃₋₁₅, dans lequel le cycloalkyle en C₃₋₁₅ de R¹ est éventuellement substitué par un ou plusieurs alkyles en C₁₋₁₂, aryles en C₆₋₂₀, -S(O)₂-alkyle en C₁₋₁₂ ou -C(O)-alkyle en C₁₋₁₂.

17. Composé selon la revendication 16, ou un stéréoisomère ou un tautomère de celui-ci, ou un sel pharmaceutiquement acceptable d'un quelconque des précédents, dans lequel R¹ représente, indépendamment à chaque occurrence, un cycloalkyle en C₃₋₆, dans lequel le cycloalkyle en C₃₋₆ de R¹ est éventuellement substitué par un ou plusieurs alkyles en C₁₋₁₂, aryles en C₆₋₂₀, -S(O)₂-alkyle en C₁₋₁₂ ou -C(O)-alkyle en C₁₋₁₂.

18. Composé selon la revendication 1, ou un stéréoisomère ou un tautomère de celui-ci, ou un sel pharmaceutiquement acceptable d'un quelconque des précédents, dans lequel le composé est choisi dans le groupe constitué par 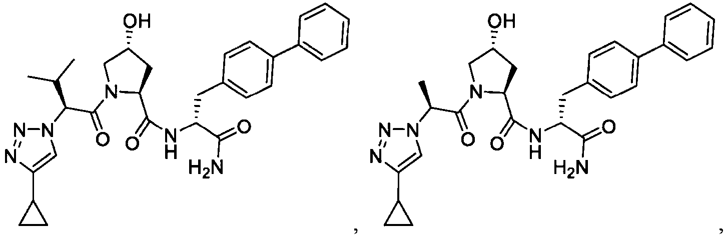 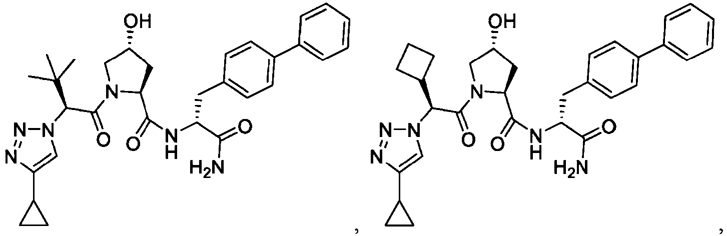 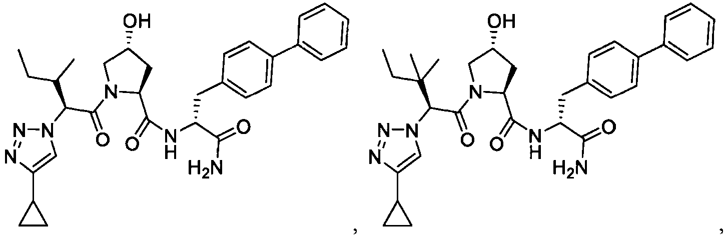 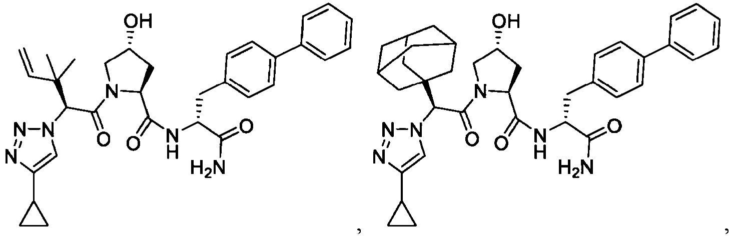 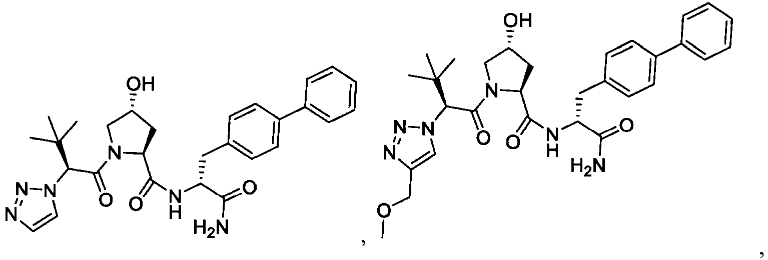 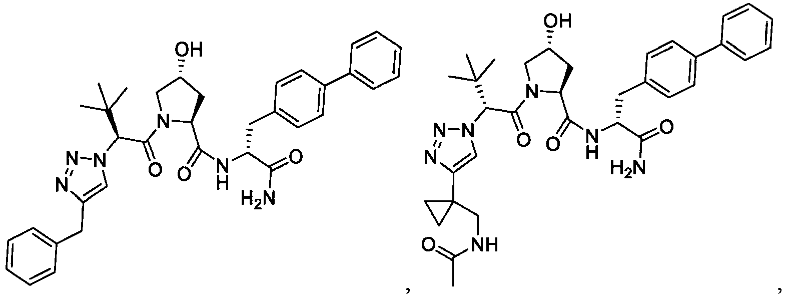 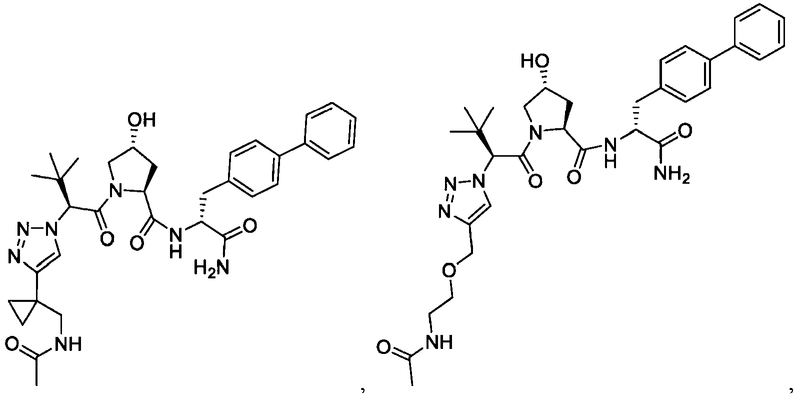 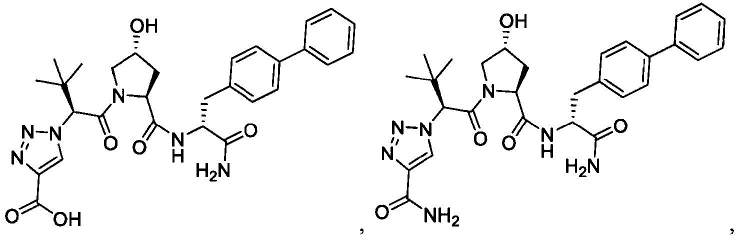 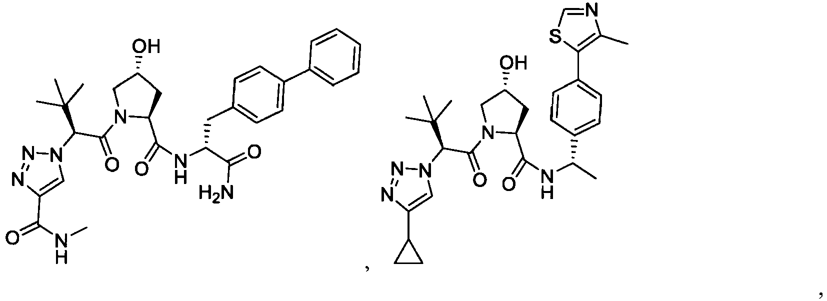 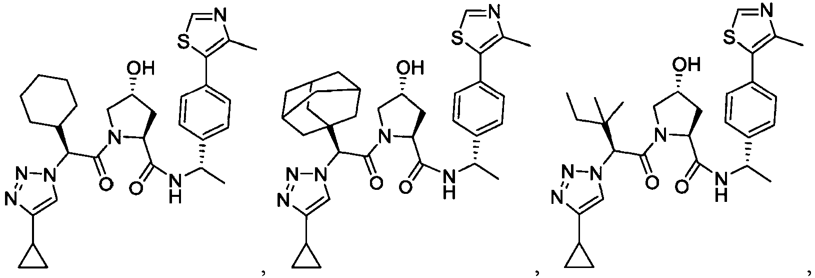 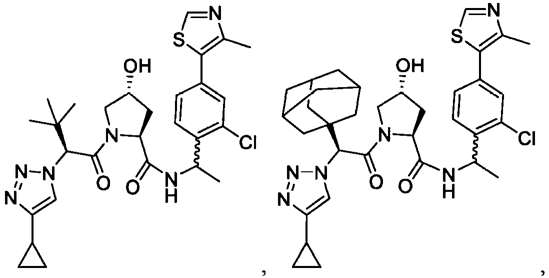 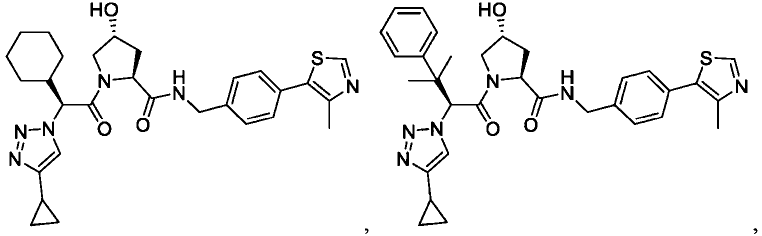 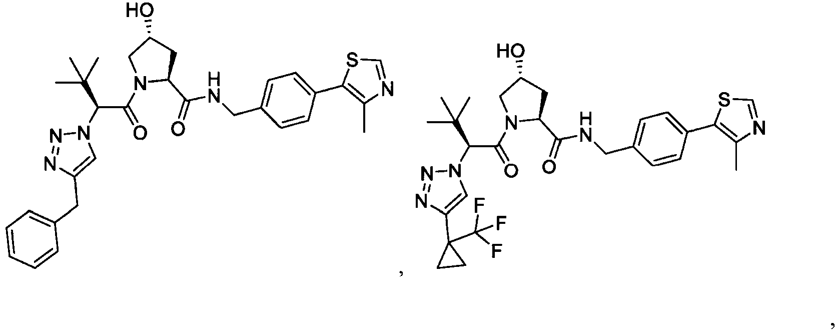 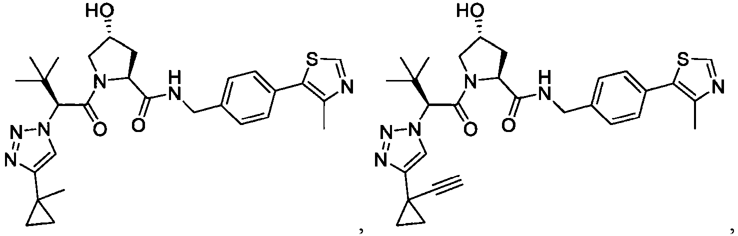 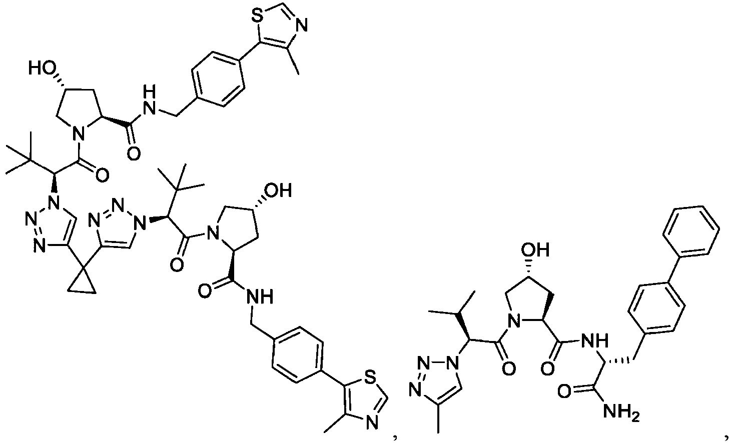 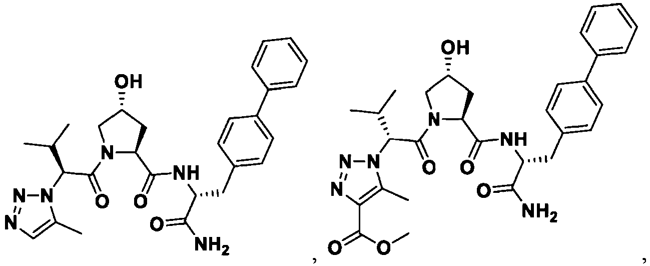 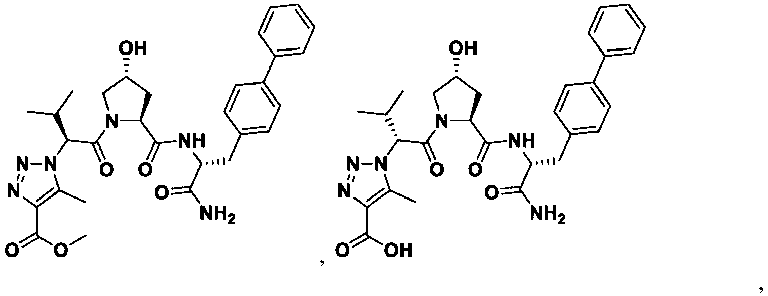 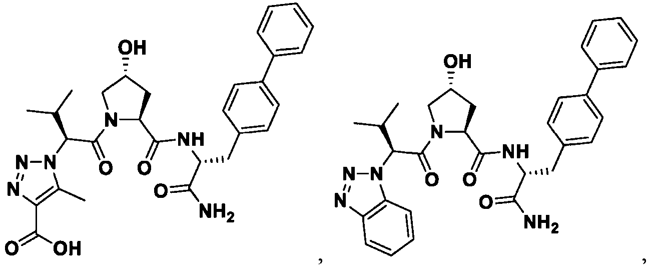 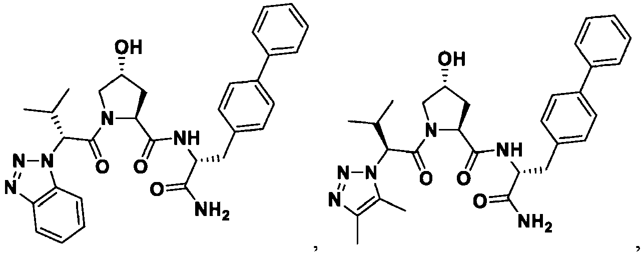 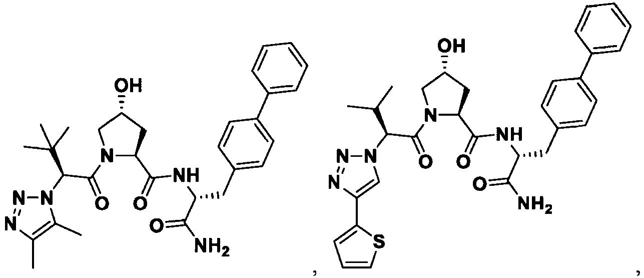 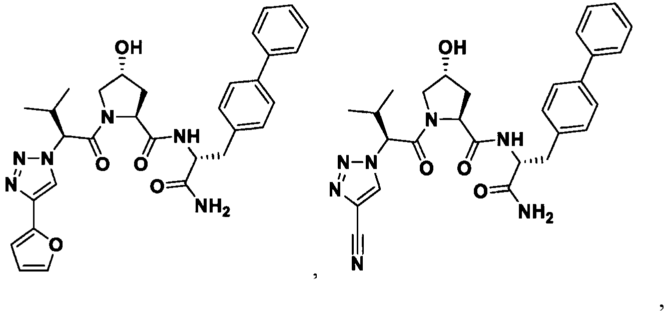 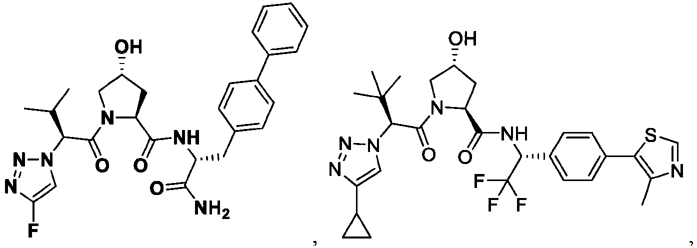 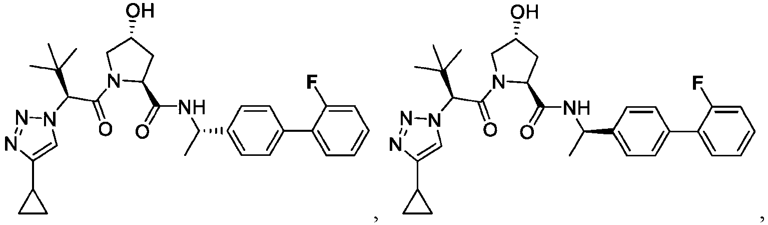 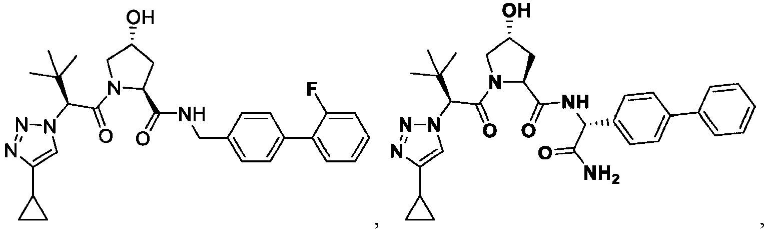 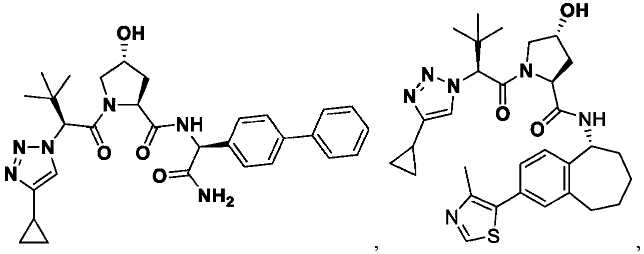 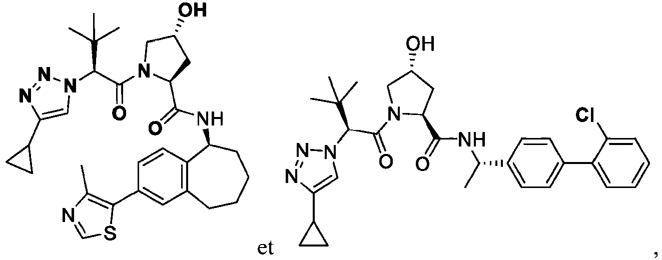 ou un stéréoisomère ou un tautomère de ceux-ci, ou un sel pharmaceutiquement acceptable d'un quelconque des précédents.

19. Composé selon la revendication 1, ou un stéréoisomère ou un tautomère de celui-ci, ou un sel pharmaceutiquement acceptable d'un quelconque des précédents, dans lequel le composé est choisi dans le groupe constitué par 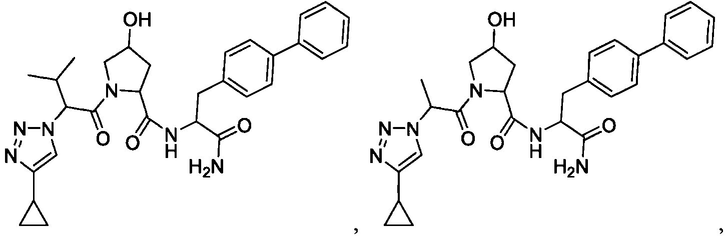 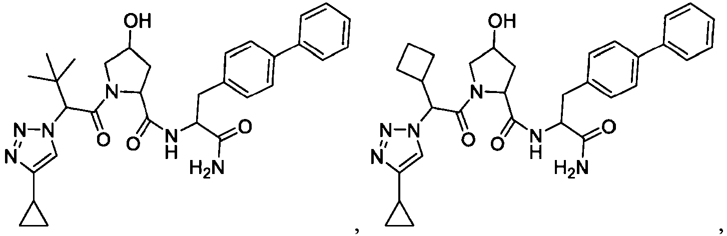 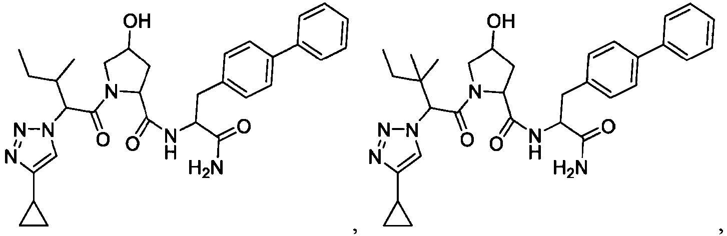 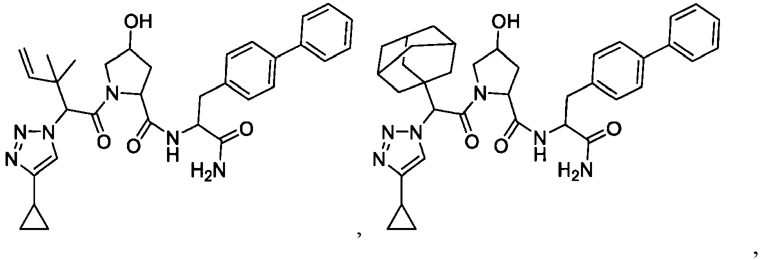 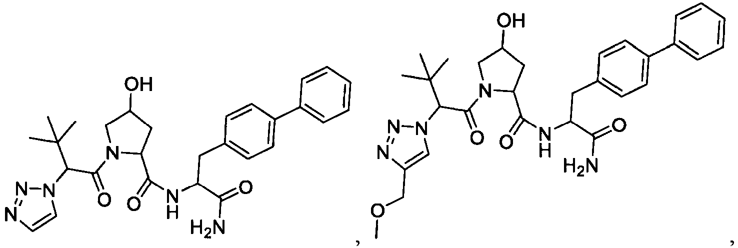 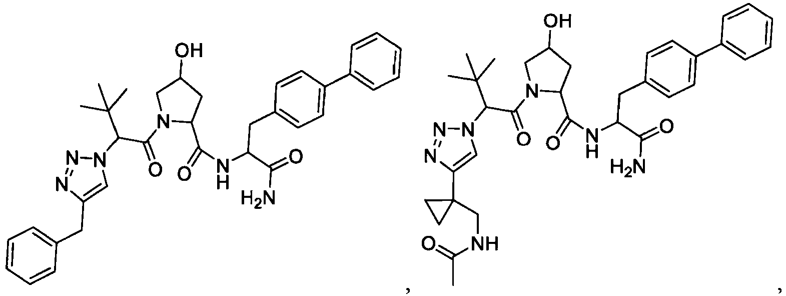 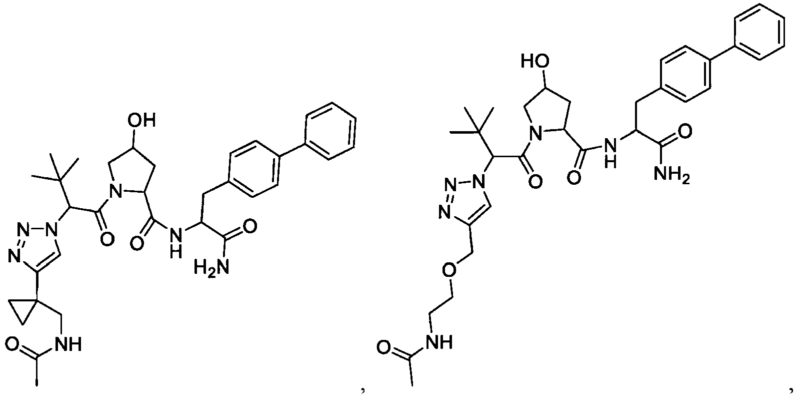 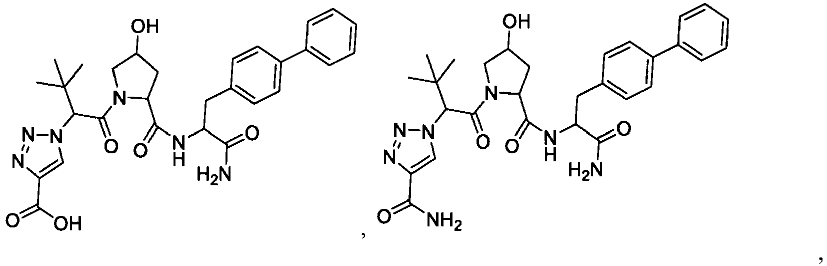 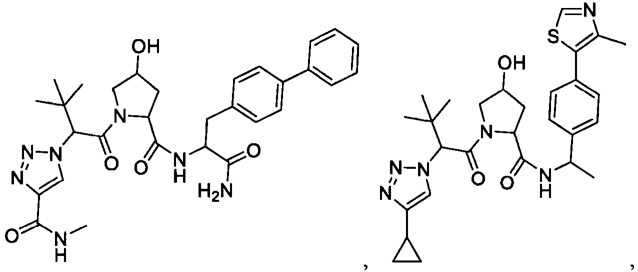 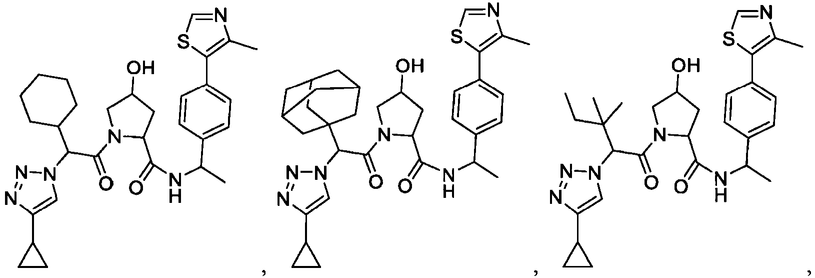 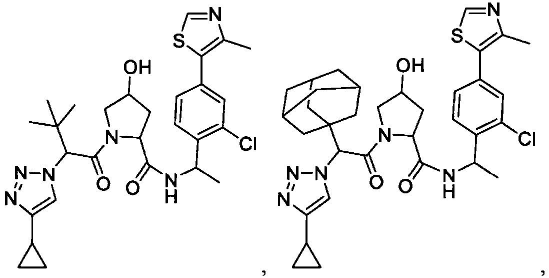 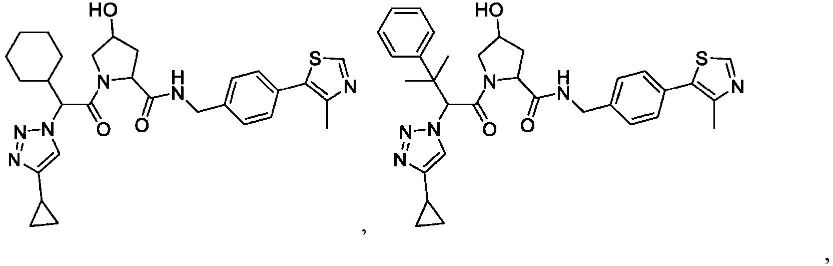 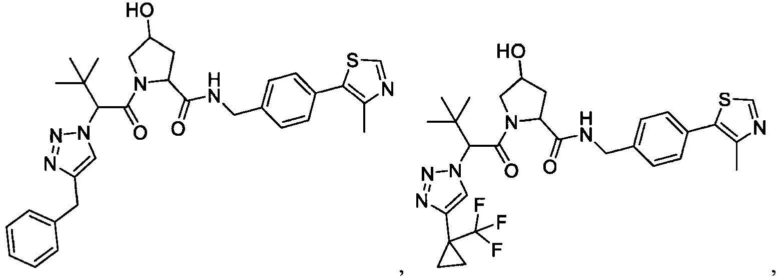 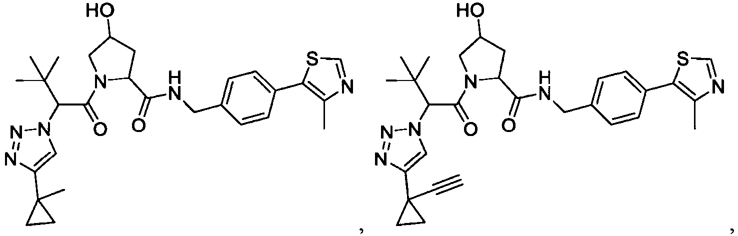 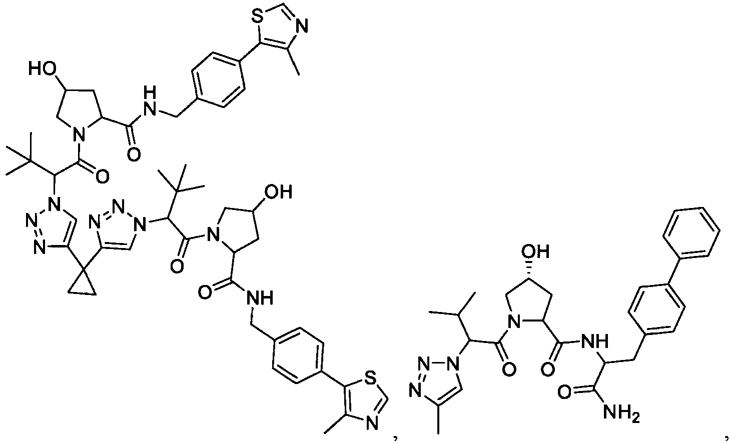 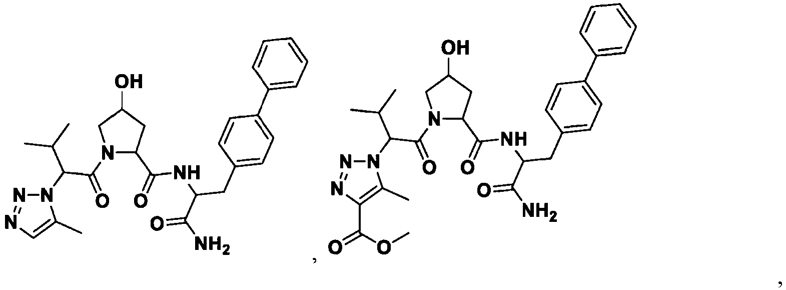 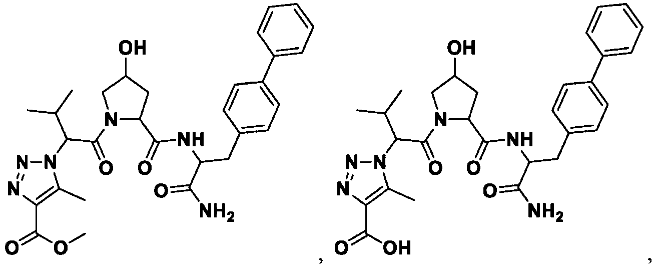 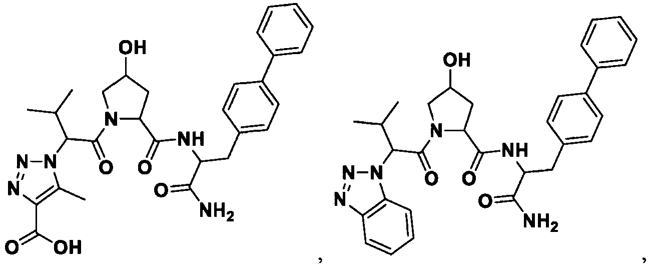 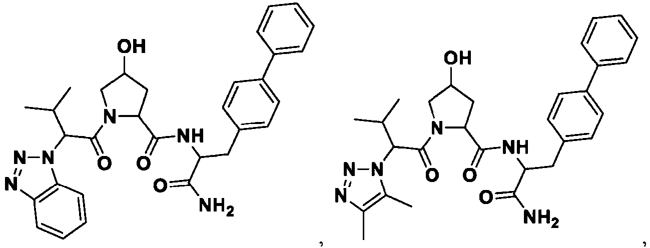 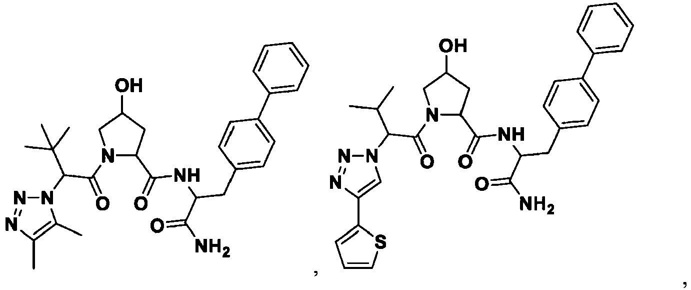 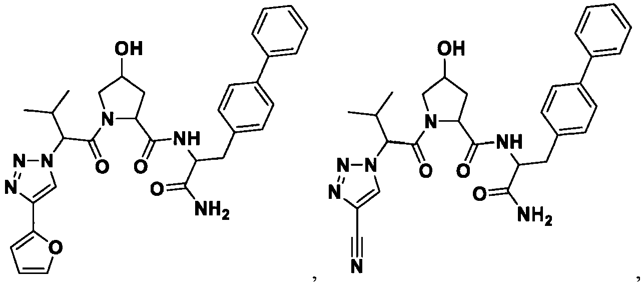 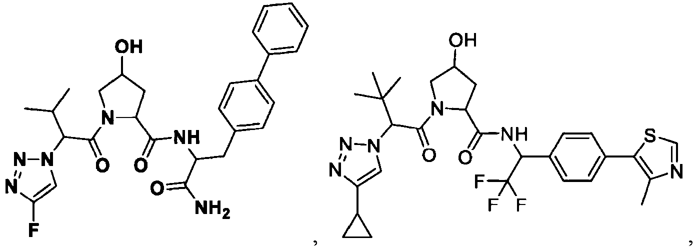 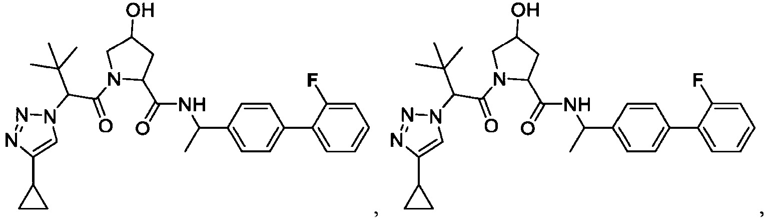 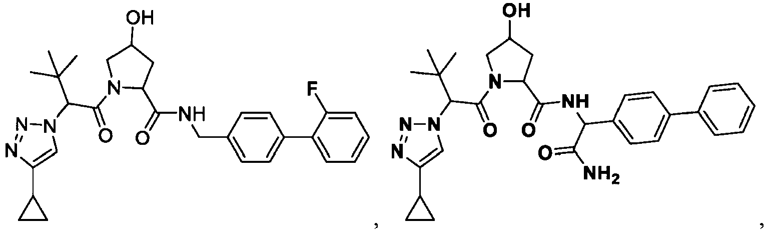 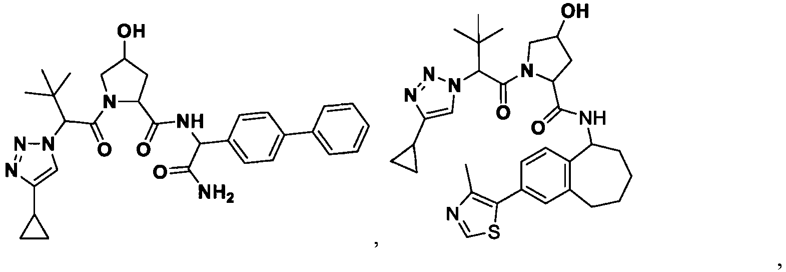 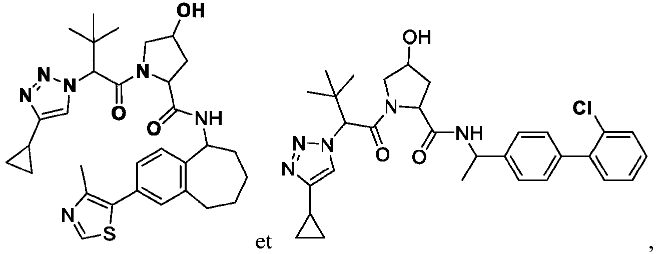 ou un stéréoisomère ou un tautomère de ceux-ci, ou un sel pharmaceutiquement acceptable d'un quelconque des précédents.

20. Composé selon l'une quelconque des revendications 1 à 19, ou un stéréoisomère ou un tautomère de celui-ci, ou un sel pharmaceutiquement acceptable d'un quelconque des précédents, pour une utilisation dans
(a) le traitement de l'anémie ;
(b) le traitement de l'anémie chronique ou de l'anémie associée à une maladie rénale chronique, une dialyse ou une chimiothérapie anticancéreuse, ou une quelconque combinaison de celles-ci ; ou
(c) le traitement de l'ischémie, de l'accident vasculaire cérébral ou d'une lésion du système cardiovasculaire pendant une ischémie, ou d'une quelconque combinaison de ceux-ci ;
(d) l'amélioration de la cicatrisation de plaie chez un humain en ayant besoin ;
(e) la réduction des cicatrices consécutives à une cicatrisation de plaie chez un humain en ayant besoin ;
(f) l'amélioration de l'angiogenèse ou de l'artériogenèse, ou des deux, chez un humain en ayant besoin ;
(g) l'amélioration de l'angiogenèse ou de l'artériogenèse, ou des deux, chez un humain, l'amélioration de l'angiogenèse ou de l'artériogenèse, ou des deux, se produisant localement chez l'humain ; ou
(h) la réduction de la probabilité d'une occlusion de stent chez un humain en ayant besoin.
